(19) Europäisches Patentamt
European Patent Office
Office européen des brevets

(11) **EP 3 960 767 A2**

(12) **EUROPEAN PATENT APPLICATION**

(43) Date of publication:
**02.03.2022 Bulletin 2022/09**

(21) Application number: **21177819.6**

(22) Date of filing: **11.03.2015**

(51) International Patent Classification (IPC):
***C07K 16/32*** (2006.01) ***A61P 35/00*** (2006.01)

(52) Cooperative Patent Classification (CPC):
**A61K 47/6803; A61K 47/6805; A61K 47/6809; A61K 47/6811; A61K 47/6845; A61K 47/6855; A61K 47/6871; A61P 35/00; C07K 16/32;**
C07K 2317/522; C07K 2317/567

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2014 US 201461952026 P**

(62) Document number(s) of the earlier application(s) in accordance with Art. 76 EPC:
**15712236.7 / 3 129 407**

(71) Applicant: **Novartis AG**
**4056 Basel (CH)**

(72) Inventors:
- **GEIERSTANGER, Bernhard Hubert**
**Solana Beach, 92075 (US)**
- **OU, Weijia**
**San Diego, 92128 (US)**

(74) Representative: **Bucher, Tamaris Clare**
**Novartis Pharma AG**
**Patent Department**
**4002 Basel (CH)**

Remarks:
This application was filed on 04-06-2021 as a divisional application to the application mentioned under INID code 62.

# (54) SPECIFIC SITES FOR MODIFYING ANTIBODIES TO MAKE IMMUNOCONJUGATES

(57) The present application provides specific sites for modifying antibodies or antibody fragments by replacing at least one native amino acid in the constant region of a parental antibody or antibody fragment with cysteine, which can be used as a site of attachment for a payload or linker-payload combination. In one embodiment, the antibodies are modified with cysteines at positions 152 and 375 of the heavy chain constant region, as defined by EU numbering format. In another embodiment, the antibodies are modified with cysteines at position 360 of the heavy chain constant region, and position 107 of the kappa light chain constant region, as defined by EU numbering format.

EP 3 960 767 A2

**Description**

FIELD OF THE INVENTION

Due to the importance of antibodies for various therapeutic applications, there is a need for robust methods to selectively modify antibodies to introduce improved properties or additional functions. The application discloses specific sites for attaching moieties to antibodies for making modified antibodies, such as for use in preparation of antibody-drug conjugates (ADCs). The selective conjugation sites are located on constant regions of the antibody and thus are useful with various antibodies.

BACKGROUND

The value of methods for modifying antibodies is well known, and many methods for conjugation of antibodies to attach various "payload" moieties have been developed. Many of these methods rely upon the natural occurrence of specific reactive amino acid residues on the antibody, such as lysine and cysteine, which can be used to attach a payload. However, relying on the native amino acids is not always desirable, because the location and amount of payload attached depend on the number and position of those reactive amino acids: too many or too few such residues make it difficult to efficiently control loading of the payload onto the antibody. In addition, placement of the reactive amino acids may make it difficult to get complete conjugation, resulting in heterogeneous products during conjugation. Heterogeneity of a pharmaceutical active ingredient, for example, is typically undesirable because it compounds the unpredictability of administering a drug to a heterogeneous population of subjects: it is far preferable to administer a homogeneous product, and far more difficult to fully characterize and predict behavior of a heterogeneous one. Site-specific conjugation of a cytotoxic drug to an antibody through, for example, engineered cysteine residues results in homogenous immunoconjugates that exhibit improved therapeutic index (Junutula et al., (2008) Nat Biotechnol. 26(8):925-932)).

Antibodies have been engineered to add certain residues like cysteine in specific positions where these residues can be used for conjugation (Lyons et al., (1990) Protein Eng., 3:703-708), but the value of specific substitutions can vary with certain antibodies, as engineered cysteine might interfere with folding of the antibody and oxidation of the proper intra-molecular disulfide bonds (Voynov et al., (2010) Bioconjug. Chem. 21(2):385-392).

Because engineered cysteines in antibodies expressed in mammalian cells are modified through disulfide bonds with glutathione (GSH) and/or cysteine during their biosynthesis (Chen et al. (2009) mAbs 1:6, 563-571), the modified cysteine(s) in the antibody drug conjugate product as initially expressed is unreactive to thiol reactive reagents. Activation of the engineered cysteine(s) requires reduction of the GSH and/or cysteine adduct (which typically results in reduction of all inter-chain disulfide bonds of the antibody), followed by reoxidation and reformation of the native, inter-chain disulfide bonds prior to conjugation (Junutula et al., (2008) Nat. Biotechnol. 26(8):925-32). Some of the sites where cysteine has been inserted interfere with the process of reoxidation and subsequently result in undesirable, non-homogeneous conjugation products. It is therefore important to identify sites on the antibody where the introduced cysteine does not interfere with the reoxidation process prior to the conjugation with a thiol reactive reagent such as maleimide or bromo-, chloro- or iodo-acetamide groups.

Conjugation of cysteine residues with bromo-acetamide, iodo-acetamide or chloro-acetamide results in the formation of a stable thioether linkage. (Alley et al., (2008) Bioconjug Chem. 19(3):759-65). However, the chemistry is less efficient than maleimide conjugation chemistry. Since forming such thiol-maleimide linkages is a popular and highly efficient method to use when attaching a payload or linker to cysteine, there is a need to identify cysteine substitution sites on an antibody where maleimide linkages can be used. More importantly, site-specifically conjugated immunoconjugates can exhibit improved therapeutic index, thus there remains a need to identify specific privileged sites for cysteine substitution in antibodies that enables conjugation of payloads onto antibodies to form efficiently, and that provide conjugates having high stability. The instant application provides such privileged cysteine substitution sites that give improved antibodies for conjugation purposes and immunoconjugates comprising such improved antibodies.

SUMMARY OF THE INVENTION

The application provides specific sites in the constant region of an antibody or antibody fragment at which cysteine ("Cys") replacement of the native amino acid on a parental antibody or antibody fragment can be performed in order to provide a Cys residue for attachment of a chemical moiety (e.g., payload/drug moiety) to form an immunoconjugate with good efficiency and stability. The application further provides engineered antibodies or antibody fragments having one or more Cys residues in one or more of these specific sites, as well as immunoconjugates made from such engineered antibodies or antibody fragments.

Methods for inserting Cys at specific locations of an antibody are known in the art, see, *e.g.,* WO 2011/005481. However, the current application discloses specific sites in the constant region of antibodies or antibody fragments where

replacing one or more native amino acids of a parental antibody or antibody fragment with Cys provides one or more of the following advantages: Good reactivity to promote efficient immunoconjugation; reduced propensity for loss of payload when a Cys-maleimide conjugation linker is used; a reduced tendency to form undesired disulfide linkages, such as cross-linking between antibodies or the formation of non-native intramolecular disulfide bonds; and low hydrophobicity of the resulting ADC.

The specific privileged sites for Cys replacement of native amino acids in the constant region of a parental antibody or antibody fragment are selected to provide efficient conjugation while minimizing undesired effects. First, the specific sites for modification are selected so that replacing the native amino acid of a parental antibody or antibody fragment with Cys in one or more of the selected locations provides antibodies or antibody fragments that are readily conjugated on the new cysteine. The specific locations are selected to be sufficiently surface-accessible to allow the sulfhydryl of the cysteine residue to be reactive towards electrophiles in aqueous solutions. The identification of suitable sites for Cys replacement of native amino acids of a parental antibody or antibody fragment involved analyzing surface exposure of the native amino acids based on crystal structure data. Because the sites described herein are sufficiently accessible and reactive, they can be used to form immunoconjugates via chemistry that is well known in the art for modifying naturally-occurring cysteine residues. Conjugation of the replacement Cys residues thus uses conventional methods.

Selected modification sites can show a low propensity for reversal of conjugation when thiol-maleimide moieties are used in the conjugation. The thiol-maleimide conjugation reaction is often highly selective and extremely efficient, and may be used either to attach a payload to the thiol of a cysteine residue of a protein or as a linker elsewhere in the linkage between protein and payload. For example, a maleimide can be attached to a protein (*e.g.,* an antibody or antibody fragment), and a payload having an attached thiol can be added to the maleimide to form a conjugate:

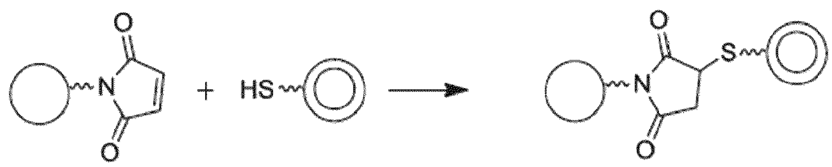

Accordingly, in this conjugation step, the protein (*e.g.,* an antibody or antibody fragment) could be either the single circle or the double circle; the other would represent the payload. The immunoconjugate stability information here specifically relates to conjugation of the substituted cysteine by reaction with a maleimide group. In some embodiments, the thiol is from a cysteine on the protein (*e.g.,* an antibody or antibody fragment), so the double circle represents the protein and the single circle represents a payload.

While the thiol-maleimide reaction is often used for making conjugates, reversal of the conjugation step as depicted below can result in loss of payload or scrambling of payload with other thiol-containing species:

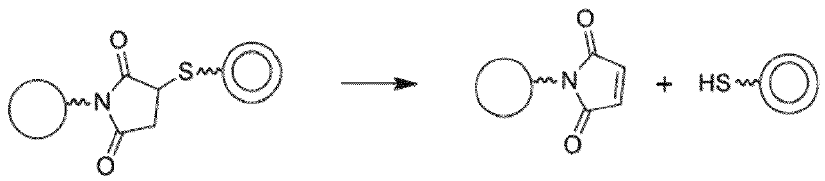

It has been reported that some sites for cysteine substitution provide more stable maleimide conjugates than others, presumably because the local chemical environment at certain points on the antibody surface around a new cysteine can promote the hydrolysis of the succinimide ring and hence prevent reversal of the thiol-maleimide linkage in the immunoconjugate (Shen et al. (2012), Nat. Biotechnol. 30(2):184-9). The identification of the advantageous sites for meeting this criterion involved inserting Cys in place of many of the native amino acids having suitable surface exposure, making immunoconjugates containing a thiol-maleimide linkage, and assessing stability of the immunoconjugate in order to eliminate sites where the stability of the conjugate was reduced by the local microenvironment around destabilizing sites. Because of this, the chemistry that can be used to attach linkers and payloads to the replacement Cys residues is not limited by the stability problems associated with the reversibility of thiol-maleimide conjugates that is discussed above. A number of methods can be used to form conjugates at cysteine, including maleimide conjugation. The sites for cysteine substitution described herein promote stability of the antibody conjugate product when using one of the most common conjugation methods, making these sites advantageous for antibody engineering, because the modified antibody can be used with the well-known and highly efficient maleimide conjugation methodology.

Selected sites can be positioned so as to minimize undesired disulfide formation that may interfere with formation

of a homogeneous conjugate. When antibodies or antibody fragments containing engineered cysteines are produced in mammalian cells, the Cys residues are typically present as disulfides to a free Cys amino acid or to glutathione (Chen et al., (2009) mAbs 16, 353-571). To free the Cys residues for conjugation with thiol reactive groups, the antibody or antibody fragment needs to be reduced, breaking all of the disulfide bonds. The antibody or antibody fragment is then reoxidized under conditions that facilitate formation of the native disulfides that stabilize the antibody or antibody fragment. Upon reoxidation, cysteine residues that are too prominently exposed on the surface of the antibody or antibody fragment can form disulfides by reaction with Cys on another antibody or antibody fragment ("inter-antibody disulfides"), or by forming undesired intra-antibody disulfides. It has been found that cysteine residues placed in the specific sites described herein are suitably accessible to be available for efficient conjugation, but are sufficiently shielded or suitably positioned to reduce or eliminate formation of inter-antibody and intra-antibody disulfide bonds that would otherwise occur during the reduction / reoxidation procedures typically needed when expressing cys-modified antibodies. Similarly, after re-oxidation some sites were found to produce non-homogenous conjugation products that appear to be due to the location of the new Cys residue engineered into the protein, and the specific sites identified herein are ones where such heterogeneity is minimized.

Conjugating drug payloads at sites where they are sequestered from solvent interactions and attachment can increase the hydrophobicity of the antibody upon payload attachment is preferred as reducing hydrophobicity of a protein therapeutic is generally considered beneficial because it might reduce aggregation and clearance from circulation. Selecting attachment sites that result in minimal changes in hydrophobicity might be particularly beneficial when 4, 6 or 8 payloads are attached per antibody, or when particularly hydrophobic payloads are used.

Sites for Cys incorporation were evaluated using these and additional methods described in the Examples herein, leading to the selection of preferred sites for Cys incorporation for engineering antibodies or antibody fragments to introduce Cys as a site for conjugation, especially for making ADCs. Additional details regarding the selection of the specific sites for replacing a natural amino acid of an antibody with Cys are provided herein.

Cysteine substitution sites are located in the constant region of an antibody or antibody fragment, and are identified herein using standard numbering conventions. It is well known, however, that portions or fragments of antibodies can be used for many purposes instead of intact full-length antibodies, and also that antibodies can be modified in various ways that affect numbering of sites in the constant region even though they do not substantially affect the functioning of the constant region. For example, insertion of an S6 tag (a short peptide) into a loop region of an antibody has been shown to allow activity of the antibody to be retained, even though it would change the numbering of many sites in the antibody. Accordingly, while the preferred cysteine substitution sites described herein are identified by a standard numbering system based on intact antibody numbering, the application includes the corresponding sites in antibody fragments or in antibodies containing other modifications, such as peptide tag insertion. The corresponding sites in those fragments or modified antibodies are thus preferred sites for cysteine substitution in fragments or modified antibodies, and references to the cysteine substitution sites by number include corresponding sites in modified antibodies or antibody fragments that retain the function of the relevant portion of the full-length antibody.

A corresponding site in an antibody fragment or modified antibody can readily be identified by aligning a segment of the antibody fragment or modified antibody with the full-length antibody to identify the site in the antibody fragment or modified antibody that matches one of the preferred cysteine substitution sites of the invention. Alignment may be based on a segment long enough to ensure that the segment matches the correct portion of the full-length antibody, such as a segment of at least 20 amino acid residues, or at least 50 residues, or at least 100 residues, or at least 150 residues. Alignment may also take into account other modifications that may have been engineered into the antibody fragment or modified antibody, thus differences in sequence due to engineered point mutations in the segment used for alignment, particularly for conservative substitutions, would be allowed. Thus, for example, an Fc domain can be excised from an antibody, and would contain amino acid residues that correspond to the cysteine substitution sites described herein, despite numbering differences: sites in the Fc domain corresponding to the cysteine substitution sites of the present disclosure would also be expected to be advantageous sites for cysteine substation in the Fc domain, and are included in the scope of this application.

In one embodiment, the application provides an immunoconjugate of Formula (I):

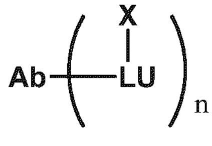

wherein Ab represents an antibody or antibody fragment comprising at least one cysteine residue at one of the preferred cysteine substitution sites described herein;

LU is a linker unit as described herein;

X is a payload or drug moiety;

and n is an integer from 1 to 16.

Typically in compounds of Formula (I), LU is attached to a cysteine at one of the cysteine substitution sites described herein, X is a drug moiety such as an anticancer drug, and n is 2-8 when Ab is an antibody, or n can be 1-8 when Ab is an antibody fragment.

In an embodiment, the application provides an immunoconjugate comprising a modified antibody or antibody fragment thereof and a drug moiety, wherein said modified antibody or antibody fragment comprises a substitution of one or more amino acids with cysteine on its constant region chosen from positions 121, 124, 152, 171, 174, 258, 292, 333, 360, and 375 of a heavy chain of said antibody or antibody fragment, and wherein said positions are numbered according to the EU system.

In an embodiment, the application provides an immunoconjugate comprising a modified antibody or antibody fragment thereof and a drug moiety, wherein said modified antibody or antibody fragment comprises a substitution of one or more amino acids with cysteine on its constant region chosen from positions 107, 108, 142, 145, 159, 161, and 165 of a light chain of said antibody or antibody fragment, wherein said positions are numbered according to the EU system, and wherein said light chain is human kappa light chain.

In an embodiment, the application provides an immunoconjugate comprising a modified antibody or antibody fragment thereof and a drug moiety, wherein said modified antibody or antibody fragment comprises a substitution of one or more amino acids with cysteine on its constant region chosen from positions 143, 147, 159, 163, and 168 of a light chain of said antibody or antibody fragment, wherein said positions are numbered according to the Kabat system, and wherein said light chain is human lambda light chain.

In an embodiment, the application provides a modified antibody or antibody fragment thereof comprising a substitution of one or more amino acids with cysteine at the positions described herein. The sites for cysteine substitution are in the constant regions of the antibody and are thus applicable to a variety of antibodies, and the sites are selected to provide stable and homogeneous conjugates. The modified antibody or fragment can have two or more cysteine substitutions, and these substitutions can be used in combination with other antibody modification and conjugation methods as described herein.

In an embodiment, the application provides pharmaceutical compositions comprising the immunoconjugate disclosed above, and methods to use the immunoconjugates.

In an embodiment, the application provides a nucleic acid encoding the modified antibody or antibody fragment described herein having at least one cysteine substitution at a site described herein. The application further provides host cells comprising these nucleic acids and methods to use the nucleic acid or host cells to express and produce the antibodies or fragments described herein.

In an embodiment, the application provides a method to select an amino acid of an antibody that is suitable for replacement by cysteine to provide a good site for conjugation, comprising:

(1) identifying amino acids in the constant region of the antibody that have a suitable surface exposure to provide a set of initial candidate sites;

(2) for each initial candidate site, expressing an antibody wherein the native amino acid at that site is replaced by cysteine;

(3) for each expressed antibody, determining whether the expressed protein is substantially homogeneous after reduction and reoxidation to provide an antibody having a free cysteine at the initial candidate site,

(4) for each expressed protein that is substantially homogeneous and functional, conjugating the cysteine at the initial candidate site with a maleimide moiety and determining whether the thiol-maleimide linkage is stable at that site;

(5) removing from the set of initial candidate sites those sites for which the expressed antibody is not substantially homogeneous and functional, and those wherein the thiol-maleimide linkage is destabilized, to provide a set of advantaged sites for cysteine substitution.

Optionally, the method further comprises a step of determining the melting temperature for the conjugate of each advantaged cysteine substitution site, and eliminating from the set any sites where cysteine substitution and conjugation causes the melting temperature to differ by 5°C or more from that of the native antibody.

In an embodiment, the application provides a method to produce an immunoconjugate, which comprises attaching a Linker Unit (LU) or a Linker Unit-Payload combination (-LU-X) to a cysteine residue in an antibody or antibody fragment, wherein the cysteine is located at a cysteine substitution site selected from 121, 124, 152, 171, 174, 258, 292, 333, 360, and 375 of a heavy chain of said antibody or antibody fragment, and positions 107, 108, 142, 145, 159, 161, and 165 of a light chain of said antibody or antibody fragment, wherein said positions are numbered according to the EU system.

Other aspects and embodiments of the application are described in greater detail herein.

The following are embodiments of the present application.

1. An immunoconjugate comprising a modified antibody or antibody fragment thereof, wherein said modified antibody or antibody fragment comprises a combination of substitution of two or more amino acids with cysteine on its constant regions wherein the combinations comprise substitutions selected from position 360 of an antibody heavy chain, and position 107 of an antibody kappa light chain, wherein said positions are numbered according to the EU system.

2. An immunoconjugate comprising a modified antibody or antibody fragment thereof, wherein said modified antibody or antibody fragment comprises a combination of substitution of two or more amino acids with cysteine on its constant regions wherein the combinations comprise substitutions selected from positions 152 and 375 of an antibody heavy chain, wherein said positions are numbered according to the EU system.

3. An immunoconjugate comprising a modified antibody or antibody fragment thereof comprising a heavy chain constant region of SEQ ID NO: 48 and a kappa light chain constant region comprising SEQ ID NO: 61.

4. An immunoconjugate comprising a modified antibody or antibody fragment thereof comprising a heavy chain constant region of SEQ ID NO: 131.

5. The immunoconjugates of any of embodiments 1-4 wherein the immunoconjugate further comprises a drug moiety.

6. The immunoconjugates of any of embodiments 1-5 wherein the drug antibody ratio is about 4.

7. The immunoconjugate of any of embodiments 1-6, wherein said drug moiety is attached to the modified antibody or antibody fragment through the sulfur of said cysteine and an optional linker.

8. The immunoconjugate of embodiments 1-7, wherein said drug moiety is connected to said sulfur of said cysteine through a cleavable or non-cleavable linker.

9. The immunoconjugate of embodiments 8, wherein said drug moiety is connected to said sulfur of said cysteine through a non-cleavable linker.

10. The immunoconjugate of embodiments 7-9, wherein said immunoconjugate comprises a thiol-maleimide linkage.

11. The immunoconjugate of embodiment 10, wherein said immunoconjugate comprises a $-S-CH_2-C(=O)-$ linkage or a disulfide linkage.

12. The immunoconjugate of embodiment 11, wherein said drug moiety is a cytotoxic agent.

13. The immunoconjugate of embodiment 12, wherein said drug moiety is selected from the group consisting of taxanes, DNA-alkylating agents (e.g., CC-1065 analogs), anthracyclines, tubulysin analogs, duocarmycin analogs, auristatin E, auristatin F, maytansinoids and Eg5 inhibitors .

14. The immunoconjugate of any of embodiments1-13, wherein said antibody is a monoclonal antibody.

15. The immunoconjugate of any of embodiments 1-13, wherein said antibody is a chimeric antibody.

16. The immunoconjugate of embodiments 1-13, wherein said antibody is a humanized or fully human antibody.

17. The immunoconjugate of any of embodiments 14-16, wherein said antibody is a bispecific or multi-specific antibody.

18. The immunoconjugate of any of embodiments 1-17, wherein said antibody or antibody fragment specifically binds to a cell surface marker on a tumor.

19. A pharmaceutical composition comprising the immunoconjugate of any of embodiments 1-18.

20. The modified antibody or antibody fragment of any of embodiments 1-19, further comprising at least one Pcl or unnatural amino acid substitution or a peptide tag for enzyme-mediated conjugation and/or combinations thereof.

21. A nucleic acid encoding the modified antibody or antibody fragment of embodiments 1-4.

22. A host cell comprising the nucleic acid of embodiment 21.

23. A method of producing a modified antibody or antibody fragment comprising incubating the host cell of embodiment 22 under suitable conditions for expressing the antibody or antibody fragment, and isolating said antibody or antibody fragment.

24. A method to produce an immunoconjugate, which comprises attaching a Linker Unit (LU) or a Linker Unit-Payload combination (-LU-X) to a cysteine residue in an antibody or antibody fragment of any of embodiments 1-4

25. The method of embodiment 24, wherein the immunoconjugate is of Formula (I):

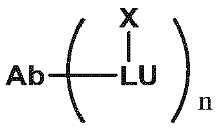

wherein Ab represents an antibody or antibody fragment comprising at least one cysteine residue at one of the preferred cysteine substitution sites described herein;

LU is a linker unit as described herein;
X is a payload or drug moiety;
and n is an integer from 1 to 16.

26. A modified antibody or antibody fragment thereof, wherein said modified antibody or antibody fragment comprises a combination of substitution of two or more amino acids with cysteine on its constant regions wherein the combinations comprise substitutions selected from position 360 of an antibody heavy chain, and position 107 of an antibody kappa light chain, wherein said positions are numbered according to the EU system.

27. A modified antibody or antibody fragment thereof, wherein said modified antibody or antibody fragment comprises a combination of substitution of two or more amino acids with cysteine on its constant regions wherein the combinations comprise substitutions selected from positions 152 and 375 of an antibody heavy chain, wherein said positions are numbered according to the EU system.

28. A modified antibody or antibody fragment therefo comprising a heavy chain constant region of SEQ ID NO: 48 and a kappa light chain constant region comprising SEQ ID NO: 61.

29. A modified antibody or antibody fragment thereof comprising a heavy chain constant region of SEQ ID NO: 131.

Definitions

The term "amino acid" refers to canonical, synthetic, and unnatural amino acids, as well as amino acid analogs and amino acid mimetics that function in a manner similar to the canonical amino acids. Canonical amino acids are proteinogenous amino acids encoded by the genetic code and include alanine, arginine, asparagine, aspartic acid, cysteine, glutamine, glutamic acid, glycine, histidine, isoleucine, leucine, lysine, methionine, phenylalanine, proline serine, threonine, tryptophan, tyrosine, valine, as well as selenocysteine, pyrrolysine and its analog pyrroline-carboxy-lysine. Amino acid analogs refer to compounds that have the same basic chemical structure as a canonical amino acid, *i.e.,* an $\alpha$-carbon that is bound to a hydrogen, a carboxyl group, an amino group, and an R group, e.g., citrulline, homoserine,

norleucine, methionine sulfoxide, methionine methyl sulfonium. Such analogs have modified R groups (e.g., norleucine) or modified peptide backbones, but retain the same basic chemical structure as a canonical amino acid.

Amino acid mimetics refer to chemical compounds that have a structure that is different from the general chemical structure of an amino acid, but that function in a manner similar to a canonical amino acid. The term "unnatural amino acid", as used herein, is intended to represent amino acid structures that cannot be generated biosynthetically in any organism using unmodified or modified genes from any organism, whether the same or different. In addition, such "unnatural amino acids" typically require a modified tRNA and a modified tRNA synthetase (RS) for incorporation into a protein. This tRNA/RS pair preferentially incorporates the unnatural amino acid over canonical amino acids. Such orthogonal tRNA/RS pair is generated by a selection process as developed by Schultz *et al.* (see, *e.g.,* Liu et al., (2010) Annu. Rev. Biochem. 79:413-444) or a similar procedure. The term "unnatural amino acid" does not include the natural occurring $22^{nd}$ proteinogenic amino acid pyrrolysine (Pyl) as well as its demethylated analog pyrroline-carboxy-lysine (Pcl), because incorporation of both residues into proteins is mediated by the unmodified, naturally occurring pyrrolysyl-tRNA/tRNA synthetase pair and because Pyl and Pcl are generated biosynthetically (see, *e.g.,* Ou et al., (2011) Proc. Natl. Acad. Sci. USA, 108:10437-10442; Cellitti et al., (2011) Nat. Chem. Biol. 27;7(8):528-30). See also U.S. provisional application 61/76236, incorporated by reference, that sites specific amino acid residues in antibody light and heavy chains that can be substituted with Pcl.

The term "antibody" as used herein refers to a polypeptide of the immunoglobulin family that is capable of binding a corresponding antigen non-covalently, reversibly, and in a specific manner. For example, a naturally occurring IgG antibody is a tetramer comprising at least two heavy (H) chains (also referred to as "antibody heavy chain") and two light (L) chains (also referred to as "antibody light chain") inter-connected by disulfide bonds. Each heavy chain is comprised of a heavy chain variable region (abbreviated herein as $V_H$) and a heavy chain constant region. The heavy chain constant region is comprised of three domains, CH1, CH2 and CH3. Each light chain is comprised of a light chain variable region (abbreviated herein as $V_L$) and a light chain constant region. The light chain constant region is comprised of one domain, $C_L$. The $V_H$ and $V_L$ regions can be further subdivided into regions of hyper variability, termed complementarity determining regions (CDR), interspersed with regions that are more conserved, termed framework regions (FR). Each $V_H$ and $V_L$ is composed of three CDRs and four FRs arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

The term "antibody" includes, but is not limited to, monoclonal antibodies, human antibodies, humanized antibodies, camelid antibodies, chimeric antibodies, and anti-idiotypic (anti-Id) antibodies (including, *e.g.,* anti-Id antibodies to antibodies of the present disclosure). The antibodies can be of any isotype/class (*e.g.,* IgG, IgE, IgM, IgD, IgA and IgY), or subclass (*e.g.,* IgG1, IgG2, IgG3, IgG4, IgA1 and IgA2).

Both the light and heavy chains are divided into regions of structural and functional homology. The terms "constant" and "variable" are used functionally. In this regard, it will be appreciated that the variable domains of both the light ($V_L$) and heavy ($V_H$) chain portions determine antigen recognition and specificity. Conversely, the constant domains of the light chain ($C_L$) and the heavy chain (CH1, CH2 or CH3) confer important biological properties such as secretion, transplacental mobility, Fc receptor binding, complement binding, and the like. By convention, the numbering of the constant region domains increases as they become more distal from the antigen binding site or amino-terminus of the antibody. The N-terminus is a variable region and at the C-terminus is a constant region; the CH3 and $C_L$ domains actually comprise the carboxy-terminal domains of the heavy and light chain, respectively.

The term "antibody fragment" as used herein refers to either an antigen binding fragment of an antibody or a non-antigen binding fragment (*e.g.,* Fc) of an antibody. The term "antigen binding fragment", as used herein, refers to one or more portions of an antibody that retain the ability to specifically interact with (*e.g.,* by binding, steric hindrance, stabilizing/destabilizing, spatial distribution) an epitope of an antigen. Examples of binding fragments include, but are not limited to, single-chain Fvs (scFv), disulfide-linked Fvs (sdFv), Fab fragments, F(ab') fragments, a monovalent fragment consisting of the $V_L$, $V_H$, $C_L$ and CH1 domains; a $F(ab)_2$ fragment, a bivalent fragment comprising two Fab fragments linked by a disulfide bridge at the hinge region; a Fd fragment consisting of the $V_H$ and CH1 domains; a Fv fragment consisting of the $V_L$ and $V_H$ domains of a single arm of an antibody; a dAb fragment (Ward et al., Nature 341:544-546, 1989), which consists of a $V_H$ domain; and an isolated complementarity determining region (CDR), or other epitope-binding fragments of an antibody.

Furthermore, although the two domains of the Fv fragment, $V_L$ and $V_H$, are coded for by separate genes, they can be joined, using recombinant methods, by a synthetic linker that enables them to be made as a single protein chain in which the $V_L$ and $V_H$ regions pair to form monovalent molecules (known as single chain Fv ("scFv"); see, *e.g.,* Bird et al., Science 242:423-426, 1988; and Huston et al., Proc. Natl. Acad. Sci. 85:5879-5883, 1988). Such single chain antibodies are also intended to be encompassed within the term "antigen binding fragment." These antigen binding fragments are obtained using conventional techniques known to those of skill in the art, and the fragments are screened

for utility in the same manner as are intact antibodies.

Antigen binding fragments can also be incorporated into single domain antibodies, maxibodies, minibodies, nanobodies, intrabodies, diabodies, triabodies, tetrabodies, v-NAR and bis-scFv (see, *e.g.,* Hollinger and Hudson, Nature Biotechnology 23:1126-1136, 2005). Antigen binding fragments can be grafted into scaffolds based on polypeptides such as fibronectin type III (Fn3) (see U.S. Pat. No. 6,703,199, which describes fibronectin polypeptide monobodies).

Antigen binding fragments can be incorporated into single chain molecules comprising a pair of tandem Fv segments ($V_H$-CH1-$V_H$-CH1) which, together with complementary light chain polypeptides, form a pair of antigen binding regions (Zapata et al., Protein Eng. 8:1057-1062, 1995; and U.S. Pat. No. 5,641,870).

The term "monoclonal antibody" or "monoclonal antibody composition" as used herein refers to polypeptides, including antibodies and antibody fragments that have substantially identical amino acid sequence or are derived from the same genetic source. This term also includes preparations of antibody molecules of single molecular composition. A monoclonal antibody composition displays a single binding specificity and affinity for a particular epitope.

The term "human antibody", as used herein, includes antibodies having variable regions in which both the framework and CDR regions are derived from sequences of human origin. Furthermore, if the antibody contains a constant region, the constant region also is derived from such human sequences, e.g., human germline sequences, or mutated versions of human germline sequences or antibody containing consensus framework sequences derived from human framework sequences analysis, for example, as described in Knappik et al., J. Mol. Biol. 296:57-86, 2000).

The human antibodies of the application may include amino acid residues not encoded by human sequences (*e.g.,* mutations introduced by random or site-specific mutagenesis *in vitro* or by somatic mutation *in vivo,* or a conservative substitution to promote stability or manufacturing).

The term "humanized" antibody, as used herein, refers to an antibody that retains the reactivity of a non-human antibody while being less immunogenic in humans. This can be achieved, for instance, by retaining the non-human CDR regions and replacing the remaining parts of the antibody with their human counterparts. See, e.g., Morrison et al., Proc. Natl. Acad. Sci. USA, 81:6851-6855 (1984); Morrison and Oi, Adv. Immunol., 44:65-92 (1988); Verhoeyen et al., Science, 239:1534-1536 (1988); Padlan,Molec. Immun., 28:489-498 (1991); Padlan, Molec. Immun., 31(3):169-217 (1994).

The term "recognize" as used herein refers to an antibody or antigen binding fragment thereof that finds and interacts (e.g., binds) with its epitope, whether that epitope is linear or conformational. The term "epitope" refers to a site on an antigen to which an antibody or antigen binding fragment of the present disclosure specifically binds. Epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, 4, 5, 6, 7, 8, 9, 10, 11, 12, 13, 14 or 15 amino acids in a unique spatial conformation. Methods of determining spatial conformation of epitopes include techniques in the art, for example, x-ray crystallography and 2-dimensional nuclear magnetic resonance (see, e.g., Epitope Mapping Protocols in Methods in Molecular Biology, Vol. 66, G. E. Morris, Ed. (1996)).

The term "affinity" as used herein refers to the strength of interaction between antibody and antigen at single antigenic sites. Within each antigenic site, the variable region of the antibody "arm" interacts through weak non-covalent forces with antigen at numerous sites; the more interactions, the stronger the affinity.

The term "isolated antibody" refers to an antibody that is substantially free of other antibodies having different antigenic specificities. An isolated antibody that specifically binds to one antigen may, however, have cross-reactivity to other antigens. Moreover, an isolated antibody may be substantially free of other cellular material and/or chemicals.

The term "conservatively modified variant" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" include individual substitutions, deletions or additions to a polypeptide sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the present disclosure. The following eight groups contain amino acids that are conservative substitutions for one

another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). In some embodiments, the term "conservative sequence modifications" are used to refer to amino acid modifications that do not significantly affect or alter the binding characteristics of the antibody containing the amino acid sequence.

The term "optimized" as used herein refers to a nucleotide sequence has been altered to encode an amino acid sequence using codons that are preferred in the production cell or organism, generally a eukaryotic cell, for example, a yeast cell, a *Pichia* cell, a fungal cell, a *Trichoderma* cell, a Chinese Hamster Ovary cell (CHO) or a human cell. The optimized nucleotide sequence is engineered to retain completely or as much as possible the amino acid sequence originally encoded by the starting nucleotide sequence, which is also known as the "parental" sequence.

The terms "percent identical" or "percent identity," in the context of two or more nucleic acids or polypeptide sequences, refers to two or more sequences or subsequences that are the same. Two sequences are "substantially identical" if two sequences have a specified percentage of amino acid residues or nucleotides that are the same (*i.e.*, 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 30 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, e.g., by the local homology algorithm of Smith and Waterman, Adv. Appl. Math. 2:482c (1970), by the homology alignment algorithm of Needleman and Wunsch, J. Mol. Biol. 48:443 (1970), by the search for similarity method of Pearson and Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, *e.g.,* Brent et al., Current Protocols in Molecular Biology, 2003).

Two examples of algorithms that are suitable for determining percent sequence identity and sequence similarity are the BLAST and BLAST 2.0 algorithms, which are described in Altschul et al., Nuc. Acids Res. 25:3389-3402, 1977; and Altschul et al., J. Mol. Biol. 215:403-410, 1990, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: The cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) or 10, M=5, N=-4 and a comparison of both strands. For amino acid sequences, the BLASTP program uses as defaults a word length of 3, and expectation (E) of 10, and the BLOSUM62 scoring matrix (see Henikoff and Henikoff, (1989) Proc. Natl. Acad. Sci. USA 89:10915) alignments (B) of 50, expectation (E) of 10, M=5, N=-4, and a comparison of both strands.

The BLAST algorithm also performs a statistical analysis of the similarity between two sequences (see, *e.g.,* Karlin and Altschul, Proc. Natl. Acad. Sci. USA 90:5873-5787, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a

reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller, Comput. Appl. Biosci. 4:11-17, 1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch, J. Mol. Biol. 48:444-453, 1970) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

Other than percentage of sequence identity noted above, another indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

The term "nucleic acid" is used herein interchangeably with the term "polynucleotide" and refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses silent variants thereof (e.g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., (1991) Nucleic Acid Res. 19:5081; Ohtsuka et al., (1985) J. Biol. Chem. 260:2605-2608; and Rossolini et al, (1994) Mol. Cell. Probes 8:91-98).

The term "operably linked" in the context of nucleic acids refers to a functional relationship between two or more polynucleotide (*e.g.,* DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, *i.e.,* they are cis-acting. However, some transcriptional regulatory sequences, such as enhancers, need not be physically contiguous or located in close proximity to the coding sequences whose transcription they enhance.

The terms "polypeptide" and "protein" are used interchangeably herein to refer to a polymer of amino acid residues. The terms apply to canonical amino acid polymers as well as to non-canonical amino acid polymers. Unless otherwise indicated, a particular polypeptide sequence also implicitly encompasses conservatively modified variants thereof.

The term "immunoconjugate" or "antibody conjugate" as used herein refers to the linkage of an antibody or an antibody fragment thereof with another agent, such as a chemotherapeutic agent, a toxin, an immunotherapeutic agent, an imaging probe, a spectroscopic probe, and the like. The linkage can be through one or multiple covalent bonds, or non-covalent interactions, and can include chelation. Various linkers, many of which are known in the art, can be employed in order to form the immunoconjugate. Additionally, the immunoconjugate can be provided in the form of a fusion protein that may be expressed from a polynucleotide encoding the immunoconjugate. As used herein, "fusion protein" refers to proteins created through the joining of two or more genes or gene fragments which originally coded for separate proteins (including peptides and polypeptides). Fusion proteins may be created by joining at the N- or C-terminus, or by insertions of genes or gene fragments into permissible regions of one of the partner proteins. Translation of the fusion gene results in a single protein with functional properties derived from each of the original proteins.

The term "subject" includes human and non-human animals. Non-human animals include all vertebrates, *e.g.,* mammals and non-mammals, such as non-human primates, sheep, dog, cow, chickens, amphibians, and reptiles. Except when noted, the terms "patient" or "subject" are used herein interchangeably.

The term "cytotoxin", or "cytotoxic agent" as used herein, refer to any agent that is detrimental to the growth and proliferation of cells and may act to reduce, inhibit, or destroy a cell or malignancy.

The term "anti-cancer agent" as used herein refers to any agent that can be used to treat a cell proliferative

disorder such as cancer, including but not limited to, cytotoxic agents, chemotherapeutic agents, radiotherapy and radiotherapeutic agents, targeted anti-cancer agents, and immunotherapeutic agents.

The term "drug moiety" or "payload" are used interchangeably and refers to a chemical moiety that is conjugated to the antibody or antibody fragment of the present disclosure, and can include any moiety that is useful to attach to an antibody or antibody fragment. For example, a drug moiety or payload can be an anti-cancer agent, an anti-inflammatory agent, an antifungal agent, an antibacterial agent, an anti-parasitic agent, an anti-viral agent, an anesthetic agent. In certain embodiments a drug moiety is selected from a V-ATPase inhibitor, a HSP90 inhibitor, an IAP inhibitor, an mTor inhibitor, a microtubule stabilizer, a microtubule destabilizers, an auristatin, a dolastatin, a maytansinoid, a MetAP (methionine aminopeptidase), an inhibitor of nuclear export of proteins CRM1, a DPPIV inhibitor, an inhibitor of phosphoryl transfer reactions in mitochondria, a protein synthesis inhibitor, a kinase inhibitor, a CDK2 inhibitor, a CDK9 inhibitor, a proteasome inhibitor, a kinesin inhibitor, an HDAC inhibitor, an Eg5 inhibitor a DNA damaging agent, a DNA alkylating agent, a DNA intercalator, a DNA minor groove binder and a DHFR inhibitor. Suitable examples include auristatins such as MMAE and MMAF; calicheamycins such as gamma-calicheamycin; and maytansinoids such as DM1 and DM4. Methods for attaching each of these to a linker compatible with the antibodies and method of the present disclosure are known in the art. See, *e.g.,* Singh et al., (2009) Therapeutic Antibodies: Methods and Protocols, vol. 525, 445-457. In addition, a payload can be a biophysical probe, a fluorophore, a spin label, an infrared probe an affinity probe, a chelator, a spectroscopic probe, a radioactive probe, a lipid molecule, a polyethylene glycol, a polymer, a spin label, DNA, RNA, a protein, a peptide, a surface, an antibody, an antibody fragment, a nanoparticle, a quantum dot, a liposome, a PLGA particle, a saccharide or a polysaccharide, a reactive functional group, or a binding agent that can connect the conjugate to another moiety, surface, etc.

The term "drug antibody ratio" (also referred to as "DAR"), refers to the number or payload or drug moieties linked to an antibody of the immunoconjugate. For example a drug antibody of ratio of 2 means that average of two drug moieties bound to an each antibody in a sample of immunoconjugates. Some individual immunoconjugates will in a sample with a drug antibody ratio of two might have none or only one drug moiety linked; others immunoconjugates in that sample will have two, three, four, or even more moieties on individual antibody. But the average in the sample will be two. There are different methods known in the art for measuring drug antibody ratios of immunoconjugates.

In an embodiment of this application, the DAR in a sample of immunoconjugates can be "homogenous". A "homogenous conjugation sample" is a sample with a narrow distribution of DAR. As an illustrative embodiment, in a homogenous conjugation sample having a DAR of 2, can contain within that sample antibodies that are not conjugated, and some antibodies having more than two moieties conjugated at about a DAR of two. "Most of the sample" means have at least over 70%, or at least over 80% or at least over 90% of the antibodies in the sample will be conjugated to two moieties.

As an illustrative embodiment, a homogenous conjugation sample having a DAR of 4, can contain within that sample antibodies that have more than four moieties or fewer than four moieties conjugated to each antibody at about a DAR of four. "Most of the sample" means have at least over 70%, or at least over 80% or at least over 90% of the antibodies in the sample will be conjugated to four moieties.

As an illustrative embodiment, a homogenous conjugation sample having a DAR of 6, can contain within that sample antibodies that have more than six moieties or fewer than six moieties conjugated to each antibody at about a DAR of six. "Most of the sample" means have at least over 70%, or at least over 80% or at least over 90% of the antibodies in the sample will be conjugated to six moieties.

As an illustrative embodiment, a homogenous conjugation sample having a DAR of 8, can contain within that sample antibodies that has some antibodies having more than eight moieties of fewer than either moieties conjugated to each antibody at about a DAR of eight. "Most of the sample" means have at least over 70%, or at least over 80% or at least over 90% of the antibodies in the sample will be conjugated to eight moieties.

An immunoconjugate having a "drug antibody ratio of about 2" refers to sample of immunoconjugates where in the drug antibody ratio can range from about 1.6-2.4 moieties/antibody, 1.8-2.3 moieties/antibody, or 1.9-2.1 moieties/antibody.

An immunoconjugate having a "drug antibody ratio of about 4" refers to sample of immunoconjugates where in the drug antibody ratio can range from about 3.4-4.4 moieties/antibody, 3.8-4.3 moieties/antibody, or 3.9-4.1 moieties/antibody.

An immunoconjugate having a "drug antibody ratio of about 6" refers to sample of immunoconjugates where in the drug antibody ratio can range from about 5.1-6.4 moieties/antibody, 5.8-6.3 moieties/antibody, or 5.9-6.1 moieties/antibody.

An immunoconjugate having a "drug antibody ratio of about 8" refers to sample of immunoconjugates where in the drug antibody ratio can range from about 7.6-8.4 moieties/antibody, 7.8-8.3 moieties/antibody, or 7.9-8.1 moieties/antibody.

"Tumor" refers to neoplastic cell growth and proliferation, whether malignant or benign, and all pre-cancerous and cancerous cells and tissues.

The term "anti-tumor activity" means a reduction in the rate of tumor cell proliferation, viability, or metastatic activity. A possible way of showing anti-tumor activity is to show a decline in growth rate of abnormal cells that arises during therapy or tumor size stability or reduction. Such activity can be assessed using accepted *in vitro* or *in vivo* tumor models, including but not limited to xenograft models, allograft models, MMTV models, and other known models known in the art to investigate anti-tumor activity.

The term "malignancy" refers to a non-benign tumor or a cancer. As used herein, the term "cancer" includes a malignancy characterized by deregulated or uncontrolled cell growth. Exemplary cancers include: carcinomas, sarcomas, leukemias, and lymphomas.

The term "cancer" includes primary malignant tumors (e.g., those whose cells have not migrated to sites in the subject's body other than the site of the original tumor) and secondary malignant tumors *(e.g.,* those arising from metastasis, the migration of tumor cells to secondary sites that are different from the site of the original tumor).

As used herein, the term "an optical isomer" or "a stereoisomer" refers to any of the various stereo isomeric configurations which may exist for a given compound of the present application and includes geometric isomers. It is understood that a substituent may be attached at a chiral center of a carbon atom. The term "chiral" refers to molecules which have the property of non-superimposability on their mirror image partner, while the term "achiral" refers to molecules which are superimposable on their mirror image partner. Therefore, the present disclosure includes enantiomers, diastereomers or racemates of the compound. "Enantiomers" are a pair of stereoisomers that are non- superimposable mirror images of each other. A 1:1 mixture of a pair of enantiomers is a "racemic" mixture. The term is used to designate a racemic mixture where appropriate. "Diastereoisomers" are stereoisomers that have at least two asymmetric atoms, but which are not mirror-images of each other. The absolute stereochemistry is specified according to the Cahn-Ingold-Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Certain compounds described herein contain one or more asymmetric centers or axes and may thus give rise to enantiomers, diastereomers, and other stereoisomeric forms that may be defined, in terms of absolute stereochemistry, as (*R*)- or (*S*)-.

Depending on the choice of the starting materials and procedures, the compounds can be present in the form of one of the possible isomers or as mixtures thereof, for example as pure optical isomers, or as isomer mixtures, such as racemates and diastereoisomer mixtures, depending on the number of asymmetric carbon atoms. The present application is meant to include all such possible isomers, including racemic mixtures, diasteriomeric mixtures and optically pure forms. Optically active (R)- and (S)-isomers may be prepared using chiral synthons or chiral reagents, or may be resolved using conventional techniques. If the compound contains a double bond, the substituent may be E or Z configuration. If the compound contains a disubstituted cycloalkyl, the cycloalkyl substituent may have a cis- or trans-configuration. All tautomeric forms are also intended to be included.

As used herein, the terms "salt" or "salts" refers to an acid addition or base addition salt of a compound of the present application. "Salts" include in particular "pharmaceutical acceptable salts". The term "pharmaceutically acceptable salts" refers to salts that retain the biological effectiveness and properties of the compounds of this application and, which typically are not biologically or otherwise undesirable. In many cases, the compounds of the present application are capable of forming acid and/or base salts by virtue of the presence of amino and/or carboxyl groups or groups similar thereto.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfonate, chloride/hydrochloride, chlorotheophyllinate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulfate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines,

substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts of the present application can be synthesized from a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the application include isotopes of hydrogen, carbon, nitrogen, oxygen, phosphorous, fluorine, and chlorine, such as $^{2}H$, $^{3}H$, $^{11}C$, $^{13}C$, $^{14}C$, $^{15}N$, $^{18}F$ $^{31}P$, $^{32}P$, $^{35}S$, $^{36}Cl$ $^{125}I$ respectively. The present disclosure includes various isotopically labeled compounds as defined herein, for example those into which radioactive isotopes, such as $^{3}H$ and $^{14}C$, or those into which non-radioactive isotopes, such as $^{2}H$ and $^{13}C$ are present. Such isotopically labeled compounds are useful in metabolic studies (with $^{14}C$), reaction kinetic studies (with, for example $^{2}H$ or $^{3}H$), detection or imaging techniques, such as positron emission tomography (PET) or single-photon emission computed tomography (SPECT) including drug or substrate tissue distribution assays, or in radioactive treatment of patients. In particular, an $^{18}F$ or labeled compound may be particularly desirable for PET or SPECT studies. Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

Further, substitution with heavier isotopes, particularly deuterium (i.e., $^{2}H$ or D) may afford certain therapeutic advantages resulting from greater metabolic stability, for example increased *in vivo* half-life or reduced dosage requirements or an improvement in therapeutic index. It is understood that deuterium in this context is regarded as a substituent of a compound of the formula (I). The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this application is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

The term "a therapeutically effective amount" of a compound of the present application refers to an amount of the compound of the present application that will elicit the biological or medical response of a subject, for example, reduction or inhibition of an enzyme or a protein activity, or ameliorate symptoms, alleviate conditions, slow or delay disease progression, or prevent a disease, etc. In one nonlimiting embodiment, the term "a therapeutically effective amount" refers to the amount of a compound of the present application that, when administered to a subject, is effective to at least partially alleviate, inhibit, prevent and/or ameliorate a condition, or a disorder or a disease, or at least partially inhibit activity of a targeted enzyme or receptor.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or

ameliorating at least one physical parameter including those which may not be discernible by the patient. In yet another embodiment, "treat", "treating" or "treatment" refers to modulating the disease or disorder, either physically, (*e.g.,* stabilization of a discernible symptom), physiologically, (*e.g.,* stabilization of a physical parameter), or both. In yet another embodiment, "treat", "treating" or "treatment" refers to preventing or delaying the onset or development or progression of the disease or disorder.

As used herein, a subject is "in need of" a treatment if such subject would benefit biologically, medically or in quality of life from such treatment.

As used herein, the term "a," "an," "the" and similar terms used in the context of the present application (especially in the context of the claims) are to be construed to cover both the singular and plural unless otherwise indicated herein or clearly contradicted by the context.

The term "thiol-maleimide" as used herein describes a group formed by reaction of a thiol with maleimide, having this general formula

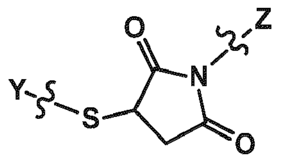

,

where Y and Z are groups to be connected via the thiol-maleimide linkage and can be linker units, and can be attached to antibodies or payloads. In some instances, Y is an engineered antibody according to the application, and the sulfur atom shown in the formula is from a cysteine at one of the substitution sites described herein; while Z represents a linker unit connected to a payload.

"Linker Unit" (LU) as used herein refers to a covalent chemical connection between two moieties, such as an antibody and a payload. Each LU can be comprised of one or more components described herein as $L_1$, $L_2$, $L_3$, $L_4$, $L_5$ and $L_6$. The linker unit can be selected to provide suitable spacing between the connected moieties, or to provide certain physicochemical properties, or to allow cleavage of the linker unit under certain conditions.

"Cleavable" as used herein refers to a linker or linker unit (LU) that connects two moieties by covalent connections, but breaks down to sever the covalent connection between the moieties under physiological conditions. Cleavage may be enzymatic or non-enzymatic, but generally releases a payload from an antibody without degrading the antibody.

"Non-cleavable" as used herein refers to a linker or linker unit (LU) that is not susceptible to breaking down under physiological conditions. While the linker may be modified physiologically, it keeps the payload connected to the antibody until the antibody is substantially degraded, *i.e.,* the antibody degradation precedes cleavage of the linker *in vivo.*

"Cyclooctyne" as used herein refers to an 8-membered ring containing a carbon-carbon triple bond (acetylene). The ring is optionally fused to one or two phenyl rings, which may be substituted with 1-4 $C_{1-4}$ alkyl, $C_{1-4}$ alkoxy, halo, hydroxyl, COOH, $COOL_1$, $-C(O)NH-L_1$, $O-L_1$, or similar groups, and which may contain N, O or S as a ring member. In preferred embodiments, cyclooctyne can be a Cs hydrocarbon ring, particularly an isolated ring that is saturated aside from the triple bond, and may be substituted with F or Hydroxy, and may be linked to a linker or LU via -O-, -C(O), C(O)NH, or C(O)O.

"Cyclooctene" as used herein refers to an 8-membered ring containing at least one double bond, especially a trans-double bond. The ring is optionally fused to one or two phenyl rings, which may be substituted with 1-4 $C_{1-4}$ alkyl, $C_{1-4}$ alkoxy, halo, hydroxyl, COOH, $COOL_1$, $-C(O)NH-L_1$, $O-L_1$, or similar groups, and which may contain N, O or S as a ring member. In preferred embodiments, cyclooctene can be an isolated Cs hydrocarbon ring that is saturated aside from the trans double bond and is optionally substituted with F or Hydroxy, and may be linked to a linker or LU via -O-, -C(O), C(O)NH, or C(O)O.

All methods described herein can be performed in any suitable order unless otherwise indicated herein or otherwise clearly contradicted by context. The use of any and all examples, or exemplary language (e.g. "such as") provided herein is intended merely to better illuminate the application and does not pose a limitation on the scope of the application otherwise claimed.

BRIEF DESCRIPTION OF THE DRAWINGS

Figure 1 depicts an amino acid sequence alignment of constant regions of trastuzumab (SEQ ID NO: 155), human IgG1 (SEQ ID NO: 151), IgG2 (SEQ ID NO: 152), IgG3 (SEQ ID NO: 153) and IgG4 (SEQ ID NO: 154).

Figure 2 is a graph depicting cell killing activity of antibody drug conjugates comprising a cKIT antibody that has two cys muations in its constant regions on cells that express cKIT. The antibody is conjugated to a linker-payload complex that inhibits Eg5. The square data points are for immunoconjugates comprising Compound A in Table 5; the triangle data points are for immunoconjugates comprising Compound B in Table 5; the square data points are for immunoconjugates comprising Compound C in Table 5.

Figure 3 depicts graphs illustrating the activity of immunoconjugates comprising cysteine-engineered cKIT antibodies in H526 tumor mouse xenograft models at dosages of 5 mg/kg (Figure 3A) and 10 mg/kg (Figure 3B) and an immunoconjugate comprising wild type cKIT antibody administered at dosages of 5.9 mg/kg (Figure 3A) and 11.8 mg/kg (Figure 3B).

Figure 4 is a graph depicting *in vivo* efficacy of anti-Her2 immunoconjugates conjugated with Eg5 inhibitor in a Her2 positive MDA-MB-231 clone 16 breast cancer xenograft model in mice.

Figure 5 is a graph depicting *in vivo* efficacy of anti-Her2 immunoconjugates conjugated with Eg5 inhibitor in a Her2 positive MDA-MB-453 breast cancer xenograft model in mice.

Figure 6 is a graph depicting *in vivo* efficacy of anti-Her2 immunoconjugates conjugated with Eg5 inhibitor in a Her2 positive HCC1954 breast cancer xenograft model in mice.

Figure 7 is a graph depicting results from an *in vivo* efficacy study of anti-cKIT ADCs conjugated with Compound F, in H526 tumor xenograft model in mice. Compound F was conjugated to cysteine-engineered or wild type cKIT antibodies. An anti-Her2 immunoconjugate was included as a non-binding control.

Figure 8 is a graph depicting results from pharmacokinetic studies of antibody anti-cKIT-HC-E152C-S375C-Compound F (Figure 8A) and antibody anti-cKIT-Compound F (Figure 8B) ADCs in naive mice at a dose of 1 mg/kg.

Figure 9 is a graph depicting *in vivo* efficacy of anti-cKIT immunoconjugates conjugated to two different compounds to two different cysteine-engineered antibodies in a H526 tumor xenograft model in mice.

DETAILED DESCRIPTION

The present application provides methods of site-specific labeling of antibodies or antibody fragments by replacing one or more amino acids of a parental antibody or antibody fragment at specific positions with cysteine amino acids ("Cys"), such that the engineered antibodies or antibody fragments are capable of conjugation to various agents (*e.g.,* cytotoxic agents). The present application also provides immunoconjugates that are produced by using the methods described herein.

When a cysteine is engineered into a parental antibody or antibody fragment, the modified antibody or antibody fragment is first recovered from the expression medium with cysteine or glutathione (GSH) attached at the engineered cysteine site(s) via a disulfide linkage (Chen et al., (2009) mAbs 16, 353-571). The attached cysteine or GSH is then removed in a reduction step, which also reduces all native inter-chain disulfide bonds of the parental antibody or antibody fragment. In a second step these disulfide bonds are re-oxidized before conjugation occurs. The present disclosure shows that when cysteine is engineered at certain sites, the re-oxidation step does not proceed well, presumably due to formation of the incorrect disulfide bonds. Accordingly, the present application provides unique sets of sites on the antibody heavy chain constant region and antibody light chain constant region, respectively, where Cys substitution as described herein produces modified antibodies or antibody fragments that perform well in the re-oxidation process, and also produce stable and well behaved immunoconjugates.

The site-specific antibody labeling according to the present application can be achieved with a variety of chemically accessible labeling reagents, such as anti-cancer agents, fluorophores, peptides, sugars, detergents, polyethylene glycols, immune potentiators, radio-imaging probes, prodrugs, and other molecules.

Accordingly, the present application provides methods of preparation of homogeneous immunoconjugates with a defined drug-to-antibody ratio for use in cancer therapy and other indications as well as imaging reagents. The present application also provides immunoconjugates prepared thereby, as well as pharmaceutical compositions comprising these immunoconjugates. The methods of the instant application can be used in combination with other conjugation methods known in the art.

The following enumerated embodiments represent certain aspects and variations of the application:

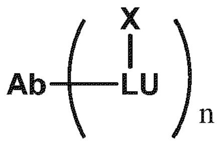

wherein Ab represents an antibody or antibody fragment comprising at least one cysteine residue at one of the preferred cysteine substitution sites described herein;

LU is a linker unit as described herein;

X is a payload or drug moiety;

and n is an integer from 1 to 16. In these embodiments, n is preferably about 2, about 4, about 6, or about 8. LU is typically a group of formula $-L_1-L_2-L_3-L_4-L_5-L_6-$, wherein $L_1$, $L_2$, $L_3$, $L_4$, $L_5$ and $L_6$ are independently selected from $-A_1-$, $-A_1X^2-$ and $-X^2-$; wherein:

$A_1$ is $-C(=O)NH-$, $-C(=O)NH(CH_2)_n-$, $-C(=O)NH(C(R^4)_2)_n-$, $-(O(CH_2)_n)_m-$, $-(O(C(R^4)_2)_n)_m-$,$-((CH_2)_nO)_m-$, $-((C(R^4)_2)_nO)_m-$, $-((CH_2)_nO)_m(CH_2)_n-$, $-((C(R^4)_2)_nO)_mC(R^4)_2)_n-$,$-(CH_2)_nC(=O)NH-$, $-(C(R^4)_2)_nC(=O)NH-$, $-(CH_2)_nNHC(=O)-$, $-(C(R^4)_2)_nNHC(=O)-$,$-NHC(=O)(CH_2)_n-$, $-NHC(=O)(C(R^4)_2)_n-$, $-C(=O)NH(CH_2)_nS-$, $-C(=O)NH(C(R^4)_2)_nS-$,$-S(CH_2)_nC(=O)NH-$, $-S(C(R^4)_2)_nC(=O)NH-$, $-C(=O)NH(CH_2)_nNHC(=O)(CH_2)_n-$, $-C(=O)NH(C(R^4)_2)_nNHC(=O)(C(R^4)_2)_n-$, $-C(=O)(CH_2)_n-$, $-C(=O)(C(R^4)_2)_n-$, $-(CH_2)_nC(=O)-$, $-(C(R^4)_2)_nC(=O)-$, $-(CH_2)_n(O(CH_2)_n)_mNHC(=O)(CH_2)_n-$-$-(C(R^4)_2)_n(O(C(R^4)_2)_n)_mNHC(=O)(C(R^4)_2)_n-$, $-(CH_2)_nNHC(=O)(CH_2)_n-$, $-(C(R^4)_2)_nNHC(=O)(C(R^4)_2)_n-$, $-(CH_2)_nNH((CH_2)_nO)_m(CH_2)_n-$-$-(C(R^4)_2)_nNH((C(R^4)_2)_nO)_m(C(R^4)_2)_n-$, $-(O(CH_2)_n)_mNHC(=O)(CH_2)_n-$, or $-(O(C(R^4)_2)_n)_mNHC(=O)(C(R^4)_2)_n-$;

each $X^2$ is independently selected from a bond, $R^8$,

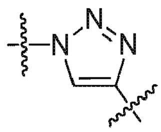
,
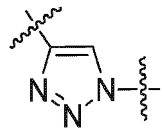
,

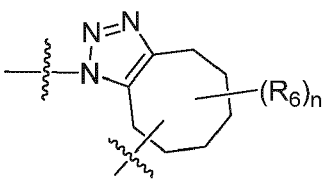
,
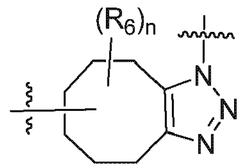
,
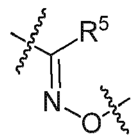
,

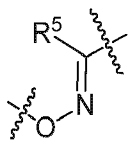
,
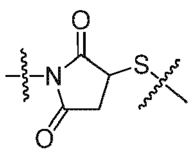
,
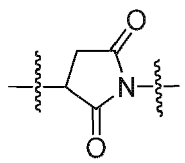
,
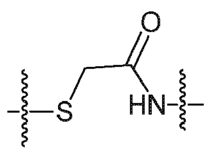
,

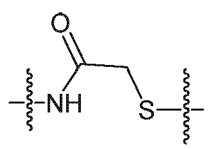
,
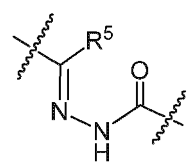
,
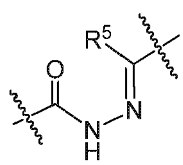
,
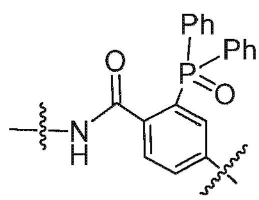
,

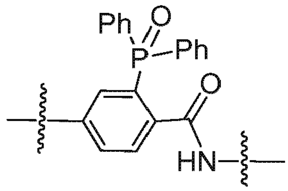

,

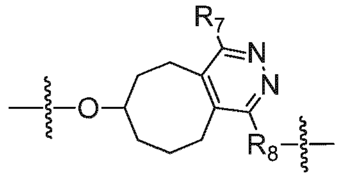

,

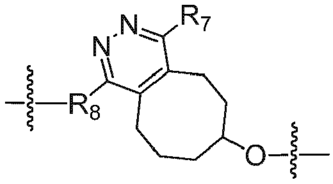

,

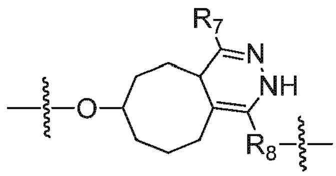

,

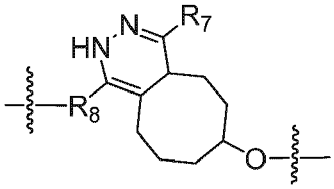

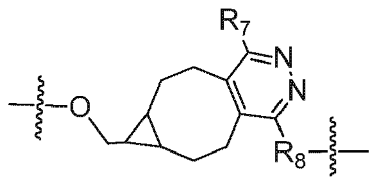

,

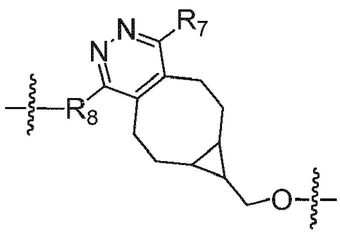

,

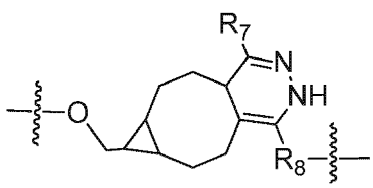

,

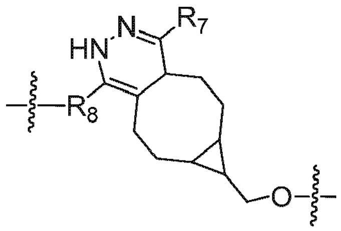

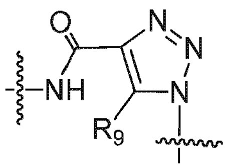

,

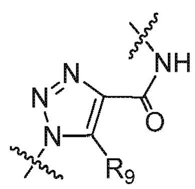

,

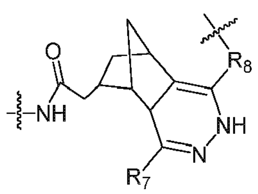

,

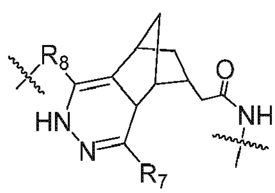

,

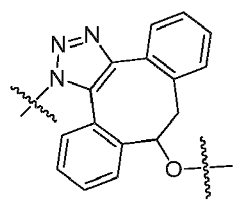

,

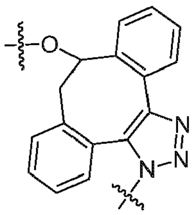 , 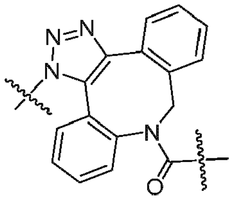 , 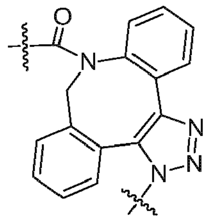

-S-, $-Si(OH)_2O-$,

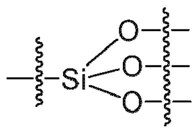 ,

$-CHR^4(CH_2)_nC(=O)NH-$, $-CHR^4(CH_2)_nNHC(=O)-$, -C(=O)NH- and-NHC(=O)-;
each $R^4$ is independently selected from H, $C_{1-4}$alkyl, side chains of known amino acids,-C(=O)OH and -OH,
each $R^5$ is independently selected from H, $C_{1-4}$alkyl, phenyl or $C_{1-4}$alkyl substituted with 1 to 3-OH groups;
each $R^6$ is independently selected from H, fluoro, benzyloxy substituted with -C(=O)OH, benzyl substituted with -C(=O)OH, $C_{1-4}$alkoxy substituted with -C(=O)OH and $C_{1-4}$alkyl substituted with -C(=O)OH;
$R^7$ is independently selected from H, $C_{1-4}$alkyl, phenyl, pyrimidine and pyridine;
$R^8$ is independently selected from

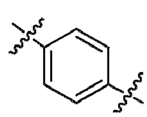 , 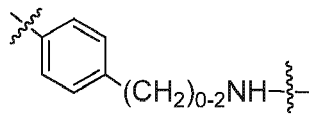 ,

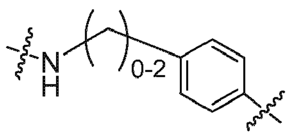 , 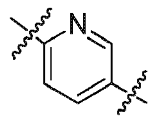 , 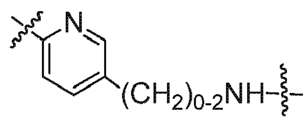 ,

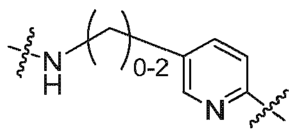 , 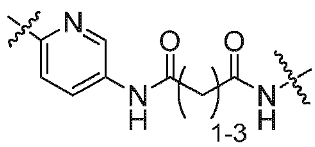 ,

and

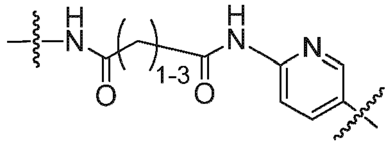 ,

$R^9$ is independently selected from H and $C_{1-6}$haloalkyl;
each n is independently selected from 1, 2, 3, 4, 5, 6, 7, 8 and 9, and

each m is independently selected from 1, 2, 3, 4, 5, 6, 7, 8 and 9.

In some of these embodiments, the immunoconjugate comprises a group of the formula

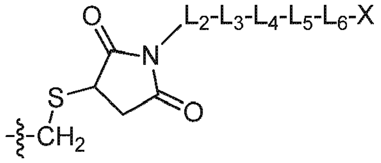

or

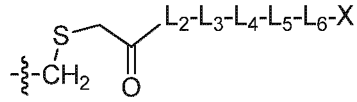

IIA IIB

wherein the sulfur atom is the sulfur of a cysteine residue in a modified antibody or antibody fragment and is located at one of the substitution sites identified herein.

In any of the foregoing embodiments, the cysteine substitution site may be a position that corresponds to one of the sites identified by a position number, even though the position of the site in the sequence has been changed by a modification or truncation of the full-length antibody. Corresponding sites can be readily identified by alignment of an antibody or fragment with a full-length antibody.

**1. Site-Specific Cysteine Engineered Antibodies**

<u>Site-specific Labeling</u>

The antibodies (*e.g.,* a parent antibody, optionally containing one or more non-canonical amino acids) of the present application are numbered according to the EU numbering system as set forth in Edelman et al., (1969) Proc. Natl. Acad. USA 63:78-85, except that the lambda light chain is numbered according to the Kabat numbering system as set forth in Kabat *et al.,* (1991) Fifth Edition. NIH Publication No. 91-3242. Human IgG1 constant region is used as a representative throughout the application. However, the present application is not limited to human IgG1; corresponding amino acid positions can be readily deduced by sequence alignment. For example, Figure 1 shows sequence alignment of antibody trastuzumab wild type heavy chain constant region (the sequence of which is STKGPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQVSLTCLVKGFYPSDI AVEWESNGQPENNYKTTPPVLDSDGSFFLYSKLTVDKSRWQQGNVFSCSVMHEALHNHYTQKS LSLSPG (SEQ ID NO: 155)), human IgG1 (the sequence of which is STKGPSVFPLAPSSKSTSGGTAALGCLVKDYFPEPVTVSWNS-GALTSGVHTFPAVLQSSGLYSLS SVVTVPSSSLGTQTYICNVNHKPSNTKVDKKVEPKSCDKTHTCPPCPAPELLG-GPSVFLFPPKPKD TLMISRTPEVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEALHNHYTQKS LSLSPG (SEQ ID NO: 151)), IgG2 (the sequence of which is STKGPSVFPLAPCSRSTSESTAALGCLVKDYFPEPVTVSWNSGALTS-GVHTFPAVLQSSGLYSLSS VVTVPSSNFGTQTYTCNVDHKPSNTKVDKTVERKCCVECPPCPAPPVAGPSVFLFPP-KPKDTLMI SRTPEVTCVVVDVSHEDPEVQFNWYVDGVEVHNAKTKPREEQFNSTFRVVSVLTVVHQDWLN GKEYKCKVSNKGLPAPIEKTISKTKGQPREPQVYTLPPSREEMTKNQVSLTCLVKGFYPSDISVE WESNGQPENNYKTTPPMLDSDGSFFLYSKLTVDKSRWQQGNVFSCSVMHEALHNHYTQKSLSL SPG (SEQ ID NO:152)), IgG3 (the sequence of which is STKGPSVFPLAPCSRSTSGGTAALGCLVKDYFPEPVTVSWNSGALTS-GVHTFPAVLQSSGLYSLS SVVTVPSSSLGTQTYTCNVNHKPSNTKVDKRVELKTPLGDTTHTCPRCPEPKSCDTPP-PCPRCPEP KSCDTPPPCPRCPEPKSCDTPPPCPRCPAPELLGGPSVFLFPPKPKDTLMISRTPEVTCVVVDVSHE DPEVQFKWYVDGVEVHNAKTKPREEQYNSTFRVVSVLTVLHQDWLNGKEYKCKVSNKALPAP IEKTISKTKGQPREPQVYTLPPSREEMTKNQVSLTCLVKGFYPSDIAVEWESSGQPENNYNTTPP MLDSDGSFFLYSKLTVDKSRWQQGNIFSCSVMHEALHNRFTQKSLSLSPG (SEQ ID NO:152)), and IgG4 (the sequence of which is STKGPSVFPLAPCSRSTSESTAALGCLVKDYFPEPVTVSWNSGALTSGVHTFPAVLQSSGLYSLSS VVTVPSSSLGTKTYTCNVDHKPSNTKVDKRVESKYGPPCPSCPAPEFLGGPSVFLFPPKPKDTLMI SRTPEVTCVVVDVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEALHNHYTQKSLSL SLG (SEQ ID NO:

153)) heavy chain constant regions, so that an identified Cys engineering site in the IgG1 constant region can be readily identified for IgG2, IgG3, and IgG4 as shown in Figure 1. For the light chain constant region, IgG1, IgG2, IgG3 and IgG4 are the same (the full-length wild type light chain sequence of human antibody trastuzumab is

DIQMTQSPSSLSASVGDRVTITCRASQDVNTAVAWYQQKPGKAPKLLIYSASFLYSGVPSRFSGS
RSGTDFTLTISSLQPEDFATYYCQQHYTTPPTFGQGTKVEIKRTVAAPSVFIFPPSDEQLKSGTASV
VCLLNNFYPREAKVQWKVDNALQSGNSQESVTEQDSKDSTYSLSSTLTLSKADYEKHKVYACE
VTHQGLSSPVTKSFNRGEC (SEQ ID NO:90)).

Table 1 below lists the amino acid positions in the constant region of the heavy chain of an antibody that can be replaced by a cysteine. Table 2A lists the amino acid positions in the constant region of the kappa light chain of an antibody that can be replaced by a cysteine. Table 2B lists the amino acid positions in the constant region of the lambda light chain of an antibody that can be replaced by a cysteine.

Table 1. Identified cysteine substitution sites in the heavy chain constant region of human IgG1 (Sites numbered according to EU numbering system).

| EU number | Residue | Surface accessibility [$Å^2$] | Selected HC Cys | SEQ ID NO. |
|---|---|---|---|---|
| 117 | SER | 128.0 | HC-S117C | 2 |
| 119 | SER | 79.1 | HC-S119C | 3 |
| 121 | LYS | 135.9 | HC-K121C | 4 |
| 124 | SER | 40.2 | HC-S124C | 5 |
| 132 | SER | 34.4 | HC-S132C | 6 |
| 134 | SER | 123.3 | HC-S134C | 7 |
| 136 | SER | 182.9 | HC-S136C | 8 |
| 139 | THR | 32.9 | HC-T139C | 9 |
| 152 | GLU | 52.1 | HC-E152C | 10 |
| 153 | PRO | 89.1 | HC-P153C | 11 |
| 155 | THR | 69.0 | HC-T155C | 12 |
| 157 | SER | 39.0 | HC-S157C | 13 |
| 164 | THR | 125.4 | HC-T164C | 14 |
| 165 | SER | 183.2 | HC-S165C | 15 |
| 169 | THR | 60.0 | HC-T169C | 16 |
| 171 | PRO | 33.3 | HC-P171C | 17 |
| 174 | LEU | 68.1 | HC-L174C | 18 |
| 176 | SER | 161.9 | HC-S176C | 19 |
| 177 | SER | 68.1 | HC-S177C | 20 |
| 189 | PRO | 86.4 | HC-P189C | 21 |
| 191 | SER | 126.8 | HC-S191C | 22 |
| 195 | THR | 111.3 | HC-T195C | 23 |
| 197 | THR | 89.8 | HC-T197C | 24 |
| 205 | LYS | 217.1 | HC-K205C | 25 |
| 207 | SER | 50.0 | HC-S207C | 26 |

(continued)

| EU number | Residue | Surface accessibility [$Å^2$] | Selected HC Cys | SEQ ID NO. |
|---|---|---|---|---|
| 212 | ASP | 97.0 | HC-D212C | 27 |
| 246 | LYS | 55.1 | HC-K246C | 28 |
| 258 | GLU | 42.1 | HC-E258C | 29 |
| 269 | GLU | 189.2 | HC-E269C | 30 |
| 274 | LYS | 137.8 | HC-K274C | 31 |
| 286 | ASN | 119.4 | HC-N286C | 32 |
| 288 | LYS | 181.8 | HC-K288C | 33 |
| 290 | LYS | 177.0 | HC-K290C | 34 |
| 292 | ARG | 251.5 | HC-R292C | 35 |
| 293 | GLU | 83.3 | HC-E293C | 36 |
| 294 | GLN | 73.5 | HC-E294C | 37 |
| 320 | LYS | 55.0 | HC-K320C | 38 |
| 322 | LYS | 78.3 | HC-K322C | 39 |
| 326 | LYS | 212.7 | HC-K326C | 40 |
| 330 | ALA | 96.3 | HC-A330C | 41 |
| 333 | GLU | 84.7 | HC-E333C | 42 |
| 334 | LYS | 49.6 | HC-K334C | 43 |
| 335 | THR | 70.1 | HC-T335C | 44 |
| 337 | SER | 15.1 | HC-S337C | 45 |
| 344 | ARG | 98.2 | HC-R344C | 46 |
| 355 | ARG | 249.4 | HC-R355C | 47 |
| 360 | LYS | 113.9 | HC-K360C | 48 |
| 362 | GLN | 40.8 | HC-Q362C | 49 |
| 375 | SER | 28.9 | HC-S375C | 50 |
| 382 | GLU | 21.8 | HC-E382C | 51 |

(continued)

| EU number | Residue | Surface accessibility [Å$^2$] | Selected HC Cys | SEQ ID NO. |
|---|---|---|---|---|
| 389 | ASN | 189.5 | HC-N389C | 52 |
| 390 | ASN | 36.4 | HC-N390C | 53 |
| 392 | LYS | 81.8 | HC-K392C | 54 |
| 393 | THR | 35.8 | HC-T393C | 55 |
| 398 | LEU | 110.9 | HC-L398C | 56 |
| 400 | SER | 81.3 | HC-S400C | 57 |
| 413 | ASP | 79.6 | HC-D413C | 58 |
| 415 | SER | 69.0 | HC-S415C | 59 |
| 422 | VAL | 80.8 | HC-V422C | 60 |

SEQ ID NO:1
EVQLVESGGGLVQPGGSLRLSCAASGFNIKDTYIHWVRQAPGKGLEWVARIYPTNGYTRYADSV
KGRFTISADTSKNTAYLQMNSLRAEDTAVYYCSRWGGDGFYAMDYWGQGTLVTVSSASTKGPS
VFPLAPSSKSTSGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPELLGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGFYPSDIAVEWES
NGQPENNYKTTPPVLDSDGSFFLYSKLTVDKSRWQQGNVFSCSVMHEALHNHYTQKSLSLSPGK

SEQ ID NO:2
CASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPKP
KDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYTQ
KSLSLSPGK

SEQ ID NO:3
SACTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPKP
KDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYTQ
KSLSLSPGK

(continued)

| EU number | Residue | Surface accessibility [Å$^2$] | Selected HC Cys | SEQ ID NO. |
|---|---|---|---|---|
| SEQ ID NO:4<br>SASTCGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:5<br>SASTKGPCVFPLAPSSKSTSGGTAALGCLVKDYFPEPVTVSWNSGALTSGVHTFPAVLQSSGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |
| SEQ ID NO:6<br>SASTKGPSVFPLAPSCKSTSGGTAALGCLVKDYFPEPVTVSWNSGALTSGVHTFPAVLQSSGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |
| SEQ ID NO:7<br>SASTKGPSVFPLAPSSKCTSGGTAALGCLVKDYFPEPVTVSWNSGALTSGVHTFPAVLQSSGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |
| SEQ ID NO:8<br>SASTKGPSVFPLAPSSKSTCGGTAALGCLVKDYFPEPVTVSWNSGALTSGVHTFPAVLQSSGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |

(continued)

| EU number | Residue | Surface accessibility [Å$^2$] | Selected HC Cys | SEQ ID NO. |
|---|---|---|---|---|
| SEQ ID NO:9<br>SASTKGPSVFPLAPSSKSTSGGCAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:10<br>SASTKGPSVFPLAPSSKSTSGGTAALGCLVKDYFPCPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:11<br>SASTKGPSVFPLAPSSKSTSGGTAALGCLVKDYFPECVTVSWNSGALTSGVHTFPAVLQSSGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |
| SEQ ID NO:12<br>SASTKGPSVFPLAPSSKSTSGGTAALGCLVKDYFPEPVCVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:13<br>SASTKGPSVFPLAPSSKSTSGGTAALGCLVKDYFPEPVTVCWNSGALTSGVHTFPAVLQSSGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |

(continued)

| EU number | Residue | Surface accessibility [Å$^2$] | Selected HC Cys | SEQ ID NO. |
|---|---|---|---|---|
| SEQ ID NO:14<br>SASTKGPSVFPLAPSSKSTSGGTAALGCLVKDYFPEPVTVSWNSGALCSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:15<br>SASTKGPSVFPLAPSSKSTSGGTAALGCLVKDYFPEPVTVSWNSGALTCGVHTFPAVLQSSGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |
| SEQ ID NO:16<br>SASTKGPSVFPLAPSSKSTSGGTAALGCLVKDYFPEPVTVSWNSGALTSGVHCFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:17<br>SASTKGPSVFPLAPSSKSTSGGTAALGCLVKDYFPEPVTVSWNSGALTSGVHTFCAVLQSSGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |
| SEQ ID NO:18<br>SASTKGPSVFPLAPSSKSTSGGTAALGCLVKDYFPEPVTVSWNSGALTSGVHTFPAVCQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |

(continued)

| EU number | Residue | Surface accessibility [Å$^2$] | Selected HC Cys | SEQ ID NO. |
|---|---|---|---|---|
| SEQ ID NO:19<br>SASTKGPSVFPLAPSSKSTSGGTAALGCLVKDYFPEPVTVSWNSGALTSGVHTFPAVLQCSGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |
| SEQ ID NO:20<br>SASTKGPSVFPLAPSSKSTSGGTAALGCLVKDYFPEPVTVSWNSGALTSGVHTFPAVLQSCGLYS<br>LSSVVTVPSSSLGTQTYICNVNHKPSNTKVDKKVEPKSCDKTHTCPPCPAPELLGGPSVFLFPPKP<br>KDTLMISRTPEVTCVVVDVSHEDPEVKFNWYVDGVEVHNAKTKPREEQYNSTYRVVSVLTVLH<br>QDWLNGKEYKCKVSNKALPAPIEKTISKAKGQPREPQVYTLPPSREEMTKNQVSLTCLVKGFYPS<br>DIAVEWESNGQPENNYKTTPPVLDSDGSFFLYSKLTVDKSRWQQGNVFSCSVMHEALHNHYTQ<br>KSLSLSPGK | | | | |
| SEQ ID NO:21<br>SASTKGPSVFPLAPSSKSTSGGTAALGCLVKDYFPEPVTVSWNSGALTSGVHTFPAVLQSSGLYSL<br>SSVVTVCSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:22<br>SASTKGPSVFPLAPSSKSTSGGTAALGCLVKDYFPEPVTVSWNSGALTSGVHTFPAVLQSSGLYSL<br>SSVVTVPSCSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:23<br>SASTKGPSVFPLAPSSKSTSGGTAALGCLVKDYFPEPVTVSWNSGALTSGVHTFPAVLQSSGLYSL<br>SSVVTVPSSSLGCQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |

(continued)

| EU number | Residue | Surface accessibility [$Å^2$] | Selected HC Cys | SEQ ID NO. |
|---|---|---|---|---|
| SEQ ID NO:24<br>SASTKGPSVFPLAPSSKSTSGGTAALGCLVKDYFPEPVTVSWNSGALTSGVHTFPAVLQSSGLYSL<br>SSVVTVPSSSLGTQCYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:25<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHCPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:26<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPCNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:27<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVCKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:28<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPCPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |

(continued)

| EU number | Residue | Surface accessibility [Å$^2$] | Selected HC Cys | SEQ ID NO. |
|---|---|---|---|---|
| SEQ ID NO:29<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPCVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:30<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHCDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:31<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVCFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:32<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHCAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:33<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNACTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |

(continued)

| EU number | Residue | Surface accessibility [Å$^2$] | Selected HC Cys | SEQ ID NO. |
|---|---|---|---|---|
| SEQ ID NO:34<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTCPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:35<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPCEEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:36<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPRCEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:37<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPRECQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:38<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYCCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |

(continued)

| EU number | Residue | Surface accessibility [$Å^2$] | Selected HC Cys | SEQ ID NO. |
|---|---|---|---|---|
| SEQ ID NO:39<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCCVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:40<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNCALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:41<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPCPIEKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:42<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPICKTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |

(continued)

| EU number | Residue | Surface accessibility [$Å^2$] | Selected HC Cys | SEQ ID NO. |
|---|---|---|---|---|
| SEQ ID NO:43<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIECTISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:44<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKCISKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:45<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTICKAKGQPREPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:46<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPCEPQVYTLPPSREEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:47<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSCEEMTKNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |

(continued)

| EU number | Residue | Surface accessibility [Å$^2$] | Selected HC Cys | SEQ ID NO. |
|---|---|---|---|---|
| SEQ ID NO:48<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTCNQVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:49<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNCVSLTCLVKGFYPSD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:50<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPCD<br>IAVEWESNGQPENNYKTTPPVLDSDGSFFLYSKLTVDKSRWQQGNVFSCSVMHEALHNHYTQKS<br>LSLSPGK | | | | |
| SEQ ID NO:51<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWCSNGQPENNYKTTPPVLDSDGSFFLYSKLTVDKSRWQQGNVFSCSVMHEALHNHYTQK<br>SLSLSPGK | | | | |
| SEQ ID NO:52<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPECNYKTTPPVLDSDGSFFLYSKLTVDKSRWQQGNVFSCSVMHEALHNHYTQKS<br>LSLSPGK | | | | |

(continued)

| EU number | Residue | Surface accessibility [Å$^2$] | Selected HC Cys | SEQ ID NO. |
|---|---|---|---|---|
| SEQ ID NO:53<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENCYKTTPPVLDSDGSFFLYSKLTVDKSRWQQGNVFSCSVMHEALHNHYTQKS<br>LSLSPGK | | | | |
| SEQ ID NO:54<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYCTTPPVLDSDGSFFLYSKLTVDKSRWQQGNVFSCSVMHEALHNHYTQKS<br>LSLSPGK | | | | |
| SEQ ID NO:55<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKCTPPVLDSDGSFFLYSKLTVDKSRWQQGNVFSCSVMHEALHNHYTQK<br>SLSLSPGK | | | | |
| SEQ ID NO:56<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVCDSDGSFFLYSKLTVDKSRWQQGNVFSCSVMHEALHNHYTQK<br>SLSLSPGK | | | | |
| SEQ ID NO:57<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDCDGSFFLYSKLTVDKSRWQQGNVFSCSVMHEALHNHYTQK<br>SLSLSPGK | | | | |

(continued)

| EU number | Residue | Surface accessibility [Å$^2$] | Selected HC Cys | SEQ ID NO. |
|---|---|---|---|---|
| SEQ ID NO:58<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVCKSRWQQGNVFSCSVMHEALHNHYTQKS<br>LSLSPGK | | | | |
| SEQ ID NO:59<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKCRWQQGNVFSCSVMHEALHNHYTQK<br>SLSLSPGK | | | | |
| SEQ ID NO:60<br>SASTKGPSVFPLAPSSKSTSGGTAALGCLVKDYFPEPVTVSWNSGALTSGVHTFPAVLQSSGLYSL<br>SSVVTVPSSSLGTQTYICNVNHKPSNTKVDKKVEPKSCDKTHTCPPCPAPELLGGPSVFLFPPKPK<br>DTLMISRTPEVTCVVVDVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTKNQVSLTCLVKGFYPSD<br>IAVEWESNGQPENNYKTTPPVLDSDGSFFLYSKLTVDKSRWQQGNCFSCSVMHEALHNHYTQKS<br>LSLSPGK | | | | |

Table 2A. Identified cysteine substitution sites in the kappa light chain constant region of human IgG1 (Sites numbered according to EU numbering system).

| EU number | Residue | Surface accessibility [$Å^2$] | Selected LC Cys | SEQ ID NO. |
|---|---|---|---|---|
| 107 | LYS | 90 | LC-K107C | 61 |
| 108 | ARG | 49 | LC-R108C | 62 |
| 109 | THR | 148 | LC-T109C | 63 |
| 112 | ALA | 50 | LC-A112C | 64 |
| 114 | SER | 39 | LC-S114C | 65 |
| 122 | ASP | 90 | LC-D122C | 66 |
| 123 | GLU | 51 | LC-E123C | 67 |
| 129 | THR | 41 | LC-T129C | 68 |
| 142 | ARG | 55 | LC-R142C | 69 |
| 143 | GLU | 117 | LC-E143C | 70 |
| 145 | LYS | 160 | LC-K145C | 71 |
| 152 | ASN | 157 | LC-N152C | 72 |
| 154 | LEU | 117 | LC-L154C | 73 |
| 156 | SER | 122 | LC-S156C | 74 |
| 159 | SER | 22 | LC-S159C | 75 |
| 161 | GLU | 66 | LC-E161C | 76 |
| 165 | GLU | 74 | LC-E165C | 77 |
| 168 | SER | 170 | LC-S168C | 78 |
| 169 | LYS | 241 | LC-K169C | 79 |
| 170 | ASP | 48 | LC-D170C | 80 |
| 182 | SER | 59 | LC-S182C | 81 |
| 183 | LYS | 131 | LC-K183C | 82 |
| 188 | LYS | 201 | LC-K188C | 83 |
| 190 | LYS | 167 | LC-K190C | 84 |
| 191 | VAL | 58 | LC-V191C | 85 |

(continued)

| EU number | Residue | Surface accessibility [Å$^2$] | Selected LC Cys | SEQ ID NO. |
|---|---|---|---|---|
| 197 | THR | 38 | LC-T197C | 86 |
| 199 | GLN | 127 | LC-Q199C | 87 |
| 203 | SER | 110 | LC-S203C | 88 |
| 206 | THR | 70 | LC-T206C | 89 |

SEQ ID NO:61
CRTVAAPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

SEQ ID NO:62
KCTVAAPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

SEQ ID NO:63
KRCVAAPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

SEQ ID NO:64
KRTVACPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

SEQ ID NO:65
KRTVAAPCVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC

SEQ ID NO:66
KRTVAAPSVFIFPPSCEQLKSGTASVVCLLNNFYPREAKVQWKVDNALQSGNSQESVTEQDSKD
STYSLSSTLTLSKADYEKHKVYACEVTHQGLSSPVTKSFNRGEC

SEQ ID NO:67
KRTVAAPSVFIFPPSDCQLKSGTASVVCLLNNFYPREAKVQWKVDNALQSGNSQESVTEQDSKD
STYSLSSTLTLSKADYEKHKVYACEVTHQGLSSPVTKSFNRGEC

SEQ ID NO:68
KRTVAAPSVFIFPPSDEQLKSGCASVVCLLNNFYPREAKVQWKVDNALQSGNSQESVTEQDSKD
STYSLSSTLTLSKADYEKHKVYACEVTHQGLSSPVTKSFNRGEC

(continued)

| EU number | Residue | Surface accessibility [Å$^2$] | Selected LC Cys | SEQ ID NO. |
|---|---|---|---|---|
| SEQ ID NO:69<br>KRTVAAPSVFIFPPSDEQLKSGTASVVCLLNNFYPCEAKVQWKVDNALQSGNSQESVTEQDSKD<br>STYSLSSTLTLSKADYEKHKVYACEVTHQGLSSPVTKSFNRGEC | | | | |
| SEQ ID NO:70<br>KRTVAAPSVFIFPPSDEQLKSGTASVVCLLNNFYPRCAKVQWKVDNALQSGNSQESVTEQDSKD<br>STYSLSSTLTLSKADYEKHKVYACEVTHQGLSSPVTKSFNRGEC | | | | |
| SEQ ID NO:71<br>KRTVAAPSVFIFPPSDEQLKSGTASVVCLLNNFYPREACVQWKVDNALQSGNSQESVTEQDSKD<br>STYSLSSTLTLSKADYEKHKVYACEVTHQGLSSPVTKSFNRGEC | | | | |
| SEQ ID NO:72<br>KRTVAAPSVFIFPPSDEQLKSGTASVVCLLNNFYPREAKVQWKVDCALQSGNSQESVTEQDSKD<br>STYSLSSTLTLSKADYEKHKVYACEVTHQGLSSPVTKSFNRGEC | | | | |
| SEQ ID NO:73<br>KRTVAAPSVFIFPPSDEQLKSGTASVVCLLNNFYPREAKVQWKVDNACQSGNSQESVTEQDSKD<br>STYSLSSTLTLSKADYEKHKVYACEVTHQGLSSPVTKSFNRGEC | | | | |
| SEQ ID NO:74<br>KRTVAAPSVFIFPPSDEQLKSGTASVVCLLNNFYPREAKVQWKVDNALQCGNSQESVTEQDSK<br>DSTYSLSSTLTLSKADYEKHKVYACEVTHQGLSSPVTKSFNRGEC | | | | |
| SEQ ID NO:75<br>KRTVAAPSVFIFPPSDEQLKSGTASVVCLLNNFYPREAKVQWKVDNALQSGNCQESVTEQDSK<br>DSTYSLSSTLTLSKADYEKHKVYACEVTHQGLSSPVTKSFNRGEC | | | | |
| SEQ ID NO:76<br>KRTVAAPSVFIFPPSDEQLKSGTASVVCLLNNFYPREAKVQWKVDNALQSGNSQCSVTEQDSKD<br>STYSLSSTLTLSKADYEKHKVYACEVTHQGLSSPVTKSFNRGEC | | | | |
| SEQ ID NO:77<br>KRTVAAPSVFIFPPSDEQLKSGTASVVCLLNNFYPREAKVQWKVDNALQSGNSQESVTCQDSKD<br>STYSLSSTLTLSKADYEKHKVYACEVTHQGLSSPVTKSFNRGEC | | | | |
| SEQ ID NO:78<br>KRTVAAPSVFIFPPSDEQLKSGTASVVCLLNNFYPREAKVQWKVDNALQSGNSQESVTEQDCK<br>DSTYSLSSTLTLSKADYEKHKVYACEVTHQGLSSPVTKSFNRGEC | | | | |

(continued)

| EU number | Residue | Surface accessibility [Å$^2$] | Selected LC Cys | SEQ ID NO. |
|---|---|---|---|---|
| SEQ ID NO:79<br>KRTVAAPSVFIFPPSDEQLKSGTASVVCLLNNFYPREAKVQWKVDNALQSGNSQESVTEQDSCD<br>STYSLSSTLTLSKADYEKHKVYACEVTHQGLSSPVTKSFNRGEC | | | | |
| SEQ ID NO:80<br>KRTVAAPSVFIFPPSDEQLKSGTASVVCLLNNFYPREAKVQWKVDNALQSGNSQESVTEQDSKC<br>STYSLSSTLTLSKADYEKHKVYACEVTHQGLSSPVTKSFNRGEC | | | | |
| SEQ ID NO:81<br>KRTVAAPSVFIFPPSDEQLKSGTASVVCLLNNFYPREAKVQWKVDNALQSGNSQESVTEQDSKD<br>STYSLSSTLTLCKADYEKHKVYACEVTHQGLSSPVTKSFNRGEC | | | | |
| SEQ ID NO:82<br>KRTVAAPSVFIFPPSDEQLKSGTASVVCLLNNFYPREAKVQWKVDNALQSGNSQESVTEQDSKD<br>STYSLSSTLTLSCADYEKHKVYACEVTHQGLSSPVTKSFNRGEC | | | | |
| SEQ ID NO:83<br>KRTVAAPSVFIFPPSDEQLKSGTASVVCLLNNFYPREAKVQWKVDNALQSGNSQESVTEQDSKD<br>STYSLSSTLTLSKADYECHKVYACEVTHQGLSSPVTKSFNRGEC | | | | |
| SEQ ID NO:84<br>KRTVAAPSVFIFPPSDEQLKSGTASVVCLLNNFYPREAKVQWKVDNALQSGNSQESVTEQDSKD<br>STYSLSSTLTLSKADYEKHCVYACEVTHQGLSSPVTKSFNRGEC | | | | |
| SEQ ID NO:85<br>KRTVAAPSVFIFPPSDEQLKSGTASVVCLLNNFYPREAKVQWKVDNALQSGNSQESVTEQDSKD<br>STYSLSSTLTLSKADYEKHKCYACEVTHQGLSSPVTKSFNRGEC | | | | |
| SEQ ID NO:86<br>KRTVAAPSVFIFPPSDEQLKSGTASVVCLLNNFYPREAKVQWKVDNALQSGNSQESVTEQDSKD<br>STYSLSSTLTLSKADYEKHKVYACEVCHQGLSSPVTKSFNRGEC | | | | |
| SEQ ID NO:87<br>KRTVAAPSVFIFPPSDEQLKSGTASVVCLLNNFYPREAKVQWKVDNALQSGNSQESVTEQDSKD<br>STYSLSSTLTLSKADYEKHKVYACEVTHCGLSSPVTKSFNRGEC | | | | |
| SEQ ID NO:88<br>KRTVAAPSVFIFPPSDEQLKSGTASVVCLLNNFYPREAKVQWKVDNALQSGNSQESVTEQDSKD<br>STYSLSSTLTLSKADYEKHKVYACEVTHQGLSCPVTKSFNRGEC | | | | |

(continued)

| EU number | Residue | Surface accessibility [$Å^2$] | Selected LC Cys | SEQ ID NO. |
|---|---|---|---|---|
| SEQ ID NO:89<br>KRTVAAPSVFIFPPSDEQLKSGTASVVCLLNNFYPREAKVQWKVDNALQSGNSQESVTEQDSKD<br>STYSLSSTLTLSKADYEKHKVYACEVTHQGLSSPVCKSFNRGEC | | | | |

Table 2B. Identified cysteine substitution sites on the lambda light chain of human IgG1.

| Kabat number | Residue | Surface accessibility [Å$^2$] | Selected LC Cys | SEQ ID NO. |
|---|---|---|---|---|
| 143 | ALA | 82 | LC-A143C | 92 |
| 145 | THR | 106 | LC-T145C | 93 |
| 147 | ALA | 14 | LC-A147C | 94 |
| 156 | LYS | 233 | LC-K156C | 95 |
| 159 | VAL | 28 | LC-V159C | 96 |
| 163 | THR | 157 | LC-T163C | 97 |
| 168 | SER | 166 | LC-S168C | 98 |
| SEQ ID NO:91 (Constant Region of human lambda ligt chain)<br>QPKAAPSVTLFPPSSEELQANKATLVCLISDFYPGAVTVAWKADSSPVKAGVETTTPSKQSN<br>NKYAASSYLSLTPEQWKSHRSYSCQVTHEGSTVEKTVAPTECS | | | | |
| SEQ ID NO:92<br>QPKAAPSVTLFPPSSEELQANKATLVCLISDFYPGCVTVAWKADSSPVKAGVETTTPSKQSN<br>NKYAASSYLSLTPEQWKSHRSYSCQVTHEGSTVEKTVAPTECS | | | | |
| SEQ ID NO:93<br>QPKAAPSVTLFPPSSEELQANKATLVCLISDFYPGAVCVAWKADSSPVKAGVETTTPSKQSN | | | | |
| NKYAASSYLSLTPEQWKSHRSYSCQVTHEGSTVEKTVAPTECS | | | | |
| SEQ ID NO:94<br>QPKAAPSVTLFPPSSEELQANKATLVCLISDFYPGAVTVCWKADSSPVKAGVETTTPSKQSN<br>NKYAASSYLSLTPEQWKSHRSYSCQVTHEGSTVEKTVAPTECS | | | | |
| SEQ ID NO:95<br>QPKAAPSVTLFPPSSEELQANKATLVCLISDFYPGAVTVAWKADSSPVCAGVETTTPSKQSN<br>NKYAASSYLSLTPEQWKSHRSYSCQVTHEGSTVEKTVAPTECS | | | | |
| SEQ ID NO:96<br>QPKAAPSVTLFPPSSEELQANKATLVCLISDFYPGAVTVAWKADSSPVKAGCETTTPSKQSN<br>NKYAASSYLSLTPEQWKSHRSYSCQVTHEGSTVEKTVAPTECS | | | | |
| SEQ ID NO:97<br>QPKAAPSVTLFPPSSEELQANKATLVCLISDFYPGAVTVAWKADSSPVKAGVETTCPSKQSN<br>NKYAASSYLSLTPEQWKSHRSYSCQVTHEGSTVEKTVAPTECS | | | | |

(continued)

| Kabat number | Residue | Surface accessibility [Å$^2$] | Selected LC Cys | SEQ ID NO. |
|---|---|---|---|---|
| SEQ ID NO:98<br>QPKAAPSVTLFPPSSEELQANKATLVCLISDFYPGAVTVAWKADSSPVKAGVETTTPSKQC<br>NNKYAASSYLSLTPEQWKSHRSYSCQVTHEGSTVEKTVAPTECS | | | | |

Because of the high sequence homology of constant regions of IgG1, IgG2, IgG3 and IgG4 antibodies, findings of the present application are not limited to any specific antibodies or antibody fragments.

In one embodiment, the present application provides immunoconjugates comprising a modified antibody or an antibody fragment thereof, and a drug moiety, wherein said modified antibody or antibody fragment thereof comprises a substitution of one or more *(e.g.,* 1, 2, 3, 4, 5, 6, 7, 8, 9, or 10) amino acids on its heavy chain constant region

In an embodiment of the present application, the amino acid substitution described herein is cysteine comprising a thiol group. In some aspects of the present application, the thiol group is utilized for chemical conjugation, and is attached to a linker unit (LU) and/or drug moiety. In some embodiments, the immunoconjugates of the present application comprise a drug moiety selected from the group consisting of a V-ATPase inhibitor, a HSP90 inhibitor, an IAP inhibitor, an mTor inhibitor, a microtubule stabilizer, a microtubule destabilizers, an auristatin, a dolastatin, a maytansinoid, a MetAP (methionine aminopeptidase), an inhibitor of nuclear export of proteins CRM1, a DPPIV inhibitor, proteasome inhibitors, an inhibitors of phosphoryl transfer reactions in mitochondria, a protein synthesis inhibitor, a kinase inhibitor, a CDK2 inhibitor, a CDK9 inhibitor, an kinesin inhibitor, an HDAC inhibitor, an Eg5 inhibitor, a DNA damaging agent, a DNA alkylating agent, a DNA intercalator, a DNA minor groove binder and a DHFR inhibitor. In some embodiments, the immunoconjugates of the present application comprise a drug moiety that is an anti-cancer agent. The modified antibody or antibody fragments of the present application can be any formats known in the art, such as a monoclonal, chimeric, humanized, fully human, bispecific, or multispecific antibody or antibody fragment thereof.

According to the present application, the modified antibody heavy chain and/or light chain (or antibody fragment thereof) may contain 1, 2, 3, 4, 5, 6, 7, 8, or more cysteine substitutions in its constant regions. In one embodiment, the modified antibodies or antibody fragments contain 2, 4, 6, 8, or more cysteine substitutions in its constant regions. In some embodiments, the modified antibody, antibody fragment or immunoconjugate thereof comprises four or more Cys substitutions.

In one embodiment, the parental antibody (antibody without cysteine substitution) is an IgG, IgM, IgE, or IgA antibody. In a specific embodiment, the parental antibody is an IgG1 antibody. In another specific embodiment, the parental antibody is an IgG2, IgG3, or IgG4 antibody.

The present application also provides modified antibodies or antibody fragments thereof comprising a substitution of one or more amino acids on its heavy chain constant region chosen from positions identified in Table 1. In some embodiments, the present application provides modified antibodies or antibody fragments thereof comprising a substitution of one or more amino acids on its light chain constant region chosen from positions identified in Table 2A or Table 2B. In other embodiments, the modified antibodies or antibody fragment thereof comprise one or more amino acids on its heavy chain constant region chosen from positions identified in Table 1 and one or more amino acids on its light chain constant region chosen from positions identified in Table 2A.

In certain embodiments, the modified antibodies or antibody fragments provided herein are labeled using the methods of the present application in combination with other conjugation methods known in the art including, but not limited to, chemoselective conjugation through lysine, histidine, tyrosine, formyl-glycine, pyrrolysine, pyrroline-carboxylysine, unnatural amino acids, and protein tags for enzyme-mediated conjugation (e.g., S6 tags).

**2. Conjugation Chemistry**

The conjugated antibody or antibody fragment thereof provided herein is produced by post-translational modification of at least one cysteine residue that was incorporated into the antibody or antibody fragment thereof as described above by site-specific labeling methods. The conjugated antibody or antibody fragment can be prepared by methods known in the art for conjugation of a payload of interest to cysteine residues that occur naturally in proteins, and by methods described for conjugation to proteins engineered to contain an additional cysteine residue substituted for another amino acid of a natural protein sequence.

In certain embodiments the modified antibodies or antibody fragment thereof provided herein are conjugated using known methods wherein the incorporated cysteine (cys) is conjugated to a maleimide derivative as Scheme Ia below. Modified antibodies of the present application that undergo this type of conjugation contain a thiol-maleimide linkage.

Scheme Ia. Conjugation via thiol-maleimide adduct formation.

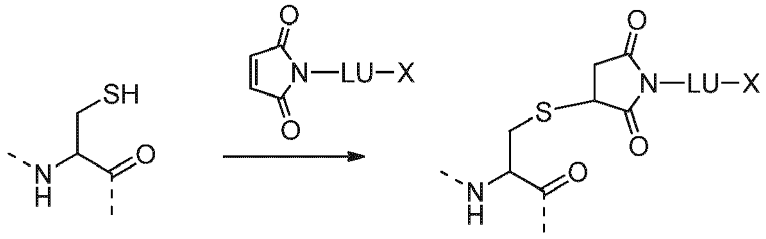

where:

LU is a Linker Unit (LU), and
X is a payload or drug moiety.

In other embodiments, the Cys incorporated into the modified antibodies or antibody fragment is conjugated by reaction with an alpha-halo carbonyl compound such as a chloro-, bromo-, or iodo-acetamide as shown in Scheme Ib below. It is understood that other leaving groups besides halogen, such as tosylate, triflate and other alkyl or aryl sulfonates, can be used as the leaving group Y. While Scheme Ib depicts reaction of a Cys thiol with an alpha-halo acetamide, the method includes any alkylation of a sulfur of an incorporated Cys with a group of the formula Y-CHR-C(=O)-, where R is H or $C_{1-4}$ alkyl, Y is a leaving group (typically Cl, Br, or I, and optionally an alkylsulfonate or arylsulfonate); it is not limited to amides.

Scheme Ib. Conjugation via reaction with an alpha-halo carbonyl compound.

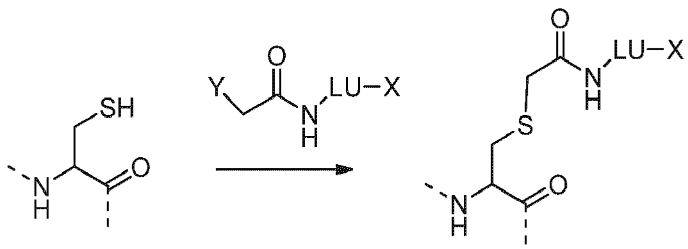

Y is a leaving group (Cl, Br, I, OTs, OTf, and the like)
LU is a linker unit
X is a payload or drug moiety

Alternatively, the Cys incorporated into the modified antibodies or antibody fragment can be conjugated by reaction with an external thiol under conditions that induce formation of a disulfide bond between the external thiol and the sulfur atom of the incorporated cysteine residue as shown in Scheme Ic below. In these examples, R can be H; however, compounds where one or both R groups represent an alkyl group, e.g., Methyl, have been found to increase the stability of the disulfide.

Scheme Ic. Conjugation via disulfide formation.

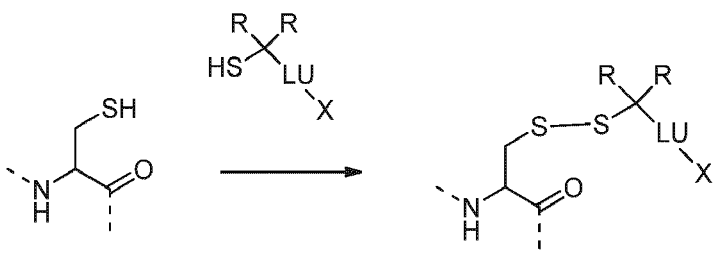

each R is independently H or $C_{1-4}$ alkyl
LU is a linker unit
X is a payload or drug moiety

By way of example only, such post-translational modifications are illustrated in Schemes (Ia)-(Ic) above, where the starting structure represents a cysteine incorporated into a light chain or heavy chain of an antibody at one of the

specific sites identified herein. Methods for performing each of these conjugation methods are well known in the art. An antibody can be modified by these methods in its light chains, or its heavy chains, or in both light and heavy chains. An antibody in which each light chain or each heavy chain has been modified to contain a single incorporated cysteine will generally contain two conjugation sites, since an antibody typically contains two light and two heavy chains.

Upon conjugation, the modified antibodies of the present application typically contain 1-12, frequently 2-8, and preferably 2, 4 or 6 -LU-X (Linker Unit-Payload) moieties. In some embodiments, an antibody light or heavy chain is modified to incorporate two new Cys residues at two of the specific sites identified herein for Cys substitutions (or alternatively one Cys is incorporated in the light chain and one in the heavy chain), so the tetrameric antibody ultimately contains four conjugation sites. Similarly the antibody can be modified by replacement of 3 or 4 of its native amino acids with Cys at the specific sites identified herein, in light chain or heavy chain or a combination thereof, resulting in 6 or 8 conjugation sites in the tetrameric antibody.

X in these conjugates represents a payload, which can be any chemical moiety that is useful to attach to an antibody. In some embodiments, X is a drug moiety selected from a cytotoxin, an anti-cancer agent, an anti-inflammatory agent, an antifungal agent, an antibacterial agent, an anti-parasitic agent, an anti-viral agent, an immune potentiator, and an anesthetic agent or any other therapeutic, or biologically active moiety or drug moiety. In other embodiments, X is a label such as a biophysical probe, a fluorophore, an affinity probe, a spectroscopic probe, a radioactive probe, a spin label, or a quantum dot. In other embodiments, X is a chemical moiety that modifies the antibody's physicochemical properties such as a lipid molecule, a polyethylene glycol, a polymer, a polysaccharide, a liposome, or a chelator. In other embodiments, X is a functional or detectable biomolecule such as a nucleic acid, a ribonucleic acid, a protein, a peptide (e.g., an enzyme or receptor), a sugar or polysaccharide, an antibody, or an antibody fragment. In other embodiments, X is an anchoring moiety such as a nanoparticle, a PLGA particle, or a surface, or any binding moiety for specifically binding the conjugate to another moiety, such as a histidine tag, poly-G, biotin, avidin, streptavidin, and the like. In other embodiments, X is a reactive functional group that can be used to attach the antibody conjugate to another chemical moiety, such as a drug moiety, a label, another antibody, another chemical moiety, or a surface.

The Linker Unit (LU) can be any suitable chemical moiety that covalently attaches the thiol-reactive group (e.g., maleimide, alpha-halo carbonyl, vinyl carbonyl (e.g., acrylate or acrylamide), vinyl sulfone, vinylpyridine, or thiol) to a payload. Many suitable LUs are known in the art. For example, LU can be comprised of one, two, three, four, five, six, or more than six linkers referred to herein as $L_1$, $L_2$, $L_3$, $L_4$, $L_5$ and $L_6$. In certain embodiments the LU comprises a linker selected from a non-enzymatically cleavable linker, a non-cleavable linker, an enzymatically cleavable linker, a photo-stable linker, a photo-cleavable linker or any combination thereof, and the LU optionally contains a self-immolative spacer.

In some embodiments, LU is a group of the formula $-L_1-L_2-L_3-L_4-$ or $-L_1-L-L_3-L_4-L_5-L_6-$. Linking groups $L_1$, $L_2$, $L_3$, $L_4$, $L_5$ and $L_6$ for use in LU include alkylene groups $-(CH_2)_n-$ (where n is 1-20, typically 1-10 or 1-6), ethylene glycol units $(-CH_2CH_2O-)_n$ (where n is 1-20, typically 1-10 or 1-6), amides -C(=O)-NH- or -NH-C(=O)-, esters -C(=O)-O- or -O-C(=O)-, rings having two available points of attachment such as divalent phenyl, $C_{3-8}$ cycloalkyl or $C_{4-8}$ heterocyclyl groups, amino acids -NH-CHR*-C=O- or -C(=O)-CHR*-NH-, where R* is the side chain of a known amino acid (frequently one of the canonical amino acids, but also including e.g. norvaline, norleucine, homoserine, homocysteine, phenylglycine, citrulline, and other named alpha-amino acids), polypeptides of known amino acids (e.g., dipeptides, tripeptides, tetrapeptides, etc.), thiol-maleimide linkages (from addition of-SH to maleimide), $-S-CR_2-$ and other thiol ethers such as $-S-CR_2-C(=O)-$ or $-C(=O)-CR_2-S-$, where R is as defined above for Scheme Ic, $-CH_2-C(=O)-$, and disulfides (-S-S-), as well as combinations of any of these with other linkers described below, e.g., a bond, a non-enzymatically cleavable linker, a non-cleavable linker, an enzymatically cleavable linker, a photo-stable linker, a photo-cleavable linker or a linker that comprises a self-immolative spacer.

In some embodiments when LU is $-L_1-L_2-L_3-L_4-L_s-L_6-$, $L_1$, $L_2$, $L_3$, $L_4$, $L_5$ and $L_6$ can be selected from:

- $A_1-$, $-A_1X^2-$ and $-X^2-$; wherein:

  $A_1$ is -C(=O)NH-, $-C(=O)NH(CH_2)_n-$, $-C(=O)NH(C(R^4)_2)_n-$, $-(O(CH_2)_n)_m-$, $-(O(C(R^4)_2)_n)_m-$,$-((CH_2)_nO)_m-$, $-((C(R^4)_2)_nO)_m-$, $-((CH_2)_nO)_m(CH_2)_n-$, $-((C(R^4)_2)_nO)_mC(R^4)_2)_n-$,$-(CH_2)_nC(=O)NH-$, $-(C(R^4)_2)_nC(=O)NH-$, $-(CH_2)_nNHC(=O)-$, $-(C(R^4)_2)_nNHC(=O)-$,$-NHC(=O)(CH_2)_n-$, $-NHC(=O)(C(R^4)_2)_n-$, $-C(=O)NH(CH_2)_nS-$, $-C(=O)NH(C(R^4)_2)_nS-$,$-S(CH_2)_nC(=O)NH-$, $-S(C(R^4)_2)_nC(=O)NH-$, $-C(=O)NH(CH_2)_nNHC(=O)(CH_2)_n-$, $-C(=O)NH(C(R^4)_2)_nNHC(=O)(C(R^4)_2)_n-$, $-C(=O)(CH_2)_n-$, $-C(=O)(C(R^4)_2)_n-$, $-(CH_2)_nC(=O)-$, $-(C(R^4)_2)_nC(=O)-$, $-(CH_2)_n(O(CH_2)_n)_mNHC(=O)(CH_2)_n-$,$-(C(R')_2)_n(O(C(R^4)_2)_n)_mNHC(=O)(C(R^4)_2)_n-$, $-(CH_2)_nNHC(=O)(CH_2)_n-$, $-(C(R^4)_2)_nNHC(=O)(C(R^4)_2)_n-$, $-(CH_2)_nNH((CH_2)_nO)_m(CH_2)_n-$$-(C(R^4)_2)_nNH((C(R^4)_2)_mO)_m(C(R^4)_2)_n-$, $-(O(CH_2)_n)_mNHC(=O)(CH_2)_n-$, or $-(O(C(R^4)_2)_n)_mNHC(=O)(C(R^4)_2)_n-$;

  each $X^2$ is independently selected from a bond, $R^8$,

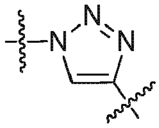 , 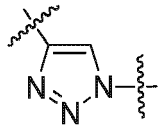 ,

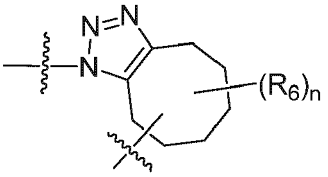 , 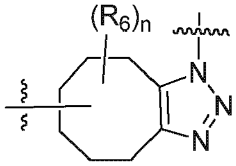 , 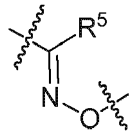 ,

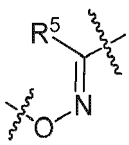 , 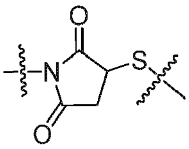 , 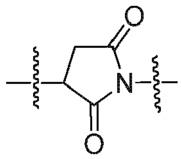 , 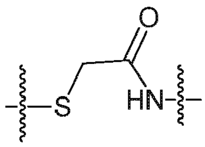 ,

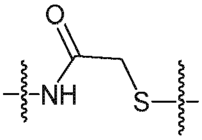 , 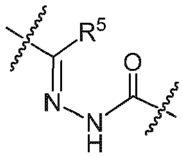 , 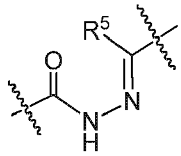 , 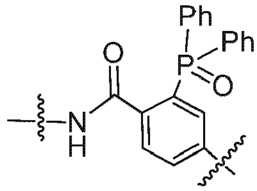 ,

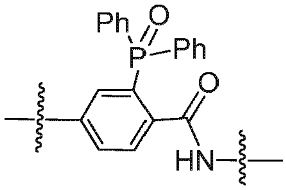 , 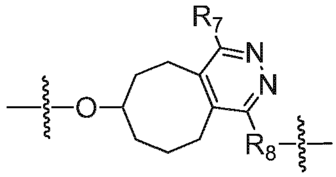 ,

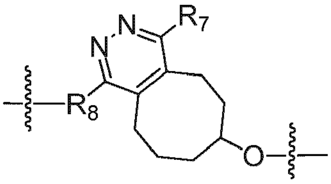 , 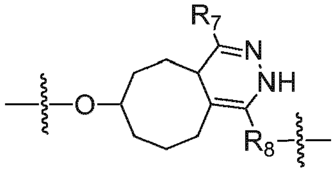 ,

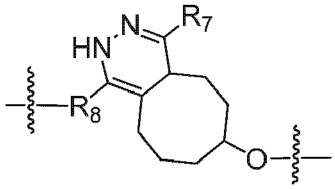 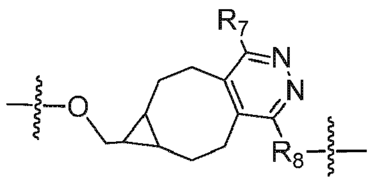 ,

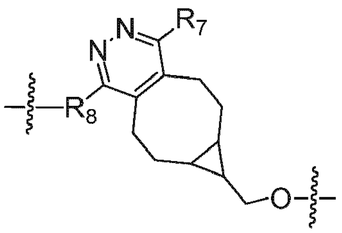 , 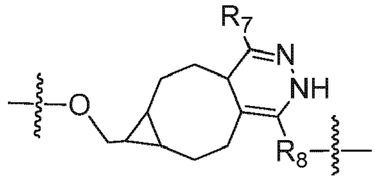 ,

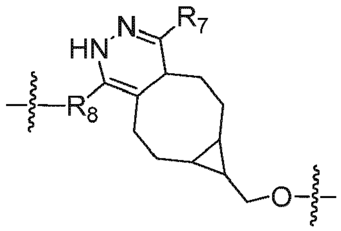 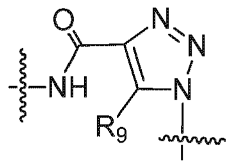 , 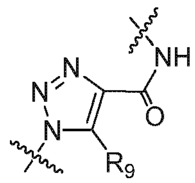 ,

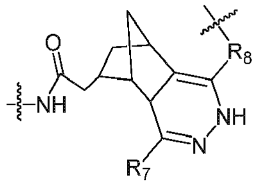 , 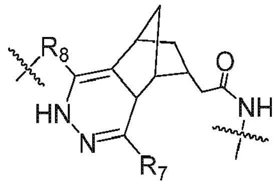 , 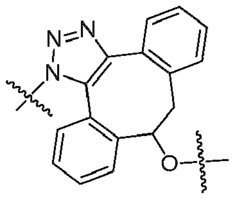 ,

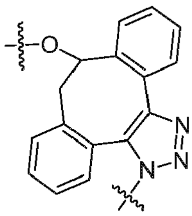 , 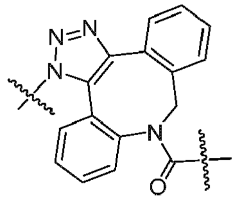 , 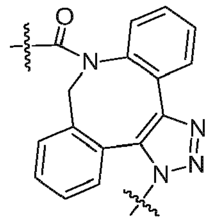

-S-, -Si(OH)$_2$O-,

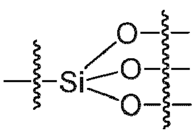

,

-CHR$^4$(CH$_2$)$_n$C(=O)NH-, -CHR$^4$(CH$_2$)$_n$NHC(=O)-, -C(=O)NH- and-NHC(=O)-;
each R$^4$ is independently selected from H, C$_{1-4}$alkyl, side chains of known amino acids,-C(=O)OH and -OH,
each R$^5$ is independently selected from H, C$_{1-4}$alkyl, phenyl or C$_{1-4}$alkyl substituted with 1 to 3-OH groups;
each R$^6$ is independently selected from H, fluoro, benzyloxy substituted with -C(=O)OH, benzyl substituted with -C(=O)OH, C$_{1-4}$alkoxy substituted with -C(=O)OH and C$_{1-4}$alkyl substituted with -C(=O)OH;
R$^7$ is independently selected from H, C$_{1-4}$alkyl, phenyl, pyrimidine and pyridine;
R$^8$ is independently selected from

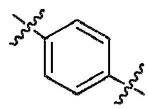 , 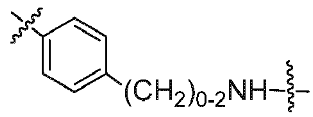 ,

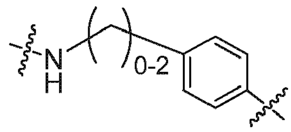 , 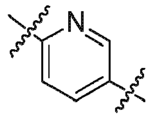 , 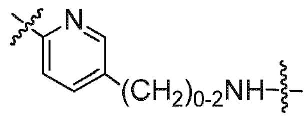 ,

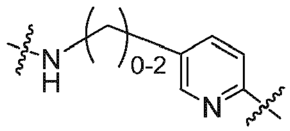 , 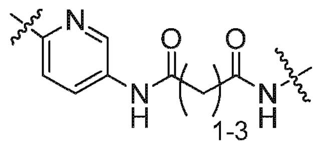 ,

and

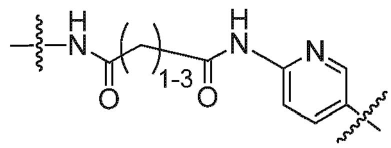 ,

$R^9$ is independently selected from H and $C_{1-6}$haloalkyl;
each n is independently selected from 1, 2, 3, 4, 5, 6, 7, 8 and 9, and
each m is independently selected from 1, 2, 3, 4, 5, 6, 7, 8 and 9.

In some embodiments, at least one of $L_1$, $L_2$, $L_3$, $L_4$, $L_5$ and $L_6$ is a stable, or non-cleavable, linker. In some embodiments, at least one of $L_1$, $L_2$, $L_3$, $L_4$, $L_5$ and $L_6$ is a cleavable linker, which may be chemically cleavable (hydrazones, disulfides) or enzymatically cleavable. In some embodiments, the enzymatically cleavable linker is one readily cleaved by a peptidase: The Val-Cit linker (valine-citrulline), a dipeptide of two known amino acids, is one such linker. In other embodiments, the enzymatically cleavable linker is one that is triggered by activity of a glucuronidase:

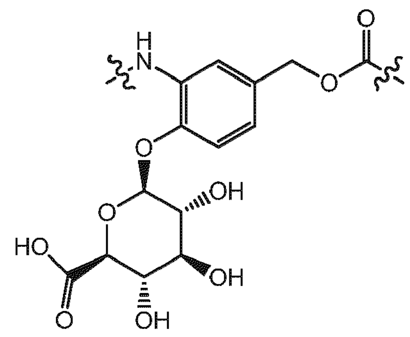

is an example of such a linker, which also comprises a self-immolative spacer that falls apart spontaneously under physiological conditions once glucuronidase cleaves the glycosidic linkage.

In some embodiments, the immunoconjugate of the present application comprises a modified cysteine residue of the formula IIA or IIB:

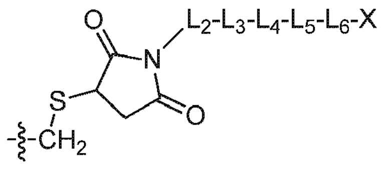

or

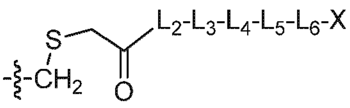

IIA

IIB

wherein $-CH_2-S-$ represents the side chain of Cys incorporated at one of the selected Cys substitution sites described herein, and $L_2$ - $L_6$ and X represent linking groups and payloads, respectively, as further described herein. In some embodiments of IIA, $L_2$ is a bond. In some embodiments of IIB, $L_2$ is NH or O. In some embodiments of both IIA and IIB, $L_3$ is selected from $(CH_2)_{1-10}$ and $(CH_2CH_2O)_{1-6}$. $L_4$, $L_5$ and $L_6$ are additional optional linkers selected from those described herein. In certain embodiments, $L_6$ can be a carbonyl (C=O) or a linker that comprises a self-immolative spacer.

In certain embodiments the Linker Unit (LU) is $-L_1-L_2-L_3-L_4-$, wherein:

$L_1$ is a bond, a non-enzymatically cleavable linker, a non-cleavable linker, an enzymatically cleavable linker, a photo-stable linker or a photo-cleavable linker;
$L_2$ is a bond, a non-enzymatically cleavable linker, a non-cleavable linker, an enzymatically cleavable linker, a photo-stable linker or a photo-cleavable linker;
$L_3$ is a bond, a non-enzymatically cleavable linker, a non-cleavable linker, an enzymatically cleavable linker, a photo-stable linker or a photo-cleavable linker, and
$L_4$ is a bond, a non-enzymatically cleavable linker, a non-cleavable linker, an enzymatically cleavable linker, a photo-stable linker, a photo-cleavable linker or a linker that comprises a self-immolative spacer.

In certain embodiments the Linker Unit (LU) is $-L_1-L_2-L_3-L_4-$, wherein

$L_1$ is a non-enzymatically cleavable linker, a non-cleavable linker, an enzymatically cleavable linker, a photo-stable linker or a photo-cleavable linker;
$L_2$ is a bond, a non-enzymatically cleavable linker, a non-cleavable linker, an enzymatically cleavable linker, a photo-stable linker or a photo-cleavable linker;
$L_3$ is a bond, a non-enzymatically cleavable linker, a non-cleavable linker, an enzymatically cleavable linker, a photo-stable linker or a photo-cleavable linker, and
$L_4$ is a bond, a non-enzymatically cleavable linker, a non-cleavable linker, an enzymatically cleavable linker, a photo-stable linker, a photo-cleavable linker or a linker that comprises a self-immolative spacer.

In some of the embodiments of LU at least one of $L_1$, $L_2$, $L_3$, $L_4$, $L_5$ and $L_6$ is a cleavable linker, and LU is considered cleavable. Similarly, in some of the embodiments of LU at least one of $L_1$, $L_2$, $L_3$, $L_4$, $L_5$ and $L_6$ is a non-cleavable linker. In certain of these embodiments, each linker of LU is non-cleavable, and LU is considered non-cleavable.

In some of the foregoing embodiments wherein LU is $-L_1-L_2-L_3-L_4-$, at least one of $L_1$, $L_2$, $L_3$ and $L_4$ is a linker selected from $-A_1-$, $-A_1X^2-$ and $-X^2-$; wherein:

$A_1$ is $-C(=O)NH-$, $-C(=O)NH(CH_2)_n-$, $-C(=O)NH(C(R^4)_2)_n-$, $-(O(CH_2)_n)_m-$, $-(O(C(R^4)_2)_n)_m-$ ,$-((CH_2)_nO)_m-$, $-((C(R^4)_2)_nO)_m-$, $-((CH_2)_nO)_m(CH_2)_n-$, $-(((C(R^4)_2)_nO)_mC(R^4)_2)_n-$,$-(CH_2)_nC(=O)NH-$, $-(C(R^4)_2)_nC(=O)NH-$, $-(CH_2)_nNHC(=O)-$, $-(C(R^4)_2)_nNHC(=O)-$,$-NHC(=O)(CH_2)_n-$, $-NHC(=O)(C(R^4)_2)_n-$, $-C(=O)NH(CH_2)_nS-$, $-C(=O)NH(C(R^4)_2)_nS-$,$-S(CH_2)_nC(=O)NH-$, $-S(C(R^4)_2)_nC(=O)NH-$, $-C(=O)NH(CH_2)_nNHC(=O)(CH_2)_n-$, $-C(=O)NH(C(R^4)_2)_nNHC(=O)(C(R^4)_2)_n-$, $-C(=O)(CH_2)_n-$, $-C(=O)(C(R^4)_2)_n-$, $-(CH_2)_nC(=O)-$, $-(C(R^4)_2)_nC(=O)-$, $-(CH_2)_n(O(CH_2)_n)_mNHC(=O)(CH_2)_n-$,$-(C(R^4)_2)_n(O(C(R^4)_2)_n)_mNHC(=O)(C(R^4)_2)_n-$, $-(CH_2)_nNHC(=O)(CH_2)_n-$, $-(C(R^4)_2)_nNHC(=O)(C(R^4)_2)_n-$, $-(CH_2)_nNH((CH_2)_nO)_m(CH_2)_n-$, $-(C(R^4)_2)_nNH((C(R^4)_2)_nO)_m(C(R^4)_2)_n-$, $-(O(CH_2)_n)_mNHC(=O)(CH_2)_n-$, or $-(O(C(R^4)_2)_n)_mNHC(=O)(C(R^4)_2)_n-$;
each $X^2$ is independently selected from a bond, $R^8$,

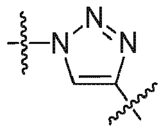 , 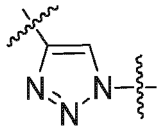 ,

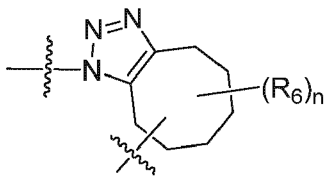 , 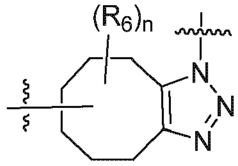 , 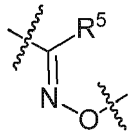 ,

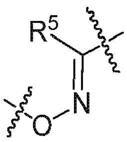 , 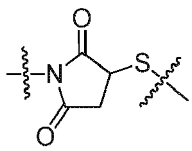 , 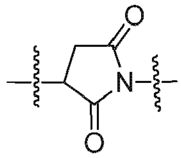 , 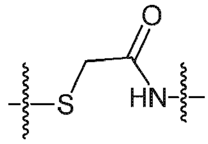 ,

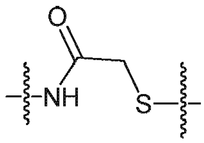 , 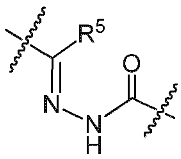 , 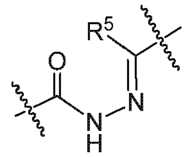 , 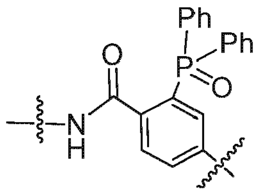 ,

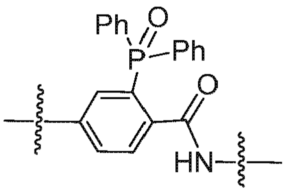 , 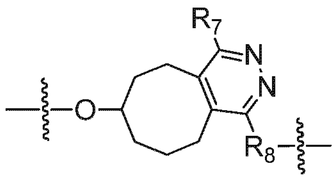 ,

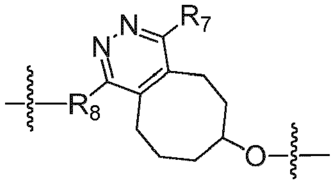 , 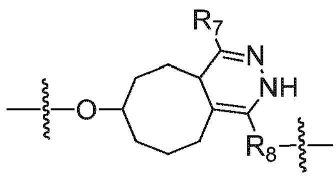 ,

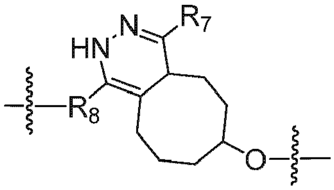 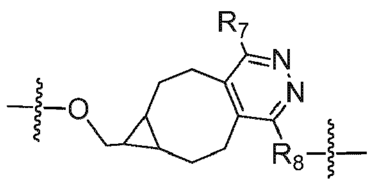 ,

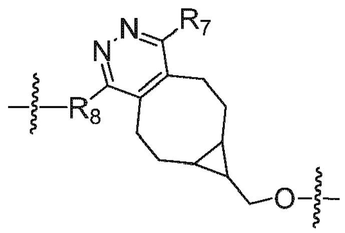 , 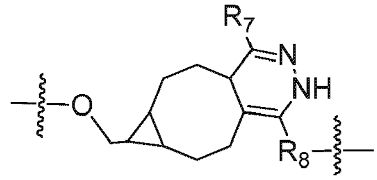 ,

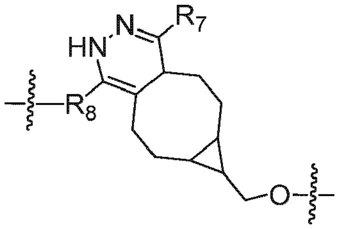 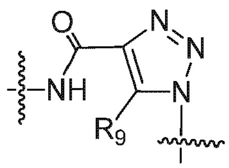 , 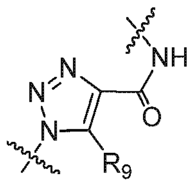 ,

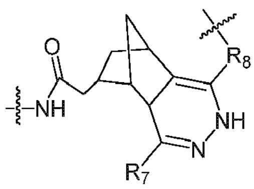 , 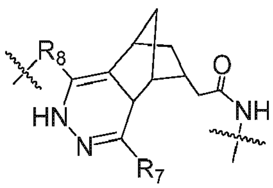 , 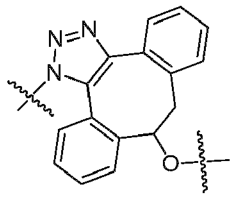 ,

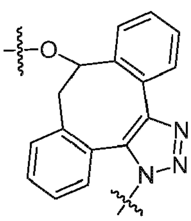 , 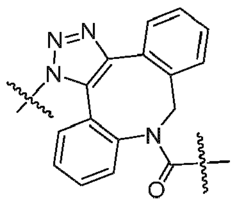 , 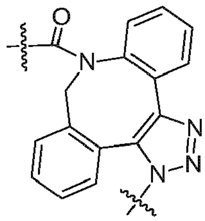

-S-, -Si(OH)$_2$O-,

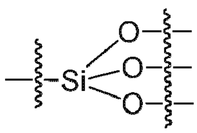 ,

-CHR$^4$(CH$_2$)$_n$C(=O)NH-, -CHR$^4$(CH$_2$)$_n$NHC(=O)-, -C(=O)NH- and-NHC(=O)-;
each R$^4$ is independently selected from H, C$_{1-4}$alkyl, side chains of known amino acids,-C(=O)OH and -OH,
each R$^5$ is independently selected from H, C$_{1-4}$alkyl, phenyl or C$_{1-4}$alkyl substituted with 1 to 3-OH groups;
each R$^6$ is independently selected from H, fluoro, benzyloxy substituted with -C(=O)OH, benzyl substituted with -C(=O)OH, C$_{1-4}$alkoxy substituted with -C(=O)OH and C$_{1-4}$alkyl substituted with -C(=O)OH;
R$^7$ is independently selected from H, C$_{1-4}$alkyl, phenyl, pyrimidine and pyridine;
R$^8$ is independently selected

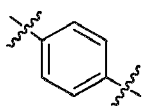 , 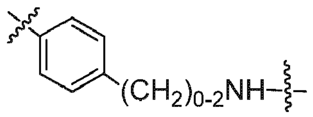 ,

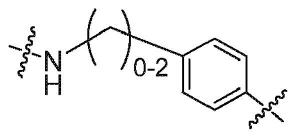 , 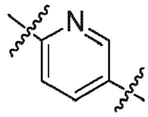 , 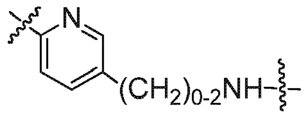 ,

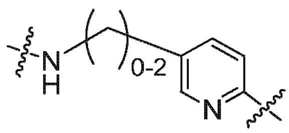 , 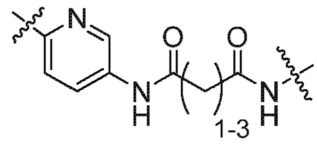 ,

and

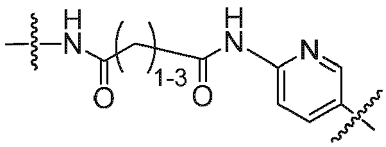 ;

$R^9$ is independently selected from H and $C_{1-6}$haloalkyl;
each n is independently selected from 1, 2, 3, 4, 5, 6, 7, 8 and 9, and
each m is independently selected from 1, 2, 3, 4, 5, 6, 7, 8 and 9.

In these embodiments, the other linkers of LU are independently selected from a bond, $-A^1-$ ,$-A_1X^2-$ , $-X^2-$ , a non-enzymatically cleavable linker, a non-cleavable linker, an enzymatically cleavable linker, a photo-stable linker, a photo-cleavable linker and a linker that comprises a self-immolative spacer.
In certain embodiments the Linker Unit (LU) is $-L_1-L_2-L_3-L_4-$, wherein

$L_1$ is a bond, $-A_1-$, $-A_1X^2-$ or $-X^2-$; where:

$A_1$ is $-C(=O)NH-$, $-C(=O)NH(CH_2)_n-$, $-C(=O)NH(C(R^4)_2)_n-$, $-(O(CH_2)_n)_m-$, $-(O(C(R^4)_2)_n)_m-$,$-((CH_2)_nO)_m-$, $-((C(R^4)_2)_nO)_m-$, $-((CH_2)_nO)_m(CH_2)_n-$, $-(((C(R^4)_2)_nO)_mC(R^4)_2)_n-$,-"$(CH_2)_nC(=O)NH-$, $-(C(R^4)_2)_nC(=O)NH-$, $-(CH_2)_nNHC(=O)-$, $-(C(R^4)_2)_nNHC(=O)-$,$-NHC(=O)(CH_2)_n-$, $-NHC(=O)(C(R^4)_2)_n-$, $-C(=O)NH(CH_2)_nS-$, $-C(=O)NH(C(R^4)_2)_nS-$,$-S(CH_2)_nC(=O)NH-$, $-S(C(R^4)_2)_nC(=O)NH-$, $-C(=O)NH(CH_2)_nNHC(=O)(CH_2)_n-$,$-C(=O)NH(C(R^4)_2)_nNHC(=O)(C(R^4)_2)_n-$, $-C(=O)(CH_2)_n-$, $-C(=O)(C(R^4)_2)_n-$, $-(CH_2)_nC(=O)-$,$-(C(R^4)_2)_nC(=O)-$, $-(CH_2)_n(O(CH_2)_n)_mNHC(=O)(CH_2)_n-$,$-(C(R^4)_2)_n(O(C(R^4)_2)_n)_mNHC(=O)(C(R^4)_2)_n-$, $-(CH_2)_nNHC(=O)(CH_2)_n-$,$-(C(R^4)_2)_nNHC(=O)(C(R^4)_2)_n-$, $-(CH_2)_nNH((CH_2)_nO)m(CH_2)_n-$,$-(C(R^4)_2)_nNH((C(R^4)_2)_nO)_m(C(R^4)_2)_n-$, $-(O(CH_2)_n)_mNHC(=O)(CH_2)_n-$, or$-(O(C(R^4)_2)_n)_mNHC(=O)(C(R^4)_2)_n-$;
each $X^2$ is independently selected from a bond, $R^8$

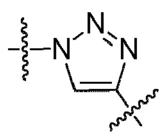 , 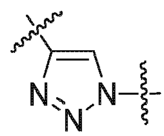 ,

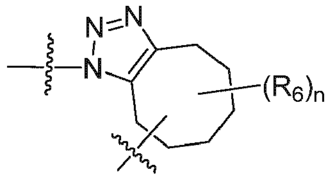 , 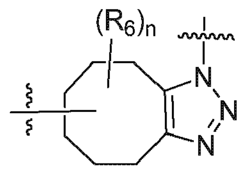 , 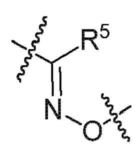 ,

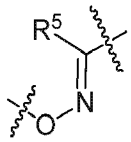 , 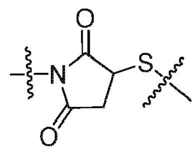 , 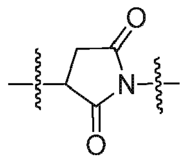 , 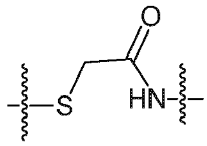 ,

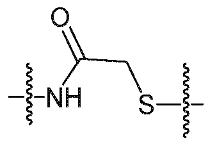 , 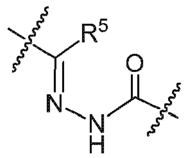 , 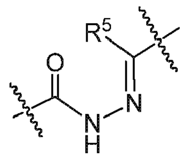 , 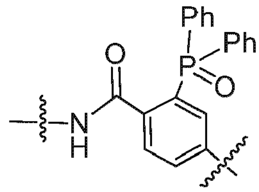 ,

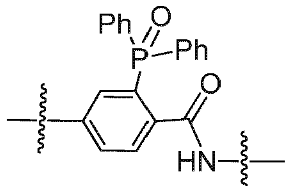 , 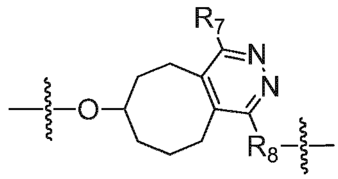 ,

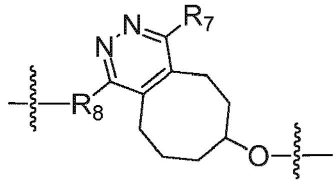 , 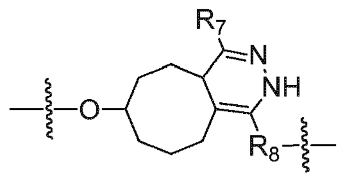 ,

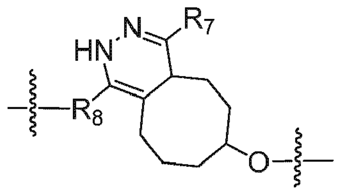 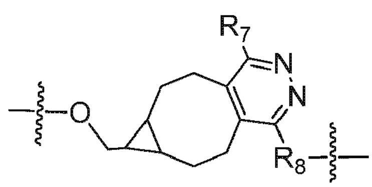 ,

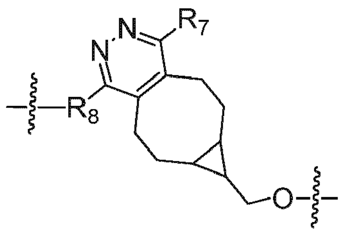

,

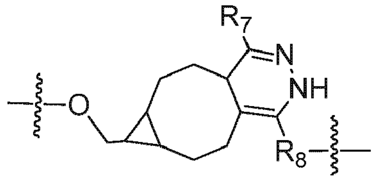

,

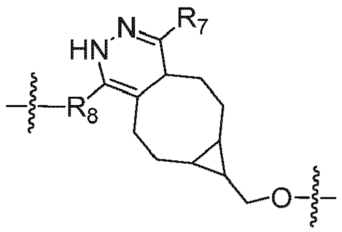

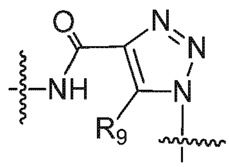

,

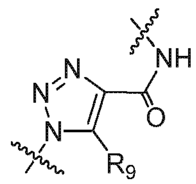

,

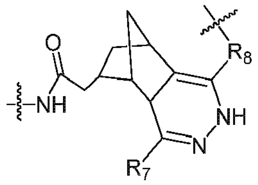

,

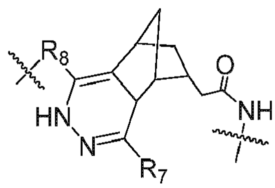

,

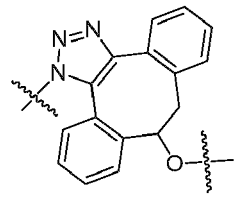

,

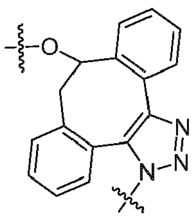

,

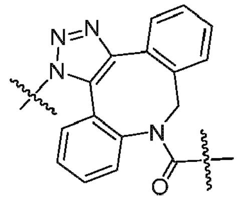

,

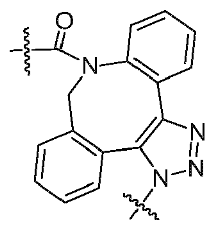

-S-, -Si(OH)$_2$O-,

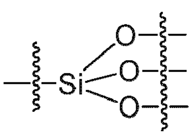

,

-CHR$^4$(CH$_2$)$_n$C(=O)NH-, -CHR$^4$(CH$_2$)$_n$NHC(=O)-, -C(=O)NH- and-NHC(=O)-;
each R$^4$ is independently selected from H, C$_{1-4}$alkyl, side chains of known amino acids,-C(=O)OH and -OH,
each R$^5$ is independently selected from H, C$_{1-4}$alkyl, phenyl or C$_{1-4}$alkyl substituted with 1 to 3-OH groups;
each R$^6$ is independently selected from H, fluoro, benzyloxy substituted with -C(=O)OH, benzyl substituted with -C(=O)OH, C$_{1-4}$alkoxy substituted with -C(=O)OH and C$_{1-4}$alkyl substituted with -C(=O)OH;
R$^7$ is independently selected from H, C$_{1-4}$alkyl, phenyl, pyrimidine and pyridine;
R$^8$ is independently selected from

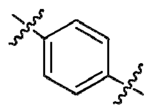 , 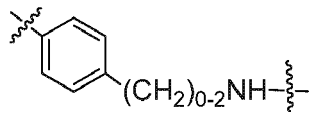 ,

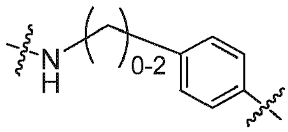 , 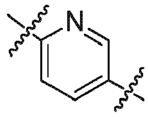 , 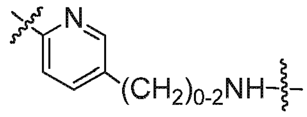 ,

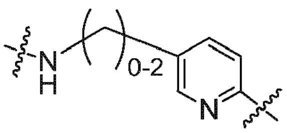 , 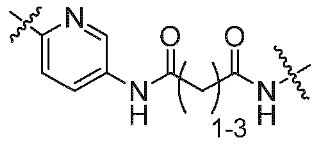 ,

and

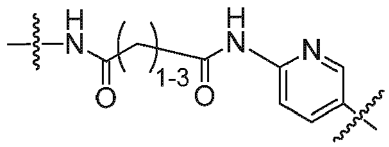 ;

$R^9$ is independently selected from H and $C_{1-6}$haloalkyl;
each n is independently selected from 1, 2, 3, 4, 5, 6, 7, 8 and 9, and
each m is independently selected from 1, 2, 3, 4, 5, 6, 7, 8 and 9;

$L_2$ is a bond, a non-enzymatically cleavable linker, a non-cleavable linker, an enzymatically cleavable linker, a photo-stable linker or a photo-cleavable linker;
$L_3$ is a bond, a non-enzymatically cleavable linker, a non-cleavable linker, an enzymatically cleavable linker, a photo-stable linker or a photo-cleavable linker, and
$L_4$ is a bond, a non-enzymatically cleavable linker, a non-cleavable linker, an enzymatically cleavable linker, a photo-stable linker, a photo-cleavable linker or a linker that comprises a self-immolative spacer.

In certain embodiments, $L_1$ is $C(=O)-CH_2CH_2-NH-C(=O)-CH_2CH_2-S-$ , so LU is $-C(=O)-CH_2CH_2-NH-C(=O)-CH_2CH_2-S-L_2-L_3-L_4-$.

In certain embodiments the Linker Unit (LU) is $-L_1-L_2-L_3-L_4-$, wherein

$L_1$ is a bond, $-A_1-$, $-A_1X^2-$ or $-X^2-$; where:

$A_1$ is $-C(=O)NH-$, $-C(=O)NH(CH_2)_n-$, $-(O(CH_2)_n)_m-$, $-((CH_2)_nO)_m-$, $-((CH_2)_nO)_m(CH_2)_n-$, $-(CH_2)_nC(=O)NH-$, $-(CH_2)_nNHC(=O)-$, $-NHC(=O)(CH_2)_n-$, $-C(=O)NH(CH_2)_nS-$, $-S(CH_2)_nC(=O)NH-$, $-C(=O)NH(CH_2)_nNHC(=O)(CH_2)_n-$, $-C(=O)(CH_2)_n-$, $-(CH_2)_nC(=O)-$, $-(CH_2)_n(O(CH_2)_n)_mNHC(=O)(CH_2)_n-$, $-(CH_2)_nNHC(=O)(CH_2)_n-$, $-(CH_2)_nNH((CH_2)_nO)_m(CH_2)_n-$, or $-(O(CH_2)_n)_mNHC(=O)(CH_2)_n-$;
each $X^2$ is independently selected from a bond, $R^8$

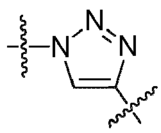 , 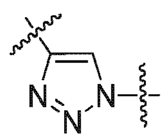 ,

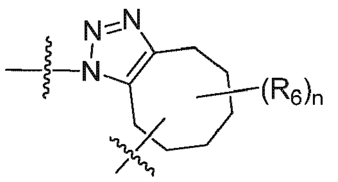 ,

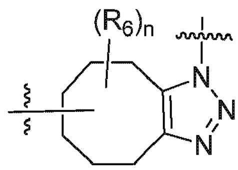 ,

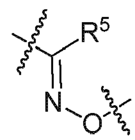 ,

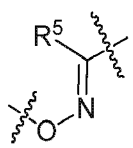 ,

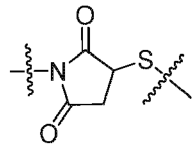 ,

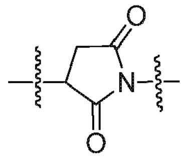 ,

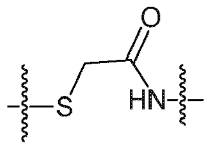 ,

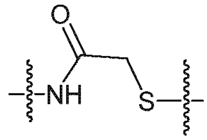 ,

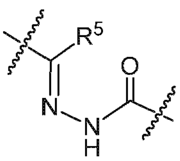 ,

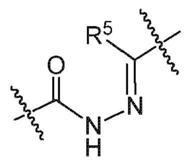 ,

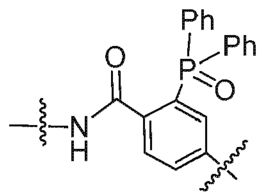 ,

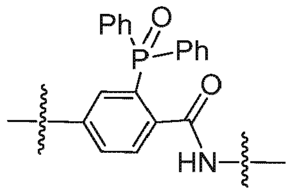 ,

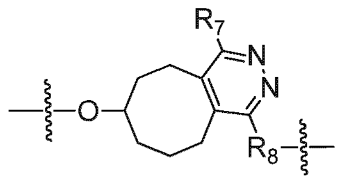 ,

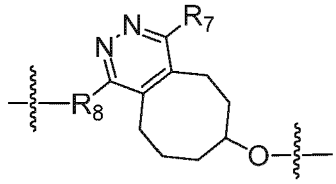 ,

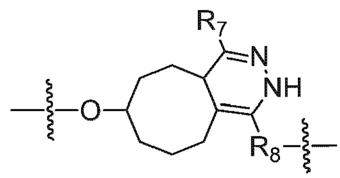 ,

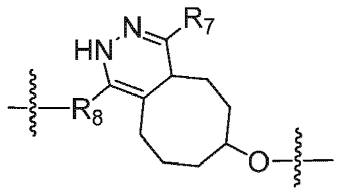

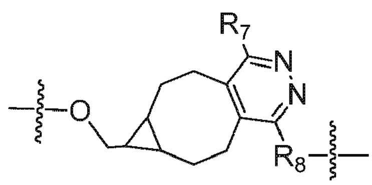 ,

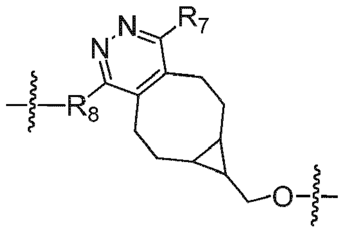,

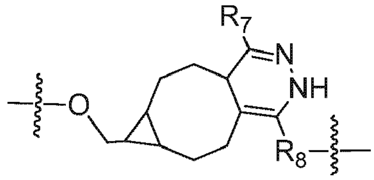,

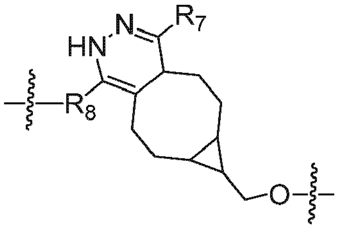

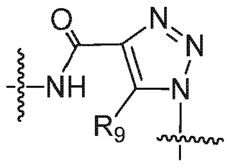,

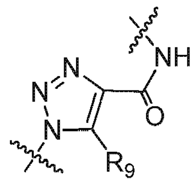,

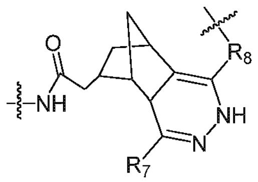,

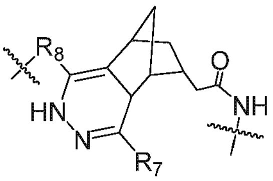,

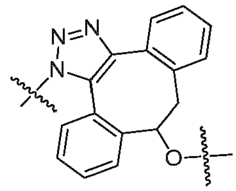,

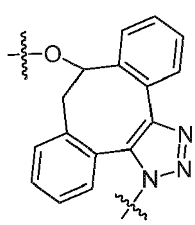,

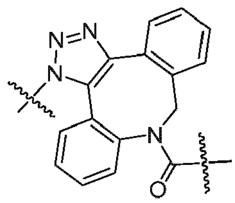,

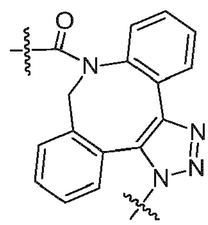

-S-, $-Si(OH)_2O-$,

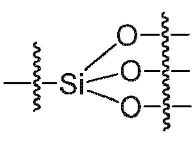,

$-CHR^4(CH_2)_nC(=O)NH-$, $-CHR^4(CH_2)_nNHC(=O)-$, -C(=O)NH- and-NHC(=O)-;
each $R^4$ is independently selected from H, $C_{1-4}$alkyl, side chains of known amino acids,-C(=O)OH and -OH,
each $R^5$ is independently selected from H, $C_{1-4}$alkyl, phenyl or $C_{1-4}$alkyl substituted with 1 to 3-OH groups;
each $R^6$ is independently selected from H, fluoro, benzyloxy substituted with -C(=O)OH, benzyl substituted with -C(=O)OH, $C_{1-4}$alkoxy substituted with -C(=O)OH and $C_{1-4}$alkyl substituted with -C(=O)OH;
$R^7$ is independently selected from H, $C_{1-4}$alkyl, phenyl, pyrimidine and pyridine;
$R^8$ is independently selected

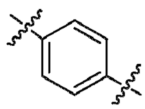,
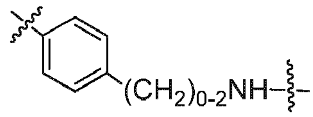,

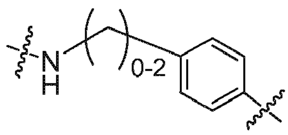,
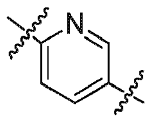,
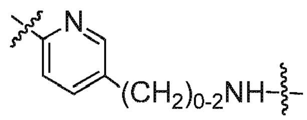,

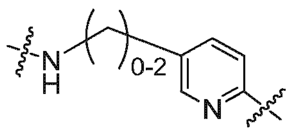,
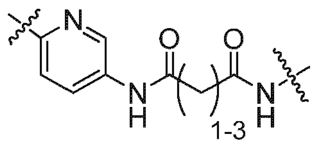,

and

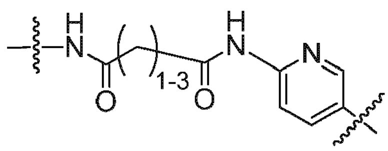;

$R^9$ is independently selected from H and $C_{1-6}$haloalkyl;
each n is independently selected from 1, 2, 3, 4, 5, 6, 7, 8 and 9, and
each m is independently selected from 1, 2, 3, 4, 5, 6, 7, 8 and 9;

$L_2$ is a bond, a non-enzymatically cleavable linker, a non-cleavable linker, an enzymatically cleavable linker, a photo-stable linker or a photo-cleavable linker;
$L_3$ is a bond, a non-enzymatically cleavable linker, a non-cleavable linker, an enzymatically cleavable linker, a photo-stable linker or a photo-cleavable linker;
$L_4$ is a bond, a non-enzymatically cleavable linker, a non-cleavable linker, an enzymatically cleavable linker, a photo-stable linker, a photo-cleavable linker or a linker that comprises a self-immolative spacer.

In certain embodiments the Linker Unit (LU) is $-L_1-L_2-L_3-L_4-$, wherein

$L_1$ is a bond, $-A_1-$, $-A_1X^2-$ or $-X^2-$; where:

$A_1$ is $-C(=O)NH-$, $-C(=O)NH(CH_2)_n-$, $-C(=O)NH(CH_2)_nS-$, $-(O(CH_2)_n)_m-$, $-((CH_2)_nO)_m(CH_2)_n-$,$-NHC(=O)(CH_2)_n-$, $-C(=O)NH(CH_2)_nNHC(=O)(CH_2)_n-$, $-(CH_2)_nNH((CH_2)_nO)_m(CH_2)_n-$ or$-(O(CH_2)_n)_mNHC(=O)(CH_2)_n-$;
each $X^2$ is independently selected from a bond, $R^8$

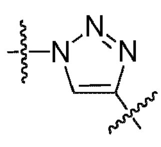,
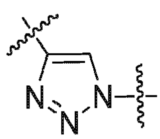,

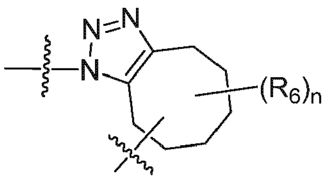
,
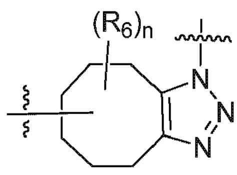
,
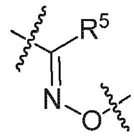
,
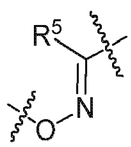
,
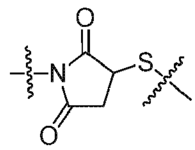
,
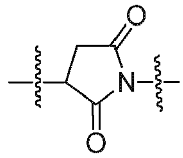
,
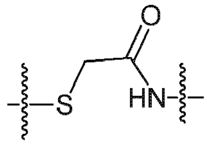
,
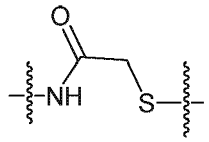
,
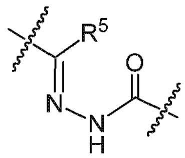
,
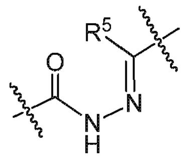
,
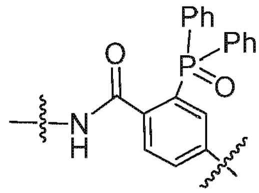
,
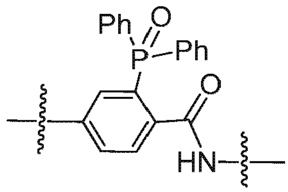
,
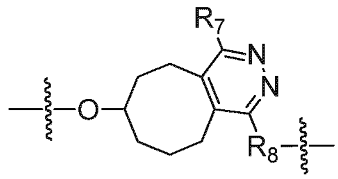
,
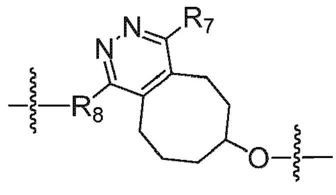
,
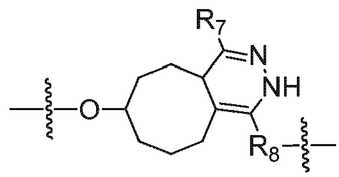
,
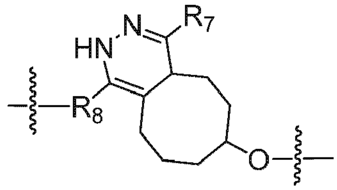
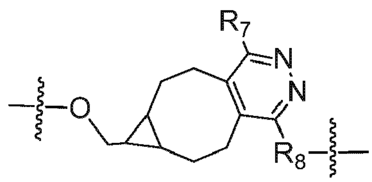
,

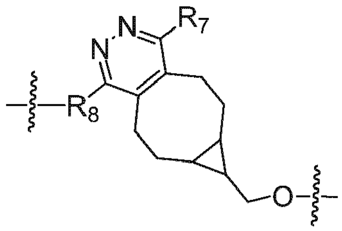 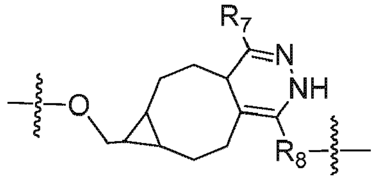

,　　　　　　　　　　,

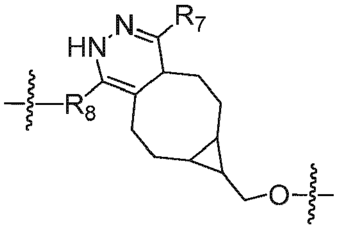 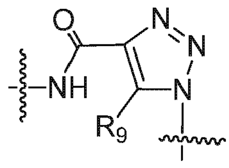 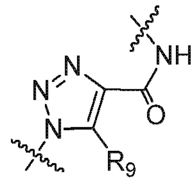

,　　　　　　　　　　,

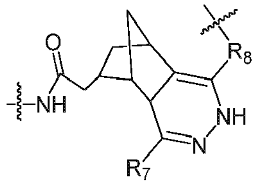 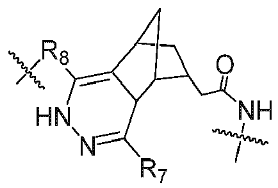 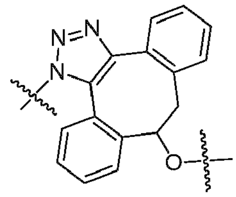

,　　　　　　　　　　,　　　　　　　　　　,

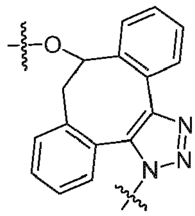 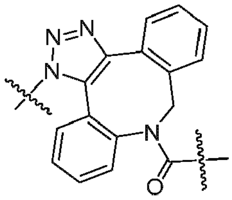 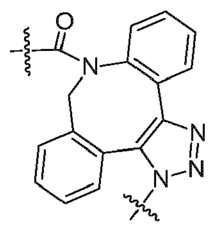

,　　　　　　　　　　,

-S-, -$Si(OH)_2O$-,

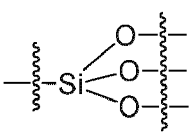

,

-$CHR^4(CH_2)_nC(=O)NH$-, -$CHR^4(CH_2)_nNHC(=O)$-, -C(=O)NH- and-NHC(=O)-;
each $R^4$ is independently selected from H, $C_{1-4}$alkyl, side chains of known amino acids,-C(=O)OH and -OH,
each $R^5$ is independently selected from H, $C_{1-4}$alkyl, phenyl or $C_{1-4}$alkyl substituted with 1 to 3-OH groups;
each $R^6$ is independently selected from H, fluoro, benzyloxy substituted with -C(=O)OH, benzyl substituted with -C(=O)OH, $C_{1-4}$alkoxy substituted with -C(=O)OH and $C_{1-4}$alkyl substituted with -C(=O)OH;
$R^7$ is independently selected from H, $C_{1-4}$alkyl, phenyl, pyrimidine and pyridine;
$R^8$ is independently selected

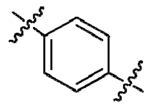 , 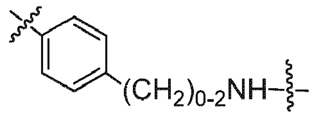 ,

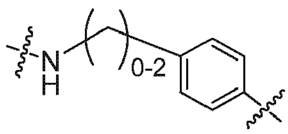 , 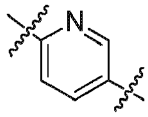 , 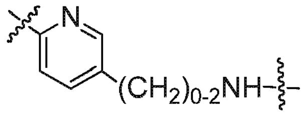 ,

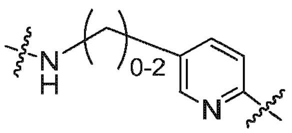 , 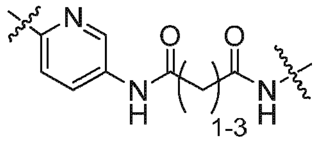 ,

and

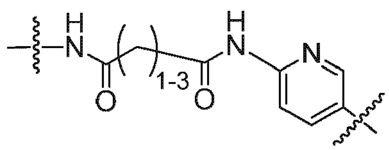 ;

$R^9$ is independently selected from H and $C_{1-6}$haloalkyl;
each n is independently selected from 1, 2, 3, 4, 5, 6, 7, 8 and 9, and
each m is independently selected from 1, 2, 3, 4, 5, 6, 7, 8 and 9;

$L_2$ is a bond, a non-enzymatically cleavable linker, a non-cleavable linker, an enzymatically cleavable linker, a photo-stable linker or a photo-cleavable linker;
$L_3$ is a bond, a non-enzymatically cleavable linker, a non-cleavable linker, an enzymatically cleavable linker, a photo-stable linker or a photo-cleavable linker, and
$L_4$ is a bond, a non-enzymatically cleavable linker, a non-cleavable linker, an enzymatically cleavable linker, a photo-stable linker, a photo-cleavable linker or a linker that comprises a self-immolative spacer.

In certain embodiments the Linker Unit (LU) is $-L_1-L_2-L_3-L_4-$, wherein

$L_1$ is a bond, $-A_1-$, $-A_1X^2-$ or $-X^2-$; where:

$A_1$ is $-C(=O)NH-$, $-C(=O)NH(CH_2)_n-$, $-C(=O)NH(CH_2)_nS-$, $-(O(CH_2)_n)_m-$, $-((CH_2)_nO)_m(CH_2)_n-$,$-NHC(=O)(CH_2)_n-$, $-C(=O)NH(CH_2)_nNHC(=O)(CH_2)_n-$, $-(CH_2)_nNH((CH_2)_nO)_m(CH_2)_n-$ or$-(O(CH_2)_n)_mNHC(=O)(CH_2)_n-$;
each $X^2$ is independently selected from a bond, $R^8$

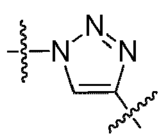 , 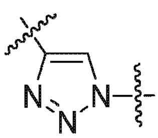 ,

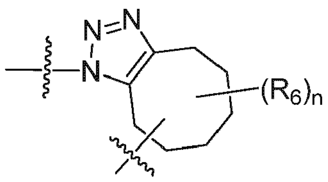 , 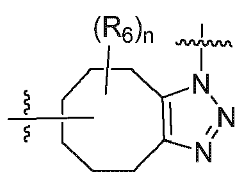 , 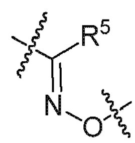 ,

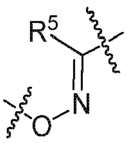 , 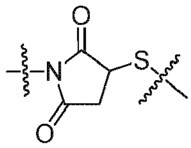 , 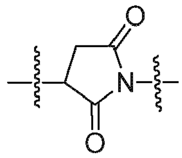 , 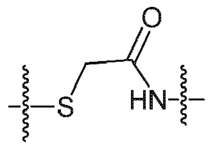 ,

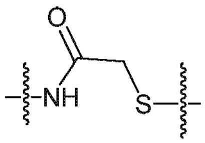 , 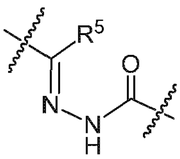 , 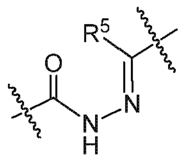 , 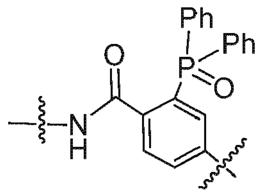 ,

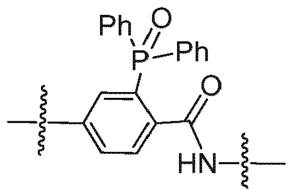 , 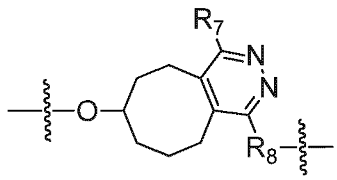 ,

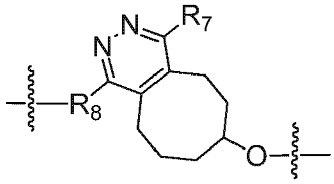 , 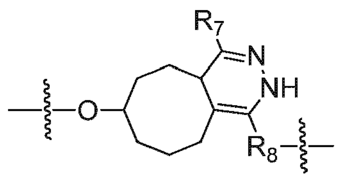 ,

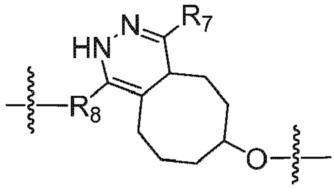 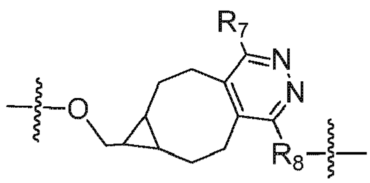 ,

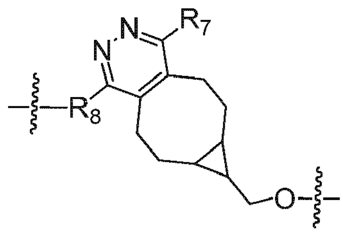

,

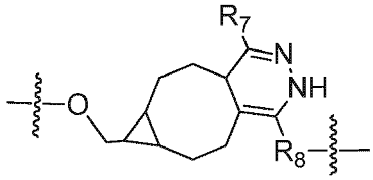

,

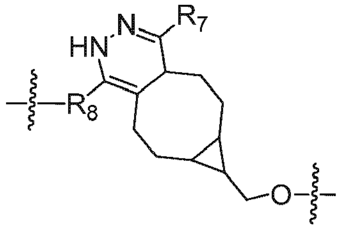

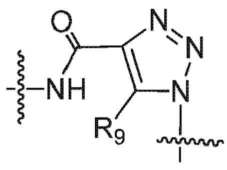

,

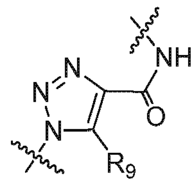

,

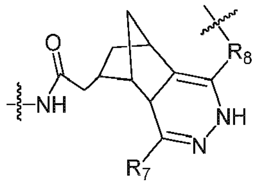

,

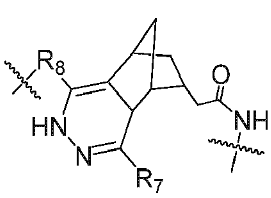

,

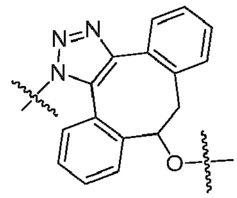

,

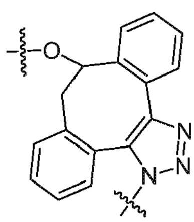

,

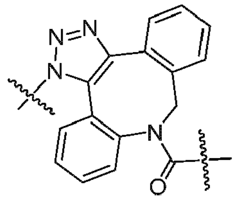

,

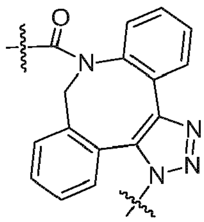

-S-, -Si$(OH)_2$O-,

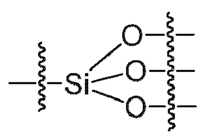

,

-CHR$^4$$(CH_2)_n$C(=O)NH-, -CHR$^4$$(CH_2)_n$NHC(=O)-, -C(=O)NH- and-NHC(=O)-;

each $R^4$ is independently selected from H, $C_{1-4}$alkyl, side chains of known amino acids,-C(=O)OH and -OH,

each $R^5$ is independently selected from H, $C_{1-4}$alkyl, phenyl or $C_{1-4}$alkyl substituted with 1 to 3 - OH groups;

each $R^6$ is independently selected from H, fluoro, benzyloxy substituted with -C(=O)OH, benzyl substituted with -C(=O)OH, $C_{1-4}$alkoxy substituted with -C(=O)OH and $C_{1-4}$alkyl substituted with -C(=O)OH;

$R^7$ is independently selected from H, $C_{1-4}$alkyl, phenyl, pyrimidine and pyridine;

$R^8$ is independently selected from

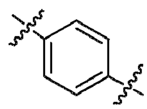, 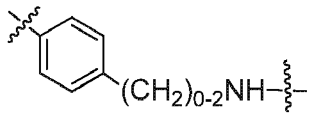,

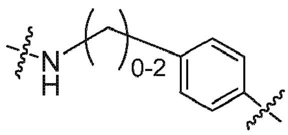, 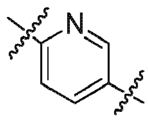, 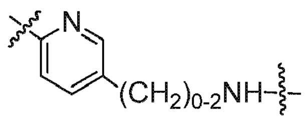,

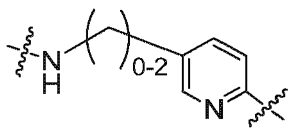, 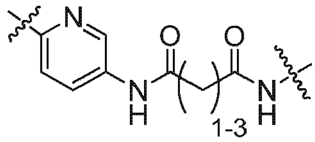,

and

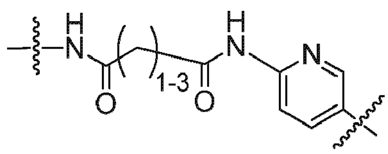;

$R^9$ is independently selected from H and $C_{1-6}$haloalkyl;
each n is independently selected from 1, 2, 3, 4, 5, 6, 7, 8 and 9, and
each m is independently selected from 1, 2, 3, 4, 5, 6, 7, 8 and 9;

$L_2$ is a bond, a non-enzymatically cleavable linker or a non-cleavable linker;
$L_3$ is a bond, a non-enzymatically cleavable linker or a non-cleavable linker;
$L_4$ is a bond, an enzymatically cleavable linker or a linker that comprises a self-immolative spacer.

In certain embodiments the Linker Unit (LU) is $-L_1-L_2-L_3-L_4-$, wherein

$L_1$ is a bond, $-A_1-$, $-A_1X^2-$ or $-X^2-$;
$L_2$ is a bond, $-A_2-$, or $-A_2X^2-$;
$L_3$ is a bond, $-A_3-$, or $-A_3X^2-$;
$L_4$ is a bond, $-A_4-$, $-A_4X^2-$,

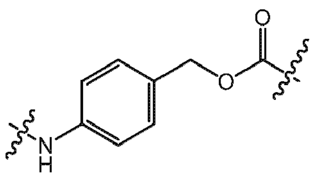, 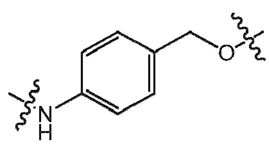,

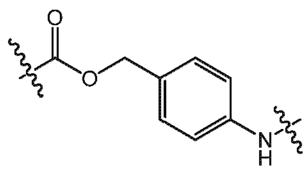, 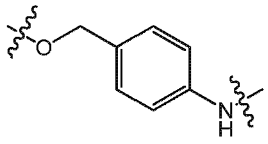, 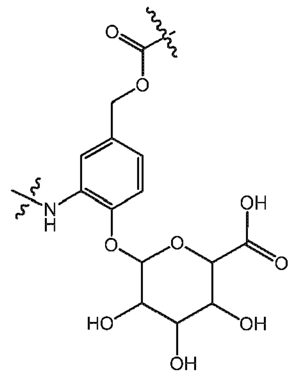,

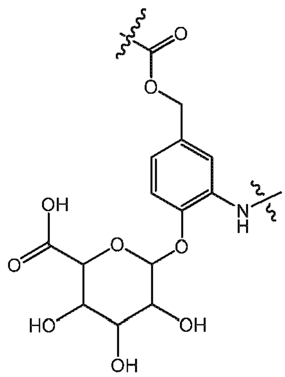, 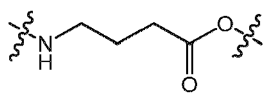, 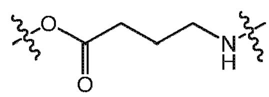,

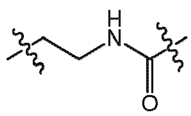, 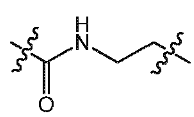, 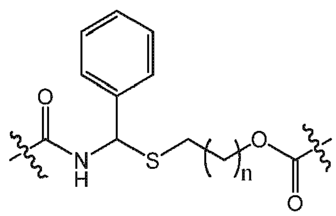,

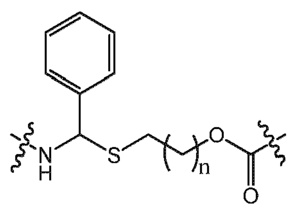, 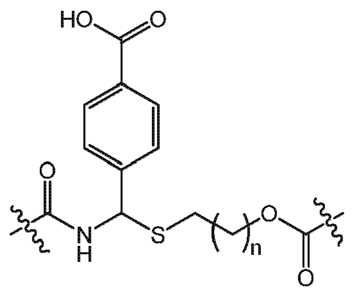, or 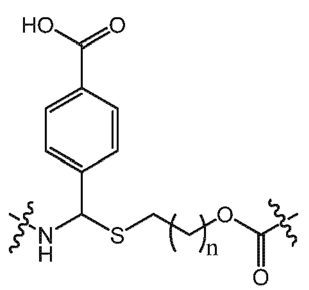;

$A_1$ is $-C(=O)NH-$, $-NHC(=O)-$, $-C(=O)NH(CH_2)_n-$, $-C(=O)NH(C(R^4)_2)_n-$, $-(O(CH_2)_n)_m-$, $-(O(C(R^4)_2)_n)_m-$,$-((CH_2)_nO)_m-$, $-((C(R^4)_2)_nO)_m-$, $-((CH_2)_nO)_m(CH_2)_n-$, $-(((C(R^4)_2)_nO)_mC(R^4)_2)_n$ -, $-(CH_2)_nC(=O)NH-$,$-(C(R^4)_2)_nC(=O)NH-$, $-(CH_2)_nNHC(=O)-$, $-(C(R^4)_2)_nNHC(=O)-$, $-NHC(=O)(CH_2)_n-$,$-NHC(=O)(C(R^4)_2)_n-$, $-C(=O)NH(CH_2)_nS-$, $-C(=O)NH(C(R^4)_2)_nS-$, $-S(CH_2)_nC(=O)NH-$,$-S(C(R^4)_2)_nC(=O)NH-$, $-C(=O)NH(CH_2)_nNHC(=O)(CH_2)_n-$, $-C(=O)NH(C(R^4)_2)_nNHC(=O)(C(R^4)_2)_n-$,$-C(=O)(CH_2)_n-$, $-C(=O)(C(R^4)_2)_n-$, $-(CH_2)_nC(=O)-$, $-(C(R^4)_2)_nC(=O)-$,$-(CH_2)_n(O(CH_2)_n)_mNHC(=O)(CH_2)_n-$, $-(C(R^4)_2)_n(O(C(R^4)_2)_n)_mN$-HC(=O)$(C(R^4)_2)_n-$,$-(CH_2)_nNHC(=O)(CH_2)_n-$, $-(C(R^4)_2)_nNHC(=O)(C(R^4)_2)_n-$, $-(CH_2)_nNH((CH_2)_nO)m(CH_2)_n-$,$-(C(R^4)_2)_nNH((C(R^4)_2)_nO)_m(C(R^4)_2)_n-$, $-(O(CH_2)_n)_mNHC(=O)(CH_2)_n-$, or$-(O(C(R^4)_2)_n)_mNHC(=O)(C(R^4)_2)_n-$;

$A_2$ is $-C(=O)NH-$, $-C(=O)NH(CH_2)_n-$, $-C(=O)NH(C(R^4)_2)_n-$, $-(O(CH_2)_n)_m-$, $-(O(C(R^4)_2)_n)_m-$, $-((CH_2)_nO)_m-$, $-((C(R^4)_2)_nO)_m-$, $-((CH_2)_nO)_m(CH_2)_n-$, $-((C(R^4)_2)_nO)_mC(R^4)_2)_n-$, $-(CH_2)_nC(=O)NH-$, $-(C(R^4)_2)_nC(=O)NR^4-$, $-(CH_2)_nNHC(=O)-$, $-(C(R^4)_2)_nNHC(=O)-$, $-NHC(=O)(CH_2)_n-$, $-NHC(=O)(C(R^4)_2)_n-$, $-C(=O)NH(CH_2)_nS-$, $-C(=O)NH(C(R^4)_2)_nS-$, $-S(CH_2)_nC(=O)NH-$, $-S(C(R^4)_2)_nC(=O)NH-$, $-(CH_2)_nS-$, $-(C(R^4)_2)_nS-$, $-S(CH_2)_n-$, $-S(C(R^4)_2)_n-$, $-(CH_2)_nNH-$, $-(C(R^4)_2)_nNH-$, $-C(=O)NH(CH_2)_nNHC(=O)(CH_2)_n-$, $-C(=O)NH(C(R^4)_2)_nNHC(=O)(C(R^4)_2)_n-$, $-C(=O)(CH_2)_n-$, $-C(=O)(C(R^4)_2)_n-$, $-(CH_2)_nC(=O)-$, $-(C(R^4)_2)_nC(=O)-$, $-(CH_2)_n(O(CH_2)_n)_mNHC(=O)(CH_2)_n-$, $-(C(R^4)_2)_n(O(C(R^4)_2)_n)_mNHC(=O)(C(R^4)_2)_n-$, $-(CH_2)_n(O(CH_2)_n)_mOC(=O)NH(CH_2)_n-$, $-(C(R^4)_2)_n(O(C(R^4)_2)_n)_mOC(=O)NH(C(R^4)_2)_n-$, $-(CH_2)_nNHC(=O)(CH_2)_n-$, $-(C(R^4)_2)_nNHC(=O)(C(R^4)_2)_n-$, $-(CH_2)_nNH((CH_2)_nO)_m(CH_2)_n-$, $-(C(R^4)_2)_nNH((C(R^4)_2)_nO)_m(C(R^4)_2)_n-$, $-(O(CH_2)_n)_mNHC(=O)(CH_2)_n-$, $-(O(C(R^4)_2)_n)_mNHC(=O)(C(R^4)_2)_n-$,

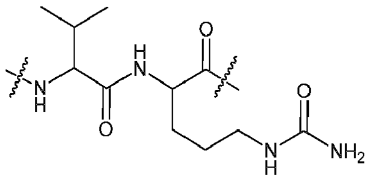

or;

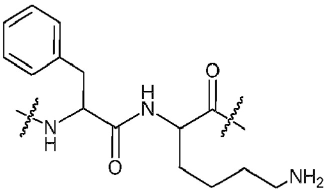

$A_3$ is $-C(=O)NH-$, $-C(=O)NH(CH_2)_N-$, $-C(=O)NH(C(R^4)_2)_n-$, $-(O(CH_2)_n)_m-$, $-(O(C(R^4)_2)_n)_m-$, $-((CH_2)_nO)_m-$, $-((C(R^4)_2)_nO)_m-$, $-((CH_2)_nO)_m(CH_2)_n-$, $-(((C(R^4)_2)_nO)_mC(R^4)_2)_n-$, $-(CH_2)_nC(=O)NH-$, $-(C(R^4)_2)_nC(=O)NH-$, $-(CH_2)_nNHC(=O)-$, $-(C(R^4)_2)_nNHC(=O)-$, $-NHC(=O)(CH_2)_n-$, $-NHC(=O)(C(R^4)_2)_n-$, $-C(=O)NH(CH_2)_nS-$, $-C(=O)NH(C(R^4)_2)_nS-$, $-S(CH_2)_nC(=O)NH-$, $-S(C(R^4)_2)_nC(=O)NH-$, $-(CH_2)_nS-$, $-(C(R^4)_2)_nS-$, $-S(CH_2)_n-$, $-S(C(R^4)_2)_n-$, $-C(=O)NH(CH_2)_nNHC(=O)(CH_2)_n-$, $-C(=O)NH(C(R^4)_2)_nNHC(=O)(C(R^4)_2)_n-$, $-C(=O)(CH_2)_n-$, $-C(=O)(C(R^4)_2)_n-$, $-(CH_2)_nC(=O)-$, $-(C(R^4)_2)_nC(=O)-$, $-(CH_2)_n(O(CH_2)_n)_mNHC(=O)(CH_2)_n-$, $-(C(R^4)_2)_n(O(C(R^4)_2)_n)_mNHC(=O)(C(R^4)_2)_n-$, $-(CH_2)_n(O(CH_2)_n)_mOC(=O)NH(CH_2)_n-$, $-(C(R^4)_2)_n(O(C(R^4)_2)_n)_mOC(=O)NH(C(R^4)_2)_n-$, $-(CH_2)_n(O(CH_2)_n)_mOC(=O)-$, $-(C(R^4)_2)_n(O(C(R^4)_2)_n)_mOC(=O)-$, $-(CH_2)_n(O(CH_2)_n)_mC(=O)-$, $-(C(R^4)_2)_n(O(C(R^4)_2)_n)_mC(=O)-$, $-(CH_2)_nNHC(=O)(CH_2)_n-$, $-(C(R^4)_2)_nNHC(=O)(C(R^4)_2)_n-$, $-(O(CH_2)_n)_mNHC(=O)(CH_2)_n-$, $-(O(C(R^4)_2)_n)_mNHC(=O)(C(R^4)_2)_n-$,

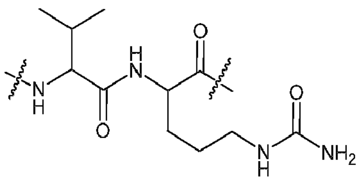

or

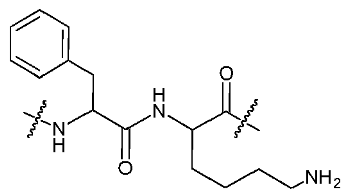

;

$A_4$ is $-C(=O)NH-$, $-C(=O)NH(CH_2)_n-$, $-C(=O)NH(C(R^4)_2)_n-$, $-(O(CH_2)_n)_m-$, $-(O(C(R^4)_2)_n)_m-$, $-((CH_2)_nO)_m-$, $-((C(R^4)_2)_nO)_m-$, $-((CH_2)_nO)_m(CH_2)_n-$, $-(((C(R^4)_2)_nO)_mC(R^4)_2)_n-$, $-(CH_2)_nC(=O)NH-$, $-(C(R^4)_2)_nC(=O)NH-$, $-(CH_2)_nNHC(=O)-$, $-(C(R^4)_2)_nNHC(=O)-$, $-NHC(=O)(CH_2)_n-$, $-NHC(=O)(C(R^4)_2)_n-$, $-C(=O)NH(CH_2)_nS-$,

$-C(=O)NH(C(R^4)_2)_nS-$, $-S(CH_2)_nC(=O)NH-$,$-S(C(R^4)_2)_nC(=O)NH-$, $-C(=O)NH(CH_2)_nNHC(=O)(CH_2)_n-$, $-C(=O)NH(C(R^4)_2)_nNHC(=O)(C(R^4)_2)_n-$,$-C(=O)(CH_2)_n-$, $-C(=O)(C(R^4)_2)_n-$, $-(CH_2)_nC(=O)-$, $-(C(R^4)_2)_nC(=O)-$,$-(CH_2)_n(O(CH_2)_n)_mNHC(=O)(CH_2)_n-$, $-(C(R^4)_2)_n(O(C(R^4)_2)_n)_mNHC(=O)(C(R^4)_2)_n-$,$-(CH_2)_nNHC(=O)(CH_2)_n-$, $-(C(R^4)_2)_nNHC(=O)(C(R^4)_2)_n-$, $-(CH_2)_nNH((CH_2)_nO)m(CH_2)_n-$,$-(C(R^4)_2)_nNH((C(R^4)_2)_nO)_m(C(R^4)_2)_n-$, $-(O(CH_2)_n)_mNHC(=O)(CH_2)_n-$, or$-(O(C(R^4)_2)_n)_mNHC(=O)(C(R^4)_2)_n-$;

each $X^2$ is independently selected from a bond, $R^8$

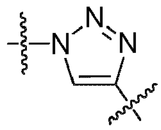 , 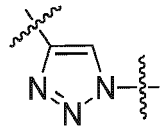 ,

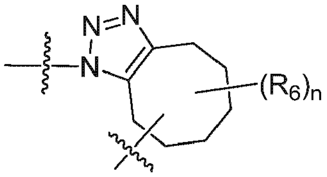 , 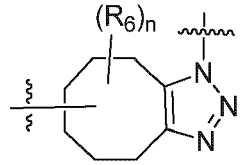 , 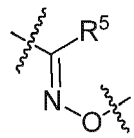 ,

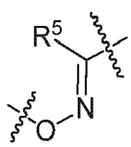 , 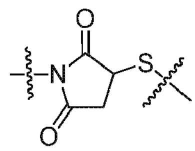 , 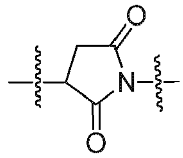 , 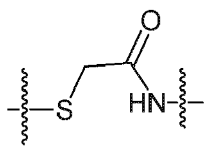 ,

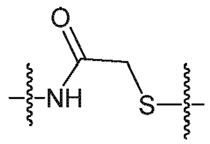 , 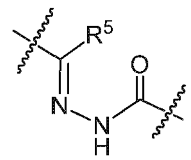 , 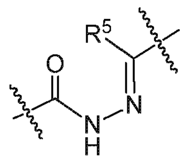 , 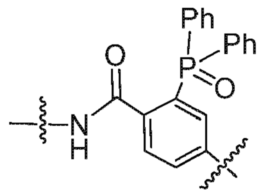 ,

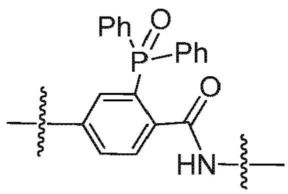 , 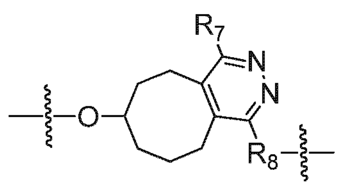 ,

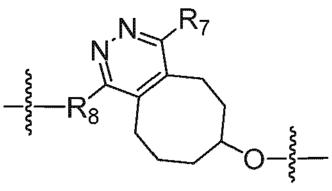 , 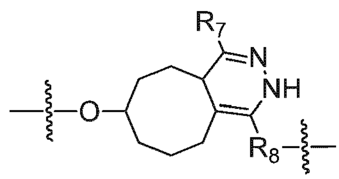 ,

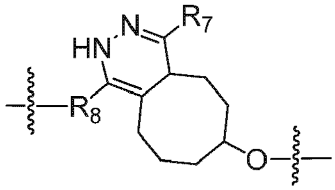

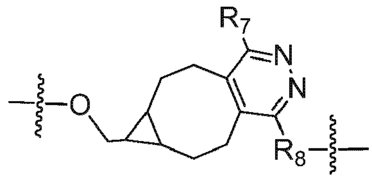

,

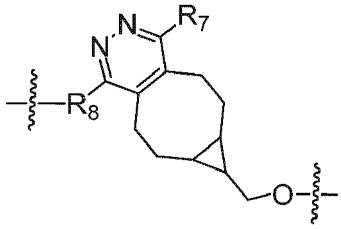

,

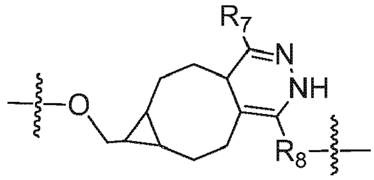

,

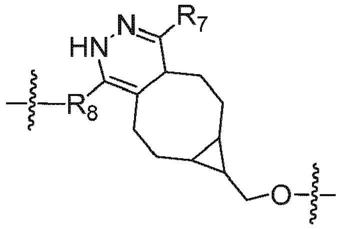

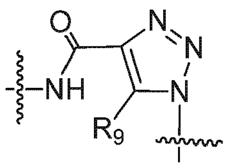

,

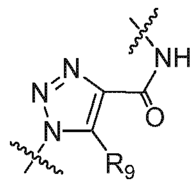

,

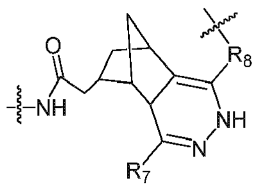

,

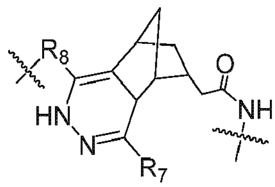

,

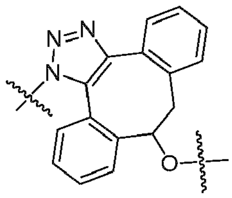

,

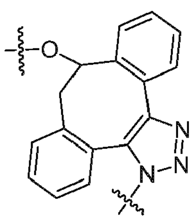

,

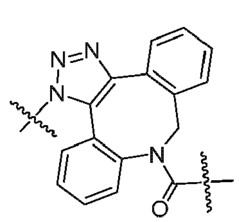

,

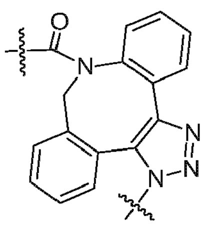

-S-, -$Si(OH)_2O$-,

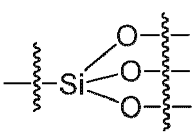

,

-$CHR^4(CH_2)_nC(=O)NH$-, -$CHR^4(CH_2)_nNHC(=O)$-, -$C(=O)NH$- and-$NHC(=O)$-;

each $R^4$ is independently selected from H, $C_{1-4}$alkyl, side chains of known amino acids, -C(=O)OH and-OH,
each $R^5$ is independently selected from H, $C_{1-4}$alkyl, phenyl or $C_{1-4}$alkyl substituted with 1 to 3 -OH groups;
each $R^6$ is independently selected from H, fluoro, benzyloxy substituted with -C(=O)OH, benzyl substituted with -C(=O)OH, $C_{1-4}$alkoxy substituted with -C(=O)OH and $C_{1-4}$alkyl substituted with-C(=O)OH;
$R^7$ is independently selected from H, $C_{1-4}$alkyl, phenyl, pyrimidine and pyridine;
$R^8$ is independently selected from

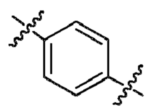
,
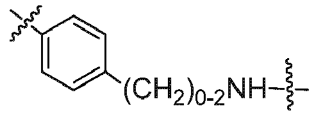
,
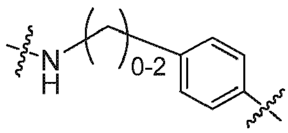
,
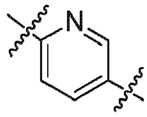
,
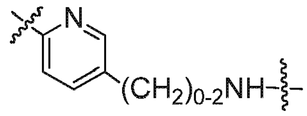
,
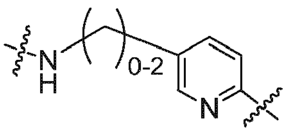
,
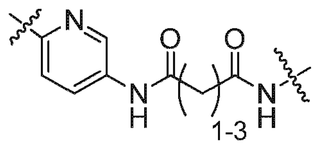
,

and

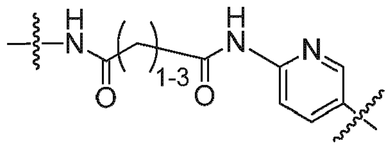
;

$R^9$ is independently selected from H and $C_{1-6}$haloalkyl;
each n is independently selected from 1, 2, 3, 4, 5, 6, 7, 8 and 9, and
each m is independently selected from 1, 2, 3, 4, 5, 6, 7, 8 and 9.

In certain embodiments the Linker Unit (LU) is $-L_1-L_2-L_3-L_4-$, wherein

$L_1$ is a bond, $-A_1-$, $-A_1X^2-$ or $-X^2-$;
$L_2$ is a bond, $-A_2-$, or $-A_2X^2-$;
$L_3$ is a bond, $-A_3-$, or $-A_3X^2-$;
$L_4$ is a bond, $-A_4-$, $-A_4X^2-$,

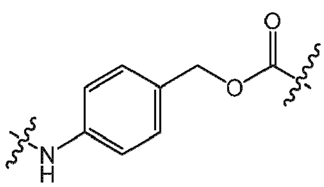
,
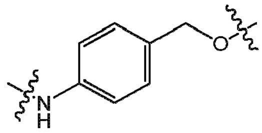
,

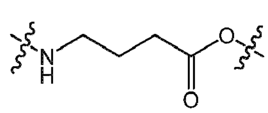, 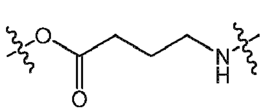, 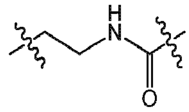, 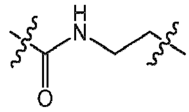,

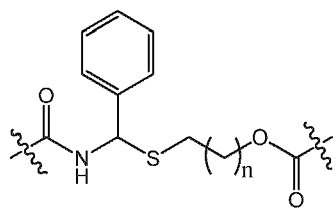, 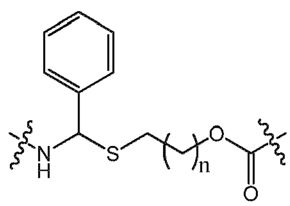, 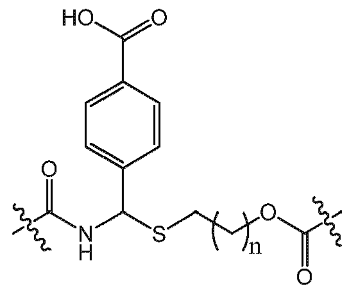,

or

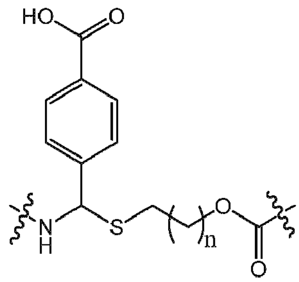;

$A_1$ is $-C(=O)NH-$, $-C(=O)NH(CH_2)_n-$, $-(O(CH_2)_n)_m-$, $-((CH_2)_nO)_m-$, $-((CH_2)_nO)_m(CH_2)_n-$,$-(CH_2)_nC(=O)NH-$, $-NHC(=O)(CH_2)_n-$, $-(CH_2)_nNHC(=O)-$, $-C(=O)NH(CH_2)_nS-$, $-S(CH_2)_nC(=O)NH-$,$-C(=O)NH(CH_2)_nNHC(=O)(CH_2)_n-$, $-C(=O)(CH_2)_n-$, $-(CH_2)_nC(=O)-$,$-(CH_2)_n(O(CH_2)_n)_mNHC(=O)(CH_2)_n-$, $-(CH_2)_nNHC(=O)(CH_2)_n-$, $-(CH_2)_nNH((CH_2)_nO)_m(CH_2)_n-$ or$-(O(CH_2)_n)_mNHC(=O)(CH_2)_n-$;

$A_2$ is $-C(=O)NH-$, $-C(=O)NH(CH_2)_n-$, $-(O(CH_2)_n)_m-$, $-((CH_2)_nO)_m-$, $-((CH_2)_nO)_m(CH_2)_n-$,$-(CH_2)_nC(=O)NH-$, $-NHC(=O)(CH_2)_n-$, $-(CH_2)_nNHC(=O)-$, $-C(=O)NH(CH_2)_nS-$, $-S(CH_2)_nC(=O)NH-$,$-C(=O)NH(CH_2)_nNHC(=O)(CH_2)_n-$, $-C(=O)(CH_2)_n-$, $-(CH_2)_nC(=O)-$,$-(CH_2)_n(O(CH_2)_n)_mNHC(=O)(CH_2)_n-$, $-(CH_2)_nNHC(=O)(CH_2)_n-$, $-(CH_2),NH((CH_2)_nO)_m(CH_2)_n-$,$-(O(CH_2)_n)_mNHC(=O)(CH_2)_n-$ or

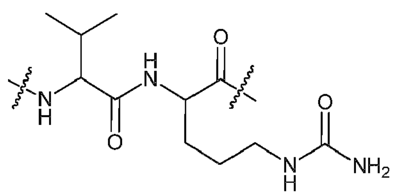;

$A_3$ is $-C(=O)NH-$, $-C(=O)NH(CH_2)_n-$, $-(O(CH_2)_n)_m-$, $-((CH_2)_nO)_m-$, $-((CH_2)_nO)_m(CH_2)_n-$,$-(CH_2)_nC(=O)NH-$, $-NHC(=O)(CH_2)_n-$, $-(CH_2)_nNHC(=O)-$, $-C(=O)NH(CH_2)_nS-$, $-S(CH_2)_nC(=O)NH-$,$-C(=O)NH(CH_2)_nNHC(=O)(CH_2)_n-$, $-C(=O)(CH_2)_n-$, $-(CH_2)_nC(=O)-$,$-(CH_2)_n(O(CH_2)_n)_mNHC(=O)(CH_2)_n-$, $-(CH_2)_nNHC(=O)(CH_2)_n-$, $-(CH_2)_nNH((CH_2)_nO)_m(CH_2)_n-$,$-(O(CH_2)_n)_mNHC(=O)(CH_2)_n-$ or

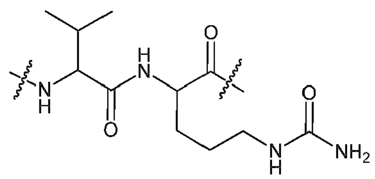

;

$A_4$ -C(=O)NH-, -C(=O)NH($CH_2$)$_n$-, -(O($CH_2$)$_n$)$_m$-, -(($CH_2$)$_n$O)$_m$-, -(($CH_2$)$_n$O)$_m$($CH_2$)$_n$-, -($CH_2$)$_n$C(=O)NH-, -NHC(=O)($CH_2$)$_n$-, -($CH_2$)$_n$NHC(=O)-, -C(=O)NH($CH_2$)$_n$S-, -S($CH_2$)$_n$C(=O)NH-,-C(=O)NH($CH_2$)$_n$NHC(=O)($CH_2$)$_n$-, -C(=O)($CH_2$)$_n$-, -($CH_2$)$_n$C(=O)-,-($CH_2$)$_n$(O($CH_2$)$_n$)$_m$NHC(=O)($CH_2$)$_n$-, -($CH_2$)$_n$NHC(=O)($CH_2$)$_n$-, -($CH_2$)$_n$NH(($CH_2$)$_n$O)$_m$($CH_2$)$_n$- or-(O($CH_2$)$_n$)$_m$NHC(=O)($CH_2$)$_n$-;
each $X^2$ is independently selected from a bond,

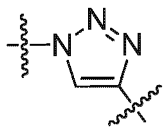 , 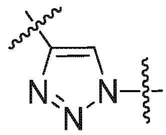 , 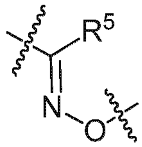

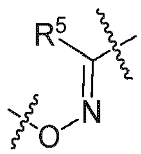 , 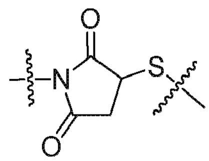 , 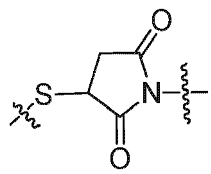 , 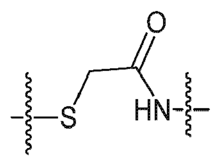 ,

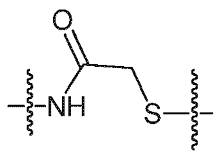 , 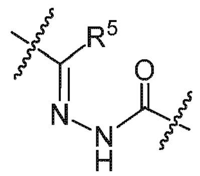 , 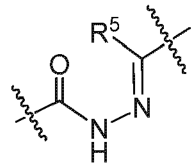 ,

-S-, -Si$(OH)_2$O-,

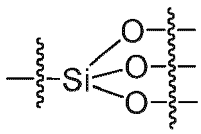

,

-$CHR^4$($CH_2$)$_n$C(=O)NH-, -$CHR^4$($CH_2$)$_n$NHC(=O)-, -C(=O)NH- and-NHC(=O)-;
each $R^4$ is independently selected from H, $C_{1-4}$alkyl, side chains of known amino acids, -C(=O)OH and-OH,
each $R^5$ is independently selected from H, $C_{1-4}$alkyl, phenyl or $C_{1-4}$alkyl substituted with 1 to 3 -OH groups;
each n is independently selected from 1, 2, 3, 4, 5, 6, 7, 8 and 9, and
each m is independently selected from 1, 2, 3, 4, 5, 6, 7, 8 and 9.

In certain embodiments the Linker Unit (LU) is -$L_1$-$L_2$-$L_3$-$L_4$-, wherein

$L_1$ is a bond, -$A_1$-, -$A_1X^2$- or -$X^2$-;
$L_2$ is a bond, -$A_2$-, or -$A_2X^2$-;
$L_3$ is a bond, -$A_3$-, or -$A_3X^2$-;
$L_4$ is a bond, -$A_4$-, -$A_4X^2$-,

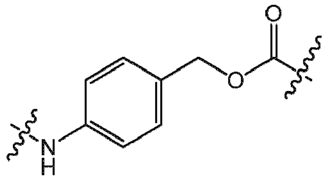

,

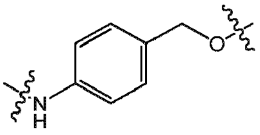

,

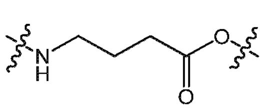

,

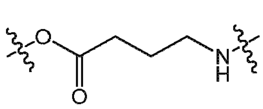

,

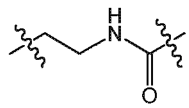

,

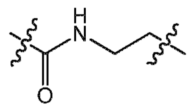

,

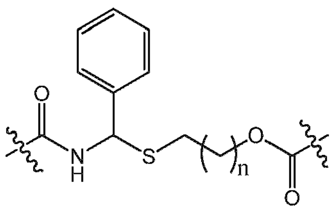

,

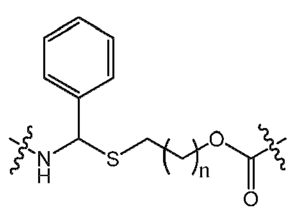

,

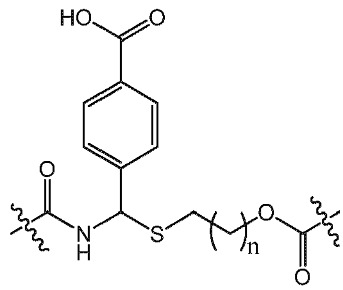

,

or

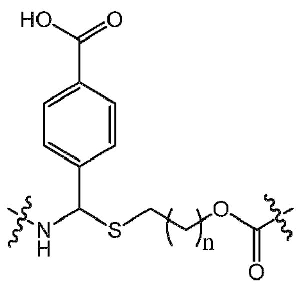

;

$A_1$ is $-C(=O)NH-$, $-C(=O)NH(CH_2)_n-$, $-C(=O)NH(CH_2)_nS-$, $-(O(CH_2)_n)_m-$, $-((CH_2)_nO)_m(CH_2)_n-$,$-NHC(=O)(CH_2)_n-$, $-(CH_2)_nNHC(=O)-$, $-C(=O)NH(CH_2)_nNHC(=O)(CH_2)_n-$,$-(CH_2)_nNH((CH_2)_nO)_m(CH_2)_n-$ or $-(O(CH_2)_n)_mNHC(=O)(CH_2)_n-$;

$A_2$ is $-C(=O)NH-$, $-C(=O)NH(CH_2)_n-$, $-C(=O)NH(CH_2)_nS-$, $-(O(CH_2)_n)_m-$,$-((CH_2)_nO)_m(CH_2)_n-$,$-NHC(=O)(CH_2)_n-$, $-(CH_2)_nNHC(=O)-$, $-C(=O)NH(CH_2)_nNHC(=O)(CH_2)_n-$,$-(CH_2)_nNH((CH_2)_nO)_m(CH_2)_n-$, $-(O(CH_2)_n)_mNHC(=O)(CH_2)_n-$ or

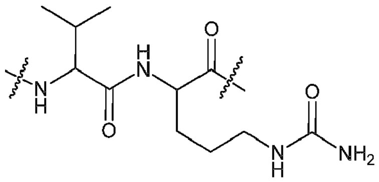

;

$A_3$ is $-C(=O)NH-$, $-C(=O)NH(CH_2)_n-$, $-C(=O)NH(CH_2)_nS-$, $-(O(CH_2)_n)_m-$, $-((CH_2)_nO)_m(CH_2)_n-$,$-NHC(=O)(CH_2)_n-$, $-(CH_2)_nNHC(=O)-$, $-C(=O)NH(CH_2)_nNHC(=O)(CH_2)_n-$,$-(CH_2)_nNH((CH_2)_nO)_m(CH_2)_n-$, $-(O(CH_2)_n)_mNHC(=O)(CH_2)_n-$ or

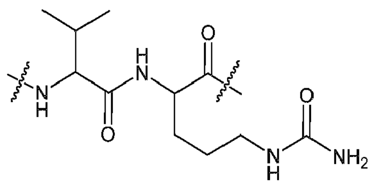

;

$A_4$ is $-C(=O)NH-$, $-C(=O)NH(CH_2)_n-$, $-C(=O)NH(CH_2)_nS-$, $-(O(CH_2)_n)_m-$, $-((CH_2)_nO)_m(CH_2)_n-$,$-NHC(=O)(CH_2)_n-$, $-(CH_2)_nNHC(=O)-$, $-C(=O)NH(CH_2)_nNHC(=O)(CH_2)_n-$,$-(CH_2)_nNH((CH_2)_nO)_m(CH_2)_n-$ or $-(O(CH_2)_n)_mNHC(=O)(CH_2)_n-$;

each $X^2$ is independently selected from a bond, $R^8$

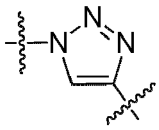 , 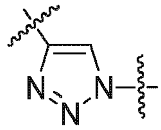 ,

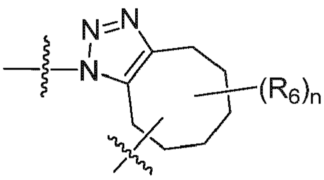 , 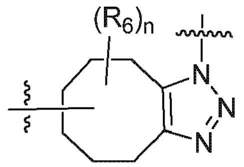 , 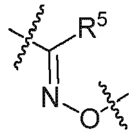 ,

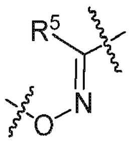 , 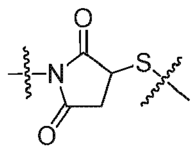 , 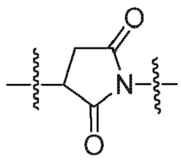 , 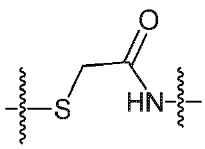 ,

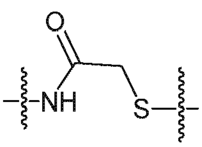 , 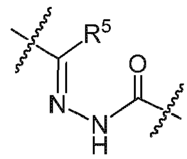 , 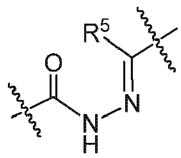 , 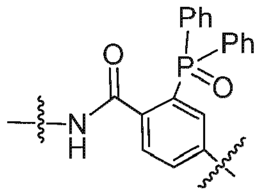 ,

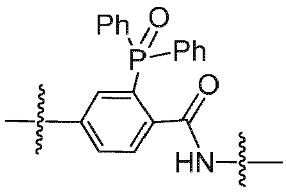 , 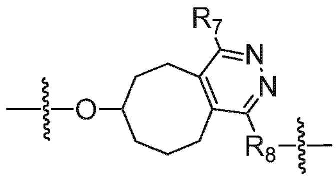 ,

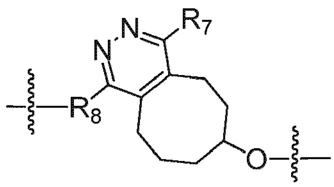 , 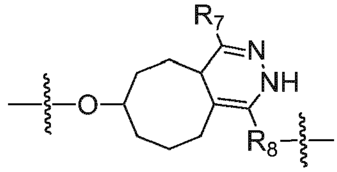 ,

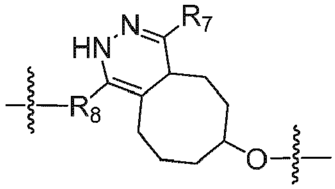 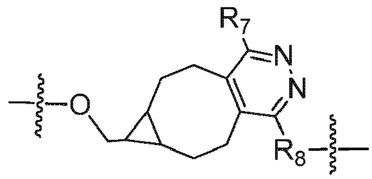 ,

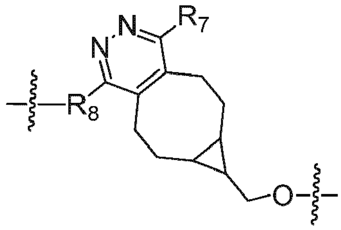 , 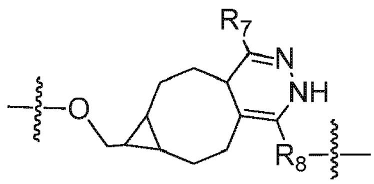 ,

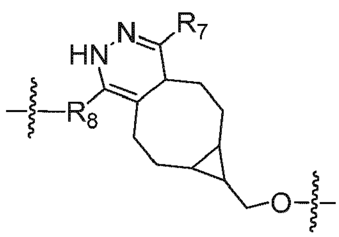 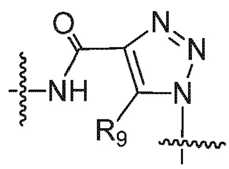 , 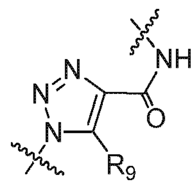 ,

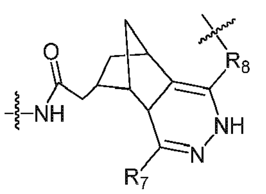 , 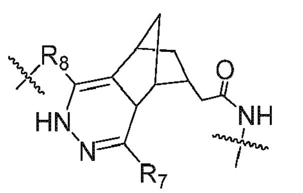 , 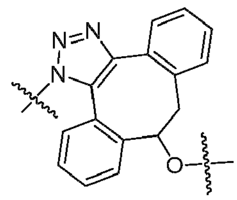 ,

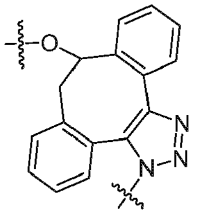 , 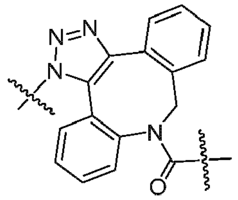 , 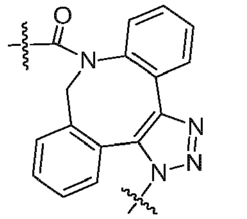

-S-, -Si(OH)$_2$O-,

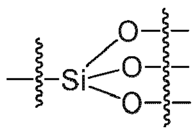

,

$-CHR^4(CH_2)_nC(=O)NH-$, $-CHR^4(CH_2)_nNHC(=O)-$, -C(=O)NH- and-NHC(=O)-;
each $R^4$ is independently selected from H, $C_{1-4}$alkyl, side chains of known amino acids, -C(=O)OH and-OH,
each $R^5$ is independently selected from H, $C_{1-4}$alkyl, phenyl or $C_{1-4}$alkyl substituted with 1 to 3 -OH groups;
each $R^6$ is independently selected from H, fluoro, benzyloxy substituted with -C(=O)OH, benzyl substituted with -C(=O)OH, $C_{1-4}$alkoxy substituted with -C(=O)OH and $C_{1-4}$alkyl substituted with-C(=O)OH;
$R^7$ is independently selected from H, $C_{1-4}$alkyl, phenyl, pyrimidine and pyridine;
$R^8$ is independently selected from

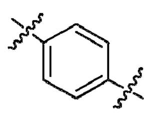

,

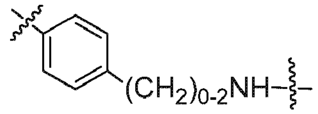

,

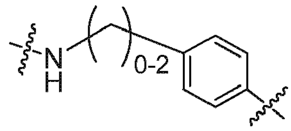

,

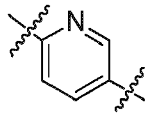

,

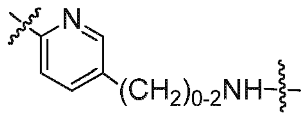

,

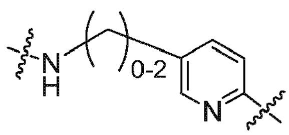

,

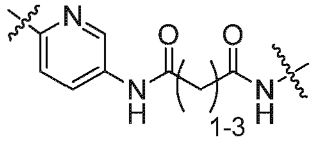

,

and

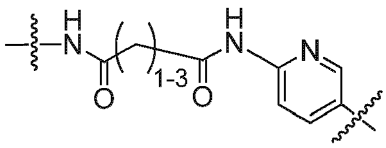

,

$R^9$ is independently selected from H and $C_{1-6}$haloalkyl;
each n is independently selected from 1, 2, 3, 4, 5, 6, 7, 8 and 9, and
each m is independently selected from 1, 2, 3, 4, 5, 6, 7, 8 and 9.

In one embodiment, $L_1$ is $-(CH_2)_{1-10}-C(=O)-$ , e.g., $-(CH_2)_5-C(=O)-$; and $L_2$, $L_3$ and $L_4$ each represent a bond.

In certain embodiments LU comprises a val-cit linker of this formula, wherein X represents a payload, typically a drug moiety such as one having anticancer activity:

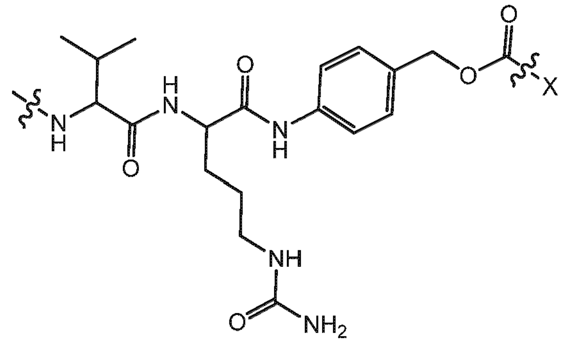

When $L_4$-$L_5$-$L_6$ is a val-cit linker as shown above, $L_3$ is preferably $-(CH_2)_{2-6}-C(=O)-$.

In certain embodiments the X group is a maytansinoid such as DM1 or DM4, or a dolastatin analog or derivative such as dolastatin 10 or 15 and auristatins MMAF or MMAE, or a calicheamicin such as N-acetyl-γ-calicheamicin, or a label or dye such as rhodamine or tetramethylrhodamine.

As used herein, a "linker" is any chemical moiety that is capable of connecting an antibody or a fragment thereof to an X group (payload) to form an immunoconjugate. Linkers can be susceptible to cleavage, such as, acid-induced cleavage, light-induced cleavage, peptidase-induced cleavage, esterase-induced cleavage, and disulfide bond cleavage, at conditions under which the compound or the antibody remains active. Alternatively, linkers can be substantially resistant to cleavage. A linker may or may not include a self-immolative spacer.

Non-limiting examples of the non-enzymatically cleavable linkers as used herein to conjugate an $X^1$ group to the modified antibodies or antibody fragment thereof provided herein include, acid-labile linkers, linkers containing a disulfide moiety, linkers containing a triazole moiety, linkers containing a hydrazone moiety, linkers containing a thioether moiety, linkers containing a diazo moiety, linkers containing an oxime moiety, linkers containing an amide moiety and linkers containing an acetamide moiety.

Non-limiting examples of the enzymatically cleavable linkers as used herein to conjugate an X group to the modified antibodies or antibody fragment thereof provided herein include, but are not limited to, linkers that are cleaved by a protease, linkers that are cleaved by an amidase, and linkers that are cleaved by β-glucuronidase or another glycosidase.

In certain embodiments, such enzyme cleavable linkers are linkers which are cleaved by cathepsin, including cathepsin Z, cathepsin B, cathepsin H and cathepsin C. In certain embodiments the enzymatically cleavable linker is a dipeptide cleaved by cathepsin, including dipeptides cleaved by cathepsin Z, cathepsin B, cathepsin H or cathepsin C. In certain embodiments the enzymatically cleavable linker is a cathepsin B-cleavable peptide linker. In certain embodiments the enzymatically cleavable linker is a cathepsin B-cleavable dipeptide linker. In certain embodiments the enzymatically cleavable dipeptide linker is valine-citrulline or phenylalanine-lysine. Other non-limiting examples of the enzymatically cleavable linkers as used herein conjugate an X group to the modified antibodies or antibody fragment thereof provided herein include, but are not limited to, linkers which are cleaved by β-glucuronidase, e.g.,

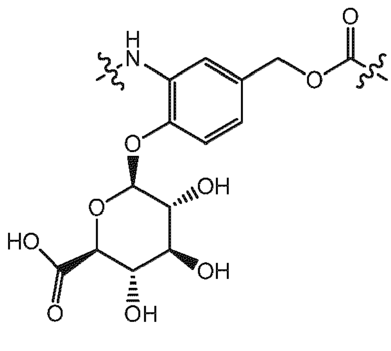

See Ducry et al, Bioconjugate Chem, (2010) vol. 21(1), 5-13.

"Self-immolative spacers" are bifunctional chemical moieties covalently linked at one terminus to a first chemical moiety and at the other terminus to a second chemical moiety, thereby forming a stable tripartate molecule. A linker can comprise a self-immolative spacer bonded to a third chemical moiety that is cleavable from the spacer either chemically or enzymatically. Upon cleavage of a bond between the self-immolative spacer and the first chemical moiety or the third

chemical moiety, self-immolative spacers undergo rapid and spontaneous intramolecular reactions and thereby separate from the second chemical moiety. These intramolecular reactions generally involve electronic rearrangements such as 1,4, or 1,6, or 1,8 elimination reactions or cyclizations to form highly favored five- or six-membered rings. In certain embodiments of the present application, the first or third moiety is an enzyme cleavable group, and this cleavage results from an enzymatic reaction, while in other embodiments the first or third moiety is an acid labile group and this cleavage occurs due to a change in pH. As applied to the present application, the second moiety is the "Payload" group as defined herein. In certain embodiments, cleavage of the first or third moiety from the self-immolative spacer results from cleavage by a proteolytic enzyme, while in other embodiments it results from cleaved by a hydrolase. In certain embodiments, cleavage of the first or third moiety from the self-immolative spacer results from cleavage by a cathepsin enzyme or a glucuronidase.

In certain embodiments, the enzyme cleavable linker is a peptide linker and the self-immolative spacer is covalently linked at one of its ends to the peptide linker and covalently linked at its other end to a drug moiety. This tripartite molecule is stable and pharmacologically inactive in the absence of an enzyme, but which is enzymatically cleavable by enzyme at a bond covalently linking the spacer moiety and the peptide moiety. The peptide moiety is cleaved from the tripartate molecule which initiates the self-immolating character of the spacer moiety, resulting in spontaneous cleavage of the bond covalently linking the spacer moiety to the drug moiety, to thereby effect release of the drug in pharmacologically active form.

In other embodiments, a linker comprises a self-immolative spacer that connects to the peptide, either directly or indirectly at one end, and to a payload at the other end; and the spacer is attached to a third moiety that can be cleaved from the spacer enzymatically, such as by a glucuronidase. Upon cleavage of the third moiety, the spacer degrades or rearranges in a way that causes the payload to be released. An example of a linker with this type of self-immolative spacer is this glucuronidase-cleavable linker, where hydrolysis of the acetal catalyzed by glucoronidase releases a phenolic compound that spontaneously decomposes under physiological conditions:

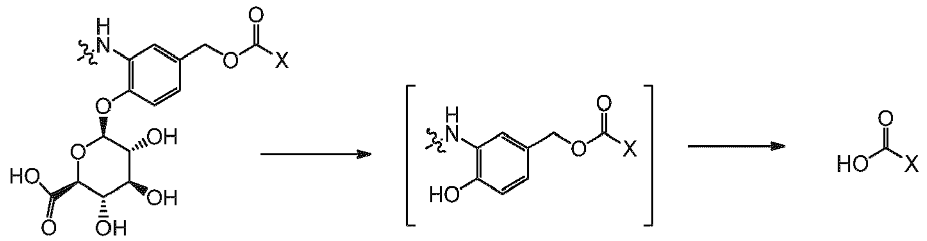

Non-limiting examples of the self-immolative spacer optionally used in the conjugation of an $X^1$ group to the modified antibodies or antibody fragment thereof provided herein include, but are not limited to, moieties which include a benzyl carbonyl moiety, a benzyl ether moiety, a 4-aminobutyrate moiety, a hemithioaminal moiety or a N-acylhemithioaminal moiety.

Other examples of self-immolative spacers include, but are not limited to, p-aminobenzyloxycarbonyl groups, aromatic compounds that are electronically similar to the p-aminobenzyloxycarbonyl group, such as 2-aminoimidazol-5-methanol derivatives and ortho or para-aminobenzylacetals. In certain embodiments, self-immolative spacers used herein which undergo cyclization upon amide bond hydrolysis, include substituted and unsubstituted 4-aminobutyric acid amides and 2-aminophenylpropionic acid amides.

In certain embodiments, the self-immolative spacer is

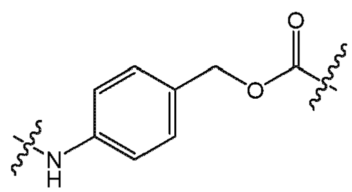

or

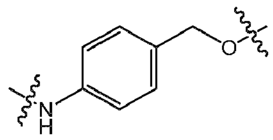

,

while in other embodiments the self-immolative spacer is

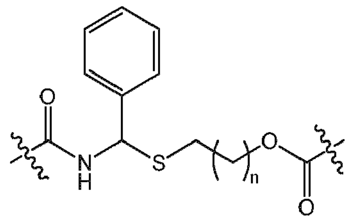

,

where n is 1 or 2. In other embodiments the self-immolative spacer is

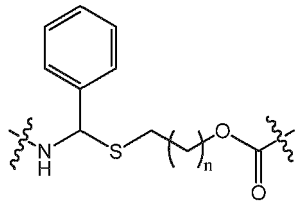

,

where n is 1 or 2. In other embodiments the self-immolative spacer is

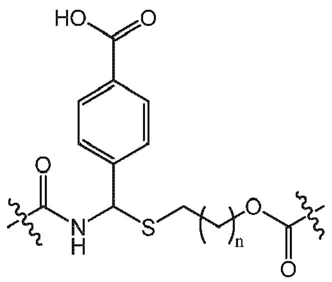

,

where n is 1 or 2. In other embodiments the self-immolative spacer is

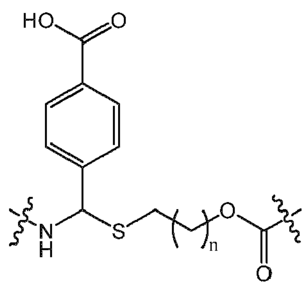

,

where n is 1 or 2. In other embodiments the self-immolative spacer is

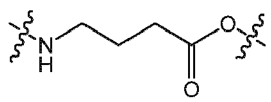,

where n is 1 or 2.

Schemes (2a-2c) illustrate the post-translational modification of the modified antibodies or antibody fragment thereof provided herein wherein the Linker Unit (LU) is $-L_1-L_2-L_3-L_4-$, and L1 in each case is the group that reacts with the new Cys.

Scheme 2a.

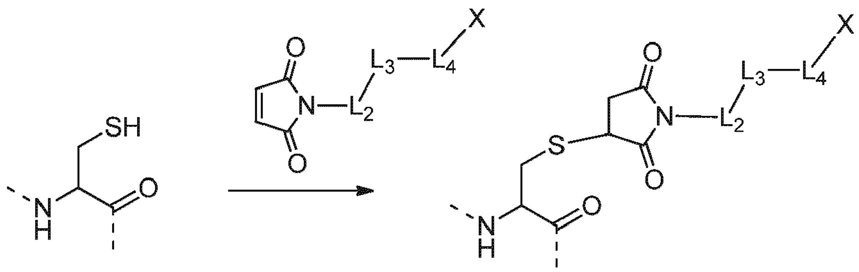

Scheme 2b.

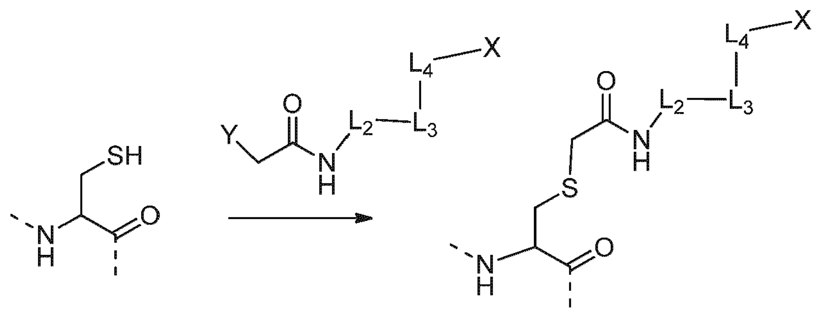

Scheme 3c.

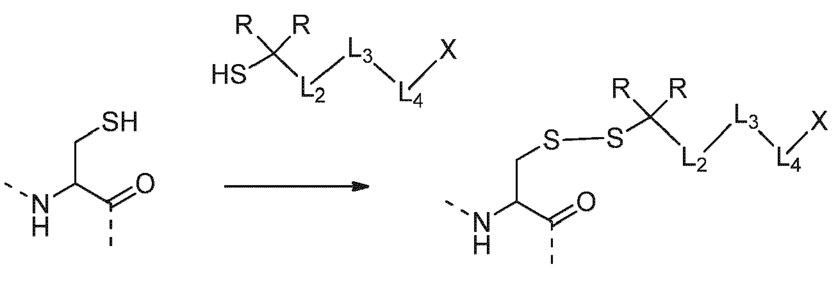

In each of Schemes 2a-2c, the starting material is the replacement Cys residue in an antibody or antibody fragment modified as described herein, where the dashed bonds indicate connection to adjoining residues of the antibody or antibody fragment; each R is H or $C_{1-4}$ alkyl, typically H or methyl; $L_2$, $L_3$ and $L_4$ are components of the linking unit LU, such as those described above; X is the payload; and the group connecting $L_2$ to the sulfur of the substitute Cys of the present application is $L_1$.

In some embodiments of the present application, X is a reactive functional group that can be used to connect the conjugated antibody to another chemical moiety, by interacting with a suitable complementary functional group.

Table 3 depicts some examples of reactive functional groups that X can represent, along with a complementary functional group that can be used to connect a conjugate comprising X to another compound. Methods for using X to connect to the corresponding complementary functional group are well known in the art. Connections using azide are typically done using 'Click' or copper-free click chemistry; reactions involving hydrazines, alkoxyamines or acyl hydrazines typically proceed through the formation of a Schiff base with one of the carbonyl functional groups.

Table 3

| X | Complementary Reactive Functional Group for X |
|---|---|
| a thiol | a thiol, a maleimide, a haloacetamide, a vinyl sulfone, or a vinylpyridine |
| an azide | an alkene, alkyne, a phosphine-(thio)ester, a cyclooctyne, a cyclooctene or an oxanobornadiene |
| a phosphine-(thio)ester) | an azide |
| an oxanobornadiene | an azide or a tetrazine |
| an alkyne | an azide or a tetrazine |
| an alkene | a tetrazine |
| a cyclooctyne | an azide or a tetrazine |
| a cyclooctene | a tetrazine |
| a norbornene | a tetrazine |
| a tetrazine | a norbornene, an alkene, alkyne, a cyclooctyne or an oxanobornadiene |
| an aldehyde | a hydroxylamine, a hydrazine or $NH_2$-NH-C(=O)- |
| a ketone | a hydroxylamine, a hydrazine or $NH_2$-NH-C(=O)- |
| a hydroxylamine | an aldehyde or a ketone |
| a hydrazine | an aldehyde or a ketone |
| $NH_2$-NH-C(=O)- | an aldehyde or a ketone |
| a haloacetamide | a thiol |
| a thiol | a thiol |
| a maleimide | a thiol |
| a vinyl sulfone | a thiol |
| a vinylpyridine | a thiol |

Exemplary products of the connections made using these components are depicted in Table 4, where $Y^1$ represents an antibody of the present application, $A_1$ represents a linking unit (LU) connecting the antibody to payload $X^a$, $-L_2-L_3-L_4-$ in Formula II-a represents a linker unit that can be present in a molecule to be connected to the conjugated antibody via $X^a$, and $X^1$ represents a payload. Payload $X^a$ is a reactive functional group, and $X^b$ on Formula II-a is the corresponding complementary functional group, and Formula II-a itself represents a molecule to be connected to the conjugated antibody. The third column in Table 4 depicts a product from reaction of $X^a$ with $X^b$.

Table 4

| $Y^1-A_1-X^a$ | $X^b-L_2-L_3-L_4-X^1$ Formula (II-a) | $Y^1-A_1-X^2-L_2-L_3-L_4-X^1$ |
|---|---|---|
| $Y^1-A_1-N_3$ | $HC{\equiv}C-L_2-L_3-L_4-X^1$ | 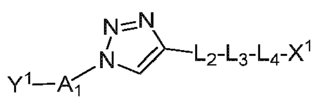 |

(continued)

| $Y^1$-$A_1$-$X^a$ | $X^b$-$L_2$-$L_3$-$L_4$-$X^1$ Formula (II-a) | $Y^1$-$A_1$-$X^2$-$L_2$-$L_3$-$L_4$-$X^1$ |
|---|---|---|
| $Y^1$-$A_1$-$N_3$ | HC=C-$L_2$-$L_3$-$L_4$-$X^1$ | 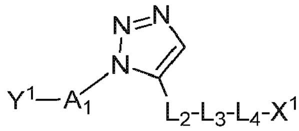 |
| $Y^1$-$A_1$-C≡CH | $N_3$-$L_2$-$L_3$-$L_4$-$X^1$ | 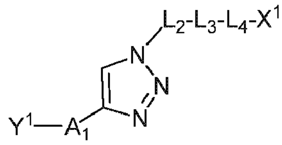 |
| $Y^1$-$A_1$-C≡CH | $N_3$-$L_2$-$L_3$-$L_4$-$X^1$ | 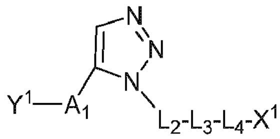 |
| 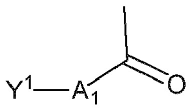 | $NH_2$-O-$L_2$-$L_3$-$L_4$-$X^1$ | 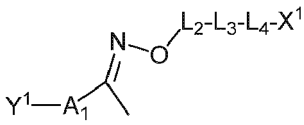 |
| 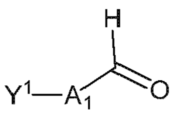 | $NH_2$-O-$L_2$-$L_3$-$L_4$-$X^1$ | 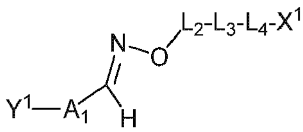 |
| 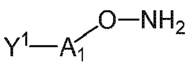 | $CH_3C(=O)$-$L_2$-$L_3$-$L_4$-$X^1$ | 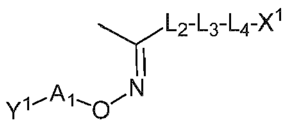 |
| 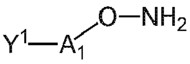 | HC(=O)-$L_2$-$L_3$-$L_4$-$X^1$ | 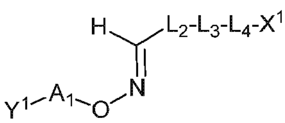 |
| 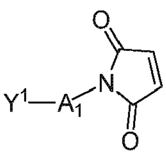 | HS-$L_2$-$L_3$-$L_4$-$X^1$ | 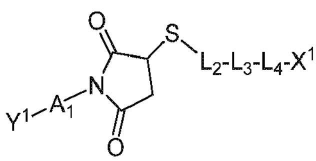 |
| 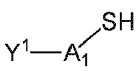 | 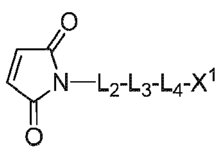 | 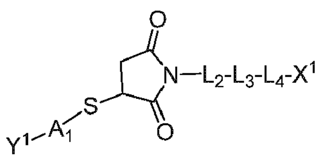 |

(continued)

| $Y^1$-$A_1$-$X^a$ | $X^b$-$L_2$-$L_3$-$L_4$-$X^1$ Formula (II-a) | $Y^1$-$A_1$-$X^2$-$L_2$-$L_3$-$L_4$-$X^1$ |
|---|---|---|
| 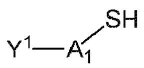 | 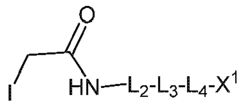 | 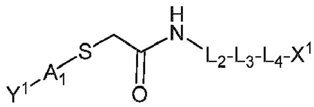 |
| 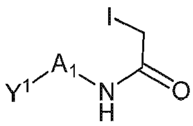 | HS—$L_2$-$L_3$-$L_4$-$X^1$ | 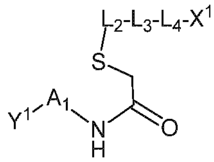 |
| 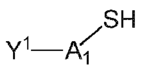 | 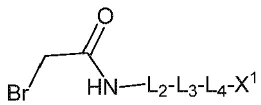 | 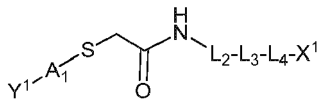 |
| 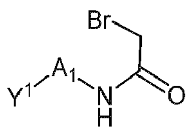 | HS—$L_2$-$L_3$-$L_4$-$X^1$ | 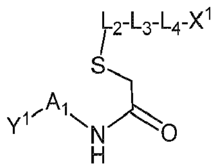 |
| 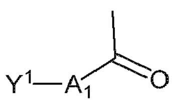 | $NH_2$-NH-C(=O)-$L_2$-$L_3$-$L_4$-$X^1$ | 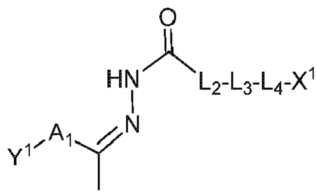 |
| 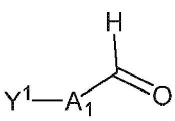 | $NH_2$-NH-C(=O)-$L_2$-$L_3$-$L_4$-$X^1$ | 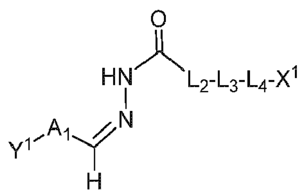 |
| 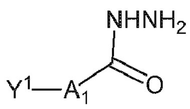 | $R_5$C(=O)-$L_2$-$L_3$-$L_4$-$X^1$ | 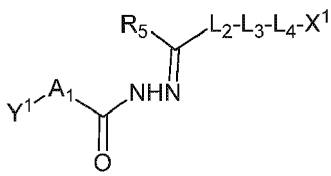 |
| 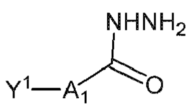 | HC(=O)-$L_2$-$L_3$-$L_4$-$X^1$ | 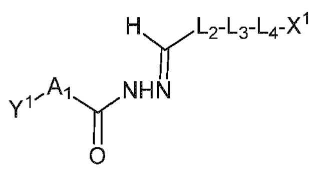 |

(continued)

| $Y^1$-$A_1$-$X^a$ | $X^b$-$L_2$-$L_3$-$L_4$-$X^1$ Formula (II-a) | $Y^1$-$A_1$-$X^2$-$L_2$-$L_3$-$L_4$-$X^1$ |
|---|---|---|
| 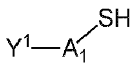 | HS-$L_2$-$L_3$-$L_4$-$X^1$ | 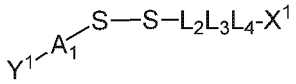 |
| $Y^1$-$A_1$-$N_3$ | 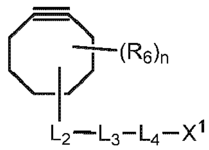 | 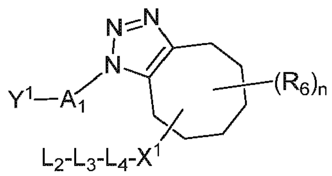 |
| 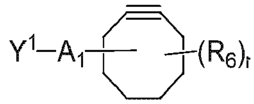 | $N_3$-$L_2$-$L_3$-$L_4$-$X^1$ | 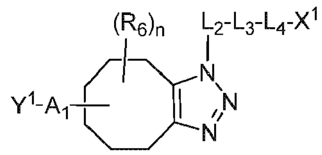 |
| $Y^1$-$A_1$-$N_3$ | 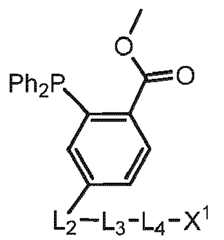 | 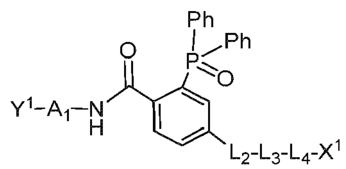 |
| 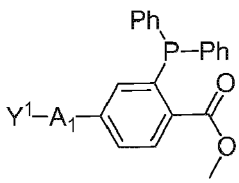 | $N_3$-$L_2$-$L_3$-$L_4$-$X^1$ | 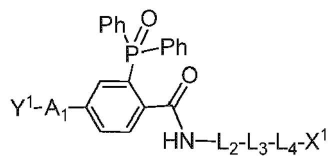 |
| $Y^1$-$A_1$-$N_3$ | 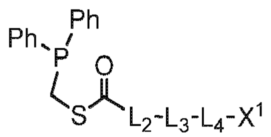 | 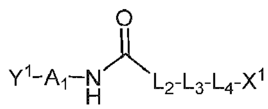 |
| 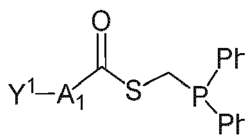 | $N_3$-$L_2$-$L_3$-$L_4$-$X^1$ | 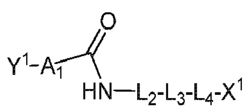 |
| 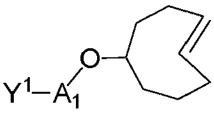 | 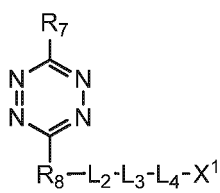 | 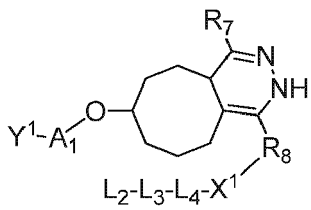 |

(continued)

| $Y^1$-$A_1$-$X^a$ | $X^b$-$L_2$-$L_3$-$L_4$-$X^1$ Formula (II-a) | $Y^1$-$A_1$-$X^2$-$L_2$-$L_3$-$L_4$-$X^1$ |
|---|---|---|
| 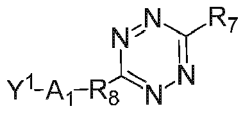 | 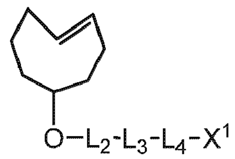 | 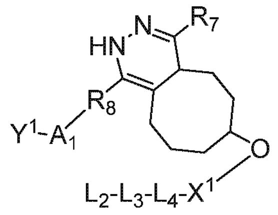 |
| 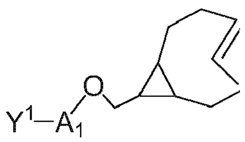 | 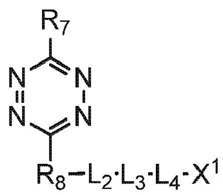 | 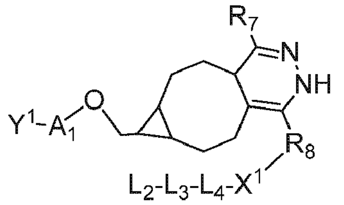 |
| 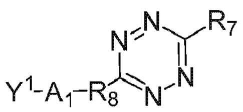 | 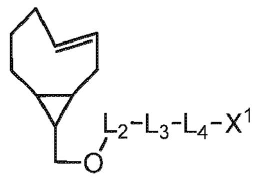 | 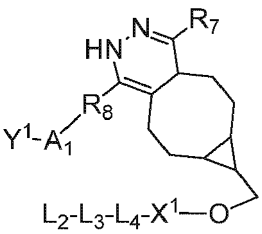 |
| 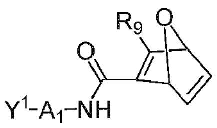 | $N_3$-$L_2$-$L_3$-$L_4$-$X^1$ | 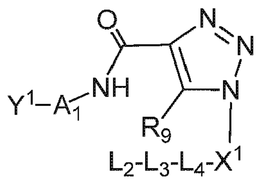 |
| $Y^1$-$A_1$-$N_3$ | 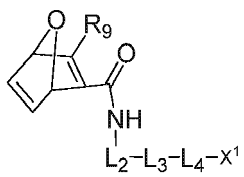 | 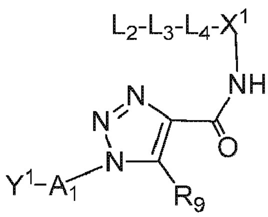 |
| 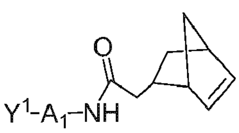 | 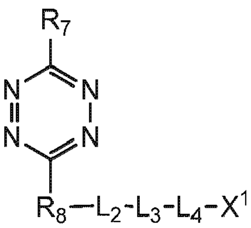 | 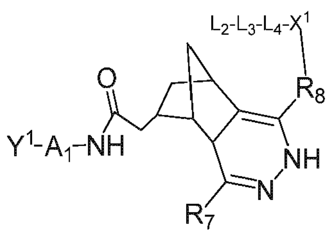 |

(continued)

| $Y^1$-$A_1$-$X^a$ | $X^b$-$L_2$-$L_3$-$L_4$-$X^1$ Formula (II-a) | $Y^1$-$A_1$-$X^2$-$L_2$-$L_3$-$L_4$-$X^1$ |
|---|---|---|
| 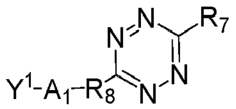 | 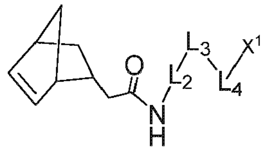 | 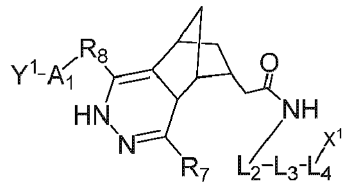 |
| $Y^1$-$A_1$-$N_3$ | 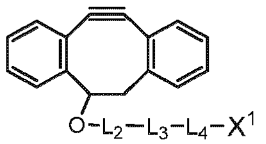 | 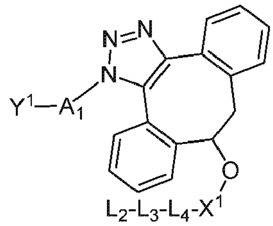 |
| 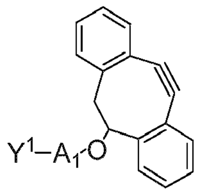 | $N_3$-$L_2$-$L_3$-$L_4$-$X^1$ | 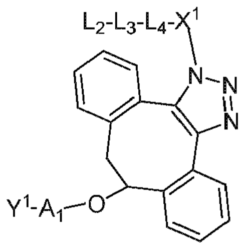 |

In certain embodiments, the modified antibody or antibody fragment thereof provided herein is conjugated with an "X group-to-antibody" (payload to antibody) ratio between about 1 and 16, such as 1-12, or 1, 2, 3, 4, 5, 6, 7, or 8, wherein the modified antibody or antibody fragment thereof contains 1, 2, 3, 4, 5, 6, 7, or 8 cysteine residues incorporated at the specific sites disclosed herein. For example, an "X group-to-antibody" ratio of 4 can be achieved by incorporating two Cys residues into the heavy chain of an antibody, which will contain 4 conjugation sites, two from each heavy chain. Immunoconjugates of such antibodies will contain up to 4 payload groups, which may be alike or different and are preferably all alike. In another example, an "X group-to-antibody" ratio of 4 can be achieved by incorporating one Cys residue into the heavy chain and a second Cys residue into the light chain of an antibody resulting in 4 conjugation sites, two in the two heavy chains and two in the two light chains. A ratio 6, 8 or higher can be achieved by combinations of 3, 4 or more cysteine substitutions of the present application in heavy and light chain of the antibody. Substituting multiple cysteine groups into an antibody can lead to inappropriate disulfide formation and other problems. Thus for loading more than 4 payload groups onto one antibody molecule, the methods of the present application can alternatively be combined with methods that do not rely upon reactions at cysteine sulfur, such as acylations at lysine, or conjugation via S6 tags or Pcl methodology.

While the payload to antibody ratio has an exact value for a specific conjugate molecule, it is understood that the value will often be an average value when used to describe a sample containing many molecules, due to some degree of in homogeneity, typically in the conjugation step. The average loading for a sample of an immunoconjugate is referred to herein as the drug to antibody ratio, or DAR. In some embodiments, the DAR is between about 4 to about 16, and typically is about 4, 5, 6, 7, 8. In some embodiments, at least 50% of a sample by weight is compound having the average ratio plus or minus 2, and preferably at least 50% of the sample is a conjugate that contains the average ratio plus or minus 1. Preferred embodiments include immunoconjugates wherein the DAR is about 2 or about 8, e.g., about 2, about 4, about 6 or about 8. In some embodiments, a DAR of 'about n' means the measured value for DAR is within 10% of n (in Formula (I)).

3. Further Alteration of the Framework of Fc Region

The present application provides site-specific labeled immunoconjugates. The immunoconjugates of the present application may comprise modified antibodies or antibody fragments thereof that further comprise modifications to framework residues within $V_H$ and/or $V_L$, *e.g.* to improve the properties of the antibody. Typically such framework modifications

are made to decrease the immunogenicity of the antibody. For example, one approach is to "back-mutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "back-mutated" to the germline sequence by, for example, site-directed mutagenesis. Such "back-mutated" antibodies are also intended to be encompassed by the present application.

Another type of framework modification involves mutating one or more residues within the framework region, or even within one or more CDR regions, to remove T-cell epitopes to thereby reduce the potential immunogenicity of the antibody. This approach is also referred to as "deimmunization" and is described in further detail in U.S. Patent Publication No. 20030153043 by Carr et al.

In addition or alternative to modifications made within the framework or CDR regions, antibodies of the present application may be engineered to include modifications within the Fc region, typically to alter one or more functional properties of the antibody, such as serum half-life, complement fixation, Fc receptor binding, and/or antigen-dependent cellular cytotoxicity. Furthermore, an antibody of the present application may be chemically modified (*e.g.,* one or more chemical moieties can be attached to the antibody) or be modified to alter its glycosylation, again to alter one or more functional properties of the antibody. Each of these embodiments is described in further detail below.

In one embodiment, the hinge region of CHI is modified such that the number of cysteine residues in the hinge region is altered, *e.g.,* increased or decreased. This approach is described further in U.S. Patent No. 5,677,425 by Bodmer et al. The number of cysteine residues in the hinge region of CHI is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Patent No. 6,165,745 by Ward et al.

In yet other embodiments, the Fc region is altered by replacing at least one amino acid residue with a different amino acid residue to alter the effector functions of the antibody. For example, one or more amino acids can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in, *e.g.,* U.S. Patent Nos. 5,624,821 and 5,648,260, both by Winter et al.

In another embodiment, one or more amino acids selected from amino acid residues can be replaced with a different amino acid residue such that the antibody has altered C1q binding and/or reduced or abolished complement dependent cytotoxicity (CDC). This approach is described in, *e.g.,* U.S. Patent Nos. 6,194,551 by Idusogie et al.

In another embodiment, one or more amino acid residues are altered to thereby alter the ability of the antibody to fix complement. This approach is described in, *e.g.,* the PCT Publication WO 94/29351 by Bodmer et al. In a specific embodiment, one or more amino acids of an antibody or antibody fragment thereof of the present application are replaced by one or more allotypic amino acid residues. Allotypic amino acid residues also include, but are not limited to, the constant region of the heavy chain of the IgG1, IgG2, and IgG3 subclasses as well as the constant region of the light chain of the kappa isotype as described by Jefferis et $\alpha$l., MAbs. 1:332-338 (2009).

In yet another embodiment, the Fc region is modified to increase the ability of the antibody to mediate antibody dependent cellular cytotoxicity (ADCC) and/or to increase the affinity of the antibody for an Fc$\gamma$ receptor by modifying one or more amino acids. This approach is described in, *e.g.,* the PCT Publication WO 00/42072 by Presta. Moreover, the binding sites on human IgG1 for FcyRI, FcyRII, FcyRIII and FcRn have been mapped and variants with improved binding have been described (see Shields et al., J. Biol. Chem. 276:6591-6604, 2001).

In still another embodiment, the glycosylation of an antibody is modified. For example, an aglycosylated antibody can be made (*i.e*., the antibody lacks glycosylation). Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in, *e.g.,* U.S. Patent Nos. 5,714,350 and 6,350,861 by Co et al.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the present application to thereby produce an antibody with

altered glycosylation. For example, EP 1,176,195 by Hang et al. describes a cell line with a functionally disrupted FUT8 gene, which encodes a fucosyl transferase, such that antibodies expressed in such a cell line exhibit hypofucosylation. PCT Publication WO 03/035835 by Presta describes a variant CHO cell line, Lecl3 cells, with reduced ability to attach fucose to Asn(297)-linked carbohydrates, also resulting in hypofucosylation of antibodies expressed in that host cell (see also Shields et al., (2002) J. Biol. Chem. 277:26733-26740). PCT Publication WO 99/54342 by Umana et al. describes cell lines engineered to express glycoprotein-modifying glycosyl transferases (*e.g.,* beta(1,4)-N acetylglucosaminyltransferase III (GnTIII)) such that antibodies expressed in the engineered cell lines exhibit increased bisecting GlcNac structures which results in increased ADCC activity of the antibodies (see also Umana et al., Nat. Biotech. 17:176-180, 1999).

In another embodiment, the antibody is modified to increase its biological half-life. Various approaches are possible. For example, one or more of the following mutations can be introduced: T252L, T254S, or T256F, as described in U.S. Patent No. 6,277,375 to Ward. Alternatively, to increase the biological half-life, the antibody can be altered within the CHI or $C_L$ region to contain a salvage receptor binding epitope taken from two loops of a CH2 domain of an Fc region of an IgG, as described in U.S. Patents 5,869,046 and 6,121,022 by Presta et al.

### 4. Antibody Conjugates

The present application provides site-specific labeling methods, modified antibodies and antibody fragments thereof, and immunoconjugates prepared accordingly. Using the methods of the present application, a modified antibody or antibody fragments thereof can be conjugated to a label, such as a drug moiety, e.g., an anti-cancer agent, an autoimmune treatment agent, an anti-inflammatory agent, an antifungal agent, an antibacterial agent, an anti-parasitic agent, an anti-viral agent, or an anesthetic agent, or an imaging reagent, such as a chelator for PET imaging, or a fluorescent label, or a MRI contrast reagent. An antibody or antibody fragments can also be conjugated using several identical or different labeling moieties combining the methods of the present application with other conjugation methods.

In certain embodiments, the immunoconjugates of the present application comprise a drug moiety selected from a V-ATPase inhibitor, a HSP90 inhibitor, an IAP inhibitor, an mTor inhibitor, a microtubule stabilizer, a microtubule destabilizer, an auristatin, a dolastatin, a maytansinoid, a MetAP (methionine aminopeptidase), an inhibitor of nuclear export of proteins CRM1, a DPPIV inhibitor, proteasome inhibitors, an inhibitor of phosphoryl transfer reactions in mitochondria, a protein synthesis inhibitor, a kinase inhibitor, a CDK2 inhibitor, a CDK9 inhibitor, an HDAC inhibitor, a DNA damaging agent, a DNA alkylating agent, a DNA intercalator, a DNA minor groove binder, topoisomerase inhibitors, RNA synthesis inhibitors, kinesin inhibitors, inhibitors of protein-protein interactions, an Eg5 inhibitor, and a DHFR inhibitor.

Further, the modified antibodies or antibody fragments of the present application may be conjugated to a drug moiety that modifies a given biological response. Drug moieties are not to be construed as limited to classical chemical therapeutic agents. For example, the drug moiety may be an immune modulator, such as an immune potentiator, a small molecule immune potentiator, a TLR agonist, a CpG oligomer, a TLR2 agonist, a TLR4 agonist, a TLR7 agonist, a TLR9 agonist, a TLR8 agonist, a T-cell epitope peptide or a like. The drug moiety may also be an oligonucleotide, a siRNA, a shRNA, a cDNA or a like. Alternatively, the drug moiety may be a protein, peptide, or polypeptide possessing a desired biological activity. Such proteins may include, for example, a toxin such as abrin, ricin A, pseudomonas exotoxin, cholera toxin, or diphtheria toxin, a protein such as tumor necrosis factor, $\alpha$-interferon, $\beta$-interferon, nerve growth factor, platelet derived growth factor, tissue plasminogen activator, a cytokine, an apoptotic agent, an anti-angiogenic agent, or, a biological response modifier such as, for example, a lymphokine.

In one embodiment, the modified antibodies or antibody fragments of the present application are conjugated to a drug moiety, such as a cytotoxin, a drug (*e.g.,* an immunosuppressant) or a radiotoxin. Examples of cytotoxin include but not limited to, taxanes (see, *e.g.,* International (PCT) Patent Application Nos. WO 01/38318 and PCT/US03/02675), DNA-alkylating agents (*e.g.,* CC-1065 analogs), anthracyclines, tubulysin analogs, duocarmycin analogs, auristatin E, auristatin F, maytansinoids, and cytotoxic agents comprising a reactive polyethylene glycol moiety (see, *e.g.,* Sasse et al., J. Antibiot. (Tokyo), 53, 879-85 (2000), Suzawa et al., Bioorg. Med. Chem., 8, 2175-84 (2000), Ichimura et al., J. Antibiot. (Tokyo), 44, 1045-53 (1991), Francisco et al., Blood (2003) (electronic publication prior to print publication), U.S. Pat. Nos. 5,475,092, 6,340,701, 6,372,738, and 6,436,931, U.S. Patent Application Publication No. 2001/0036923 A1, Pending U.S. patent application Ser. Nos. 10/024,290 and 10/116,053, and International (PCT) Patent Application No. WO 01/49698), taxol, cytochalasin B, gramicidin D, ethidium bromide, emetine, mitomycin, etoposide, tenoposide, colchicine, doxorubicin, daunorubicin, dihydroxy anthracin dione, mitoxantrone, mithramycin, actinomycin D, 1-dehydrotestosterone, glucocorticoids, and puromycin and analogs or homologs thereof. Therapeutic agents also include, for example, anti-metabolites (*e.g.*, methotrexate, 6-mercaptopurine, 6-thioguanine, cytarabine, 5-fluorouracil decarbazine), ablating agents (*e.g.*, mechlorethamine, thiotepa chlorambucil, meiphalan, carmustine (BSNU) and lomustine (CCNU), cyclophosphamide, busulfan, dibromomannitol, streptozotocin, mitomycin C, and cis-dichlorodiamine platinum (II) (DDP) cisplatin, anthracyclines (*e.g.,* daunorubicin (formerly daunomycin) and doxorubicin), antibiotics (e.g., dactinomycin

(formerly actinomycin), bleomycin, mithramycin, and anthramycin (AMC)), and anti-mitotic agents (*e.g.,* vincristine and vinblastine). (See *e.g.,* Seattle Genetics US20090304721).

Other examples of therapeutic cytotoxins that can be conjugated to the modified antibodies or antibody fragments of the present application include duocarmycins, calicheamicins, maytansines and auristatins, and derivatives thereof. An example of a calicheamicin antibody conjugate is commercially available (Mylotarg$^{Tm}$; Wyeth-Ayerst).

For further discussion of types of cytotoxins, linkers and methods for conjugating therapeutic agents to antibodies, see also Saito et al., (2003) Adv. Drug Deliv. Rev. 55:199-215; Trail et al., (2003) Cancer Immunol. Immunother. 52:328-337; Payne, (2003) Cancer Cell 3:207-212; Allen, (2002) Nat. Rev. Cancer 2:750-763; Pastan and Kreitman, (2002) Curr. Opin. Investig. Drugs 3:1089-1091; Senter and Springer, (2001) Adv. Drug Deliv. Rev. 53:247-264.

According to the present application, modified antibodies or antibody fragments thereof can also be conjugated to a radioactive isotope to generate cytotoxic radiopharmaceuticals, referred to as radioimmunoconjugates. Examples of radioactive isotopes that can be conjugated to antibodies for use diagnostically or therapeutically include, but are not limited to, iodine$^{131}$, indium$^{11}$, yttrium$^{90}$, and lutetium$^{177}$. Methods for preparing radioimmunoconjugates are established in the art. Examples of radioimmunoconjugates are commercially available, including Zevalin™ (DEC Pharmaceuticals) and Bexxar™ (Corixa Pharmaceuticals), and similar methods can be used to prepare radioimmunoconjugates using the antibodies of the present application. In certain embodiments, the macrocyclic chelator is 1,4,7,10-tetraazacyclododecane-N,N',N",N"'-tetraacetic acid (DOTA) which can be attached to the antibody via a linker molecule. Such linker molecules are commonly known in the art and described in Denardo et al., (1998) Clin. Cancer Res. 4(10):2483-90; Peterson et al., (1999) Bioconjug. Chem. 10(4):553-7; and Zimmerman et al., (1999) Nucl. Med. Biol. 26(8):943-50, each incorporated by reference in their entireties.

The present application further provides modified antibodies or fragments thereof that specifically bind to an antigen. The modified antibodies or fragments may be conjugated or fused to a heterologous protein or polypeptide (or fragment thereof, preferably to a polypeptide of at least 10, at least 20, at least 30, at least 40, at least 50, at least 60, at least 70, at least 80, at least 90 or at least 100 amino acids) to generate fusion proteins. In particular, the present application provides fusion proteins comprising an antibody fragment described herein (*e.g.,* a Fab fragment, Fd fragment, Fv fragment, F(ab)2 fragment, a $V_H$ domain, a $V_H$ CDR, a $V_L$ domain or a $V_L$ CDR) and a heterologous protein, polypeptide, or peptide.

In some embodiments, modified antibody fragments without antigen binding specificity, such as but not limited to, modified Fc domains with engineered cysteine residue(s) according to the present application, are used to generate fusion proteins comprising such an antibody fragment (e.g., engineered Fc) and a heterologous protein, polypeptide, or peptide.

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of antibodies of the present application or fragments thereof (*e.g.,* antibodies or fragments thereof with higher affinities and lower dissociation rates). See, generally, U.S. Patent Nos. 5,605,793, 5,811,238, 5,830,721, 5,834,252, and 5,837,458; Patten et al., (1997) Curr. Opinion Biotechnol. 8:724-33; Harayama, (1998) Trends Biotechnol. 16(2):76-82; Hansson et al., (1999) J. Mol. Biol. 287:265-76; and Lorenzo and Blasco, (1998) Biotechniques 24(2):308-313 (each of these patents and publications are hereby incorporated by reference in its entirety). Antibodies or fragments thereof, or the encoded antibodies or fragments thereof, may be altered by being subjected to random mutagenesis by error-prone PCR, random nucleotide insertion or other methods prior to recombination. A polynucleotide encoding an antibody or fragment thereof that specifically binds to an antigen may be recombined with one or more components, motifs, sections, parts, domains, fragments, etc. of one or more heterologous molecules.

Moreover, the modified antibodies or antibody fragments thereof of the present application can be conjugated to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., (1989) Proc. Natl. Acad. Sci. USA 86:821-824, for instance, hexa-histidine provides for convenient purification of the fusion protein. Other peptide tags useful for purification include, but are not limited to, the hemagglutinin ("HA") tag, which corresponds to an epitope derived from the influenza hemagglutinin protein (Wilson et al., (1984) Cell 37:767), and the "FLAG" tag (A. Einhauer et al., J. Biochem. Biophys. Methods 49: 455-465, 2001). According to the present application, antibodies or antibody fragments can also be conjugated to tumor-penetrating peptides in order to enhance their efficacy.

In other embodiments, modified antibodies or antibody fragments of the present application are conjugated to a diagnostic or detectable agent. Such immunoconjugates can be useful for monitoring or prognosing the onset, development, progression and/or severity of a disease or disorder as part of a clinical testing procedure, such as determining the efficacy of a particular therapy. Such diagnosis and detection can accomplished by coupling the antibody to detectable substances including, but not limited to, various enzymes, such as, but not limited to, horseradish peroxidase, alkaline phosphatase, beta-galactosidase, or acetylcholinesterase; prosthetic groups, such as, but not limited to, streptavidin/biotin and avidin/biotin; fluorescent materials, such as, but not limited to, Alexa Fluor 350, Alexa Fluor 405, Alexa Fluor

430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, Alexa Fluor 750, umbelliferone, fluorescein, fluorescein isothiocyanate, rhodamine, dichlorotriazinylamine fluorescein, dansyl chloride or phycoerythrin; luminescent materials, such as, but not limited to, luminol; bioluminescent materials, such as but not limited to, luciferase, luciferin, and aequorin; radioactive materials, such as, but not limited to, iodine ($^{131}I$, $^{125}I$, $^{123}I$, and $^{121}I$,), carbon ($^{14}C$) , sulfur ($^{35}S$), tritium ($^{3}H$), indium ($^{115}In$, $^{113}In$, $^{112}In$, and $^{111}In$,), technetium ($^{99}Tc$), thallium ($^{201}Ti$), gallium ($^{68}Ga$, $^{67}Ga$), palladium ($^{103}Pd$), molybdenum ($^{99}Mo$), xenon ($^{133}Xe$), fluorine ($^{18}F$), $^{153}Sm$, $^{177}Lu$, $^{159}Gd$, $^{149}Pm$, $^{140}La$, $^{175}Yb$, $^{166}Ho$, $^{90}Y$, 47Sc, $^{186}Re$, $^{188}Re$, $^{142}Pr$, $^{105}Rh$, $^{97}Ru$, $^{68}Ge$, $^{57}Co$, $^{65}Zn$, $^{85}Sr$, $^{32}P$, $^{153}Gd$, $^{169}Yb$, $^{51}Cr$, $^{54}Mn$, $^{75}Se$, $^{64}Cu$, $^{113}Sn$, and $^{117}Sn$; and positron emitting metals using various positron emission tomographies, and non-radioactive paramagnetic metal ions.

Modified antibodies or antibody fragments of the present application may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

**5. Pharmaceutical Composition**

To prepare pharmaceutical or sterile compositions including immunoconjugates, the immunoconjugates of the present application are mixed with a pharmaceutically acceptable carrier or excipient. The compositions can additionally contain one or more other therapeutic agents that are suitable for treating or preventing cancer (breast cancer, colorectal cancer, lung cancer, multiple myeloma, ovarian cancer, liver cancer, gastric cancer, pancreatic cancer, acute myeloid leukemia, chronic myeloid leukemia, osteosarcoma, squamous cell carcinoma, peripheral nerve sheath tumors (*e.g.,* schwannoma), head and neck cancer, bladder cancer, esophageal cancer, Barretts esophageal cancer, glioblastoma, clear cell sarcoma of soft tissue, malignant mesothelioma, neurofibromatosis, renal cancer, melanoma, prostate cancer, benign prostatic hyperplasia (BPH), gynacomastica, and endometriosis).

Formulations of therapeutic and diagnostic agents can be prepared by mixing with physiologically acceptable carriers, excipients, or stabilizers in the form of, *e.g.,* lyophilized powders, slurries, aqueous solutions, lotions, or suspensions (see, *e.g.,* Hardman et al., Goodman and Gilman's The Pharmacological Basis of Therapeutics, McGraw-Hill, New York, N.Y., 2001; Gennaro, Remington: The Science and Practice of Pharmacy, Lippincott, Williams, and Wilkins, New York, N.Y., 2000; Avis, et al. (eds.), Pharmaceutical Dosage Forms: Parenteral Medications, Marcel Dekker, NY, 1993; Lieberman, et al. (eds.), Pharmaceutical Dosage Forms: Tablets, Marcel Dekker, NY, 1990; Lieberman, et al. (eds.) Pharmaceutical Dosage Forms: Disperse Systems, Marcel Dekker, NY, 1990; Weiner and Kotkoskie, Excipient Toxicity and Safety, Marcel Dekker, Inc., New York, N.Y., 2000).

Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules is available (see, *e.g.*, Wawrzynczak, Antibody Therapy, Bios Scientific Pub. Ltd, Oxfordshire, UK, 1996; Kresina (ed.), Monoclonal Antibodies, Cytokines and Arthritis, Marcel Dekker, New York, N.Y., 1991; Bach (ed.), Monoclonal Antibodies and Peptide Therapy in Autoimmune Diseases, Marcel Dekker, New York, N.Y., 1993; Baert et al., New Engl. J. Med. 348:601-608, 2003; Milgrom et al., New Engl. J. Med. 341:1966-1973, 1999; Slamon et al., New Engl. J. Med. 344:783-792, 2001; Beniaminovitz et al., New Engl. J. Med. 342:613-619, 2000; Ghosh et al., New Engl. J. Med. 348:24-32, 2003; Lipsky et al., New Engl. J. Med. 343:1594-1602, 2000).

Determination of the appropriate dose is made by the clinician, *e.g.*, using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, *e.g.,* the inflammation or level of inflammatory cytokines produced.

Actual dosage levels of the active ingredients in the pharmaceutical compositions of the present application may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present application employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors known in the medical arts.

Compositions comprising antibodies or fragments thereof of the present application can be provided by continuous infusion, or by doses at intervals of, *e.g.,* one day, one week, or 1-7 times per week. Doses may be provided intravenously, subcutaneously, topically, orally, nasally, rectally, intramuscular, intracerebrally, or by inhalation. A specific dose protocol is one involving the maximal dose or dose frequency that avoids significant undesirable side effects.

For the immunoconjugates of the present application, the dosage administered to a patient may be 0.0001 mg/kg to 100 mg/kg of the patient's body weight. The dosage may be between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight. The dosage of the antibodies or fragments thereof of the present application may be calculated using the patient's weight in kilograms (kg) multiplied by the dose to be administered in mg/kg.

Doses of the immunoconjugates the present application may be repeated and the administrations may be separated by at least 1 day, 2 days, 3 days, 5 days, 10 days, 15 days, 30 days, 45 days, 2 months, 75 days, 3 months, or at least 6 months. In a specific embodiment, does of the immunoconjugates of the present application are repeated every 3 weeks.

An effective amount for a particular patient may vary depending on factors such as the condition being treated, the overall health of the patient, the method route and dose of administration and the severity of side effects (see, *e.g.,* Maynard et al., A Handbook of SOPs for Good Clinical Practice, Interpharm Press, Boca Raton, Fla., 1996; Dent, Good Laboratory and Good Clinical Practice, Urch Publ., London, UK, 2001).

The route of administration may be by, *e.g.,* topical or cutaneous application, injection or infusion by intravenous, intraperitoneal, intracerebral, intramuscular, intraocular, intraarterial, intracerebrospinal, intralesional, or by sustained release systems or an implant (see, *e.g.,* Sidman et al., Biopolymers 22:547-556, 1983; Langer et al., J. Biomed. Mater. Res. 15:167-277, 1981; Langer, Chem. Tech. 12:98-105, 1982; Epstein et al., Proc. Natl. Acad. Sci. USA 82:3688-3692, 1985; Hwang et al., Proc. Natl. Acad. Sci. USA 77:4030-4034, 1980; U.S. Pat. Nos. 6,350,466 and 6,316,024). Where necessary, the composition may also include a solubilizing agent and a local anesthetic such as lidocaine to ease pain at the site of the injection. In addition, pulmonary administration can also be employed, *e.g.,* by use of an inhaler or nebulizer, and formulation with an aerosolizing agent. See, *e.g.,* U.S. Pat. Nos. 6,019,968, 5,985,320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entirety.

A composition of the present application may also be administered via one or more routes of administration using one or more of a variety of methods known in the art. As will be appreciated by the skilled artisan, the route and/or mode of administration will vary depending upon the desired results. Selected routes of administration for the immunoconjugates of the present application include intravenous, intramuscular, intradermal, intraperitoneal, subcutaneous, spinal or other parenteral routes of administration, for example by injection or infusion. Parenteral administration may represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the present application can be administered via a non-parenteral route, such as a topical, epidermal or mucosal route of administration, for example, intranasally, orally, vaginally, rectally, sublingually or topically. In one embodiment, the immunoconjugates of the present application is administered by infusion. In another embodiment, the immunoconjugates of the present application is administered subcutaneously.

If the immunoconjugates of the present application are administered in a controlled release or sustained release system, a pump may be used to achieve controlled or sustained release (see Langer, supra; Sefton, CRC Crit. Ref Biomed. Eng. 14:20, 1987; Buchwald et al., Surgery 88:507, 1980; Saudek et al., N. Engl. J. Med. 321:574, 1989). Polymeric materials can be used to achieve controlled or sustained release of the therapies of the present application (see *e.g.,* Medical Applications of Controlled Release, Langer and Wise (eds.), CRC Pres., Boca Raton, Fla., 1974; Controlled Drug Bioavailability, Drug Product Design and Performance, Smolen and Ball (eds.), Wiley, New York, 1984; Ranger and Peppas, J. Macromol. Sci. Rev. Macromol. Chem. 23:61, 1983; see also Levy et al., Science 228:190, 1985; During et al., Ann. Neurol. 25:351, 1989; Howard et al., J. Neurosurg. 7 1:105, 1989; U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, *e.g.,* Goodson, in Medical Applications of Controlled Release, supra, vol. 2, pp. 115-138, 1984).

Controlled release systems are discussed in the review by Langer, Science 249:1527-1533, 1990). Any technique known to one of skill in the art can be used to produce sustained release formulations comprising one or more immunoconjugates of the present application. See, *e.g.,* U.S. Pat. No. 4,526,938, PCT publication WO 91/05548, PCT publication WO 96/20698, Ning et al., Radiotherapy & Oncology 39:179-189, 1996; Song et al., PDA Journal of Pharmaceutical Science & Technology 50:372-397, 1995; Cleek et al., Pro. Int'l. Symp. Control. Rel. Bioact. Mater. 24:853-854, 1997; and Lam et al., Proc. Int'l. Symp. Control Rel. Bioact. Mater. 24:759-760, 1997, each of which is incorporated herein by reference in their entirety.

If the immunoconjugates of the present application are administered topically, they can be formulated in the form of an ointment, cream, transdermal patch, lotion, gel, shampoo, spray, aerosol, solution, emulsion, or other form well-known to one of skill in the art. See, *e.g.,* Remington's Pharmaceutical Sciences and Introduction to Pharmaceutical Dosage Forms, 19th ed., Mack Pub. Co., Easton, Pa. (1995). For non-sprayable topical dosage forms, viscous to semi-solid or solid forms comprising a carrier or one or more excipients compatible with topical application and having a dynamic viscosity, in some instances, greater than water are typically employed. Suitable formulations include, without limitation, solutions, suspensions, emulsions, creams, ointments, powders, liniments, salves, and the like, which are, if desired, sterilized or mixed with auxiliary agents (*e.g.,* preservatives, stabilizers, wetting agents, buffers, or salts) for influencing various properties, such as, for example, osmotic pressure. Other suitable topical dosage forms include sprayable aerosol preparations wherein the active ingredient, in some instances, in combination with a solid or liquid inert carrier, is packaged in a mixture with a pressurized volatile (*e.g.,* a gaseous propellant, such as Freon™) or in a squeeze bottle. Moisturizers or humectants can also be added to pharmaceutical compositions and dosage forms if desired. Examples of such additional ingredients are well-known in the art.

If the compositions comprising the immunoconjugates are administered intranasally, it can be formulated in an aerosol form, spray, mist or in the form of drops. In particular, prophylactic or therapeutic agents for use according to the present application can be conveniently delivered in the form of an aerosol spray presentation from pressurized packs or a nebulizer, with a suitable propellant (*e.g.,* dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, *e.g.,* gelatin) for use in an inhaler or insufflator may be formulated containing a powder mix of the compound and a suitable powder base such as lactose or starch.

Methods for co-administration or treatment with a second therapeutic agent, *e.g.,* a cytokine, steroid, chemotherapeutic agent, antibiotic, or radiation, are known in the art (see, *e.g.,* Hardman et al., (eds.) (2001) Goodman and Gilman's The Pharmacological Basis of Therapeutics, 10th ed., McGraw-Hill, New York, N.Y.; Poole and Peterson (eds.) (2001) Pharmacotherapeutics for Advanced Practice: A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%; at least 40%, or at least 50%.

Additional therapies (*e.g.,* prophylactic or therapeutic agents), which can be administered in combination with the immunoconjugates of the present application may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the immunoconjugates of the present application. The two or more therapies may be administered within one same patient visit.

In certain embodiments, the immunoconjugates of the present application can be formulated to ensure proper distribution *in vivo.* For example, the blood-brain barrier (BBB) excludes many highly hydrophilic compounds. To ensure that the therapeutic compounds of the present application cross the BBB (if desired), they can be formulated, for example, in liposomes. For methods of manufacturing liposomes, see, *e.g.,* U.S. Pat. Nos. 4,522,811; 5,374,548; and 5,399,331. The liposomes may comprise one or more moieties which are selectively transported into specific cells or organs, thus enhance targeted drug delivery (see, *e.g.,* Ranade, (1989) J. Clin. Pharmacol. 29:685). Exemplary targeting moieties include folate or biotin (see, *e.g.,* U.S. Pat. No. 5,416,016 to Low et al.); mannosides (Umezawa et al., (1988) Biochem. Biophys. Res. Commun. 153:1038); antibodies (Bloeman et al., (1995) FEBS Lett. 357:140; Owais et al., (1995) Antimicrob. Agents Chemother. 39:180); surfactant protein A receptor (Briscoe et al., (1995) Am. J. Physiol. 1233:134); p 120 (Schreier et al, (1994) J. Biol. Chem. 269:9090); see also K. Keinanen; M. L. Laukkanen (1994) FEBS Lett. 346:123; J. J. Killion; I. J. Fidler (1994) Immunomethods 4:273.

The present application provides protocols for the administration of pharmaceutical composition comprising immunoconjugates of the present application alone or in combination with other therapies to a subject in need thereof.

The therapies (*e.g.,* prophylactic or therapeutic agents) of the combination therapies of the present application can be administered concomitantly or sequentially to a subject. The therapy (*e.g.,* prophylactic or therapeutic agents) of the combination therapies of the present application can also be cyclically administered. Cycling therapy involves the administration of a first therapy (*e.g.,* a first prophylactic or therapeutic agent) for a period of time, followed by the administration of a second therapy (*e.g.*, a second prophylactic or therapeutic agent) for a period of time and repeating this sequential administration, *i.e.*, the cycle, in order to reduce the development of resistance to one of the therapies (*e.g.,* agents) to avoid or reduce the side effects of one of the therapies (*e.g.,* agents), and/or to improve, the efficacy of the therapies.

The therapies (*e.g.,* prophylactic or therapeutic agents) of the combination therapies of the present application can be administered to a subject concurrently.

The term "concurrently" is not limited to the administration of therapies (*e.g.,* prophylactic or therapeutic agents) at exactly the same time, but rather it is meant that a pharmaceutical composition comprising antibodies or fragments thereof the present application are administered to a subject in a sequence and within a time interval such that the antibodies of the present application can act together with the other therapy or therapies to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (*e.g.,* prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (*e.g.,* prophylactic or therapeutic agents) are administered to a within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

The present application having been fully described, it is further illustrated by the following examples and claims, which are illustrative and are not meant to be further limiting.

EXAMPLES

**Example 1. Payload Compounds.**

Table 5 below lists structures of various payload compounds used in making antibody drug conjugates as described in the Examples in this application. Compounds A-E and methods of synthesizing the compounds, are disclosed, for example, in PCT/US2014/024795, and Compound F is disclosed, for example, in PCT/US2014/070800, both of which are incorporated herein by reference in their entirety. A synthetic method for Compound G is disclosed below.

**Compound G: Synthetic Procedure**

Synthesis of (S)-2-((2R,3R)-3-((S)-1-((3R,4S,5S)-4-((S)-2-((S)-2-(6-(2,5-dioxo-2,5-dihydro-1H-pyrrol-1-yl)-N-methylhexanamido)-3-methylbutanamido)-N,3-dimethylbutanamido)-3-methoxy-5-methylheptanoyl)pyrrolidin-2-yl)-3-methoxy-2-methylpropanamido)-3-phenylpropanoic acid **(MC-MMAF, Compound G)**

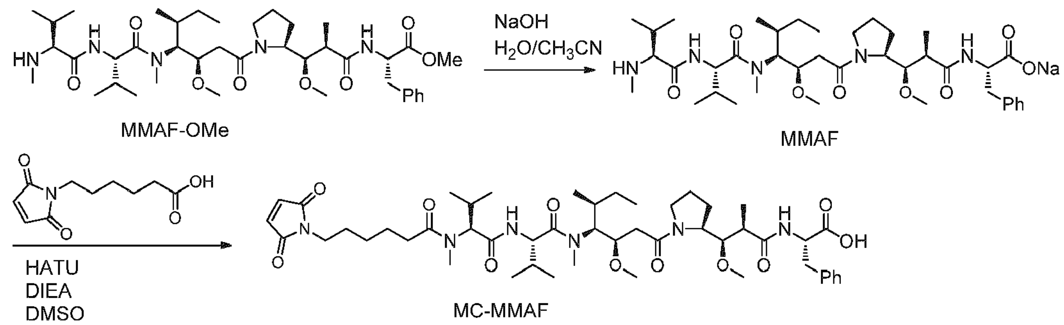

MMAF-OMe
MMAF
MC-MMAF

MMAF-OMe (135mg, Concortis Biosystems) was dissoved in CH3CN (10mL). To the resulting clear solution was added 5mL water, followed by 0.375mL of IN aqueous sodium hydroxide (certified, Fisher Scientific). The reaction mixture was stirred magnetically at 21°C for 18 hours, at which time LCMS analysis indicated a complete reaction. The reaction mixture mixture was frozen and lyophilized, affording MMAF sodium salt. LCMS retention time 0.911 minutes. MS (ESI+) m/z 732.5 (M+1). The whole MMAF sodium salt thus obtained in previous reaction was dissoved in 10mL DMSO. In a separate reaction vessel, 6-(2,5-dioxo-2,5-dihydro-1H-pyrrol-1-yl)hexanoic acid (95mg) was treated with HATU (165mg) and DIEA (0.126mL) in 3.0mL DMSO at at 21°C for 25min. The whole reaction mixture of the activated ester was added to the solution of MMAF sodium salt, and The reaction mixture was stirred at the same temperature for 3 hours. The reaction mixture mixture was partitioned between 40mL of EtOAc and 20 mL of 5% aqueous citric acid. The organic layer was separated, and the aqueous layer was extracted with 20mL of EtOAc. The combined organic layers were washed with 10mL saturated aqueous NaCl, dryed over anhydrous MgSO4, filtered and concentrated under reduced pressure. The residue was purified on an ISCO CombiFlash instrument using an ISCO C18gold 15.5g column. The desired material was eluted with 50% $CH_3CN$ in $H_2O$. The fractions containing the desired product was combined and lyophilized, affording compound as white solid. LCMS retention time 1.392 minutes. MS (ESI+) m/z 925.6 (M+1).

Table 5: Linker-payloads tested.

| Compound | Structure |
|---|---|
| Compound A (Eg5 inhibitor) | 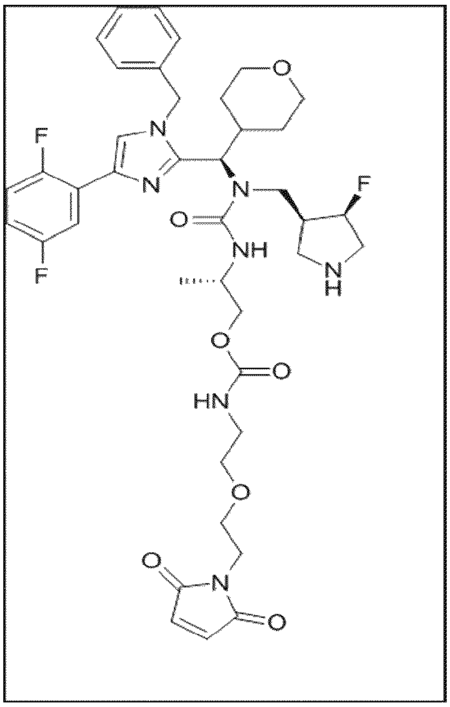 |
| Compound B (Eg5 inhibitor) | 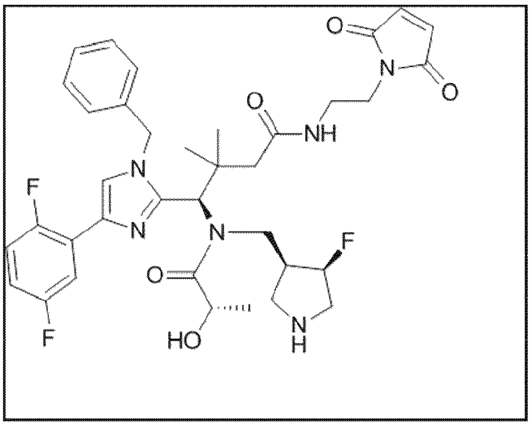 |

(continued)

| Compound | Structure |
| --- | --- |
| Compound C (Eg5 inhibitor) | 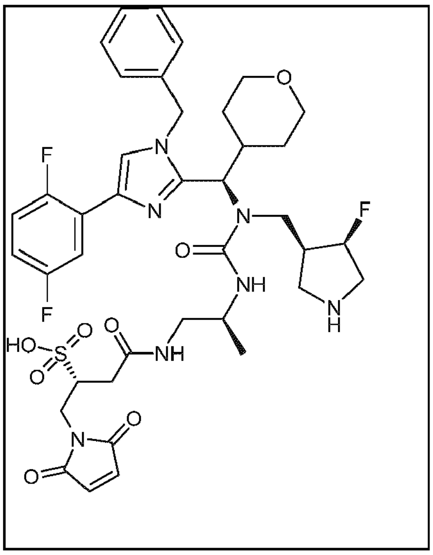 |
| Compound D (Eg5 inhibitor) | 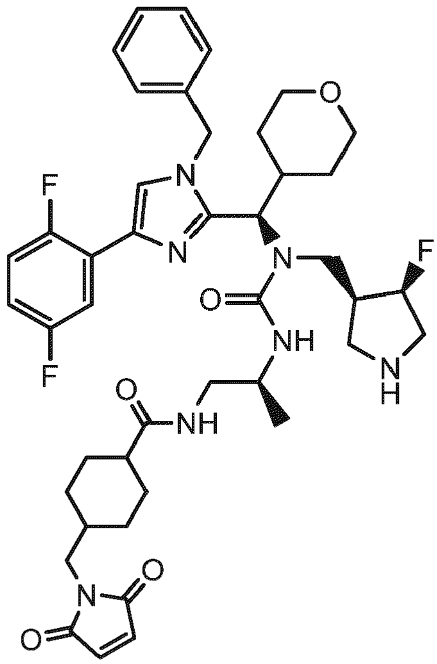 |
| Compound E (Eg5 inhibitor) | 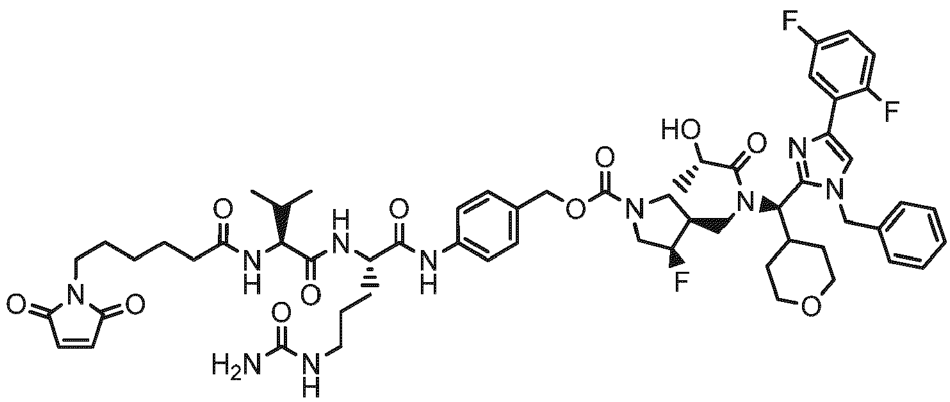 |

(continued)

| Compound | Structure |
|---|---|
| Compound F (cytotoxic peptide) | 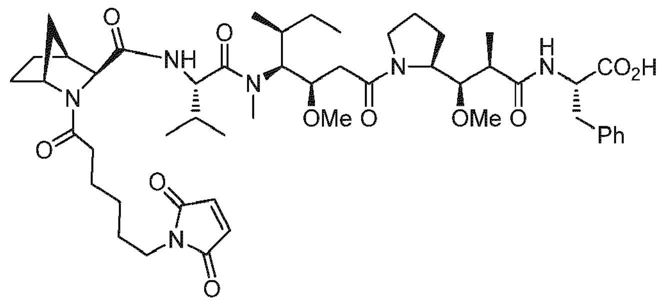 |
| Compound G (MMAF) | 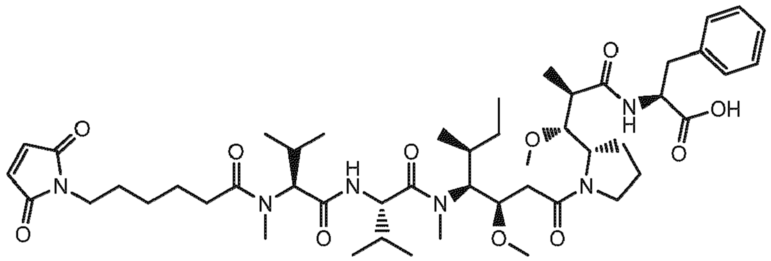 |

## Example 2. Preparation of trastuzumab Cys mutant antibodies.

DNA encoding variable regions of heavy and light chains of trastuzumab, an anti-HER2 antibody (the terms "trastuzumab," "anti-HER2 antibody," and "TBS" are used interchangeably herein), were chemically synthesized and cloned into two mammalian expression vectors, pOG-HC and pOG-LC that contain constant regions of human IgG1 and human kappa light chain, resulting in two wild type constructs, pOG-trastuzumab HC and pOG-trastuzumab LC, respectively. In the vectors the expression of antibody heavy and light chain constructs in mammalian cells is driven by a CMV promoter. The vectors contain a synthetic 24 amino acid signal sequence: MKTFILLLWVLLLWVIFLLPGATA (SEQ ID NO: 99), in the N-terminal of heavy chain or light chain to guide their secretion from mammalian cells. The signal sequence has been validated to be efficient in directing protein secretion in hundreds of mammalian proteins in 293 Freestyle™ cells.

Oligonucleotide directed mutagenesis was employed to prepare Cys mutant constructs in trastuzumab. Pairs of mutation primers were chemically synthesized for each Cys mutation site (Table 6). The sense and anti-sense mutation primer pairs were mixed prior to PCR amplification. PCR reactions were performed by using PfuUltra II Fusion HS DNA Polymerase (Stratagene) with pOG-trastuzumab HC and pOG-trastuzumab LC as the templates. After PCR reactions, the PCR products were confirmed on agarose gels, and treated with Dpn I followed by transformation in DH10b cells (Klock et al., (2009) Methods Mol Biol. 498:91-103).

Table 6. DNA sequences of mutation primers used to prepare 11 individual Cys mutations heavy and light chains of human IgG1.

| Mutation sites | Primer name | Sequence | SEQ ID NO. |
|---|---|---|---|
| LC-K107C | LC-CYS-S1 | GTGGAGATCTGTCGAACGGTGGCCGCTCCCAGCGTGTTCA | 100 |
| | LC-CYS-A1 | ACCGTTCGACAGATCTCCACCTTGGTACCCTGTCCGAAC | 101 |
| LC-S159C | LC-CYS-S18 | AGCGGCAACTGTCAGGAGAGCGTCACCGAGCAGGACAGCAA | 102 |
| | LC-CYS-A18 | CTCTCCTGACAGTTGCCGCTCTGCAGGGCGTTGTCCACCT | 103 |

(continued)

| Mutation sites | Primer name | Sequence | SEQ ID NO. |
|---|---|---|---|
| LC-E165C | LC-CYS-S20 | GAGCGTCACCTGTCAGGACAGCAAGGACTCCACCTACAGC | 104 |
| | LC-CYS-A20 | CTGTCCTGACAGGTGACGCTCTCCTGGCTGTTGCCGCTCT | 105 |
| HC-E152C | HC-CYS-S9 | TACTTCCCTGTCCCGTGACCGTGTCCTGGAACAGCGGA | 106 |
| | HC-CYS-A9 | GGTCACGGGACAGGGGAAGTAGTCCTTCACCAGGCAGC | 107 |
| HC-P171C | HC-CYS-S16 | CACACCTTCTGTGCCGTGCTGCAGAGCAGCGGCCTGTACA | 109 |
| | HC-CYS-A16 | CAGCACGGCACAGAAGGTGTGCACGCCGGAGGTCAGGGCT | 110 |
| HC-P247C | HC-CYS-S247 | CTGTTCCCACCCAAGTCTAAGGACACCCTGATGATCAG | 111 |
| | HC-CYS-A247 | CTTGGGTGGGAACAGGAACACGGAGGGTCCGCCCAG | 112 |
| HC-A327C | HC-CYS-S327 | TGCAAGGTCTCCAACAAGTGTCTGCCAGCCCCCATCGAAAAG | 113 |
| | HC-CYS-A327 | GTTGGAGACCTTGCACTTGTATTCCTTGCCGTTCAGCCAG | 114 |
| HC-K334C | HC-CYS-S46 | CCCATCGAATGCACCATCAGCAAGGCCAAGGGCCAGCCA | 115 |
| | HC-CYS-A46 | GCTGATGGTGCATTCGATGGGGGCTGGCAGGGCCTTGTTG | 116 |
| HC-A339C | HC-CYS-S339 | CTTGCTGATGGTCTTTTCGATGGGGGCTGGCAGGGCCTTG | 117 |
| | HC-CYS-A339 | AAGACCATCAGCAAGTGTAAGGGCCAGCCACGGGAG | 118 |
| HC-K360C | HC-CYS-S52 | AGCTGACCTGCAACCAGGTGTCCCTGACCTGTCTGGTGA | 119 |
| | HC-CYS-A52 | CACCTGGTTGCAGGTCAGCTCGTCCCGGGATGGAGGCAGG | 120 |
| HC-Y373C | HC-CYS-S373 | CTGGTGAAGGGCTTCTGTCCCAGCGACATCGCCGTGGAGTG | 121 |
| | HC-CYS-A373 | GAAGCCCTTCACCAGACAGGTCAGGGACACCTGGTTCTTG | 122 |

(continued)

| Mutation sites | Primer name | Sequence | SEQ ID NO. |
|---|---|---|---|
| HC-S375C | HC-CYS-S54 | TTCTACCCCTGCGACATCGCCGTGGAGTGGGAGAGCAACG | 123 |
| | HC-CYS-A54 | GGCGATGTCGCAGGGGTAGAAGCCCTTCACCAGACAGGTCA | 124 |
| HC-Y391C | HC-CYS-S391 | AACAACTGTAAGACCACACCTCCAGTGCTGGACAGCGAC | 125 |
| | HC-CYS-A391 | GGTCTTACAGTTGTTCTCGGGCTGGCCGTTGCTCTCCCAC | 126 |
| HC-P396C | HC-CYS-S396 | ACACCTTGTGTGCTGGACAGCGACGGCAGCTTCTTCCTG | 127 |
| | HC-CYS-A396 | CAGCACACAAGGTGTGGTCTTGT AGTTGTTCTCGGGCTG | 128 |

In some cases, two or more mutations were made in the same chain of trastuzumab. Oligonucleotide directed mutagenesis was employed to prepare the multiple Cys mutant constructs using the same method as above but using a pOG-trastuzumab-Cys mutant plasmid as the template for serial rounds of mutagenesis.

Sequences of all Cys mutant constructs were confirmed by DNA sequencing. The encoded protein sequence of the constant region of the HC and LC Cys mutant IgG1 constructs are shown in Table 7 and Table 8, respectively. Amino acid residues in human IgG1 heavy chain and human kappa light chain are numbered by EU numbering system (Edelman et al, (1969) Proc Natl Acad Sci USA, 63:78-85).

Table 7. Amino acid sequences of the constant region of Cys mutant constructs in human IgG1 heavy chain. SEQ ID NO:1 is the sequence for full-length trastuzumab with the constant region underlined. Additional sequences are Cys mutant constructs in human IgG1 heavy chain, showing only the sequences of the constant region. The mutant cys positions are shown by bold and underlined text.

| |
|---|
| SEQ ID NO: 1<br>EVQLVESGGGLVQPGGSLRLSCAASGFNIKDTYIHWVRQAPGKGLEWV ARIYPTNGYTRYADSVKGRFTISADTSKNTAYLQMNSLRAEDTAVYYC SRWGGDGFYAMDYWGQGTLVTVSSASTKGPSVFPLAPSSKSTSGGTAA LGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPS VFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKT ISKAKGQPREPQVYTLPPSREEMTKNQVSLTCLVKGFYPSDIAVEWESN GQPENNYKTTPPVLDSDGSFFLYSKLTVDKSRWQQGNVFSCSVMHEAL HNHYTQKSLSLSPGK |
| SEQ ID NO: 10 (Cysteine substitution at position 152)<br>SASTKGPSVFPLAPSSKSTSGGTAALGCLVKDYFP**C**PVTVSWNSGALTS GVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQ DWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSK LTVDKSRWQQGNVFSCSVMHEALHNHYTQKSLSLSPGK |
| SEQ ID NO: 18 (Cysteine substitution at position 174) |

(continued)

| |
|---|
| SASTKGPSVFPLAPSSKSTSGGTAALGCLVKDYFPEPVTVSWNSGALTS<br>GVHTFPAV**C**QSSGLYSLSSVVTVPSSSLGTQTYICNVNHKPSNTKVDKK<br>VEPKSCDKTHTCPPCPAPELLGGPSVFLFPPKPKDTLMISRTPEVTCVVV<br>DVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTK<br>NQVSLTCLVKGFYPSDIAVEWESNGQPENNYKTTPPVLDSDGSFFLYSK<br>LTVDKSRWQQGNVFSCSVMHEALHNHYTQKSLSLSPGK |
| SEO ID NO:42 (Cysteine substitution at position 333)<br>SASTKGPSVFPLAPSSKSTSGGTAALGCLVKDYFPEPVTVSWNSGALTS<br>GVHTFPAVLQSSGLYSLSSVVTVPSSSLGTQTYICNVNHKPSNTKVDKK<br>VEPKSCDKTHTCPPCPAPELLGGPSVFLFPPKPKDTLMISRTPEVTCVVV<br>DVSHEDPEVKFNWYVDGVEVHNAKTKPREEQYNSTYRVVSVLTVLHQ<br>DWLNGKEYKCKVSNKALPAPI**C**KTISKAKGQPREPQVYTLPPSREEMT<br>KNQVSLTCLVKGFYPSDIAVEWESNGQPENNYKTTPPVLDSDGSFFLYS<br>KLTVDKSRWQQGNVFSCSVMHEALHNHYTQKSLSLSPGK |
| SEO ID NO:48 (Cysteine substitution at position 360)<br>SASTKGPSVFPLAPSSKSTSGGTAALGCLVKDYFPEPVTVSWNSGALTS<br>GVHTFPAVLQSSGLYSLSSVVTVPSSSLGTQTYICNVNHKPSNTKVDKK<br>VEPKSCDKTHTCPPCPAPELLGGPSVFLFPPKPKDTLMISRTPEVTCVVV<br>DVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMT**C**<br>NQVSLTCLVKGFYPSDIAVEWESNGQPENNYKTTPPVLDSDGSFFLYSK<br>LTVDKSRWQQGNVFSCSVMHEALHNHYTQKSLSLSPGK |
| SEQ ID NO:50 (Cysteine substitution at position 375)<br>SASTKGPSVFPLAPSSKSTSGGTAALGCLVKDYFPEPVTVSWNSGALTS<br>GVHTFPAVLQSSGLYSLSSVVTVPSSSLGTQTYICNVNHKPSNTKVDKK<br>VEPKSCDKTHTCPPCPAPELLGGPSVFLFPPKPKDTLMISRTPEVTCVVV<br>DVSHEDPEVKFNWYVDGVEVHNAKTKPREEQYNSTYRVVSVLTVLHQ<br>DWLNGKEYKCKVSNKALPAPIEKTISKAKGQPREPQVYTLPPSREEMTK<br>NQVSLTCLVKGFYP**C**DIAVEWESNGQPENNYKTTPPVLDSDGSFFLYSK<br>LTVDKSRWQQGNVFSCSVMHEALHNHYTQKSLSLSPGK |
| SEQ ID NO 129: (Cysteine substitution at positions 334 and 375)<br>SASTKGPSVFPLAPSSKSTSGGTAALGCLVKDYFPEPVTVSWNSGALTSG<br>VHTFPAVLQSSGLYSLSSVVTVPSSSLGTQTYICNVNHKPSNTKVDKKVEP<br>KSCDKTHTCPPCPAPELLGGPSVFLFPPKPKDTLMISRTPEVTCVVVDVSH<br>EDPEVKFNWYVDGVEVHNAKTKPREEQYNSTYRVVSVLTVLHQDWLNG<br>KEYKCKVSNKALPAPIE**C**TISKAKGQPREPQVYTLPPSREEMTKNQVSLT<br>CLVKGFYP**C**DIAVEWESNGQPENNYKTTPPVLDSDGSFFLYSKLTVDKSR<br>WQQGNVFSCSVMHEALHNHYTQKSLSLSPGK |
| SEQ ID NO: 130 (Cysteine substitution at positions 334 and 392)<br>SASTKGPSVFPLAPSSKSTSGGTAALGCLVKDYFPEPVTVSWNSGALTSG<br>VHTFPAVLQSSGLYSLSSVVTVPSSSLGTQTYICNVNHKPSNTKVDKKVEP<br>KSCDKTHTCPPCPAPELLGGPSVFLFPPKPKDTLMISRTPEVTCVVVDVSH<br>EDPEVKFNWYVDGVEVHNAKTKPREEQYNSTYRVVSVLTVLHQDWLNG<br>KEYKCKVSNKALPAPIE**C**TISKAKGQPREPQVYTLPPSREEMTKNQVSLT<br>CLVKGFYPSDIAVEWESNGQPENNY**C**TTPPVLDSDGSFFLYSKLTVDKSR<br>WQQGNVFSCSVMHEALHNHYTQKSLSLSPGK |
| SEQ ID NO: 131 (Cysteine substitution at positions 152 and 375) |

(continued)

| |
|---|
| SASTKGPSVFPLAPSSKSTSGGTAALGCLVKDYFP**C**PVTVSWNSGALTSG<br>VHTFPAVLQSSGLYSLSSVVTVPSSSLGTQTYICNVNHKPSNTKVDKKVEP<br>KSCDKTHTCPPCPAPELLGGPSVFLFPPKPKDTLMISRTPEVTCVVVDVSH<br>EDPEVKFNWYVDGVEVHNAKTKPREEQYNSTYRVVSVLTVLHQDWLNG<br>KEYKCKVSNKALPAPIEKTISKAKGQPREPQVYTLPPSREEMTKNQVSLT<br>CLVKGFYP**C**DIAVEWESNGQPENNYKTTPPVLDSDGSFFLYSKLTVDKSR<br>WQQGNVFSCSVMHEALHNHYTQKSLSLSPGK |
| SEQ ID NO: 132 (Cysteine substitution at positions 339 and 396) |
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISK**C**KGQPREPQ<br>VYTLPPSREEMTKNQVSLTCLVKGFYPSDIAVEWESNGQPENNYKT<br>TP**C**VLDSDGSFFLYSKLTVDKSRWQQGNVFSCSVMHEALHNHYTQ<br>KSLSLSPGK |
| SEQ ID NO: 133 (Cysteine substitution at positions 152 and 171) |
| SASTKGPSVFPLAPSSKSTSGGTAALGCLVKDYFP**C**PVTVSWNSGA<br>LTSGVHTF**C**AVLQSSGLYSLSSVVTVPSSSLGTQTYICNVNHKPSNT<br>KVDKKVEPKSCDKTHTCPPCPAPELLGGPSVFLFPPKPKDTLMISRT<br>PEVTCVVVDVSHEDPEVKFNWYVDGVEVHNAKTKPREEQYNSTY<br>RVVSVLTVLHQDWLNGKEYKCKVSNKALPAPIEKTISKAKGQPREP<br>QVYTLPPSREEMTKNQVSLTCLVKGFYPSDIAVEWESNGQPENNYK<br>TTPPVLDSDGSFFLYSKLTVDKSRWQQGNVFSCSVMHEALHNHYT<br>QKSLSLSPGK |
| SEQ ID NO: 134 (Cystine substitution at position 334 and 396) |
| SEQ ID NO: 134 (Cysteine substitution at positions 334 and 396)<br>SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIE**C**TISKAKGQPREPQ<br>VYTLPPSREEMTKNQVSLTCLVKGFYPSDIAVEWESNGQPENNYKT<br>TP**C**VLDSDGSFFLYSKLTVDKSRWQQGNVFSCSVMHEALHNHYTQ<br>KSLSLSPGK |
| SEQ ID NO: 135 (Cysteine substitution at position 396) |
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISKAKGQPREPQ<br>VYTLPPSREEMTKNQVSLTCLVKGFYPSDIAVEWESNGQPENNYKT<br>TP**C**VLDSDGSFFLYSKLTVDKSRWQQGNVFSCSVMHEALHNHYTQ<br>KSLSLSPGK |
| SEQ ID NO: 136 (Cysteine substitution at positions 375 and 396) |

(continued)

| |
|---|
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISKAKGQPREPQ<br>VYTLPPSREEMTKNQVSLTCLVKGFYP**C**DIAVEWESNGQPENNYKT<br>TP**C**VLDSDGSFFLYSKLTVDKSRWQQGNVFSCSVMHEALHNHYTQ<br>KSLSLSPGK |
| SEQ ID NO: 137 (Cysteine substitution at positions 375 and 391) |
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISKAKGQPREPQ<br>VYTLPPSREEMTKNQVSLTCLVKGFYP**C**DIAVEWESNGQPENN**C**KT<br>TPPVLDSDGSFFLYSKLTVDKSRWQQGNVFSCSVMHEALHNHYTQ<br>KSLSLSPGK |
| SEO ID NO: 138 (Cysteine substitution at positions 391 and 396) |
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISKAKGQPREPQ<br>VYTLPPSREEMTKNQVSLTCLVKGFYPSDIAVEWESNGQPENN**C**KT<br>TP**C**VLDSDGSFFLYSKLTVDKSRWQQGNVFSCSVMHEALHNHYTQ<br>KSLSLSPGK |
| SEQ ID NO: 139 (Cysteine substitution at positions 152 and 396) |
| SASTKGPSVFPLAPSSKSTSGGTAALGCLVKDYFP**C**PVTVSWNSGA<br>LTSGVHTFPAVLQSSGLYSLSSVVTVPSSSLGTQTYICNVNHKPSNT<br>KVDKKVEPKSCDKTHTCPPCPAPELLGGPSVFLFPPKPKDTLMISRT<br>PEVTCVVVDVSHEDPEVKFNWYVDGVEVHNAKTKPREEQYNSTY<br>RVVSVLTVLHQDWLNGKEYKCKVSNKALPAPIEKTISKAKGQPREP<br>QVYTLPPSREEMTKNQVSLTCLVKGFYPSDIAVEWESNGQPENNYK<br>TTP**C**VLDSDGSFFLYSKLTVDKSRWQQGNVFSCSVMHEALHNHYT<br>QKSLSLSPGK |
| SEQ ID NO: 140 (Cysteine substitution at positions 327 and 339) |
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNK**C**LPAPIEKTISK**C**KGQPREPQ<br>VYTLPPSREEMTKNQVSLTCLVKGFYPSDIAVEWESNGQPENNYKT<br>TPPVLDSDGSFFLYSKLTVDKSRWQQGNVFSCSVMHEALHNHYTQ<br>KSLSLSPGK |
| SEQ ID NO: 141 (Cysteine substitution at position 391) |

(continued)

| |
|---|
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISKAKGQPREPQ<br>VYTLPPSREEMTKNQVSLTCLVKGFYPSDIAVEWESNGQPENN**C**KT<br>TPPVLDSDGSFFLYSKLTVDKSRWQQGNVFSCSVMHEALHNHYTQ<br>KSLSLSPGK |
| SEQ ID NO: 142 (Cysteine substitution at positions 152 and 339) |
| SASTKGPSVFPLAPSSKSTSGGTAALGCLVKDYFP**C**PVTVSWNSGA<br>LTSGVHTFPAVLQSSGLYSLSSVVTVPSSSLGTQTYICNVNHKPSNT<br>KVDKKVEPKSCDKTHTCPPCPAPELLGGPSVFLFPPKPKDTLMISRT<br>PEVTCVVVDVSHEDPEVKFNWYVDGVEVHNAKTKPREEQYNSTY<br>RVVSVLTVLHQDWLNGKEYKCKVSNKALPAPIEKTISK**C**KGQPREP<br>QVYTLPPSREEMTKNQVSLTCLVKGFYPSDIAVEWESNGQPENNYK<br>TTPPVLDSDGSFFLYSKLTVDKSRWQQGNVFSCSVMHEALHNHYT<br>QKSLSLSPGK |
| SEO ID NO: 143 (Cysteine substitution at positions 339 and 375) |
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISK**C**KGQPREPQ<br>VYTLPPSREEMTKNQVSLTCLVKGFYP**C**DIAVEWESNGQPENNYKT<br>TPPVLDSDGSFFLYSKLTVDKSRWQQGNVFSCSVMHEALHNHYTQ<br>KSLSLSPGK |
| SEQ ID NO: 144 (Cysteine substitution at positions 152 and 327) |
| SASTKGPSVFPLAPSSKSTSGGTAALGCLVKDYFP**C**PVTVSWNSGA<br>LTSGVHTFPAVLQSSGLYSLSSVVTVPSSSLGTQTYICNVNHKPSNT<br>KVDKKVEPKSCDKTHTCPPCPAPELLGGPSVFLFPPKPKDTLMISRT<br>PEVTCVVVDVSHEDPEVKFNWYVDGVEVHNAKTKPREEQYNSTY<br>RVVSVLTVLHQDWLNGKEYKCKVSNK**C**LPAPIEKTISKAKGQPREP<br>QVYTLPPSREEMTKNQVSLTCLVKGFYPSDIAVEWESNGQPENNYK<br>TTPPVLDSDGSFFLYSKLTVDKSRWQQGNVFSCSVMHEALHNHYT<br>QKSLSLSPGK |
| SEQ ID NO: 145 (Cysteine substitution at position 373) |
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISKAKGQPREPQ<br>VYTLPPSREEMTKNQVSLTCLVKGF**C**PSDIAVEWESNGQPENNYKT<br>TPPVLDSDGSFFLYSKLTVDKSRWQQGNVFSCSVMHEALHNHYTQ<br>KSLSLSPGK |
| SEQ ID NO: 146 (Cysteine substitution at positions 327 and 375) |

(continued)

| |
|---|
| SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNK**C**LPAPIEKTISKAKGQPREPQ<br>VYTLPPSREEMTKNQVSLTCLVKGFYP**C**DIAVEWESNGQPENNYKT<br>TPPVLDSDGSFFLYSKLTVDKSRWQQGNVFSCSVMHEALHNHYTQ<br>KSLSLSPGK |
| SEQ ID NO: 147 (Cysteine substitution at position 247)<br>SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPK**C**KDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISKAKGQPREPQ<br>VYTLPPSREEMTKNQVSLTCLVKGFYPSDIAVEWESNGQPENNYKT<br>TPPVLDSDGSFFLYSKLTVDKSRWQQGNVFSCSVMHEALHNHYTQ<br>KSLSLSPGK |
| SEQ ID NO:150 (Constant region of the wild type heavy chain of anti-cKIT and anti-Her2 antibodies)<br>SASTKGPSVFPLAPSSKSTSGGTAALGCLVKDYFPEPVTVSWNSGAL<br>TSGVHTFPAVLQSSGLYSLSSVVTVPSSSLGTQTYICNVNHKPSNTK<br>VDKKVEPKSCDKTHTCPPCPAPELLGGPSVFLFPPKPKDTLMISRTP<br>EVTCVVVDVSHEDPEVKFNWYVDGVEVHNAKTKPREEQYNSTYR<br>VVSVLTVLHQDWLNGKEYKCKVSNKALPAPIEKTISKAKGQPREPQ<br>VYTLPPSREEMTKNQVSLTCLVKGFYPSDIAVEWESNGQPENNYKT<br>TPPVLDSDGSFFLYSKLTVDKSRWQQGNVFSCSVMHEALHNHYTQ<br>KSLSLSPGK |

Table 8. Amino acid sequences of the constant region of 3 human kappa light chain Cys mutant constructs. SEQ ID NO:90 sequence for full-length trastuzumab (human kappa light chain) with the constant region underlined. Additional sequences are the sequence ID numbers for Cys mutant constructs in the constant region of human kappa light chain.

| |
|---|
| SEQ ID NO:90 (anti-Her2 light chain)<br>DIQMTQSPSSLSASVGDRVTITCRASQDVNTAVAWYQQKPGKAPKL<br>LIYSASFLYSGVPSRFSGSRSGTDFTLTISSLQPEDFATYYCQQHYTTP<br>PTFGQGTKVEIKRTVAAPSVFIFPPSDEQLKSGTASVVCLLNNFYPRE<br>AKVQWKVDNALQSGNSQESVTEQDSKDSTYSLSSTLTLSKADYEKH<br>KVYACEVTHQGLSSPVTKSFNRGEC |
| SEQ ID NO:61 (Cysteine substitution at position 107)<br>**C**RTVAAPSVFIFPPSDEQLKSGTASVVCLLNNFYPREAKVQWKVDNA<br>LQSGNSQESVTEQDSKDSTYSLSSTLTLSKADYEKHKVYACEVTHQG<br>LSSPVTKSFNRGEC |
| SEQ ID NO:75 (Cysteine substitution at position 159)<br>KRTVAAPSVFIFPPSDEQLKSGTASVVCLLNNFYPREAKVQWKVDNAL<br>QSGN**C**QESVTEQDSKDSTYSLSSTLTLSKADYEKHKVYACEVTHQGLS<br>SPVTKSFNRGEC |
| SEQ ID NO:77 (Cysteine substitution at position 165) |

(continued)

| |
|---|
| KRTVAAPSVFIFPPSDEQLKSGTASVVCLLNNFYPREAKVQWKVDNA LQSGNSQESVT**C**QDSKDSTYSLSSTLTLSKADYEKHKVYACEVTHQG LSSPVTKSFNRGEC |
| SEO ID NO: 148 (Cvsteine substitution at positions 159 and 165) |
| KRTVAAPSVFIFPPSDEQLKSGTASVVCLLNNFYPREAKVQWKVDNA LQSGN**C**QESVT**C**QDSKDSTYSLSSTLTLSKADYEKHKVYACEVTHQ GLSSPVCKSFNRGEC |
| SEQ ID NO:149 (Constant region of wild type light chain for anti-Her2 and anti-cKIT) |
| KRTVAAPSVFIFPPSDEQLKSGTASVVCLLNNFYPREAKVQWKVDNA LQSGNSQESVTEQDSKDSTYSLSSTLTLSKADYEKHKVYACEVTHQG LSSPVTKSFNRGEC |

Example 3. Transfer of the trastuzumab heavy chain and light chain Cys mutations to different antibodies.

For trastuzumab, all Cys mutations for the attachment of drug payloads were chosen to be in the constant region of its human IgG1 heavy and human kappa light chain. Because constant regions of antibodies are highly conserved in primary sequence and structure, Cys mutant residues that are identified as good payload attachment sites in the context of trastuzumab will also serve as preferred attachment residues in other antibodies. To demonstrate the transferability of these generic conjugation sites to other antibodies, we cloned a set of Cys mutations into an anti-cKIT antibody. The anti-cKIT antibody is an antibody with a human IgG1 heavy chain and a human kappa light chain that binds to the cKIT protein. The DNA encoding variable regions of antibody anti-cKIT were cloned into three selected pOG trastuzumab HC Cys mutant plasmid constructs and two selected pOG trastuzumab LC Cys mutant plasmid constructs (SEQ ID NOs listed in Table 9) to replace the variable regions of trastuzumab constructs in the plasmids as described in Example 2. As a result, the amino acid sequences of the heavy chain and light chain constant regions in corresponding five Cys constructs of the anti-cKIT antibody and of trastuzumab are identical. Subsequent examples show that these sites can be conjugated readily. Conversely, due to a high degree of similarity in primary sequences and in tertiary structures for different human IgG isotypes, Cys mutations on the kappa light chain of trastuzumab can readily be transferred to equivalent light chains on human antibodies containing different isotype heavy chains. In the same way, the sites identified in the constant region of IgG1 may be transferred to IgG2, IgG3 and IgG4.

Table 9. Sequence ID numbers of trastuzumab Cys constructs used for cloning of the variable region of the anti-cKIT antibody.

| Sequence ID NO: of trastuzumab Cys construct |
|---|
| SEQ ID NO:48 |
| SEQ ID NO:61 |
| SEQ ID NO:77 |
| SEQ ID NO:129 |
| SEQ ID NO:131 |

**Example 4. Expression and purification of Cys mutant antibodies in 293 Freestyle™ cells.**

Antibody conjugates produced through conjugation to lysine residues or partially reduced native disulfide bonds often feature drug-to-antibody-ratios (DAR) of between 3 and 4. Cys engineered antibodies more typically feature a DAR of 2. For certain indications, it may be desirable to produce ADCs with higher DAR which can in principle be achieved by introducing multiple Cys mutations in the antibody. As the number of Cys mutation increases, the likelihood that such mutations interfere with the required re-oxidation process during ADC preparation and hence result in heterogeneous products also increases. In this study, a large number of single site heavy and light chain Cys mutants with good re-oxidation behavior were identified. To demonstrate that several conjugation sites can be combined for the production of ADCs with DAR greater than two, several single site Cys constructs of light and heavy chain of trastuzumab and anti-cKIT antibody (Table 10) were co-expressed in 293 Freestyle™ cells.

Table 10. Sequence IDs for constant regions of Cys engineered antibodies resulting in ADCs with DAR of 4 or 6

| .Cys-drug ADC (DAR=4 to 6) | LC SEQ ID NO | HC SEQ ID NO | Target DAR |
|---|---|---|---|
| anti-cKIT-HC-E152C-S375C | 149 | 131 | 4 |
| trastuzumab-HC-E152C-S375C | 149 | 131 | 4 |
| anti-cKIT -HC-K360C-LC-K107C | 61 | 48 | 4 |
| trastuzumab-HC-K360C-LC-K107C | 61 | 48 | 4 |
| trastuzumab-HC-A339C-P396C | 149 | 132 | 4 |
| trastuzumab-HC-E152C-LC-E165C | 77 | 10 | 4 |
| trastuzumab-HC-E152C-LC-S 159C-E165C | 148 | 10 | 6 |
| trastuzumab-HC-E152C-P171C | 149 | 133 | 4 |
| trastuzumab-HC-K334C-P396C | 149 | 134 | 4 |
| trastuzumab-HC-K334C-S375C | 149 | 129 | 4 |
| anti-cKIT-HC-K334C-S375C-LC-E165C | 77 | 129 | 6 |
| trastuzumab-HC-P396C-LC-E165C | 77 | 135 | 4 |
| trastuzumab-HC-S375C-P396C | 149 | 136 | 4 |
| trastuzumab-HC-S375C-Y391C | 149 | 137 | 4 |
| trastuzumab-HC-Y391C-P396C | 149 | 138 | 4 |
| trastuzumab-LC-S159C-E165C | 148 | 150 | 4 |

Cys mutant antibody were expressed in 293 Freestyle™ cells by co-transfecting heavy chain and light chain plasmids using transient transfection method as described previously (Meissner, et al., Biotechnol Bioeng. 75:197-203 (2001)). The DNA plasmids used in co-transfection were prepared using Qiagen plasmid preparation kit according to manufacturer's protocol. 293 Freestyle™ cells were cultured in suspension in Freestyle™ expression media (Invitrogen) at 37°C under 5% $CO_2$. Three days before transfection, cells were split to 0.25 x $10^6$ cells/ml into fresh media. On the day of transfection, the cell density typically reached 1.5-2 x $10^6$ cells/ml. The cells were transfected with a mixture of heavy chain and light chain plasmids at the ratio of 1:1 using PEI method (Meissner, et al., Biotechnol Bioeng. 75:197-203 (2001)). The transfected cells were further cultured for five days. The media from the culture was harvested by centrifugation of the culture at 2000 g for 20 min and filtered through 0.2 micrometer filters. The expressed antibodies were purified from the filtered media using Protein A-Sepharose™ (GE Healthcare Life Sciences). Antibody IgGs were eluted from the Protein A-Sepharose™ column by the elution buffer (pH 3.0) and immediately neutralized with 1 M Tris-HCl (pH 8.0) followed by a buffer exchange to PBS.

Expression levels of trastuzumab and anti-cKIT Cys mutant antibodies in transiently transfected 293 Freestyle™ are similar to that of wild type antibodies, with yields ranging from 12-25 mg/L, suggesting that single to triple point mutations in the selected sites did not significantly alter retention of the expressed antibody by the cells' secretion machinery. Analysis of the purified Cys mutant antibodies using non-reducing SDS PAGE indicates that the Cys mutant antibodies did not form oligomers disulfide-linked by the engineered cysteines.

**Example 5. Reduction, re-oxidation and conjugation of Cys mutant antibodies with various payloads.**

Because engineered Cys in antibodies expressed in mammalian cells are typically modified by adducts (disulfides) such as glutathione (GSH) and/or Cysteine during their biosynthesis (Chen *et al.* 2009), the modified Cys in the product as initially expressed is unreactive to thiol reactive reagents such as maleimido or bromo-or iodo-acetamide groups. To conjugate the engineered cysteine after expression, the glutathione or cysteine adducts need to be removed by reducing these disulfides, which generally entails reducing all of the disulfides in the expressed protein. This can be accomplished by first exposing the antibody to a reducing agent such as dithiothreitol (DTT) followed by a procedure that allows for the re-oxidation of all native disulfide bonds of the antibody to restore and/or stabilize the functional antibody structure. Accordingly, in order to reduce all native disulfide bonds and the disulfide bound between the cysteine or GSH adducts of the engineered cysteine residue, freshly prepared DTT was added to purified Cys mutants of trastuzumab and anti-cKIT antibody, to a final concentration of 10 or 20 mM DTT. After the antibody incubation with DTT at 37°C for 1 hour, the mixtures were dialyzed against PBS for three days with daily buffer exchange to remove DTT and re-oxidize the native disulfide bonds. The re-oxidation process was monitored by reverse-phase HPLC, which is able to separate full IgG from individual heavy and light chain molecules. The conjugation reaction mixtures were analyzed on a PRLP-S 4000A column (50 mm x 2.1 mm, Agilent) heated to 80°C and elution of the column was carried out by a linear gradient of 30-60% acetonitrile in water containing 0.1% TFA at a flow rate of 1.5 ml/min. The elution of proteins from the column was monitored at 280 nm. Dialysis was allowed to continue until reoxidation was complete. Reoxidation restores intra-chain disulfides, while dialysis allows cysteines and glutathiones connected to the newly-introduced cysteine(s) to dialyze away.

After re-oxidation, the antibodies are ready for conjugation. Maleimide-containing compounds were added to re-oxidized antibodies in PBS buffer (pH 7.2) at ratios of typically 1.5:1, 2:1, or 10:1. The incubations were carried out from 1 hour to 24 hours. The conjugation process was monitored by reverse-phase HPLC, which is able to separate conjugated antibodies from nonconjugated ones in most cases. The elution of proteins from the column was monitored by UV absorbance at wavelengths of 280 nm, 254 nm and 215 nm.

When the conjugation mixtures were analyzed by reverse-phase HPLC, many Cys sites generated homogenous conjugation products, as suggested by uniform, single peak elution profiles, while some Cys sites generated heterogeneous conjugation products or showed only peaks matching the unconjugated antibodies.

The procedures described above involve reduction and re-oxidation of native disulfide bonds as well as the reduction of bonds between the cysteine and GSH adducts of the engineered cysteine residues. During the re-oxidation process, the engineered cysteine residue may interfere with reforming of the proper native disulfide bonds through a process of disulfide shuffling. This may lead to the formation of mismatched disulfide bonds, either between the engineered cysteine and a native cysteine residue or between incorrectly matched native disulfide bonds. Such mismatched disulfide bonds may affect the retention of the antibody on the reverse-phase HPLC column. The mismatch processes may also result in unpaired cysteine residues other than the desired engineered cysteine. Attachment of the maleimide-compound to different positions on the antibody affects the retention time differently (see discussion of homogenously conjugated ADCs below). In addition, incomplete re-oxidation will leave the antibody with native cysteine residues that will react with maleimide-compound in addition to the desired conjugation with the engineered cysteine residue. Any process that hinders proper and complete formation of the native disulfide bonds will result in a complex HPLC profile upon conjugation to Cys reactive compounds. Although sites were chosen to be surface exposed, there may also be heterogeneity in the final product if the introduced free cysteine is not accessible to or is otherwise unable to interact productively with the maleimide-drug in some or all conformations of the antibody. If the free cysteine is non-reactive, the final DAR will be lowered and the product likely to be a heterogenous mixture of fully, partially, and unmodified Cys mutant antibody. In the case of an antibody with two or more introduced free cysteines, there can be additional complexity introduced if drug attachment at one site interferes (i.e. by steric crowding) with the binding of a second drug at a second site. Such competition will lead to lower final DAR and a heterogeneous product. If two introduced cysteines are very close and properly oriented, then they may also form a non-native disulfide bond rather than forming two free cysteines. In this case, the antibody will not be reactive towards a maleimide-drug compound and the result will be a lower final DAR or even a uniform unconjugated product. The yield of the uniform ADC as measured by UV absorption by RP-HPLC the unpurified reaction mixtures, varied depending on the Cys mutations as well as the linker-payload compound used. Using the reduction/re-oxidation protocol and conjugation procedures described above 26 of 45 multiple Cys mutant trastuzumab or anti-cKIT antibodies described here resulted in homogeneous conjugation products of acceptable final DAR (DAR 3.4-4.4 for double Cys mutant, 5.1-6.0 for triple Cys mutant, Table 11) for such small test conjugations. These Cys sites and drug combinations are advantageous when making ADCs.

Table 11. DAR calculated from RP-HPLC analysis and verified by LCMS of intact, reduced, deglycosylated antibody chains for 45 multiple Cys mutant antibody samples conjugated to various drug by the methods described above.

| Cys mutant antibody | Linker-payload compound | Expected DAR | Observed DAR |
|---|---|---|---|
| trastuzumab-HC-A339C-P396C | Compound A | 4.0 | 2.0 |
| trastuzumab-HC-A329C-P396C | Compound G | 4.0 | 3.6 |
| trastuzumab-HC-A339C-P396C | Compound G | 4.0 | 3.6 |
| trastuzumab-HC-E152C-LC-E165C | Compound A | 4.0 | 3.0 |
| trastuzumab-HC-E152C-LC-E165C | Compound E | 4.0 | 3.0 |

(continued)

| Cys mutant antibody | Linker-payload compound | Expected DAR | Observed DAR |
|---|---|---|---|
| trastuzumab-HC-E152C-LC-E165C | Compound F | 4.0 | 3.7 |
| trastuzumab-HC-E152C-LC-E165C | Compound G | 4.0 | 2.9 |
| trastuzumab-HC-E152C-LC-S159C-E165C | Compound A | 6.0 | 4.0 |
| trastuzumab-HC-E152C-LC-S159C-E165C | Compound E | 6.0 | 5.2 |
| trastuzumab-HC-E152C-LC-S159C-E165C | Compound G | 6.0 | 5.2 |
| trastuzumab-HC-E152C-P174C | Compound A | 4.0 | 1.9 |
| trastuzumab-HC-E152C-P174C | Compound F | 4.0 | 3.7 |
| anti-cKIT-HC-E152C-S375C | Compound A | 4.0 | 3.9 |
| anti-cKIT-HC-E152C-S375C | Compound F | 4.0 | 3.8 |
| trastuzumab-HC-E152C-S375C | Compound A | 4.0 | 3.7 |
| trastuzumab-HC-E152C-S375C | Compound G | 4.0 | 3.7 |
| trastuzumab-HC-K334C-P396C | Compound A | 4.0 | 0.6 |
| trastuzumab-HC-K334C-P396C | Compound F | 4.0 | 3.7 |
| trastuzumab-HC-K334C-P396C | Compound G | 4.0 | 3.5 |
| trastuzumab-HC-K334C-S375C | Compound A | 4.0 | 2.6 |
| trastuzumab-HC-K334C-S375C | Compound F | 4.0 | 3.8 |
| trastuzumab-HC-K334C-S375C | Compound G | 4.0 | 3.0 |
| anti-cKIT-HC-K334C-S375C-LC-E165C | Compound E | 6.0 | 5.8 |
| anti-cKIT-HC-K334C-S375C-LC-E165C | Compound G | 6.0 | 5.2 |
| trastuzumab-HC-K334C-S375C-LC-E165C | Compound E | 6.0 | 6.0 |
| trastuzumab-HC-K334C-S375C-LC-E165C | Compound G | 6.0 | 6.0 |
| anti-KIT-HC-K360C-LC-K107C | Compound A | 4.0 | 4.0 |
| anti-KIT-HC-K360C-LC-K107C | Compound F | 4.0 | 4.0 |
| trastuzumab-HC-K360C-LC-K107C | Compound A | 4.0 | 4.0 |
| trastuzumab-HC-K360C-LC-K107C | Compound G | 4.0 | 3.9 |
| trastuzumab-HC-P396C-LC-E165C | Compound A | 4.0 | 1.6 |
| trastuzumab-HC-P396C-LC-E165C | Compound F | 4.0 | 3.8 |

(continued)

| Cys mutant antibody | Linker-payload compound | Expected DAR | Observed DAR |
|---|---|---|---|
| trastuzumab-HC-P396C-LC-E165C | Compound G | 4.0 | 3.4 |
| trastuzumab-HC-S375C-P396C | Compound A | 4.0 | 0.0 |
| trastuzumab-HC-S375C-P396C | Compound F | 4.0 | 0.0 |
| trastuzumab-HC-S375C-P396C | Compound G | 4.0 | 0.0 |
| trastuzumab-HC-S375C-Y391C | Compound A | 4.0 | 2.3 |
| trastuzumab-HC-S375C-Y391C | Compound G | 4.0 | 3.2 |
| trastuzumab-HC-Y391C-P396C | Compound A | 4.0 | 0.0 |
| trastuzumab-HC-Y391C-P396C | Compound F | 4.0 | 3.6 |
| trastuzumab-HC-Y391C-P396C | Compound G | 4.0 | 2.9 |
| trastuzumab-LC-S159C-E165C | Compound A | 4.0 | 2.0 |
| trastuzumab-LC-S159C-E165C | Compound D | 4.0 | 3.5 |
| trastuzumab-LC-S159C-E165C | Compound E | 4.0 | 3.6 |

A subset of the 45 ADC samples in Table 11 were analyzed in details in various assays: Differential scanning fluorimetry (DSF) was used to measure thermal stability. Analytical size exclusion chromatograph (AnSEC) and multi-angle light scattering (MALS) were used to measure aggregation. *In vitro* antigen dependent cell killing potency was measured by cell viability assays and pharmacokinetics behavior was measured in mice. In general, the multiple Cys mutant ADCs showed thermal stability similar to single Cys mutant ADCs. The ADCs were predominantly monomeric as determined by analytical size exclusion chromatography.

**Example 6. Preparation of anti-cKIT and trastuzumab Cys mutant ADCs conjugated with various compounds.**

Antibody drug conjugates of trastuzumab and anti-cKIT cys mutant antibodies HC-E152C-S375C and HC-K360C-LC-K107C were prepared using several payloads as described above. Some of the properties of these ADCs are shown in Table 12. The *in vitro* cell killing potency of these ADCs was tested as described in Example 7 and the results are summarized in Table 13 and Table 14. The compounds were further subjected to pharmacokinetic (PK) studies in naiive mice as described in Example 8. The PK properties are summarized in Table 15 and Table 16.

Table 12. Properties of anti-Her2 Cvs mutant ADCs conjugated with various compounds.

| ADC name[a] | DAR[b] | Aggregation (%)[c] |
|---|---|---|
| trastuzumab-HC-E152C-S375C-Compound A | 3.8 | 0.4 |
| trastuzumab-HC-E152C-S375C-Compound B | 4.0 | BLQ |
| trastuzumab-HC-E152C-S375C-Compound C | 3.7 | 0.6 |
| trastuzumab-HC-E152C-S375C-Compound F | 3.8 | 0.3 |
| trastuzumab-HC-K360C-LC-K107C-Compound A | 3.9 | 3.8 |
| trastuzumab-HC-K360C-LC-K107C-Compound B | 3.8 | 2.0 |
| trastuzumab-HC-K360C-LC-K107C-Compound C | 4.0 | 5.1 |
| trastuzumab-HC-K360C-LC-K107C-Compound F | 3.8 | 3.5 |
| anti-cKIT-HC-E152C-S375C-Compound A | 3.9 | BLQ |
| anti-cKIT-HC-E152C-S375C-Compound F | 3.8 | 1.5 |
| anti-cKIT-HC-K360C-LC-K107C-Compound A | 4 | BLQ |

(continued)

| ADC name[a] | DAR[b] | Aggregation (%)[c] |
|---|---|---|
| anti-cKIT-HC-K360C-LC-K107C-Compound F | 4 | 1.5 |
| [a] Name consists of a description of the mutated antibody and the name of the compound used in the chemical conjugation step.<br>[b] Drug-to-antibody ratio according to reverse-phase HPLC.<br>[c] Aggregation measured by analytical size exclusion chromatography; includes dimeric and oligomeric species. BLQ = below limit of quantitation. | | |

**Example 7. Cell proliferation assays to measure *In vitro* cell killing potency of Cys mutant ADCs.**

Cells that naturally express target antigens or cell lines engineered to express target antigens are frequently used to assay the activity and potency of ADCs. For evaluation of the cell killing potency of trastuzumab ADCs *in vitro,* two engineered cell lines, MDA-MB231 clone 16 and clone 40, and HCC1954 cells were employed (Gazdar A, Rabinovsky R, Yefenof E, Gordon B, Vitetta ES. Breast Cancer Res Treat. (2000) 61:217-228). MDA-MB231 clone 16 cells stably express high copy numbers (~$5x10^5$ copies /cell) of recombinant human Her2 while clone 40 expresses low copy numbers (~$5x10^3$ copies /cell) of human Her2. HCC1954 cells endogenously express high level (~$5x10^5$ copies /cell) of human Her2 in the surface. For determination of the cell killing potency of anti-cKIT ADCs, H526, KU812, CMK11-5 and Jurkat cells were used. While CMK11-5, H526 and KU812 cells express a high level of the antigen for the anti-cKIT antibody in the cell surface there is no detectable antigen expression in Jurkat cells. An antigen dependent cytotoxic effect should only kill cells that express sufficient antigen in the cell surface and not cells lacking the antigen. The cell proliferation assays were conducted with Cell-Titer-Glo™ (Promega) five days after cells were incubated with various concentrations of ADCs (Riss et al., (2004) Assay Drug Dev Technol. 2:51-62). In some studies, the cell based assays are high throughput and conducted in an automated system (Melnick et al., (2006) Proc Natl Acad Sci USA. 103:3153-3158).

Trastuzumab Cys mutant ADCs specifically killed Her2 expressing MDA-MB231 clone 16 and HCC1954 but not MDA-MB231 clone 40 cells that express Her2 at very low levels (Table 13). Trastuzumab ADCs prepared with Compound F also killed JimT1 cells. $IC_{50}$ of the trastuzumab Cys mutant ADCs varied by cell type and depending on the compound used (Table 13). Similarly, anti-cKIT Cys mutant ADCs displayed antigen-dependent cell killing in cell proliferation assays. Anti-cKIT Cys-drug ADCs killed antigen expressing NCI-H526, KU812 and CMK115 cells but not antigen negative Jurkat cells. The $IC_{50}$ of the anti-cKIT ADCs varied with cell type and compound used (Table 14).

Table 13. *In vitro* cell killing potency of anti-Her2 ADCs conjugated with various compounds.

| | $IC_{50}$ (μM)[b] | | | |
|---|---|---|---|---|
| ADC name[a] | MDA231-40 | HCC1954 | JimT1 | MDA231-16 |
| trastuzumab-HC-E152C-S375C-Compound A | 6.7E-02 | 1.4E-04 | 4.8E-02 | 6.7E-02 |
| trastuzumab-HC-E152C-S375C-Compound B | 6.7E-02 | 1.6E-04 | 6.7E-02 | 2.6E-04 |
| trastuzumab-HC-E152C-S375C-Compound C | 6.7E-02 | 1.8E-04 | 5.6E-02 | 3.6E-04 |
| trastuzumab-HC-E152C-S375C-Compound F | 6.7E-02 | 1.6E-04 | 1.7E-04 | 1.8E-04 |
| trastuzumab-HC-K360C-LC-K107C-Compound A | 6.7E-02 | 1.7E-04 | 6.7E-02 | 4.5E-02 |
| trastuzumab-HC-K360C-LC-K107C-Compound B | 6.7E-02 | 8.3E-05 | 6.7E-02 | 6.7E-02 |
| trastuzumab-HC-K360C-LC-K107C-Compound C | 6.7E-02 | 1.7E-04 | 6.7E-02 | 4.5E-02 |
| trastuzumab-HC-K360C-LC-K107C-Compound F | 6.7E-02 | 5.4E-05 | 1.3E-04 | 8.1E-05 |
| [a] Name consists of a description of the mutated antibody and the name of the compound used in the chemical conjugation step.<br>[b] The highest concentration used in the assay was 6.7E-02 μM. $IC_{50}$ values of 6.7E-02 μM therefore refer to inactivity of the ADC in the assay. | | | | |

Table 14. In *vitro cell* killing activity of anti-cKIT ADCs conjugated with various compounds.

| ADC name[a] | $IC_{50}$ (μM)[b] | | | |
|---|---|---|---|---|
| | Jurkat | H526 | KU812 | CMK115 |
| anti-cKIT-HC-E152C-S375C-Compound A | 6.7E-02 | 1.9E-04 | 6.7E-02 | 6.7E-02 |
| anti-cKIT-HC-E152C-S375C-Compound F | 6.7E-02 | 5.3E-05 | 5.7E-05 | 6.1E-05 |
| anti-cKIT-HC-K360C-LC-K107C-Compound A | 6.7E-02 | 2.0E-04 | 6.7E-02 | 6.7E-02 |
| anti-cKIT-HC-K360C-LC-K107C-Compound F | 5.2E-02 | 5.7E-05 | 6.1E-05 | 9.9E-05 |

[a] Name consists of a description of the mutated antibody and the name of the compound used in the chemical conjugation step.
[b] The highest concentration used in the assay was 6.7E-02 μM. $IC_{50}$ values of 6.7E-02 μM therefore refer to inactivity of the ADC in the assay.

**Example 8. Pharmacokinetic study of Cys mutant ADCs.**

It has been demonstrated that a long serum half-life is critical for high *in vivo* efficacy of ADCs (Hamblett, et al., "Effects of drug loading on the antitumor activity of a monoclonal antibody drug conjugate," Clin Cancer Res., 10:7063-7070 (2004); Alley et al., Bioconjug Chem. 19:759-765 (2008)). Attaching a usually hydrophobic drug payload to an antibody can significantly affect the properties of an antibody, and this may lead to a fast clearance of the ADCs *in vivo* (Hamblett *et al.,* 2004) and poor *in vivo* efficacy. To evaluate the effects of different conjugation sites on clearance of multi-Cys-drug ADCs *in vivo,* pharmacokinetic studies in non-tumor bearing mice were carried out. To detect drug containing ADCs in murine plasma, an anti-MMAF antibody was generated. ELISA assays for the detection of ADCs were developed on a Gyros™ platform using an anti-human IgG (anti-hIgG) to capture human IgG molecules from the plasma and an anti-human IgG (anti-hIgG) antibody and an anti-MMAF antibody for signal generation in two separate assays. The two ELISA assays measure the serum concentration of the antibody and the "intact" ADC respectively as discussed in more detail below. Three mice per group were administered with a single dose of the Cys ADCs at 1 mg/kg. Eight plasma samples were collected over the course of three weeks and assayed by ELISA as described above. Standard curves were generated for each ADC separately using the same material as was injected into the mice. As measured by anti-hIgG ELISA, the Cys mutant ADCs (Tables 15 and 16) displayed a pharmacokinetic profile similar to unconjugated wild type antibodies, indicating that mutation and payload conjugation to these sites did not significantly affect the antibody's clearance. To determine the chemical stability of the linkage between the maleimide payload and the antibody at the various Cys sites during circulation in mouse, ADC concentrations as measured by the anti-MMAF ELISA assay and as measured by the anti-hIgG ELISA assay were compared to each other for ADCs prepared with Compound F which is readily detected with the anti-MMAF ELISA (Tables 15 and 16). For these ADCs, values for the area-under-the-plasma-concentration-versus-time-curve (AUC) were calculated from measurements with the anti-MMAF (AUC-MMAF) and the anti-hIgG ELISA (AUC-IgG). Previous results for similar analyses suggest uncertainties of >25%. Since the ratio should remain near 1 if no drug loss occurs, a ratio >0.7 indicates that within the accuracy of the measurement, little drug loss was observed after administration in mice for trastuzumab and anti-cKIT ADCs prepared with Compound F (Tables 15 and 16).

To further understand the retention of ADC drug payloads especially for payloads that are not detectable by the anti-MMAF ELISA (such as Compounds A-E), samples were analyzed by immuno-precipitation mass spectrometry (IP-MS). In particular, ADCs were affinity-purified from mouse serum collected through terminal bleeding and the drug payloads attached to ADCs were analyzed by MS analysis. In a typical process, 200 μl of plasma was diluted with an equal amount of PBS containing 10 mM EDTA. To the dilution, 10 μl of affinity resin (IgG Select Sepharose 6 Fast flow; GE Healthcare 17-0969-01; 50% slurry) was added. Incubation of the resin with the diluted plasma samples was performed for 1 hr at room temperature by applying mild agitation to avoid resin settling. The resin was then filtered off and washed two times with 200 μl of PBS. To deglycosylate the antibody, 10 μl of PNGase F (1 mg/mL, 1/2x TBS pH 7.4, 2.5 mM EDTA, 50% Glycerol) diluted with 10 μl of PBS was added to the resin and the mixtures were incubated for 2-3 hrs at 37°C. After PNGase F was removed by washing the affinity resin twice with 200 μl PBS, the sample was eluted twice from the affinity resin by adding 20 μl of 1% formic acid and filtering off the resin. The combined eluates were diluted with 20 μl of 6 M guanidine hydrochloride and 5 μl of reduction buffer (0.66 M TCEP, 3.3 M ammonium acetate, pH 5). To effectively reduce the antibody, samples were incubated for at least 30 min at room temperature before analysis. LCMS was performed with an Agilent Technologies 6550-iFunnel QTOF MS / Agilent 1260 HPLC system. A standard reversed-phase chromatography was used for sample desalting with a PLRS column (8 μm, 2.1 x 50 mm,

1000Å, Agilent) at a flow rate of 0.5 ml/min at 80°C. Elution was carried out using a linear gradient of 20%- to 60%-acetonitrile containing 0.1% formic acid in 6 min. Agilent Qualitative Analysis was used for processing of the spectral record and spectral deconvolution. For analysis the spectral record was summed over the time interval covering elution of all relevant species. Summed spectra were deconvoluted in charge state and images of the deconvoluted spectra were recorded. The values of peak intensity were extracted for assignable species. Assignments of DAR state and fragment species were made based on values of calculated mass from the sequence of the analyzed antibodies and the expected mass shifts of the conjugates with drug molecules. The average DAR was calculated using the relative peak heights of all DAR states across a distribution. Average antibody DAR was calculated as the sum of DARs from 2 average light chains and 2 average heavy chains.

The average DAR of ADCs purified from plasma after three weeks in mouse circulation, as measured by MS, was compared to the DAR in the original ADC preparations. "Payload retention" (Tables 15 and 16) was calculated from the ratio of the two DARs (DAR of ADC isolated from mouse plasma divided by the DAR of original ADC preparation), and represent the percentage of payloads retained on the ADC after three weeks in mouse circulation. Payload retention of ADCs as measured by MS is largely in agreement with results obtained by the aforementioned ELISA assay for ADCs prepared with Compound F (Tables 15 and Table 16). The data indicate a high degree of stability of the drug-antibody linkage during circulation in mice over a three week period for the Cys ADCs described herein.

Table 15. Pharmacokinetic properties of anti-cKIT Cys mutant ADCs conjugated with various compounds.

| ADC name[a] | AUC Payload[b] (μg/ml*h) | AUC IgG[c] (μg/ml*h) | AUC ratio (Payload AUC/IgG AUC) | Payload retention[d] |
|---|---|---|---|---|
| anti-cKit-HC-E152C-S375C-Compound A | n.a. | 7016 | n.a. | 0.82 |
| anti-cKit-HC-EI52C-S375C-Compound F | 2565 | 3912 | 0.66 | 0.64 |
| anti-cKit-HC-K360C-LC-K107C-Compound A | n.a. | 5112 | n.a. | 0.88 |
| anti-cKit-HC-K360C-LC-K107C-Compound F | 4582 | 5051 | 0.91 | 0.78 |

[a] Name consists of a description of the mutated antibody and the name of the compound used in the chemical conjugation step.
[b] AUC readout by anti-MMAF ELISA.
[c] AUC readout by anti-IgG ELISA.
[d] Payload retention as measured by IP-MS after 3 weeks of circulation in mouse.
n.a: not applicable. Anti-MMAF ELISA does not detect payload.

Table 16. Pharmacokinetic properties of anti-Her2 Cys mutant ADCs conjugated with various compounds.

| ADC name[a] | AUC Payload[b] (μg/ml*h) | AUC IgG[c] (μg/ml*h) | AUC ratio (Payload AUC/IgG AUC) | Payload retention[d] |
|---|---|---|---|---|
| trastuzumab-HC-E152C-S375C-Compound A | n.a. | 1859 | n.a. | 0.74 |
| trastuzumab-HC-E152C-S375C-Compound B | n.a. | 2172 | n.a. | 0.73 |
| trastuzumab-HC-E152C-S375C-Compound C | n.a. | 2506 | n.a. | 0.76 |
| trastuzumab-HC-E152C-S375C-Compound F | 1367 | 2414 | 0.57 | 0.50 |
| trastuzumab-HC-K360C-LC-K107C-Compound A | n.a. | 3870 | n.a. | 0.86 |
| trastuzumab-HC-K360C-LC-K107C-Compound B | n.a. | 3280 | n.a. | 0.91 |

(continued)

| ADC name[a] | AUC Payload[b] (μg/ml*h) | AUC IgG[c] (μg/ml*h) | AUC ratio (Payload AUC/IgG AUC) | Payload retention[d] |
|---|---|---|---|---|
| trastuzumab-HC-K360C-LC-K107C-Compound C | n.a. | 3258 | n.a. | 0.84 |
| trastuzumab-HC-K360C-LC-K107C-Compound F | 2622 | 3842 | 0.68 | 0.82 |

[a] Name consists of a description of the mutated antibody and the name of the compound used in the chemical conjugation step.
[b] AUC readout by anti-MMAF ELISA.
[c] AUC readout by anti-IgG ELISA.
[d] Payload retention as measured by IP-MS after 3 weeks of circulation in mouse.
n.a: not applicable. Anti-MMAF ELISA does not detect payload.

**Example 9. Preparation and trastuzumab and anti-cKIT ADC conjugated with Eg5 Inhibitor.**

Engineered Cys ADCs have been reported to be better tolerated in mice and rat animal models than ADCs made by conjugation to partially reduced native disulfides or through native lysine residues (Junutula et al., (2008) Nat Biotechnol. 26(8):925-932). To evaluate differences in *in vivo* efficacy between ADCs conjugated through engineered Cys antibodies and ADCs conjugated to partially reduced native disulfide bonds (Doronina et al., (2003) Nat. Biotechnol. 21, 778-784), Cys mutants of trastuzumab and the anti-cKIT antibody were expressed in 293 Freestyle™ cells and purified as described in Example 4 and ADCs were prepared as described in Example 5.

Eg5 linker-payload Compound A in Table 5 was conjugated to antibody anti-cKIT-HC-E152C-S375C double mutant (also referred to as cKITB, the immunoconjugates are referred to as cKitB-Cmpd A or cKitB-5B) and anti-cKIT-HC-K360C-LC-K107C double mutant (also referred to as cKitC, immunoconjugates are referred to as cKitC-Cmpd A or cKitC-5B) as well as wild type anti-cKIT antibody (immunoconjugates also referred to as cKitA-Cmpd A or cKitA-5B). (Residue Numbers are EU numbers). The sequences of the constant regions of the antibodies are set forth in Table 17.

Table 17: Sequence information for wild type and cys-substituted constant regions of antibodies.

**SEQ ID NO:150 (Constant region of the heavy chain wild type of antibody anti-cKIT)**

SASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTVD
KSRWQQGNVFSCSVMHEALHNHYTQKSLSLSPGK

**SEQ ID NO:149 (Constant region of the light chain wild type of antibody anti-cKIT)**

KRTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

**SEQ ID NO:50 (Constant region of the mutant heavy chain of antibody anti-cKIT with mutation HC-S375C)**

SASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPS
REEMTKNQVSLTCLVKGFYPCDIAVEWESNGQPENNYKTTPPVLDSDGSFFLYSKLTVD
KSRWQQGNVFSCSVMHEALHNHYTQKSLSLSPGK

**SEQ ID NO:61 (Constant region of the mutant light chain of antibody anti-cKIT with mutation LC-K107C)**

CRTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

(continued)

| |
|---|
| **SEQ ID NO: 131 (Constant region of the mutant heavy chain of antibody anti-cKIT with mutations HC-E152C-S375C)** |
| SASTKGPSVFPLAPSSKSTSGGTAALGCLVKDYFPCPVTVSWNSGALTSGVHTFPAVLQS SGLYSLSSVVTVPSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPS REEMTKNQVSLTCLVKGFYPCDIAVEWESNGQPENNYKTTPPVLDSDGSFFLYSKLTVD KSRWQQGNVFSCSVMHEALHNHYTQKSLSLSPGK |
| **SEQ ID NO: 48 (Constant region of mutant heavy chain antibody (anti-cKIT with mutation at HC-K360C)** |
| SASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPS REEMTCNQVSLTCLVKGFYPSDIAVEWESNGQPENNYKTTPPVLDSDGSFFLYSKLTVD KSRWQQGNVFSCSVMHEALHNHYTQKSLSLSPGK |

Specifically, reoxidized antibodies were conjugated with Compound A by incubating 5 mg/ml antibody with 0.35 mM Compound A for 1 hour in 50 mM sodium phosphate buffer (pH 7.2). The completeness of the reaction was monitored by RP-HPLC and a DAR of 3.9 and 4.0 were obtained for the cKitB and cKitC conjugates, respectively. DAR measurements were further verified by MS. ADCs were shown to be potent and *in vitro* cell killing assays and had pharmacokinetics properties similar to unconjugated antibody in non-tumor bearing mice.

The ADC with Compound A conjugated to the native disulfide bonds of cKitA was prepared as follows in a 2-step process. The antibody at a concentration of 5-10 mg/ml in PBS containing 2 mM EDTA, was first partially reduced for 1 hour at 37 °C with 50 mM mercaptoethylamine (added as a solid). After desalting and addition of 1% w/v PS-20 detergent, the partially reduced antibody (1-2 mg/ml) was reacted overnight at 4 °C with an amount of 0.5-1 mg of compound A, dissolved at 10 mg/ml in DMSO, per 10 mg antibody. The ADC was purified by Protein A chromatography. After base-line washing with PBS, the conjugate was eluted with 50 mM citrate, pH 2.7, 140 mM NaCl, neutralized and sterile filtered. The average DAR was 3.2.

Table 18: Properties of three anti-cKIT ADCs conjugated with Compound A.

| ADC name[a] | DAR[b] | Aggregation[c] |
|---|---|---|
| Anti-cKIT-Compound A (cKitA-5B) | 3.2 | 0.8% |
| anti-cKIT-HC-EI52C-S375C-Compound A (cKitB-5B) | 3.9 | 1.5% |
| anti-cKIT-HC-K360C-LC-K107C-Compound A (cKitC-5B) | 4.0 | 3.2% |

[a] Name consists of a description of the mutated antibody and the name of the compound used in the chemical conjugation step.
[b]Drug-to-antibody ratio according to reverse-phase HPLC or HIC.
[c] Aggregation measured by analytical size exclusion chromatography; includes dimeric and oligomeric species.

Similarly, immunoconjugates with the following combinations of payloads with anti-cKIT and trastuzumab mutated antibodies having cysteine substitutions were prepared and characterized by the same methods. Note that the engineered antibodies consistently provided DAR near 4, the expected loading if the four added cysteine residues per antibody complex are all conjugated to payload (Tables 18 and 19):

Table 19. Summary of anti-cKIT and trastuzumab Cys mutant ADCs with Eg5 inhibitor payloads.

| ADC name[a] | Conc. (mg/ml) | DAR[b] | Aggregation (%)[c] | Endotoxin (Eu/mg) |
|---|---|---|---|---|
| trastuzumab-HC-E152C-S375C-Compound A (TBS--5B) | 2 | 3.9 | 3.4 | <0.1 |

(continued)

| ADC name[a] | Conc. (mg/ml) | DAR[b] | Aggregation (%)[c] | Endotoxin (Eu/mg) |
|---|---|---|---|---|
| trastuzumab-HC-E152C-S375C-Compound B (TBS--5E) | 2 | 3.8 | 2 | <0.1 |
| trastuzumab-HC-E152C-S375C-Compound C (TBS-5D) | 2 | 4 | 5.1 | <0.1 |
| anti-cKIT-HC-E152C-S375C-Compound A (cKitB--5B) | 4 | 3.8 | 0 | <0.1 |
| anti-cKIT-HC-E152C-S375C-Compound B (cKitB--5E) | 4 | 3.9 | 0.1 | <0.1 |
| anti-cKIT-HC-E152C-S375C-Compound C (cKitB--5D) | 4 | 3.9 | 0.2 | 0.2 |
| trastuzumab-HC-K360C-LC-K107C-Compound A (5B) | 4 | 3.8 | 0.4 | <0.1 |
| trastuzumab-HC-K360C-LC-K107C-Compound B (5E) | 4 | 4 | 0 | 0.1 |
| trastuzumab-HC-K360C-LC-K107C-Compound C (5D) | 3 | 3.7 | 0.6 | <0.1 |

[a] Name consists of a description of the mutated antibody and the name of the compound used in the chemical conjugation step.
[b]Drug-to-antibody ratio according to reverse-phase HPLC.
[c] Aggregation was measured analytical size exclusion chromatography; includes dimeric and oligomeric species.

**Example 10. *In vitro* potency and *In vivo* efficacy of ADCs prepared with Eg5 inhibiting payloads.**

Immunoconjugates were prepared from each of the Eg5 inhibiting linker-payload compounds shown in Table 5 conjugated with an anti-cKIT antibody (also referred to as cKitA) and HC-E152C-S375C Cys-mutated versions of anti-cKIT antibody (cKitB). The constant region for anti-cKIT (cKitA) wild type and Cys-substituted mutants are shown in Table 17 above. Conjugates having a drug to antibody ratio (DAR) between 3.5 and 4.0 were prepared for each payload by the methods described above. The immunoconjugates were tested for activity in a cell line expected to be recognized by antibodies to cKit.

Figure 2 shows inhibition of cell growth by immunoconjugates with the HC-E152C-S375C Cys-substituted cKIT immunoconjugates comprising Compounds A, B, and C. Jurkat cells are a cKIT negative cell line, and were not sensitive to the three anti-cKIT (cKitA) immunoconjugates. However, proliferation of H526 cells, a cKIT positive cell line, was inhibited by all three anti-cKIT (cKitA) conjugates with $IC_{50}$s ranging from 100 to 500 pM. The H526 cell line was selected as a xenograft model for *in vivo* efficacy studies.

*In vivo* xenograft tumor models simulate biological activity observed in humans and consist of grafting relevant and well characterized human primary tumors or tumor cell lines into immune-deficient nude mice. Studies on treatment of tumor xenograft mice with anti-cancer reagents have provided valuable information regarding *in vivo* efficacy of the tested reagents (Sausville and Burger, (2006) Cancer Res. 66:3351-3354).

All animal studies were conducted in accordance with the Guide for the Care and Use of Laboratory Animals (NIH publication; National Academy Press, 8th edition, 2001). H526 cells were implanted in nu/nu mice subcutaneously (Morton and Houghton, Nat Protoc. 2007;2:247-250). After the tumor size reached ~200 $mm^3$, ADCs were administered into the mice by i.v. injection in a single dose. Tumor growth was measured periodically after ADC injection. An example of such an *in vivo* efficacy study is shown in Figure 3.

Figure 3 summarizes the activity of two ADCs made with cysteine-engineered anti-cKIT antibodies, namely anti-cKIT-HC-E152C-S375C-Compound A (cKitB-5B) and anti-cKIT-HC-K360C-LC-K107C-Compound A (cKitC-5B), which inhibited growth of H526 tumor xenografts in mice at doses of 5 mg/kg (Figure 3A) and 10 mg/kg (Figure 3B). Anti-cKIT-Compound A (cKitA-5B) prepared with the wild type antibody through partial reduction, because of lower DAR, was administered at higher doses to match the molar payload dose. 6 mice were injected per group for each ADC tested. No significant weight loss was observed associated with the ADC treatment in any group suggesting low systemic toxicity.

The cysteine-engineered anti- cKIT ADCs of Compound A were more active than the ADC prepared through partial reduction of the wild type antibody Anti-cKIT-Compound A (cKitA-5B). Thus, while immunoconjugates of Eg5 inhibitors were active with various cKit antibodies including unmodified ones, this demonstrates that protein engineering to introduce new cysteine residues into the constant region and using the new cysteine residues as attachment points for the payload/linker group can provide improved immunoconjugates.

The Cys-substituted cKitA immunoconjugates were also tested in murine xenograft model. Both of the Cys substituted immunoconjugates showed greater activity than the nonsubstituted immunoconjugates, as measured by tumor volume post-implant.

**Example 11: Dose dependent *in vivo* efficacy of an anti-Her2 ADC conjugated with an Eg5 inhibitor in the Her2 positive MDA-MB-231 clone 16 breast cancer model in mice.**

The anti-tumor efficacy of the anti-Her2 ADC trastuzumab-HC-E152C-S375C-Compound A was prepared by conjugating trastuzumab HC-E152C-S375C Cys mutant antibody with Eg5 inhibitor Compound A was evaluated in the Her2 positive MDA-MB-231 HER2 clone 16 breast cancer xenograft model. Female athymic nude-Foxn1 mice were implanted subcutaneously with 5 x $10^6$ cells containing 50% Matrigel™ (BD Biosciences) in phosphate-buffered saline (PBS) solution. The total injection volume containing cells in suspension was 200 $\mu$l. Mice were enrolled in the study 13 days post implantation of tumor cells with average tumor volumes of ~220$mm^3$. After being randomly assigned to one of eight groups (n=5/group), mice were administered a single *i.v.* dose of PBS, a non-specific isotype control-HC-E152C-S375C-Compound A (10 mg/kg) or trastuzumab-HC-E152C-S375C-Compound A (2.5, 5 or 10 mg/kg). Tumor volumes (Figure 4) and body weights were measured at least twice weekly.

On Day 40 post-tumor cell implant, mice treated with a single administration of 2.5 mg/kg of trastuzumab-HC-E152C-S375C-Compound A had tumors that showed a percent mean change in tumor volume compared to the vehicle control (T/C) of 8.22%. Mice treated with a single administration of 5 mg/kg and 10 mg/kg of trastuzumab-HC-E152C-S375C-Compound A had tumors that showed a regression in volume of 74.14% and 76.35%, respectively, both of which were statistically different from the vehicle alone and non-specific isotype ADC controls ($p < 0.05$, ANOVA, Tukey's post-hoc test). The treatments were well tolerated at all dose levels.

Table 20: TBS-HC-E152C-S375C-Compound A dose response in the Her2 positive MDA-MB-231clone 16, breast cancer model in mice on Day 40.

| Drug | Dose (mg/kg) | Schedule | Tumor Response | | | Host Response | |
|---|---|---|---|---|---|---|---|
| | | | Mean change of tumor volume vs control (T/C) (%) | Regression (%) | Mean change of tumor volume (mm3±SEM) | Mean change of body weight (%±SEM) | Survival (Survivors/ total) |
| Vehicle | 0 | Single dose IV | 100 | - | 1112.82 ± 254.74 | 7.15 ± 6.63 | 5/5 |
| Isotype Control-HC-E152C-S375C-Compound A | 10 | Single dose IV | 81.54 | - | 907.35 ± 246.84 | 2.78 ± 2.05 | 5/5 |
| TBS-HC-E152C-S375C-Compound A | 2.5 | Single dose IV | 8.22 | - | 91.47 ± 99.08 | 2.92 ± 0.80 | 5/5 |
| TBS-HC-E152C-S375C-Compound A | 5 | Single dose IV | - | 74.14 | -151.46 ± 25.52 | 4.01 ± 0.78 | 5/5 |

(continued)

| Drug | Dose (mg/kg) | Schedule | Tumor Response | | | Host Response | |
|---|---|---|---|---|---|---|---|
| | | | Mean change of tumor volume vs control (T/C) (%) | Regression (%) | Mean change of tumor volume (mm3±SEM) | Mean change of body weight (%±SEM) | Survival (Survivors/ total) |
| TBS-HC-E152C-S375C-Compound A | 10 | Single dose IV | - | 76.35 | -163.29 ± 20.63 | 0.52 ± 1.80 | 5/5 |

**Example 12*: In vivo* efficacy of an anti-Her2 ADC conjugated with an Eg5 inhibitor in the Her2 positive MDA-MB-453 human breast cancer xenograft mouse model.**

The anti-tumor efficacy of the anti-Her2 trastuzumab-HC-E152C-S375C-Compound A ADC was also evaluated in the Her2 positive MDA-MB-453 human breast cancer xenograft model. Female SCID beige mice were implanted subcutaneously with 5 x $10^6$ cells containing 50% Matrigel™ (BD Biosciences) in phosphate-buffered saline (PBS) solution. The total injection volume containing cells in suspension was 200 μl. Mice were enrolled in the study seven days post implantation of tumor cells with tumor volumes of approximately 168 $mm^3$-216 $mm^3$. After being randomly assigned to one of four groups (n=6/group), mice were administered a single i.v. dose of PBS, a non-specific isotype control-HC-E152C-S375C-Compound A (10 mg/kg) or trastuzumab-HC-E152C-S375C-Compound A (10 mg/kg). Tumor volumes (Figure 5) and body weights were measured at least twice weekly.

On Day 45 post-implant, mice treated with trastuzumab-HC-E152C-S375C-Compound A (10 mg/kg) had tumors that showed a regression in volume of 71.2%, which was statistically different from the vehicle alone and non-specific isotype ADC controls ($p < 0.05$, ANOVA, Tukey's post-hoc test). The treatments were well tolerated at all dose levels.

Table 21: ADC efficacy of of trastuzumab-HC-E152C-S375C-Compound A at 10 mg/kg in the Her2 positive MDA-MB-453 human breast cancer xenograft mouse model on Day 45.

| Drug | Dose (mg/kg) | Schedule | Tumor Response | | | Host Response | |
|---|---|---|---|---|---|---|---|
| | | | Mean change of tumor volume vs control (T/C) (%) | Regression (%) | Mean change of tumor volume (mm3±SEM) | Mean change of bodyweight (%±SEM) | Survival (Survivors/ total) |
| Vehicle | 0 | Single dose IV | 100 | | 654±69.5 | 12±1.93 | 6/6 |
| Isotype Control-HC-E152C-S3 75 C-Compound A | 10 | Single dose IV | 128.3 | | 827.9±96.7 | 11.1±0.85 | 6/6 |
| Trastuzumab-HC-E152C-S375C-Compound A | 10 | Single dose IV | -- | 71.2 | -138.5±22.2 | 11.7±3.8 | 6/6 |

**Example 13: *In vivo* efficacy of an anti-Her2 ADC conjugated with an Eg5 inhibitor in the Her2 positive HCC1954 human breast cancer xenograft mouse model.**

The anti-tumor efficacy of the anti-Her2 trastuzumab-HC-E152C-S375C-Compound A ADC was further evaluated in the Her2 positive HCC1954 breast cancer xenograft model. Female athymic nude-Foxn1 mice were implanted

subcutaneously with 5 x $10^6$ cells containing 50% Matrigel™ (BD Biosciences) in phosphate-buffered saline (PBS) solution. The total injection volume containing cells in suspension was 200 μl. Mice were enrolled in the study 11 days post implantation with tumor volumes of approximately 148 $mm^3$- 216 $mm^3$. After being randomly assigned to one of four groups (n=6/group), mice were administered a single i.v. dose of PBS, a non-specific isotype control-HC-E152C-S375C-Compound A (10 mg/kg) or trastuzumab-HC-E152C-S375C-Compound A (10 mg/kg. Tumor volumes (Figure 6) and body weights were measured at least twice weekly.

On Day 45 post-implant, mice treated with trastuzumab-HC-E152C-S372C-Compound A (10 mg/kg) had tumors that showed a regression in volume of 63.0%, which was statistically different from the vehicle alone and non-specific isotype ADC controls ($p < 0.05$, ANOVA, Tukey's post-hoc test). The treatments were well tolerated at all dose levels.

Table 22: ADC efficacy of trastuzumab-HC-E152C-S375C-Compound A at 10 mg/kg in the Her2 positive HCC1954 human breast cancer xenograft mouse model on Day 45.

| Drug | Dose (mg/kg) | Schedule | Tumor Response | | | Host Response | |
|---|---|---|---|---|---|---|---|
| | | | Mean change of tumor volume vs control (T/C) (%) | Regression (%) | Mean change of tumor volume (mm3±SEM) | Mean change of body weight (%±SEM) | Survival (Survivors/ total) |
| Vehicle | 0 | Single dose IV | 100 | | 555.2±122.5 | 5.9±1.4 | 6/6 |
| Isotype control antibody-HC-E152C-S375C-Compound A | 10 | Single dose IV | 117.9 | | 654.6±200.1 | 8.6±0.9 | 6/6 |
| trastuzumab - HC-E152C-S375 C-Compound A | 10 | Single dose IV | -- | 63.0 | -112.0±15.95 | 8.0±1.4 | 6/6 |

**Example 14: *In vivo* efficacy study comparing anti-cKIT Cys mutant ADCs to ADCs prepared by partial reduction of a non-engineered antibody.** The *in vivo* efficacy of two anti-cKIT ADCs: anti-cKIT-HC-E152C-S375C-Compound F and anti-cKIT-Compound F, were compared in the H526 xenograft mouse model (Figure 7). The two ADCs were prepared with the same payload; Compound F (Table 5), conjugated to different Cys sites using two different methods. Conjugate anti-cKIT-HC-E152C-S375C-Compound F was prepared with a Cys mutant antibody, as described in Example 5 with Compound F conjugated to engineered Cys residues, HC-E152C and HC-S375C. Conjugate anti-cKIT-Compound F was prepared by applying the partial reduction method described in Example 9 to wild type anti-cKIT antibody with Compound F conjugated to native Cys residues. Anti-cKIT-Compound F had a slightly higher DAR (DAR 4.6) and aggregation (2.9%) than anti-cKIT-HC-E152C-S375C-Compound F (DAR 3.9, 0.6% aggregation). Pharmacokinetic studies in non-tumor bearing mice showed that the two ADCs retained the same payload to a very different extent during three weeks of circulation in mouse: As illustrated by ELISA (Figure 8A, Figure 8B) and as determined by IP-MS (see Example 8), anti-cKIT-HC-E152C-S375C-Compound F displayed much better payload retention (56%) than anti-cKIT-Compound F (20%).

In the H526 xenograft model, the same dosage of anti-cKIT-HC-E152C-S375C-Compound F is more efficacious in inhibiting tumors than anti-cKIT-Compound F (Figure 7). Anti-Her2-HC-E152C-S375C-Compound F (see Table 12 for properties), whose antigen is not expressed in H526 cells, was included as control and did not show any tumor inhibiting activity.

**Example 15. *In vivo* efficacy study comparing anti-cKIT Cys mutant ADCs conjugated at different sites with Compound F and Compound A.**

In another example, the *in vivo* efficacy of anti-cKIT-HC-E152C-S375C-Compound F, anti-cKIT-HC-K360C-LC-K107C-Compound F, anti-cKIT-HC-E152C-S375C-Compound A, and anti-cKIT-HC-K360C-LC-K107C-Compound A ADCs were compared in the H526 xenograft model (Figure 9). The two payloads, Compound F and Compound A,

were conjugated to different Cys sites using two different antibodies as described in Example 7. The properties of the ADCs are summarized in Table 12. The DAR measured was close to the theoretical DAR of 4 for all four conjugates and little aggregation was observed for the resulting ADCs (Table 12). Single doses of 3.5 mg/kg of the ADCs were injected *i.v.* into animals bearing H526 tumors. The results of tumor volume measurements in the H526 xenograft model are shown in Figure 9. In this model, the same dosage of anti-cKIT-Compound F ADC was more efficacious in inhibiting tumors than ADCs prepared with Compound A. There is not statistically significant difference in tumor inhibiting activity between ADCs conjugated to the two different sets of Cys mutants.

**Example 16. Hydrophobicity of trastuzumab ADCs conjugated with Compound G.**

To further optimize the selection of Cys mutants and mutant combinations for the preparation of ADCs with DAR 2, 4, 6 and greater, the properties of trastuzumab Cys mutant ADCs were analyzed with respect to hydrophobicity. Cys mutants ADCs conjugated with Compound G (MC-MMAF) were prepared as described above. The final DAR as determined experimentally as described were generally close to the target and are listed in Table 23 below. The hydrophobicity of these ADCs was measured by hydrophobic interaction chromatography as follows.

Analytical HIC data for trastuzumab Cys-MMAF ADCs were collected using a Tosoh Bioscience (King of Prussia, PA, USA) TSKgel Butyl-NPR column (100 mm $\times$ 4.6 mm, 2.5 $\mu$m) installed on an Agilent 1260 LC system (Santa Clara, CA, USA). The method consisted of a binary gradient of buffer A (20 mM His-HCl, 1.5 M ammonium sulfate, pH 6.0) and buffer B (20 mM His-HCl, 15% isopropanol, pH 6.0). Samples were prepared by diluting approximately 20 $\mu$g of antibody (PBS) with 0.5 volume of 3 M ammonium sulfate. The gradient consisted of 5 min holding at 100% A, followed a linear gradient of 0 to 100% B over 30 min, a return to 100% A over 5 min, and finally re-equilibrating at initial conditions for 10 min prior to the next injection. The separation was monitored by UV absorption at 280 nm and analyzed using Chromelion software (Dionex).

Surprisingly, it was observed that the retention times of the DAR 4 ADCs varied greatly although the only difference is the site of Compound G attachment. In addition, the range of retention times overlapped substantially with the range observed for DAR 2 ADCs included for comparison (Table 23). HIC separates molecules on the basis of the hydrophobicity. All DAR 2 ADCs have a HIC retention time larger than that of unconjugated antibody (retention time = 45 min) which is to be expected when a hydrophobic drug molecule such as Compound G is attached to an antibody. However, attaching the payload at different sites increases the hydrophobicity of the ADC to various extents.

Table 23. DAR and analytical HIC retention times for trastuzumab-Cys-Compound G ADCs with DAR = 2, 4, or 6.

| Trastuzumab Cys mutation site | DAR | Retention time (min) |
|---|---|---|
| HC-E152C | 1.8 | 15.5 |
| HC-K334C-P396C | 3.5 | 15.8 |
| HC-P396C | 2.0 | 15.9 |
| HC-E152C-P396C | 3.3 | 16.1 |
| HC-E152C-LC-E165C | 2.9 | 16.2 |
| HC-A327C-A339C | 3.5 | 16.2 |
| LC-E165C-HC-P396C | 3.4 | 16.3 |
| HC-Y391C | 2.0 | 16.4 |
| HC-E152C-S375C | 3.7 | 16.5 |
| LC-E165C-HC-S375C | 4.0 | 16.7 |
| HC-E152C-A339C | 3.7 | 17.1 |
| HC-E152C-LC-R142C | 3.8 | 17.1 |
| HC-A339C-S375C | 3.3 | 17.2 |
| HC-E333C | 1.9 | 17.2 |
| HC-E152C-A327C | 3.7 | 17.3 |
| LC-E165C-HC-L174C | 3.4 | 17.4 |
| HC-S375C-Y391C | 3.2 | 17.4 |

(continued)

| Trastuzumab Cys mutation site | DAR | Retention time (min) |
|---|---|---|
| HC-A339C-P396C | 3.6 | 17.5 |
| LC-S159C-HC-E152C | 3.8 | 17.5 |
| HC-Y373C | 2.0 | 17.7 |
| LC-E165C-HC-K334C-S375C | 6.0 | 18.1 |
| HC-A327C-S375C | 3.8 | 18.2 |
| LC-E165C-HC-K334C-K392C | 5.8 | 18.2 |
| HC-P247C | 2.0 | 18.9 |
| LC-K107C-HC-K360C | 3.9 | 21.5 |

The surprisingly large differences in retention times can be rationalized from the inspection of location of the attachment sites on the structure of an antibody: The retention times are higher if the drug payload is attached at an exposed site on the outside of an antibody, for example at HC-P247C where retention time of almost 19 min were measured. Conversely, if the payload is attached at an interior site such as the cavity formed between variable and CH1 domains (for example, HC-E152C) or the large opening between CH2 and CH3 domains of the antibody (for example, HC-P396C), the HIC retention time is below 16 min because the payload is partially sequestered from interacting with solvent and the HIC column. Likewise, for DAR 4 ADCs that include two relatively interior sites (for examples HC-E152C-P396C and HC-E152C-S375C), the retention time remains short, on the order of 15.5-16.5 min, while DAR 4 ADCs that include very exposed sites (for example, LC-K107C-HC-K360C) can show retention times greater than 21 min.

Reducing hydrophobicity of a protein drug including ADCs is generally considered beneficial because it may reduce aggregation and clearance from circulation. Conjugating drug payloads at sites where they are sequestered from solvent interactions and attachment minimally increases the hydrophobicity of the antibody upon drug attachment should be beneficial independent of the conjugation chemistry and payload class. Carefully selecting attachment sites that result in minimal changes in hydrophobicity may be particularly beneficial when 4, 6 or more drugs are attached per antibody, or when particularly hydrophobic payloads are used.

**Example 17. Hydrophobicity of anti-Her2 Cys mutant ADCs conjugated with various compounds.**

A subset of the trastuzumab-HC-E152C-S375C and trastuzumab-HC-K360C-LC-K107C ADCs prepared in Example 7 (see Table 12 for properties) were also characterized by hydrophobic interaction chromatograpy as described in detail below (Table 24). ADCs conjugated to the combination of exposed Cys residues (positions HC-K360C-LC-K107C) are more hydrophobic than ADCs with drugs attached to the HC-E152C-S375C antibody. The effect is more pronounced for the Eg5 inhibitor payloads Compound A and Compound C compared to the cytotoxic peptide Compound F.

As discussed in Example 16, attaching drugs at sites where they are sequestered from solvent interactions such as HC-E152C-S375C appears to increase the hydrophobicity of the antibody to a lesser degree than when attached at more solvent exposed positions such as HC-K360C and LC-K107C. Although beneficial for many applications, particularly for the attachment of hydrophobic payloads, conjugating payloads at more solvent exposed positions will have beneficial utility in other applications.

Table 24: Hydrophobicity scores of various Cys mutant anti-Her2-ADCs conjugated with different payloads

| ADC name[a] | Hydrophobicity score[b] |
|---|---|
| trastuzumab-HC-E152C-S375C-Compound F | 0.91 |
| trastuzumab-HC-E152C-S375C-Compound A | 0.90 |
| trastuzumab-HC-E152C-S375C-Compound C | 0.87 |
| trastuzumab-HC-K360C-LC-K107C-Compound F | 0.51 |
| trastuzumab-HC-K360C-LC-K107C-Compound A | 0.33 |

(continued)

| ADC name[a] | Hydrophobicity score[b] |
|---|---|
| trastuzumab-HC-K360C-LC-K107C-Compound C | 0.31 |

[a] Name consists of a description of the mutated antibody and a description of the compound used in the chemical conjugation step.
[b] Hydrophobic Interaction Chromatography (HIC) measurements: The separation of the different species was carried out on a TSKgel Butyl-NPR column (4.6 mm ID x 35 mm L, Tosoh Bioscience) connected to an Agilent 1260 Infinity LC System (Agilent Technologies). The system was equilibrated firstly with mobile phase B (17 mM His/HCl, pH 6.0 containing 15% isopropanol) and subsequently with mobile phase A (20 mM His/HCl pH 6.0, containing 1.5 M $(NH_4)_2SO_4$) until a stable baseline was reached. 10 to 50 ug of sample, stored at 4°C in the auto-sampler, was injected and separated at a flow rate of 1.0 mL/min at a constant temperature of 25°C. Elution of species with different hydrophobicity was achieved using a gradient from 100% mobile phase A to 100% mobile phase B within 30 column volumes. Eluting species were detected at 280 nm and the retention time of the peak maximum was used to calculate the hydrophobicity index. This index is determined with respect to the linear regression (plot retention time vs. hydrophobicity index) of three reference molecules of defined hydrophobicity. This procedure allows compensating for potential run-to-run variability and variations due to differences between column batches and is independent of the exact absolute ammonium sulfate concentration. The lower the hydrophobicity index (= late elution from HIC), the more hydrophobic is the molecule and the higher is the risk of unfavorable behavior during production or storage of the drug substance and drug product.

It is understood that the examples and embodiments described herein are for illustrative purposes only and that various modifications or changes in light thereof will be suggested to persons skilled in the art and are to be included within the spirit and purview of this application and the scope of the appended claims.

## Claims

1. An immunoconjugate comprising a modified antibody or antibody fragment thereof, wherein said modified antibody or antibody fragment comprises a combination of substitution of two or more amino acids with cysteine on its constant regions wherein the combinations comprise substitutions selected from position 360 of an antibody heavy chain, and position 107 of an antibody kappa light chain, wherein said positions are numbered according to the EU system.

2. An immunoconjugate comprising a modified antibody or antibody fragment thereof, wherein said modified antibody or antibody fragment comprises a combination of substitution of two or more amino acids with cysteine on its constant regions wherein the combinations comprise substitutions selected from positions 152 and 375 of an antibody heavy chain, wherein said positions are numbered according to the EU system.

3. An immunoconjugate comprising a modified antibody or antibody fragment therefo comprising a heavy chain constant region of SEQ ID NO: 48 and a kappa light chain constant region comprising SEQ ID NO: 61.

4. An immunoconjugate comprising a modified antibody or antibody fragment thereof comprising a heavy chain constant region of SEQ ID NO: 131.

5. The immunoconjugates of any of claims 1-4 wherein the immunoconjugate further comprises a drug moiety.

6. The immunoconjugates of any of claims 1-5 wherein the drug antibody ratio is about 4.

7. The immunoconjugate of any of claim 1-6, wherein said drug moiety is attached to the modified antibody or antibody fragment through the sulfur of said cysteine and an optional linker.

8. The immunoconjugate of claim 7, wherein said drug moiety is connected to said sulfur of said cysteine through a cleavable or non-cleavable linker.

**9.** The immunoconjugate of claim 8, wherein said drug moiety is connected to said sulfur of said cysteine through a non-cleavable linker.

**10.** The immunoconjugate of claim 7-9, wherein said immunoconjugate comprises a thiol-maleimide linkage.

**11.** The immunoconjugate of claim 10, wherein said immunoconjugate comprises a $-S-CH_2-C(=O)-$ linkage or a disulfide linkage.

**12.** The immunoconjugate of claim 11, wherein said drug moiety is a cytotoxic agent.

**13.** The immunoconjugate of claim 12, wherein said drug moiety is selected from the group consisting of taxanes, DNA-alkylating agents (e.g., CC-1065 analogs), anthracyclines, tubulysin analogs, duocarmycin analogs, auristatin E, auristatin F, maytansinoids, and Eg5 inhibitors .

**14.** The immunoconjugate of any of claims 1-13, wherein said antibody is a monoclonal antibody.

**15.** The immunoconjugate of any of claims 1-13, wherein said antibody is a chimeric antibody.

**16.** The immunoconjugate of claim 1-13, wherein said antibody is a humanized or fully human antibody.

**17.** The immunoconjugate of any of claims 14-16, wherein said antibody is a bispecific or multi-specific antibody.

**18.** The immunoconjugate of any of claims 1-17, wherein said antibody or antibody fragment specifically binds to a cell surface marker on a tumor.

**19.** A pharmaceutical composition comprising the immunoconjugate of any of claims 1-18.

**20.** The modified antibody or antibody fragment of any of claims 1-19, further comprising at least one Pcl or unnatural amino acid substitution or a peptide tag for enzyme-mediated conjugation and/or combinations thereof.

**21.** A nucleic acid encoding the modified antibody or antibody fragment of any of claims 1-4.

**22.** A host cell comprising the nucleic acid of claim 21.

**23.** A method of producing a modified antibody or antibody fragment comprising incubating the host cell of claim 22 under suitable conditions for expressing the antibody or antibody fragment, and isolating said antibody or antibody fragment.

**24.** A method to produce an immunoconjugate, which comprises attaching a Linker Unit (LU) or a Linker Unit-Payload combination (-LU-X) to a cysteine residue in an antibody or antibody fragment of any of claims 1-4

**25.** The method of claim 24, wherein the immunoconjugate is of Formula (I):

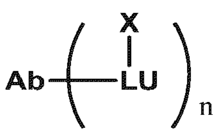

wherein Ab represents an antibody or antibody fragment comprising at least one cysteine residue at one of the preferred cysteine substitution sites described herein;
LU is a linker unit as described herein;
X is a payload or drug moiety;
and n is an integer from 1 to 16.

**26.** A modified antibody or antibody fragment thereof, wherein said modified antibody or antibody fragment comprises a combination of substitution of two or more amino acids with cysteine on its constant regions wherein the combinations comprise substitutions selected from position 360 of an antibody heavy chain, and position 107 of an antibody

kappa light chain, wherein said positions are numbered according to the EU system.

**27.** A modified antibody or antibody fragment thereof, wherein said modified antibody or antibody fragment comprises a combination of substitution of two or more amino acids with cysteine on its constant regions wherein the combinations comprise substitutions selected from positions 152 and 375 of an antibody heavy chain, wherein said positions are numbered according to the EU system.

**28.** A modified antibody or antibody fragment therefo comprising a heavy chain constant region of SEQ ID NO: 48 and a kappa light chain constant region comprising SEQ ID NO: 61.

**29.** A modified antibody or antibody fragment thereof comprising a heavy chain constant region of SEQ ID NO: 131.

```
trastuzumab, Ig gamma-1 chain C region                      119 STKGPSVFPLAPSSKSTSGGTAALGCLVKDYFPEPVTVSWNSGALTSGVHTFPAVLQSSGLYSLSSVVTVPSSSLGTQT 197
P01857[1-330], Ig gamma-1 chain C region, Homo sapiens      119 STKGPSVFPLAPSSKSTSGGTAALGCLVKDYFPEPVTVSWNSGALTSGVHTFPAVLQSSGLYSLSSVVTVPSSSLGTQT 197
P01859[1-326], Ig gamma-2 chain C region, Homo sapiens      119 STKGPSVFPLAPCSRSTSESTAALGCLVKDYFPEPVTVSWNSGALTSGVHTFPAVLQSSGLYSLSSVVTVPSSNFGTQT 197
P01860[1-377], Ig gamma-3 chain C region, Homo sapiens      119 STKGPSVFPLAPCSRSTSGGTAALGCLVKDYFPEPVTVSWNSGALTSGVHTFPAVLQSSGLYSLSSVVTVPSSSLGTQT 197
P01861[1-327], Ig gamma-4 chain C region, Homo sapiens      119 STKGPSVFPLAPCSRSTSESTAALGCLVKDYFPEPVTVSWNSGALTSGVHTFPAVLQSSGLYSLSSVVTVPSSSLGTKT 197

trastuzumab, Ig gamma-1 chain C region                      198 YICNVNHKPSNTKVDKKVE-----------------------------------------------PKSCDKTHTCPPCP 230
P01857[1-330], Ig gamma-1 chain C region, Homo sapiens      198 YICNVNHKPSNTKVDKKVE-----------------------------------------------PKSCDKTHTCPPCP 230
P01859[1-326], Ig gamma-2 chain C region, Homo sapiens      198 YTCNVDHKPSNTKVDKTVER--------------------------------------------------KCCVECPPCP 227
P01860[1-377], Ig gamma-3 chain C region, Homo sapiens      198 YTCNVNHKPSNTKVDKRVELKTPLGDTTHTCPRCPEPKSCDTPPPCPRCPEPKSCDTPPPCPRCPEPKSCDTPPPCPRCP 277
P01861[1-327], Ig gamma-4 chain C region, Homo sapiens      198 YTCNVDHKPSNTKVDKRVES--------------------------------------------------KYGPPCPSCP 227

trastuzumab, Ig gamma-1 chain C region                      231 APELLGGPSVFLFPPKPKDTLMISRTPEVTCVVVDVSHEDPEVKFNWYVDGVEVHNAKTKPREEQYNSTYRVVSVLTVLH 310
P01857[1-330], Ig gamma-1 chain C region, Homo sapiens      231 APELLGGPSVFLFPPKPKDTLMISRTPEVTCVVVDVSHEDPEVKFNWYVDGVEVHNAKTKPREEQYNSTYRVVSVLTVLH 310
P01859[1-326], Ig gamma-2 chain C region, Homo sapiens      228 APPVAG-PSVFLFPPKPKDTLMISRTPEVTCVVVDVSHEDPEVQFNWYVDGVEVHNAKTKPREEQFNSTFRVVSVLTVVH 306
P01860[1-377], Ig gamma-3 chain C region, Homo sapiens      278 APELLGGPSVFLFPPKPKDTLMISRTPEVTCVVVDVSHEDPEVQFKWYVDGVEVHNAKTKPREEQYNSTFRVVSVLTVLH 357
P01861[1-327], Ig gamma-4 chain C region, Homo sapiens      228 APEFLGGPSVFLFPPKPKDTLMISRTPEVTCVVVDVSQEDPEVQFNWYVDGVEVHNAKTKPREEQFNSTYRVVSVLTVLH 307

trastuzumab, Ig gamma-1 chain C region                      311 QDWLNGKEYKCKVSNKALPAPIEKTISKAKGQPREPQVYTLPPSREEMTKNQVSLTCLVKGFYPSDIAVEWESNGQPENN 390
P01857[1-330], Ig gamma-1 chain C region, Homo sapiens      311 QDWLNGKEYKCKVSNKALPAPIEKTISKAKGQPREPQVYTLPPSRDELTKNQVSLTCLVKGFYPSDIAVEWESNGQPENN 390
P01859[1-326], Ig gamma-2 chain C region, Homo sapiens      307 QDWLNGKEYKCKVSNKGLPAPIEKTISKTKGQPREPQVYTLPPSREEMTKNQVSLTCLVKGFYPSDISVEWESNGQPENN 386
P01860[1-377], Ig gamma-3 chain C region, Homo sapiens      358 QDWLNGKEYKCKVSNKALPAPIEKTISKTKGQPREPQVYTLPPSREEMTKNQVSLTCLVKGFYPSDIAVEWESSGQPENN 437
P01861[1-327], Ig gamma-4 chain C region, Homo sapiens      308 QDWLNGKEYKCKVSNKGLPSSIEKTISKAKGQPREPQVYTLPPSQEEMTKNQVSLTCLVKGFYPSDIAVEWESNGQPENN 387

trastuzumab, Ig gamma-1 chain C region                      391 YKTTPPVLDSDGSFFLYSKLTVDKSRWQQGNVFSCSVMHEALHNHYTQKSLSLSPG 446 (SEQ ID NO: 155)
P01857[1-330], Ig gamma-1 chain C region, Homo sapiens      391 YKTTPPVLDSDGSFFLYSKLTVDKSRWQQGNVFSCSVMHEALHNHYTQKSLSLSPG 446 (SEQ ID NO: 151)
P01859[1-326], Ig gamma-2 chain C region, Homo sapiens      387 YKTTPPMLDSDGSFFLYSKLTVDKSRWQQGNVFSCSVMHEALHNHYTQKSLSLSPG 442 (SEQ ID NO: 152)
P01860[1-377], Ig gamma-3 chain C region, Homo sapiens      438 YNTTPPMLDSDGSFFLYSKLTVDKSRWQQGNIFSCSVMHEALHNRFTQKSLSLSPG 493 (SEQ ID NO: 153)
P01861[1-327], Ig gamma-4 chain C region, Homo sapiens      388 YKTTPPVLDSDGSFFLYSRLTVDKSRWQEGNVFSCSVMHEALHNHYTQKSLSLSLG 443 (SEQ ID NO: 154)
```

Figure 1

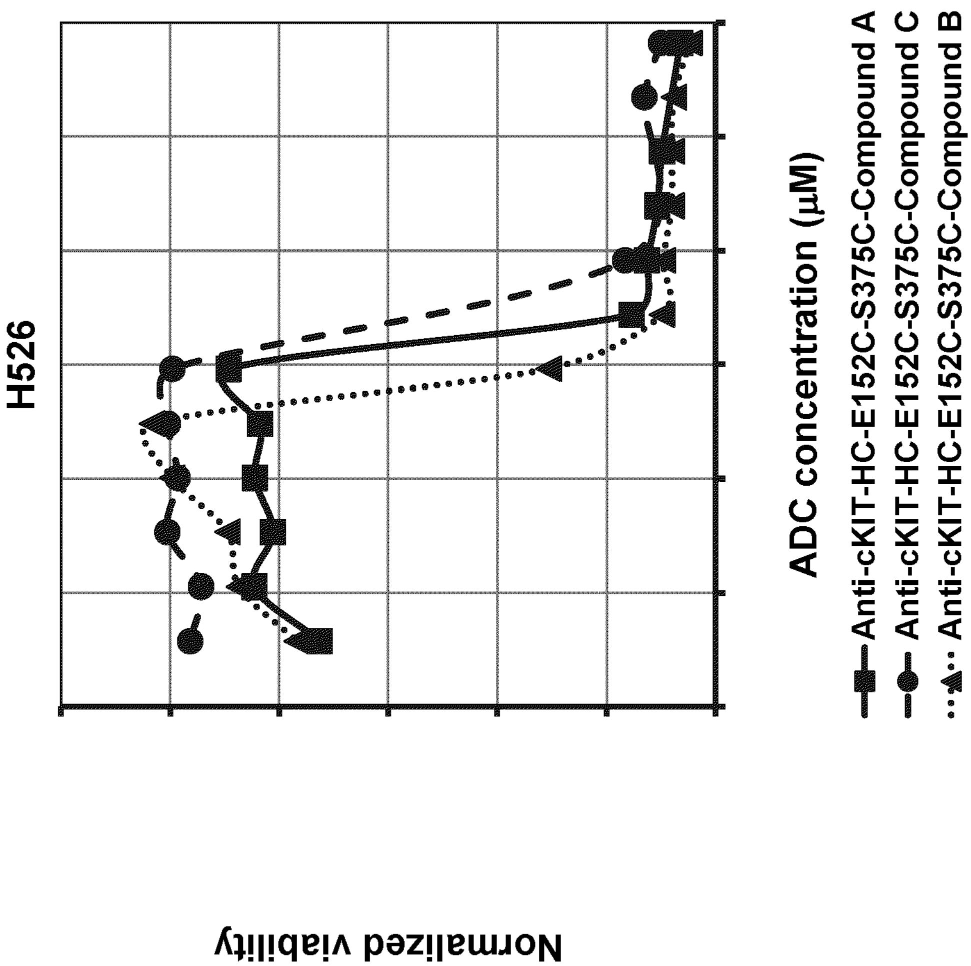
H526
Normalized viability
ADC concentration (μM)
Anti-cKIT-HC-E152C-S375C-Compound A
Anti-cKIT-HC-E152C-S375C-Compound C
Anti-cKIT-HC-E152C-S375C-Compound B

Figure 2

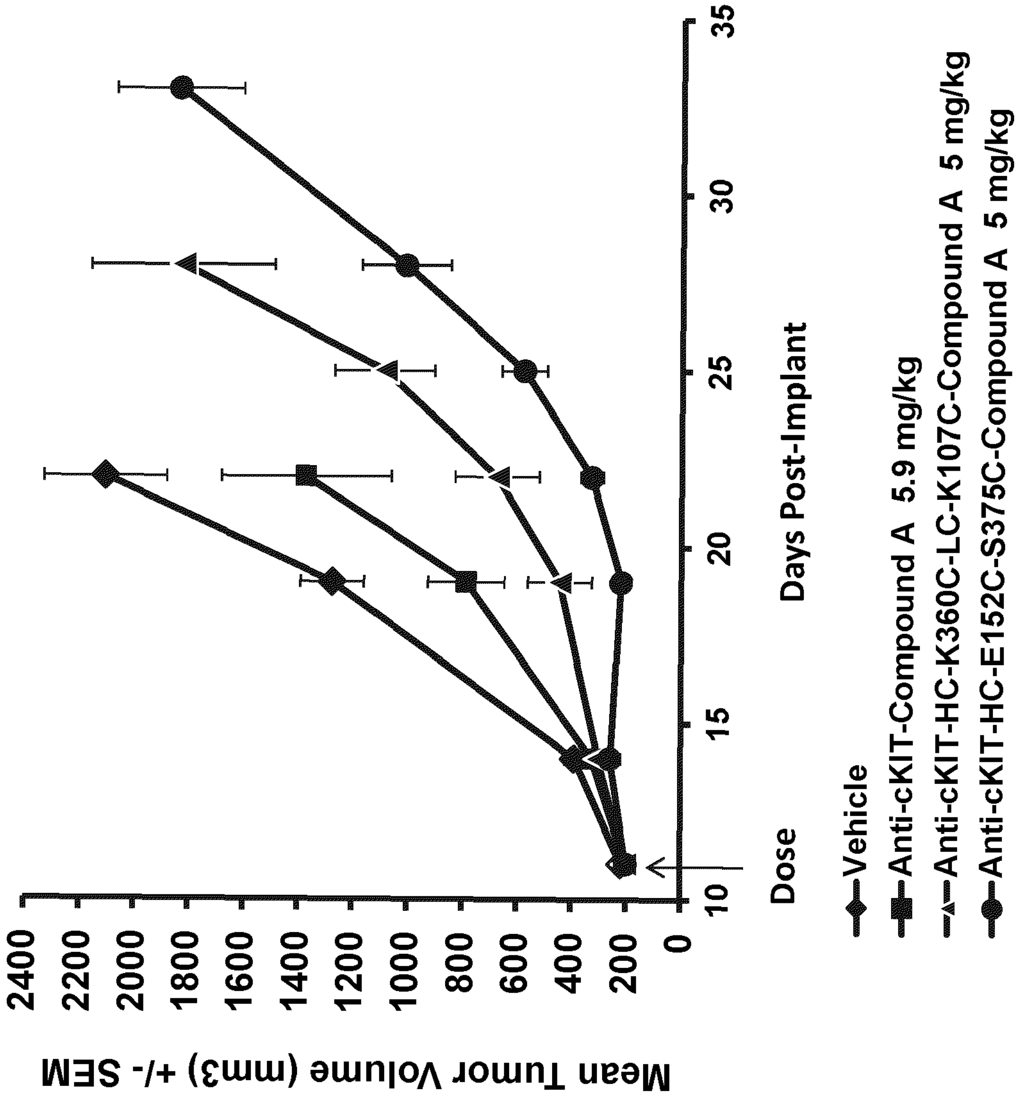
Mean Tumor Volume (mm3) +/- SEM
0
200
400
600
800
1000
1200
1400
1600
1800
2000
2200
2400
10
15
20
25
30
35
Dose
Days Post-Implant
Vehicle
Anti-cKIT-Compound A 5.9 mg/kg
Anti-cKIT-HC-K360C-LC-K107C-Compound A 5 mg/kg
Anti-cKIT-HC-E152C-S375C-Compound A 5 mg/kg

Figure 3A

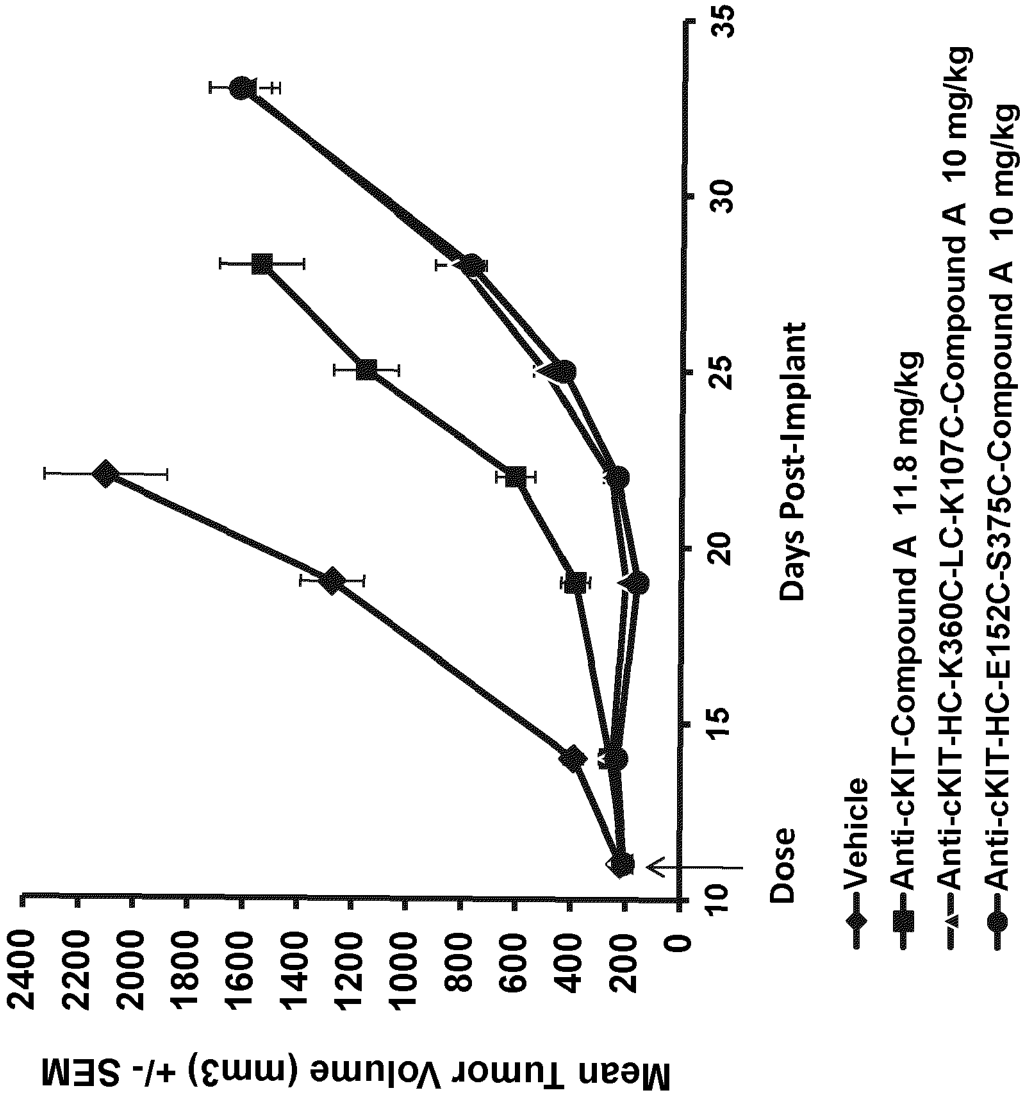
Mean Tumor Volume (mm3) +/- SEM
0
200
400
600
800
1000
1200
1400
1600
1800
2000
2200
2400
10
15
20
25
30
35
Dose
Days Post-Implant
Vehicle
Anti-cKIT-Compound A 11.8 mg/kg
Anti-cKIT-HC-K360C-LC-K107C-Compound A 10 mg/kg
Anti-cKIT-HC-E152C-S375C-Compound A 10 mg/kg

Figure 3B

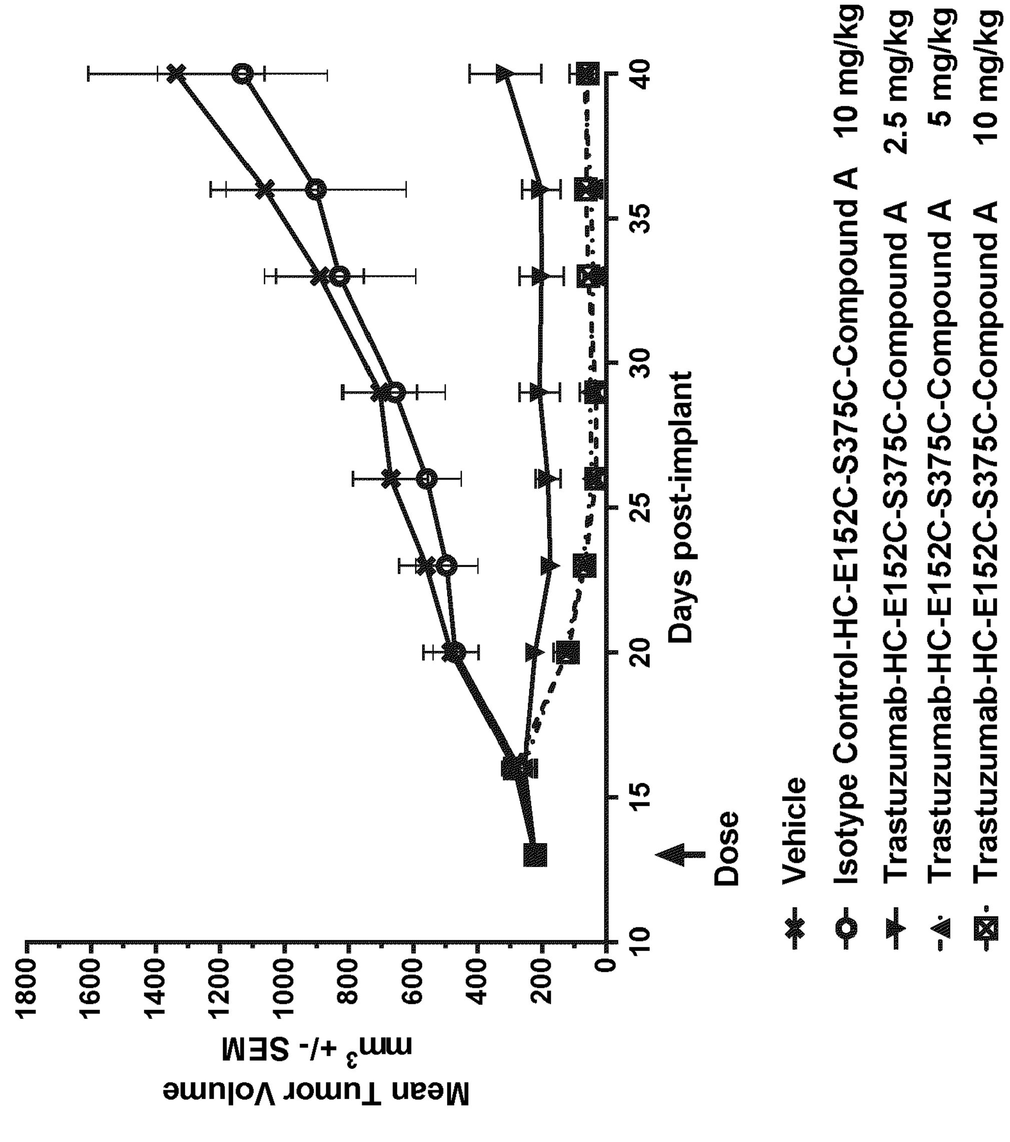
Mean Tumor Volume
mm$^3$ +/- SEM
0
200
400
600
800
1000
1200
1400
1600
1800
10
15
20
25
30
35
40
Days post-implant
Dose
Vehicle
Isotype Control-HC-E152C-S375C-Compound A 10 mg/kg
Trastuzumab-HC-E152C-S375C-Compound A 2.5 mg/kg
Trastuzumab-HC-E152C-S375C-Compound A 5 mg/kg
Trastuzumab-HC-E152C-S375C-Compound A 10 mg/kg

Figure 4

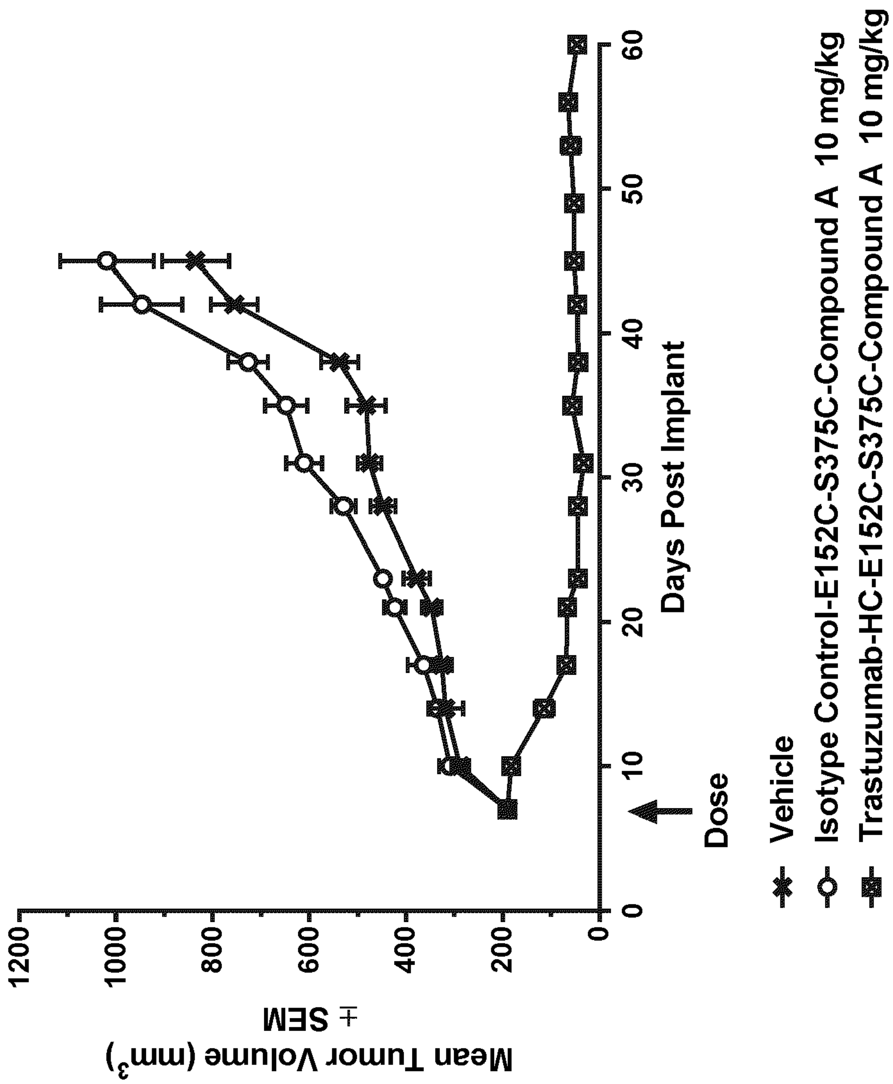
Mean Tumor Volume (mm3) ± SEM
1200
1000
800
600
400
200
0
0
10
20
30
40
50
60
Days Post Implant
Dose
Vehicle
Isotype Control-E152C-S375C-Compound A 10 mg/kg
Trastuzumab-HC-E152C-S375C-Compound A 10 mg/kg

Figure 5

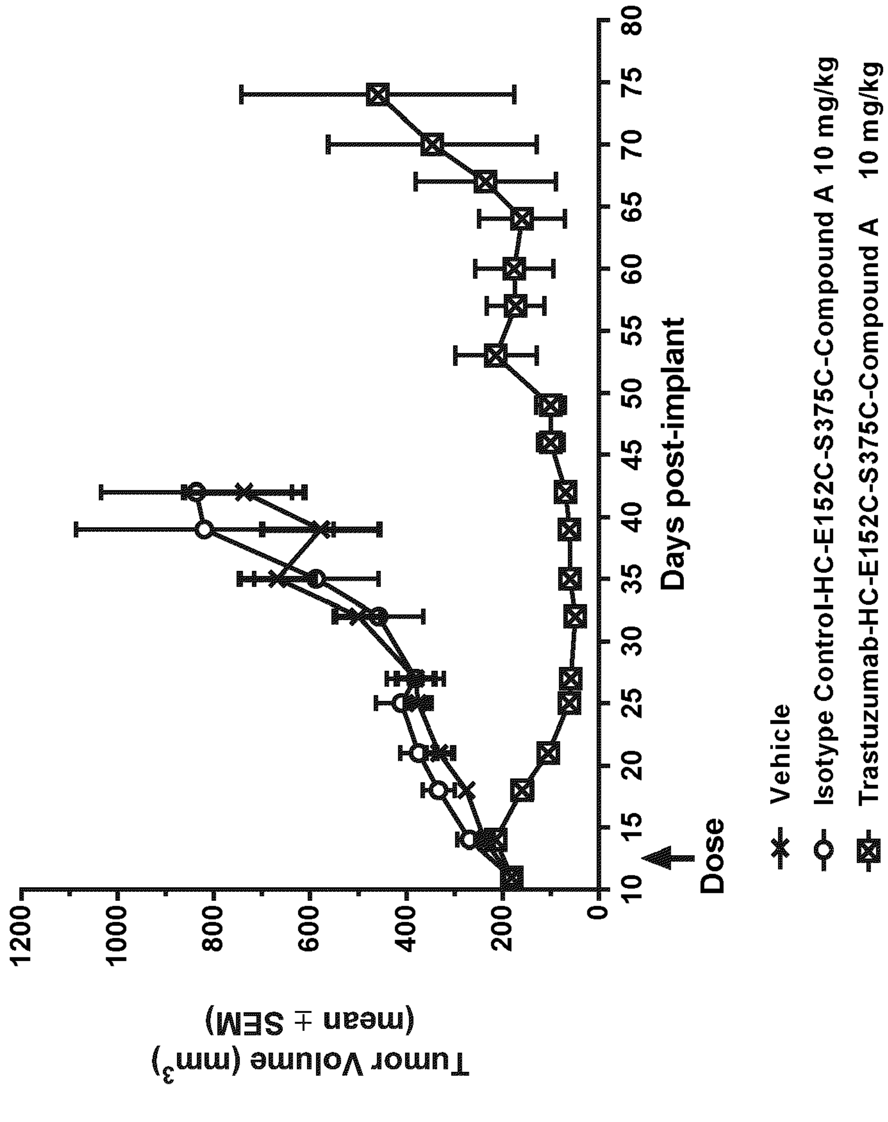
Tumor Volume (mm3)
(mean ± SEM)
1200
1000
800
600
400
200
0
10
15
20
25
30
35
40
45
50
55
60
65
70
75
80
Days post-implant
Dose
Vehicle
Isotype Control-HC-E152C-S375C-Compound A 10 mg/kg
Trastuzumab-HC-E152C-S375C-Compound A 10 mg/kg

Figure 6

Figure 7

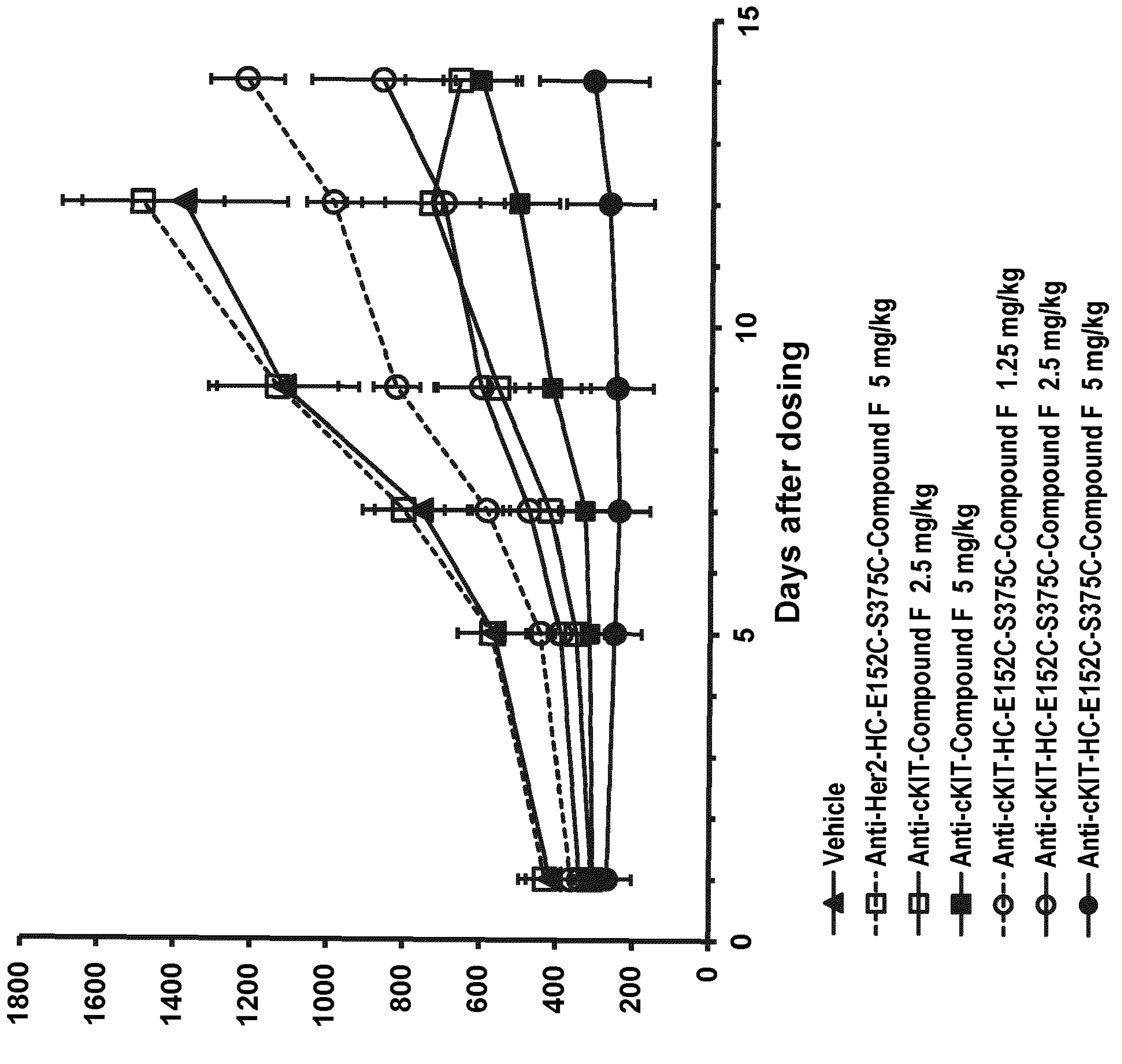
Mean tumor volume (mm³ +/- SEM)
1800
1600
1400
1200
1000
800
600
400
200
0
0
5
10
15
Days after dosing
Vehicle
Anti-Her2-HC-E152C-S375C-Compound F 5 mg/kg
Anti-cKIT-Compound F 2.5 mg/kg
Anti-cKIT-Compound F 5 mg/kg
Anti-cKIT-HC-E152C-S375C-Compound F 1.25 mg/kg
Anti-cKIT-HC-E152C-S375C-Compound F 2.5 mg/kg
Anti-cKIT-HC-E152C-S375C-Compound F 5 mg/kg

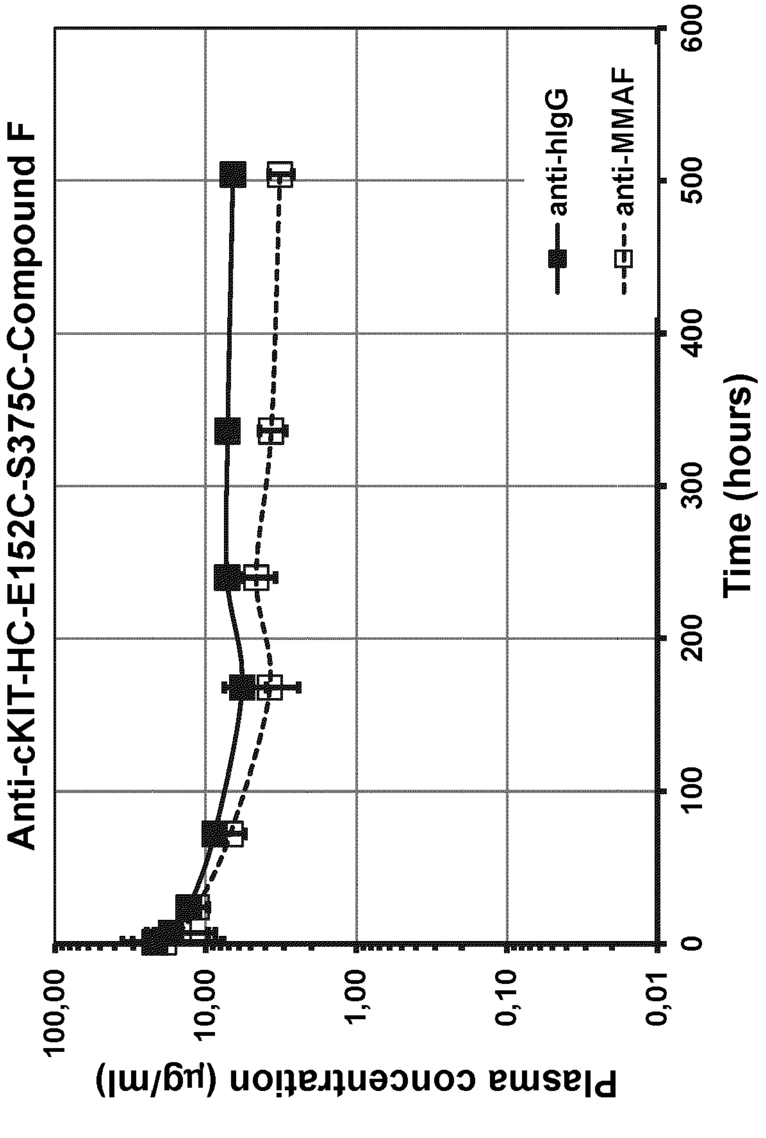
Anti-cKIT-HC-E152C-S375C-Compound F
Plasma concentration (µg/ml)
100,00
10,00
1,00
0,10
0,01
0
100
200
300
400
500
600
Time (hours)
anti-hIgG
anti-MMAF

Figure 8A

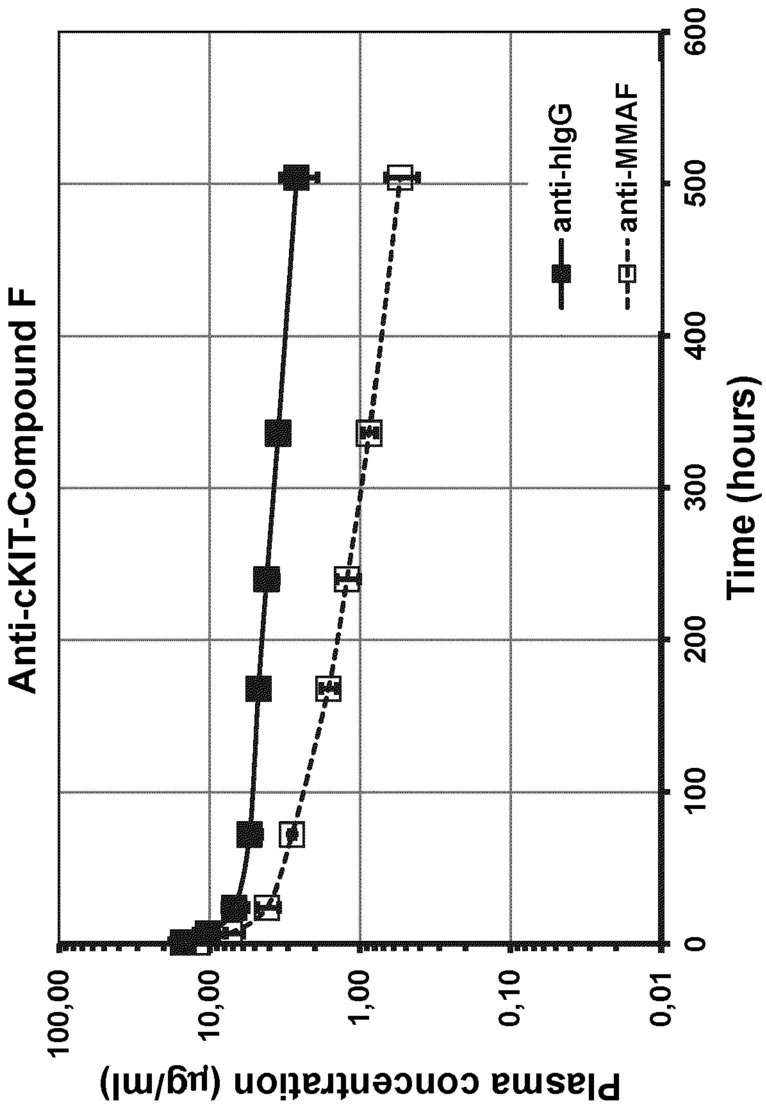
Anti-cKIT-Compound F
Plasma concentration (µg/ml)
100,00
10,00
1,00
0,10
0,01
0
100
200
300
400
500
600
Time (hours)
anti-hIgG
anti-MMAF

Figure 8B

Figure 9

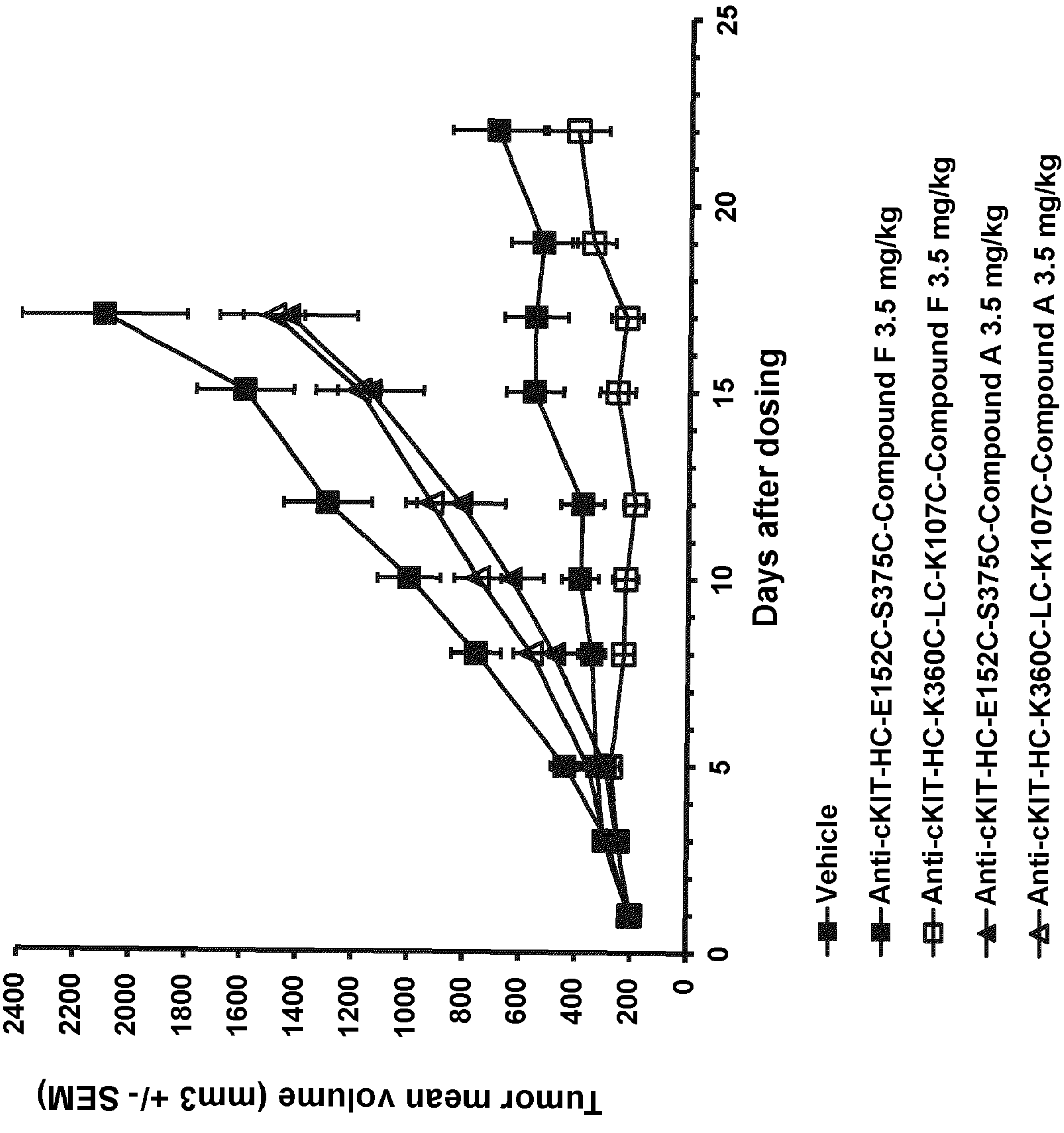
Tumor mean volume (mm3 +/- SEM)
0
200
400
600
800
1000
1200
1400
1600
1800
2000
2200
2400
Days after dosing
0
5
10
15
20
25
Vehicle
Anti-cKIT-HC-E152C-S375C-Compound F 3.5 mg/kg
Anti-cKIT-HC-K360C-LC-K107C-Compound F 3.5 mg/kg
Anti-cKIT-HC-E152C-S375C-Compound A 3.5 mg/kg
Anti-cKIT-HC-K360C-LC-K107C-Compound A 3.5 mg/kg

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011005481 A
- US 6176236 A
- US 6703199 B
- US 5641870 A
- US 20030153043 A, Carr
- US 5677425 A, Bodmer
- US 6165745 A, Ward
- US 5624821 A
- US 5648260 A, Winter
- US 6194551 B, Idusogie
- WO 9429351 A, Bodmer
- WO 0042072 A, Presta
- US 5714350 A
- US 6350861 B, Co
- EP 1176195 A, Hang
- WO 03035835 A, Presta
- WO 9954342 A, Umana
- US 6277375 B, Ward
- US 5869046 A
- US 6121022 A, Presta
- WO 0138318 A
- US 0302675 W
- US 5475092 A
- US 6340701 B
- US 6372738 B
- US 6436931 B
- US 20010036923 A1
- US 024290
- US 10116053 B
- WO 0149698 A
- US 20090304721 A
- US 5605793 A
- US 5811238 A
- US 5830721 A
- US 5834252 A
- US 5837458 A
- US 6350466 B
- US 6316024 B
- US 6019968 A
- US 5985320 A
- US 5985309 A
- US 5934272 A
- US 5874064 A
- US 5855913 A
- US 5290540 A
- US 4880078 A
- WO 9219244 A
- WO 9732572 A
- WO 9744013 A
- WO 9831346 A
- WO 9966903 A
- US 5679377 A
- US 5916597 A
- US 5912015 A
- US 5989463 A
- US 5128326 A
- WO 9915154 A
- WO 9920253 A
- US 4526938 A
- WO 9105548 A
- WO 9620698 A
- US 4522811 A
- US 5374548 A
- US 5399331 A
- US 5416016 A, Low
- US 2014024795 W
- US 2014070800 W

### Non-patent literature cited in the description

- **JUNUTULA et al.** *Nat Biotechnol.,* 2008, vol. 26 (8), 925-932
- **LYONS et al.** *Protein Eng.,* 1990, vol. 3, 703-708
- **VOYNOV et al.** *Bioconjug. Chem.,* 2010, vol. 21 (2), 385-392
- **CHEN et al.** *mAbs,* 2009, vol. 1 (6), 563-571
- **JUNUTULA et al.** *Nat. Biotechnol.,* 2008, vol. 26 (8), 925-32
- **ALLEY et al.** *Bioconjug Chem.,* 2008, vol. 19 (3), 759-65
- **SHEN et al.** *Nat. Biotechnol.,* 2012, vol. 30 (2), 184-9
- **CHEN et al.** *mAbs,* 2009, vol. 16, 353-571
- **LIU et al.** *Annu. Rev. Biochem.,* 2010, vol. 79, 413-444
- **OU et al.** *Proc. Natl. Acad. Sci. USA,* 2011, vol. 108, 10437-10442
- **CELLITTI et al.** *Nat. Chem. Biol.,* 2011, vol. 7 (8), 528-30
- **WARD et al.** *Nature,* 1989, vol. 341, 544-546

- **BIRD et al.** *Science,* 1988, vol. 242, 423-426
- **HUSTON et al.** *Proc. Natl. Acad. Sci.,* 1988, vol. 85, 5879-5883
- **HOLLINGER ; HUDSON.** *Nature Biotechnology,* 2005, vol. 23, 1126-1136
- **ZAPATA et al.** *Protein Eng.,* 1995, vol. 8, 1057-1062
- **KNAPPIK et al.** *J. Mol. Biol.,* 2000, vol. 296, 57-86
- **MORRISON et al.** *Proc. Natl. Acad. Sci. USA,* 1984, vol. 81, 6851-6855
- **MORRISON ; OI.** *Adv. Immunol.,* 1988, vol. 44, 65-92
- **VERHOEYEN et al.** *Science,* 1988, vol. 239, 1534-1536
- **PADLAN.** *Molec. Immun.,* 1991, vol. 28, 489-498
- **PADLAN.** *Molec. Immun.,* 1994, vol. 31 (3), 169-217
- Epitope Mapping Protocols in Methods in Molecular Biology. 1996, vol. 66
- **CREIGHTON.** *Proteins,* 1984
- **SMITH ; WATERMAN.** *Adv. Appl. Math.,* 1970, vol. 2, 482c
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **BRENT et al.** *Current Protocols in Molecular Biology,* 2003
- **ALTSCHUL et al.** *Nuc. Acids Res.,* 1977, vol. 25, 3389-3402
- **ALTSCHUL et al.** *J. Mol. Biol.,* 1990, vol. 215, 403-410
- **HENIKOFF ; HENIKOFF.** *Proc. Natl. Acad. Sci. USA,* 1989, vol. 89, 10915
- **KARLIN ; ALTSCHUL.** *Proc. Natl. Acad. Sci. USA,* 1993, vol. 90, 5873-5787
- **E. MEYERS ; W. MILLER.** *Comput. Appl. Biosci.,* 1988, vol. 4, 11-17
- **NEEDLEMAN ; WUNSCH.** *J. Mol. Biol.,* 1970, vol. 48, 444-453
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **SINGH et al.** *Therapeutic Antibodies: Methods and Protocols,* 2009, vol. 525, 445-457
- Remington's Pharmaceutical Sciences. Mack Publishing Company, 1985
- **STAHL ; WERMUTH.** Handbook of Pharmaceutical Salts: Properties, Selection, and Use. Wiley-VCH, 2002
- Remington's Pharmaceutical Sciences. Mack Printing Company, 1990, 1289-1329
- **EDELMAN et al.** *Proc. Natl. Acad. USA,* 1969, vol. 63, 78-85
- **DUCRY et al.** *Bioconjugate Chem,* 2010, vol. 21 (1), 5-13
- **JEFFERIS.** *MAbs,* 2009, vol. 1, 332-338
- **SHIELDS et al.** *J. Biol. Chem.,* 2001, vol. 276, 6591-6604
- **SHIELDS et al.** *J. Biol. Chem.,* 2002, vol. 277, 26733-26740
- **UMANA et al.** *Nat. Biotech.,* 1999, vol. 17, 176-180
- **SASSE et al.** *J. Antibiot. (Tokyo),* 2000, vol. 53, 879-85
- **SUZAWA et al.** *Bioorg. Med. Chem.,* 2000, vol. 8, 2175-84
- **ICHIMURA et al.** *J. Antibiot. (Tokyo),* 1991, vol. 44, 1045-53
- **FRANCISCO et al.** *Blood,* 2003
- **SAITO et al.** *Adv. Drug Deliv. Rev.,* 2003, vol. 55, 199-215
- **TRAIL et al.** *Cancer Immunol. Immunother.,* 2003, vol. 52, 328-337
- **PAYNE.** *Cancer Cell,* 2003, vol. 3, 207-212
- **ALLEN.** *Nat. Rev. Cancer,* 2002, vol. 2, 750-763
- **PASTAN ; KREITMAN.** *Curr. Opin. Investig. Drugs,* 2002, vol. 3, 1089-1091
- **SENTER ; SPRINGER.** *Adv. Drug Deliv. Rev.,* 2001, vol. 53, 247-264
- **DENARDO et al.** *Clin. Cancer Res.,* 1998, vol. 4 (10), 2483-90
- **PETERSON et al.** *Bioconjug. Chem.,* 1999, vol. 10 (4), 553-7
- **ZIMMERMAN et al.** *Nucl. Med. Biol.,* 1999, vol. 26 (8), 943-50
- **PATTEN et al.** *Curr. Opinion Biotechnol.,* 1997, vol. 8, 724-33
- **HARAYAMA.** *Trends Biotechnol,* 1998, vol. 16 (2), 76-82
- **HANSSON et al.** *J. Mol. Biol.,* 1999, vol. 287, 265-76
- **LORENZO ; BLASCO.** *Biotechniques,* 1998, vol. 24 (2), 308-313
- **GENTZ et al.** *Proc. Natl. Acad. Sci. USA,* 1989, vol. 86, 821-824
- **WILSON et al.** *Cell,* 1984, vol. 37, 767
- **A. EINHAUER et al.** *J. Biochem. Biophys. Methods,* 2001, vol. 49, 455-465
- **HARDMAN et al.** Goodman and Gilman's The Pharmacological Basis of Therapeutics. McGraw-Hill, 2001
- **GENNARO.** Remington: The Science and Practice of Pharmacy. Lippincott, Williams, and Wilkins, 2000
- Pharmaceutical Dosage Forms: Parenteral Medications. Marcel Dekker, 1993
- Pharmaceutical Dosage Forms: Tablets. Marcel Dekker, 1990

- Pharmaceutical Dosage Forms: Disperse Systems. Marcel Dekker, 1990
- **WEINER ; KOTKOSKIE.** Excipient Toxicity and Safety. Marcel Dekker, Inc, 2000
- **WAWRZYNCZAK.** Antibody Therapy. Bios Scientific Pub. Ltd, 1996
- Monoclonal Antibodies, Cytokines and Arthritis. Marcel Dekker, 1991
- Monoclonal Antibodies and Peptide Therapy in Autoimmune Diseases. Marcel Dekker, 1993
- **BAERT et al.** *New Engl. J. Med.,* 2003, vol. 348, 601-608
- **MILGROM et al.** *New Engl. J. Med.,* 1999, vol. 341, 1966-1973
- **SLAMON et al.** *New Engl. J. Med.,* 2001, vol. 344, 783-792
- **BENIAMINOVITZ et al.** *New Engl. J. Med.,* 2000, vol. 342, 613-619
- **GHOSH et al.** *New Engl. J. Med.,* 2003, vol. 348, 24-32
- **LIPSKY et al.** *New Engl. J. Med.,* 2000, vol. 343, 1594-1602
- **MAYNARD et al.** A Handbook of SOPs for Good Clinical Practice. Interpharm Press, 1996
- **DENT.** Good Laboratory and Good Clinical Practice. Urch Publ, 2001
- **SIDMAN et al.** *Biopolymers,* 1983, vol. 22, 547-556
- **LANGER et al.** *J. Biomed. Mater. Res.,* 1981, vol. 15, 167-277
- **LANGER.** *Chem. Tech.,* 1982, vol. 12, 98-105
- **EPSTEIN et al.** *Proc. Natl. Acad. Sci. USA,* 1985, vol. 82, 3688-3692
- **HWANG et al.** *Proc. Natl. Acad. Sci. USA,* 1980, vol. 77, 4030-4034
- **SEFTON.** *CRC Crit. Ref Biomed. Eng.,* 1987, vol. 14, 20
- **BUCHWALD et al.** *Surgery,* 1980, vol. 88, 507
- **SAUDEK et al.** *N. Engl. J. Med.,* 1989, vol. 321, 574
- Medical Applications of Controlled Release. CRC Pres, 1974
- Controlled Drug Bioavailability, Drug Product Design and Performance. Wiley, 1984
- **RANGER ; PEPPAS.** *J. Macromol. Sci. Rev. Macromol. Chem.,* 1983, vol. 23, 61
- **LEVY et al.** *Science,* 1985, vol. 228, 190
- **DURING et al.** *Ann. Neurol.,* 1989, vol. 25, 351
- **HOWARD et al.** *J. Neurosurg,* 1989, vol. 7 1, 105
- **LANGER.** *Science,* 1990, vol. 249, 1527-1533
- **NING et al.** *Radiotherapy & Oncology,* 1996, vol. 39, 179-189
- **SONG et al.** *PDA Journal of Pharmaceutical Science & Technology,* 1995, vol. 50, 372-397
- **CLEEK et al.** *Pro. Int'l. Symp. Control. Rel. Bioact. Mater.,* 1997, vol. 24, 853-854
- **LAM et al.** *Proc. Int'l. Symp. Control Rel. Bioact. Mater.,* 1997, vol. 24, 759-760
- Remington's Pharmaceutical Sciences and Introduction to Pharmaceutical Dosage Forms. Mack Pub. Co, 1995
- Goodman and Gilman's The Pharmacological Basis of Therapeutics. McGraw-Hill, 2001
- Pharmacotherapeutics for Advanced Practice: A Practical Approach. Lippincott, Williams & Wilkins, 2001
- Cancer Chemotherapy and Biotherapy. Lippincott, Williams & Wilkins, 2001
- **RANADE.** *J. Clin. Pharmacol.,* 1989, vol. 29, 685
- **UMEZAWA et al.** *Biochem. Biophys. Res. Commun.,* 1988, vol. 153, 1038
- **BLOEMAN et al.** *FEBS Lett.,* 1995, vol. 357, 140
- **OWAIS et al.** *Antimicrob. Agents Chemother,* 1995, vol. 39, 180
- **BRISCOE et al.** *Am. J. Physiol.,* 1995, vol. 1233, 134
- **SCHREIER et al.** *J. Biol. Chem.,* 1994, vol. 269, 9090
- **K. KEINANEN ; M. L. LAUKKANEN.** *FEBS Lett.,* 1994, vol. 346, 123
- **J. J. KILLION ; I. J. FIDLER.** *Immunomethods,* 1994, vol. 4, 273
- **KLOCK et al.** *Methods Mol Biol.,* 2009, vol. 498, 91-103
- **EDELMAN et al.** *Proc Natl Acad Sci USA,* 1969, vol. 63, 78-85
- **MEISSNER et al.** *Biotechnol Bioeng.,* 2001, vol. 75, 197-203
- **GAZDAR A ; RABINOVSKY R ; YEFENOF E ; GORDON B ; VITETTA ES.** *Breast Cancer Res Treat,* 2000, vol. 61, 217-228
- **RISS et al.** *Assay Drug Dev Technol.,* 2004, vol. 2, 51-62
- **MELNICK et al.** *Proc Natl Acad Sci USA.,* 2006, vol. 103, 3153-3158
- **HAMBLETT et al.** Effects of drug loading on the antitumor activity of a monoclonal antibody drug conjugate. *Clin Cancer Res.,* 2004, vol. 10, 7063-7070
- **ALLEY et al.** *Bioconjug Chem,* 2008, vol. 19, 759-765
- **DORONINA et al.** *Nat. Biotechnol.,* 2003, vol. 21, 778-784
- **SAUSVILLE ; BURGER.** *Cancer Res,* 2006, vol. 66, 3351-3354
- Guide for the Care and Use of Laboratory Animals. NIH publication; National Academy Press, 2001
- **MORTON ; HOUGHTON.** *Nat Protoc,* 2007, vol. 2, 247-250

SEQUENCE LISTING

<110> IRM LLC

<120> SPECIFIC SITES FOR MODIFYING ANTIBODIES TO MAKE IMMUNOCONJUGATES

<130> PAT056151-WO-PCT

<140> PCT/US2015/019984
<141> 2015-03-11

<150> 61/952,026
<151> 2014-03-12

<160> 156

<170> PatentIn version 3.5

<210> 1
<211> 450
<212> PRT
<213> Homo sapiens

<400> 1

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160
```

```
Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415
```

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 2
<211> 331
<212> PRT
<213> Artificial Sequence

<220>
<221> source
<223> /note="Description of Artificial Sequence: Synthetic polypeptide"

<400> 2
Cys Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 3
<211> 331
<212> PRT
<213> Artificial Sequence

<220>
<221> source
<223> /note="Description of Artificial Sequence: Synthetic polypeptide"

<400> 3
Ser Ala Cys Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

```
Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
```

```
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330


<210> 4
<211> 331
<212> PRT
<213> Artificial Sequence

<220>
<221> source
<223> /note="Description of Artificial Sequence: Synthetic
      polypeptide"

<400> 4
Ser Ala Ser Thr Cys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
```

145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 5
<211> 331
<212> PRT
<213> Artificial Sequence

<220>
<221> source
<223> /note="Description of Artificial Sequence: Synthetic polypeptide"

<400> 5
Ser Ala Ser Thr Lys Gly Pro Cys Val Phe Pro Leu Ala Pro Ser Ser
1 5 10 15

Lys Ser Thr Ser Gly Gly Thr Ala Ala Leu Gly Cys Leu Val Lys Asp

20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

```
Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330


<210> 6
<211> 331
<212> PRT
<213> Artificial Sequence

<220>
<221> source
<223> /note="Description of Artificial Sequence: Synthetic
      polypeptide"

<400> 6
Ser Ala Ser Thr Lys Gly Pro Ser Val Phe Pro Leu Ala Pro Ser Cys
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140
```

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 7
<211> 331
<212> PRT
<213> Artificial Sequence

<220>
<221> source
<223> /note="Description of Artificial Sequence: Synthetic
      polypeptide"

<400> 7
Ser Ala Ser Thr Lys Gly Pro Ser Val Phe Pro Leu Ala Pro Ser Ser
1               5                   10                  15

Lys Cys Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 8
<211> 331
<212> PRT
<213> Artificial Sequence

<220>
<221> source
<223> /note="Description of Artificial Sequence: Synthetic
      polypeptide"

<400> 8
Ser Ala Ser Thr Lys Gly Pro Ser Val Phe Pro Leu Ala Pro Ser Ser
1               5                   10                  15

Lys Ser Thr Cys Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 9
<211> 331
<212> PRT
<213> Artificial Sequence

<220>
<221> source
<223> /note="Description of Artificial Sequence: Synthetic polypeptide"

<400> 9
Ser Ala Ser Thr Lys Gly Pro Ser Val Phe Pro Leu Ala Pro Ser Ser
1 5 10 15

Lys Ser Thr Ser Gly Gly Cys Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 10
<211> 331
<212> PRT
<213> Artificial Sequence

<220>
<221> source
<223> /note="Description of Artificial Sequence: Synthetic polypeptide"

<400> 10
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Cys Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 11
<211> 331
<212> PRT
<213> Artificial Sequence

<220>
<221> source
<223> /note="Description of Artificial Sequence: Synthetic polypeptide"

<400> 11
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
            20                  25                  30

Tyr Phe Pro Glu Cys Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu

260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 12
<211> 331
<212> PRT
<213> Artificial Sequence

<220>
<221> source
<223> /note="Description of Artificial Sequence: Synthetic polypeptide"

<400> 12
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Cys Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr

130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 13
<211> 331
<212> PRT
<213> Artificial Sequence

<220>
<221> source
<223> /note="Description of Artificial Sequence: Synthetic polypeptide"

<400> 13
Ser Ala Ser Thr Lys Gly Pro Ser Val Phe Pro Leu Ala Pro Ser Ser

```
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Cys Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255
```

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 14
<211> 331
<212> PRT
<213> Artificial Sequence

<220>
<221> source
<223> /note="Description of Artificial Sequence: Synthetic polypeptide"

<400> 14
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Cys
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 15
<211> 331
<212> PRT
<213> Artificial Sequence

<220>
<221> source
<223> /note="Description of Artificial Sequence: Synthetic
polypeptide"

<400> 15

Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Cys Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

```
Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330


<210> 16
<211> 331
<212> PRT
<213> Artificial Sequence

<220>
<221> source
<223> /note="Description of Artificial Sequence: Synthetic
      polypeptide"

<400> 16
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Cys Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125
```

```
Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 17
<211> 331
<212> PRT
<213> Artificial Sequence

<220>
<221> source
<223> /note="Description of Artificial Sequence: Synthetic
      polypeptide"
```

<400> 17

```
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Cys Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255
```

```
Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330


<210> 18
<211> 331
<212> PRT
<213> Artificial Sequence

<220>
<221> source
<223> /note="Description of Artificial Sequence: Synthetic
      polypeptide"

<400> 18
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Cys Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125
```

```
Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330


<210> 19
<211> 331
<212> PRT
<213> Artificial Sequence

<220>
<221> source
<223> /note="Description of Artificial Sequence: Synthetic
      polypeptide"
```

<400> 19

Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Cys Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe

245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 20
<211> 331
<212> PRT
<213> Artificial Sequence

<220>
<221> source
<223> /note="Description of Artificial Sequence: Synthetic polypeptide"

<400> 20
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Cys Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro

115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 21
<211> 331
<212> PRT
<213> Artificial Sequence

<220>
<221> source
<223> /note="Description of Artificial Sequence: Synthetic

polypeptide"

<400> 21
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Cys Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 22
<211> 331
<212> PRT
<213> Artificial Sequence

<220>
<221> source
<223> /note="Description of Artificial Sequence: Synthetic polypeptide"

<400> 22
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Cys Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 23
<211> 331
<212> PRT
<213> Artificial Sequence

<220>
<221> source

<223> /note="Description of Artificial Sequence: Synthetic
polypeptide"

<400> 23

Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Cys Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

```
Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330


<210> 24
<211> 331
<212> PRT
<213> Artificial Sequence

<220>
<221> source
<223> /note="Description of Artificial Sequence: Synthetic
      polypeptide"

<400> 24
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Cys Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110
```

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 25
<211> 331
<212> PRT
<213> Artificial Sequence

<220>

<221> source
<223> /note="Description of Artificial Sequence: Synthetic polypeptide"

<400> 25
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Cys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 26
<211> 331
<212> PRT
<213> Artificial Sequence

<220>
<221> source
<223> /note="Description of Artificial Sequence: Synthetic polypeptide"

<400> 26
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Cys Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 27
<211> 331
<212> PRT
<213> Artificial Sequence

<220>
<221> source
<223> /note="Description of Artificial Sequence: Synthetic polypeptide"

<400> 27

```
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Cys
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
```

225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 28
<211> 331
<212> PRT
<213> Artificial Sequence

<220>
<221> source
<223> /note="Description of Artificial Sequence: Synthetic polypeptide"

<400> 28
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro

100 105 110

Cys Pro Ala Pro Glu Leu Leu Gly Gly Pro Ser Val Phe Leu Phe Pro
115 120 125

Pro Cys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 29
<211> 331
<212> PRT
<213> Artificial Sequence

<220>
<221> source
<223> /note="Description of Artificial Sequence: Synthetic
      polypeptide"

<400> 29
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Cys Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 30
<211> 331
<212> PRT
<213> Artificial Sequence

<220>
<221> source
<223> /note="Description of Artificial Sequence: Synthetic polypeptide"

<400> 30
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Cys Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 31
<211> 331
<212> PRT

<213> Artificial Sequence

<220>
<221> source
<223> /note="Description of Artificial Sequence: Synthetic polypeptide"

<400> 31
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Cys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 32
<211> 331
<212> PRT
<213> Artificial Sequence

<220>
<221> source
<223> /note="Description of Artificial Sequence: Synthetic polypeptide"

<400> 32
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

```
Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Cys Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330
```

<210> 33
<211> 331

<212> PRT
<213> Artificial Sequence

<220>
<221> source
<223> /note="Description of Artificial Sequence: Synthetic
      polypeptide"

<400> 33
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Cys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

```
Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330


<210> 34
<211> 331
<212> PRT
<213> Artificial Sequence

<220>
<221> source
<223> /note="Description of Artificial Sequence: Synthetic
      polypeptide"

<400> 34
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95
```

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Cys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 35

<211> 331
<212> PRT
<213> Artificial Sequence

<220>
<221> source
<223> /note="Description of Artificial Sequence: Synthetic polypeptide"

<400> 35

```
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Cys
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
```

210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 36
<211> 331
<212> PRT
<213> Artificial Sequence

<220>
<221> source
<223> /note="Description of Artificial Sequence: Synthetic polypeptide"

<400> 36
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp

85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Cys Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 37
<211> 331
<212> PRT
<213> Artificial Sequence

<220>
<221> source
<223> /note="Description of Artificial Sequence: Synthetic
polypeptide"

<400> 37
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Cys Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 38
<211> 331
<212> PRT
<213> Artificial Sequence

<220>
<221> source
<223> /note="Description of Artificial Sequence: Synthetic polypeptide"

<400> 38
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

```
Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Cys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330
```

<210> 39
<211> 331
<212> PRT
<213> Artificial Sequence

<220>
<221> source
<223> /note="Description of Artificial Sequence: Synthetic polypeptide"

<400> 39

```
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Cys Val Ser
        195                 200                 205
```

```
Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330
```

<210> 40
<211> 331
<212> PRT
<213> Artificial Sequence

<220>
<221> source
<223> /note="Description of Artificial Sequence: Synthetic polypeptide"

<400> 40

```
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80
```

```
Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Cys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330
```

<210> 41
<211> 331
<212> PRT
<213> Artificial Sequence

<220>
<221> source
<223> /note="Description of Artificial Sequence: Synthetic polypeptide"

<400> 41
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Cys Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 42
<211> 331
<212> PRT
<213> Artificial Sequence

<220>
<221> source
<223> /note="Description of Artificial Sequence: Synthetic
      polypeptide"

<400> 42
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Cys Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys

325 330

<210> 43
<211> 331
<212> PRT
<213> Artificial Sequence

<220>
<221> source
<223> /note="Description of Artificial Sequence: Synthetic polypeptide"

<400> 43
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser

195 200 205

Asn Lys Ala Leu Pro Ala Pro Ile Glu Cys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 44
<211> 331
<212> PRT
<213> Artificial Sequence

<220>
<221> source
<223> /note="Description of Artificial Sequence: Synthetic polypeptide"

<400> 44
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln

```
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Cys Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320
```

```
Thr Gln Lys Ser Leu Ser Leu Ser Pro Gly Lys
                325                 330


<210> 45
<211> 331
<212> PRT
<213> Artificial Sequence

<220>
<221> source
<223> /note="Description of Artificial Sequence: Synthetic
      polypeptide"

<400> 45
Ser Ala Ser Thr Lys Gly Pro Ser Val Phe Pro Leu Ala Pro Ser Ser
1               5                   10                  15


Lys Ser Thr Ser Gly Gly Thr Ala Ala Leu Gly Cys Leu Val Lys Asp
            20                  25                  30


Tyr Phe Pro Glu Pro Val Thr Val Ser Trp Asn Ser Gly Ala Leu Thr
        35                  40                  45


Ser Gly Val His Thr Phe Pro Ala Val Leu Gln Ser Ser Gly Leu Tyr
    50                  55                  60


Ser Leu Ser Ser Val Val Thr Val Pro Ser Ser Ser Leu Gly Thr Gln
65                  70                  75                  80


Thr Tyr Ile Cys Asn Val Asn His Lys Pro Ser Asn Thr Lys Val Asp
                85                  90                  95


Lys Lys Val Glu Pro Lys Ser Cys Asp Lys Thr His Thr Cys Pro Pro
            100                 105                 110


Cys Pro Ala Pro Glu Leu Leu Gly Gly Pro Ser Val Phe Leu Phe Pro
        115                 120                 125


Pro Lys Pro Lys Asp Thr Leu Met Ile Ser Arg Thr Pro Glu Val Thr
    130                 135                 140


Cys Val Val Val Asp Val Ser His Glu Asp Pro Glu Val Lys Phe Asn
145                 150                 155                 160


Trp Tyr Val Asp Gly Val Glu Val His Asn Ala Lys Thr Lys Pro Arg
                165                 170                 175


Glu Glu Gln Tyr Asn Ser Thr Tyr Arg Val Val Ser Val Leu Thr Val
            180                 185                 190
```

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Cys Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 46
<211> 331
<212> PRT
<213> Artificial Sequence

<220>
<221> source
<223> /note="Description of Artificial Sequence: Synthetic polypeptide"

<400> 46
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

```
Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Cys Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320
```

Thr Gln Lys Ser Leu Ser Leu Ser Pro Gly Lys
325 330

<210> 47
<211> 331
<212> PRT
<213> Artificial Sequence

<220>
<221> source
<223> /note="Description of Artificial Sequence: Synthetic polypeptide"

<400> 47
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Cys Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 48
<211> 331
<212> PRT
<213> Artificial Sequence

<220>
<221> source
<223> /note="Description of Artificial Sequence: Synthetic polypeptide"

<400> 48
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

```
Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Cys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320
```

Thr Gln Lys Ser Leu Ser Leu Ser Pro Gly Lys
325 330

<210> 49
<211> 331
<212> PRT
<213> Artificial Sequence

<220>
<221> source
<223> /note="Description of Artificial Sequence: Synthetic polypeptide"

<400> 49
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Cys Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 50
<211> 331
<212> PRT
<213> Artificial Sequence

<220>
<221> source
<223> /note="Description of Artificial Sequence: Synthetic polypeptide"

<400> 50
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Cys Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr

305 310 315 320

Thr Gln Lys Ser Leu Ser Leu Ser Pro Gly Lys
325 330

<210> 51
<211> 331
<212> PRT
<213> Artificial Sequence

<220>
<221> source
<223> /note="Description of Artificial Sequence: Synthetic polypeptide"

<400> 51
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val

180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Cys Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 52
<211> 331
<212> PRT
<213> Artificial Sequence

<220>
<221> source
<223> /note="Description of Artificial Sequence: Synthetic
polypeptide"

<400> 52
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr

50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Cys Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 53
<211> 331
<212> PRT
<213> Artificial Sequence

<220>
<221> source
<223> /note="Description of Artificial Sequence: Synthetic polypeptide"

<400> 53
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Cys Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 54
<211> 331
<212> PRT
<213> Artificial Sequence

<220>
<221> source
<223> /note="Description of Artificial Sequence: Synthetic polypeptide"

<400> 54
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Cys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 55
<211> 331
<212> PRT
<213> Artificial Sequence

<220>
<221> source
<223> /note="Description of Artificial Sequence: Synthetic polypeptide"

<400> 55
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Cys Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 56
<211> 331
<212> PRT
<213> Artificial Sequence

<220>
<221> source
<223> /note="Description of Artificial Sequence: Synthetic polypeptide"

<400> 56
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Cys Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 57
<211> 331
<212> PRT
<213> Artificial Sequence

<220>
<221> source
<223> /note="Description of Artificial Sequence: Synthetic polypeptide"

<400> 57
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Cys Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 58
<211> 331
<212> PRT
<213> Artificial Sequence

<220>
<221> source
<223> /note="Description of Artificial Sequence: Synthetic polypeptide"

<400> 58
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Cys Lys Ser Arg Trp Gln Gln Gly

290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 59
<211> 331
<212> PRT
<213> Artificial Sequence

<220>
<221> source
<223> /note="Description of Artificial Sequence: Synthetic polypeptide"

<400> 59
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg

165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Cys Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 60
<211> 331
<212> PRT
<213> Artificial Sequence

<220>
<221> source
<223> /note="Description of Artificial Sequence: Synthetic polypeptide"

<400> 60
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr

```
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285
```

Phe Leu Tyr Ser Lys Leu Thr Val Asp Lys Ser Arg Trp Gln Gln Gly
    290                 295                 300

Asn Cys Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 61
<211> 108
<212> PRT
<213> Artificial Sequence

<220>
<221> source
<223> /note="Description of Artificial Sequence: Synthetic polypeptide"

<400> 61
Cys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
            100                 105

<210> 62
<211> 108
<212> PRT
<213> Artificial Sequence

<220>
<221> source
<223> /note="Description of Artificial Sequence: Synthetic polypeptide"

<400> 62
Lys Cys Thr Val Ala Ala Pro Ser Val Phe Ile Phe Pro Pro Ser Asp

1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Glu Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 63
<211> 108
<212> PRT
<213> Artificial Sequence

<220>
<221> source
<223> /note="Description of Artificial Sequence: Synthetic polypeptide"

<400> 63
Lys Arg Cys Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Glu Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys

100 105

<210> 64
<211> 108
<212> PRT
<213> Artificial Sequence

<220>
<221> source
<223> /note="Description of Artificial Sequence: Synthetic polypeptide"

<400> 64
Lys Arg Thr Val Ala Cys Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Glu Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 65
<211> 108
<212> PRT
<213> Artificial Sequence

<220>
<221> source
<223> /note="Description of Artificial Sequence: Synthetic polypeptide"

<400> 65
Lys Arg Thr Val Ala Ala Pro Cys Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
            100                 105

<210> 66
<211> 108
<212> PRT
<213> Artificial Sequence

<220>
<221> source
<223> /note="Description of Artificial Sequence: Synthetic polypeptide"

<400> 66
Lys Arg Thr Val Ala Ala Pro Ser Val Phe Ile Phe Pro Pro Ser Cys
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
            100                 105

<210> 67
<211> 108
<212> PRT
<213> Artificial Sequence

<220>

<221> source
<223> /note="Description of Artificial Sequence: Synthetic polypeptide"

<400> 67
Lys Arg Thr Val Ala Ala Pro Ser Val Phe Ile Phe Pro Pro Ser Asp
1               5                   10                  15

Cys Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
            100                 105

<210> 68
<211> 108
<212> PRT
<213> Artificial Sequence

<220>
<221> source
<223> /note="Description of Artificial Sequence: Synthetic polypeptide"

<400> 68
Lys Arg Thr Val Ala Ala Pro Ser Val Phe Ile Phe Pro Pro Ser Asp
1               5                   10                  15

Glu Gln Leu Lys Ser Gly Cys Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 69
<211> 108
<212> PRT
<213> Artificial Sequence

<220>
<221> source
<223> /note="Description of Artificial Sequence: Synthetic polypeptide"

<400> 69
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Cys Glu Ala Lys Val Gln Trp Lys Val Asp Asn Ala Leu
35 40 45

Gln Ser Gly Asn Ser Gln Glu Ser Val Thr Glu Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 70
<211> 108
<212> PRT
<213> Artificial Sequence

<220>
<221> source
<223> /note="Description of Artificial Sequence: Synthetic polypeptide"

<400> 70
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Cys Ala Lys Val Gln Trp Lys Val Asp Asn Ala Leu
35 40 45

Gln Ser Gly Asn Ser Gln Glu Ser Val Thr Glu Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 71
<211> 108
<212> PRT
<213> Artificial Sequence

<220>
<221> source
<223> /note="Description of Artificial Sequence: Synthetic polypeptide"

<400> 71
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Cys Val Gln Trp Lys Val Asp Asn Ala Leu
35 40 45

Gln Ser Gly Asn Ser Gln Glu Ser Val Thr Glu Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 72
<211> 108
<212> PRT
<213> Artificial Sequence

<220>
<221> source
<223> /note="Description of Artificial Sequence: Synthetic polypeptide"

<400> 72
Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Cys Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
            100                 105

<210> 73
<211> 108
<212> PRT
<213> Artificial Sequence

<220>
<221> source
<223> /note="Description of Artificial Sequence: Synthetic polypeptide"

<400> 73
Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Cys
        35                  40                  45

Gln Ser Gly Asn Ser Gln Glu Ser Val Thr Glu Gln Asp Ser Lys Asp

50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 74
<211> 108
<212> PRT
<213> Artificial Sequence

<220>
<221> source
<223> /note="Description of Artificial Sequence: Synthetic polypeptide"

<400> 74
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Cys Gly Asn Ser Gln Glu Ser Val Thr Glu Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 75
<211> 108
<212> PRT
<213> Artificial Sequence

<220>
<221> source
<223> /note="Description of Artificial Sequence: Synthetic polypeptide"

<400> 75
Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Cys Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
            100                 105

<210> 76
<211> 108
<212> PRT
<213> Artificial Sequence

<220>
<221> source
<223> /note="Description of Artificial Sequence: Synthetic
      polypeptide"

<400> 76
Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Cys Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
100 105

<210> 77
<211> 108
<212> PRT
<213> Artificial Sequence

<220>
<221> source
<223> /note="Description of Artificial Sequence: Synthetic polypeptide"

<400> 77
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Cys Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 78
<211> 108
<212> PRT
<213> Artificial Sequence

<220>
<221> source
<223> /note="Description of Artificial Sequence: Synthetic polypeptide"

<400> 78
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

```
Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Cys Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
            100                 105


<210> 79
<211> 108
<212> PRT
<213> Artificial Sequence

<220>
<221> source
<223> /note="Description of Artificial Sequence: Synthetic
      polypeptide"

<400> 79
Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Cys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
            100                 105


<210> 80
<211> 108
<212> PRT
```

<213> Artificial Sequence

<220>
<221> source
<223> /note="Description of Artificial Sequence: Synthetic polypeptide"

<400> 80
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Glu Gln Asp Ser Lys Cys
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 81
<211> 108
<212> PRT
<213> Artificial Sequence

<220>
<221> source
<223> /note="Description of Artificial Sequence: Synthetic polypeptide"

<400> 81
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Glu Gln Asp Ser Lys Asp
50 55 60

Ser Thr Tyr Ser Leu Ser Ser Thr Leu Thr Leu Cys Lys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 82
<211> 108
<212> PRT
<213> Artificial Sequence

<220>
<221> source
<223> /note="Description of Artificial Sequence: Synthetic polypeptide"

<400> 82
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Glu Gln Asp Ser Lys Asp
50 55 60

Ser Thr Tyr Ser Leu Ser Ser Thr Leu Thr Leu Ser Cys Ala Asp Tyr
65 70 75 80

Glu Lys His Lys Val Tyr Ala Cys Glu Val Thr His Gln Gly Leu Ser
85 90 95

Ser Pro Val Thr Lys Ser Phe Asn Arg Gly Glu Cys
100 105

<210> 83
<211> 108
<212> PRT
<213> Artificial Sequence

<220>
<221> source
<223> /note="Description of Artificial Sequence: Synthetic polypeptide"

<400> 83
Lys Arg Thr Val Ala Ala Pro Ser Val Phe Ile Phe Pro Pro Ser Asp

```
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Cys His Lys Val Tyr Ala Cys Glu Val Thr His Gln Gly Leu Ser
                85                  90                  95

Ser Pro Val Thr Lys Ser Phe Asn Arg Gly Glu Cys
            100                 105


<210> 84
<211> 108
<212> PRT
<213> Artificial Sequence

<220>
<221> source
<223> /note="Description of Artificial Sequence: Synthetic
      polypeptide"

<400> 84
Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Cys Val Tyr Ala Cys Glu Val Thr His Gln Gly Leu Ser
                85                  90                  95

Ser Pro Val Thr Lys Ser Phe Asn Arg Gly Glu Cys
```

100 105

<210> 85
<211> 108
<212> PRT
<213> Artificial Sequence

<220>
<221> source
<223> /note="Description of Artificial Sequence: Synthetic polypeptide"

<400> 85
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Glu Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Cys Tyr Ala Cys Glu Val Thr His Gln Gly Leu Ser
85 90 95

Ser Pro Val Thr Lys Ser Phe Asn Arg Gly Glu Cys
100 105

<210> 86
<211> 108
<212> PRT
<213> Artificial Sequence

<220>
<221> source
<223> /note="Description of Artificial Sequence: Synthetic polypeptide"

<400> 86
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Cys His Gln Gly Leu Ser
                85                  90                  95

Ser Pro Val Thr Lys Ser Phe Asn Arg Gly Glu Cys
            100                 105

<210> 87
<211> 108
<212> PRT
<213> Artificial Sequence

<220>
<221> source
<223> /note="Description of Artificial Sequence: Synthetic
      polypeptide"

<400> 87
Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Cys Gly Leu Ser
                85                  90                  95

Ser Pro Val Thr Lys Ser Phe Asn Arg Gly Glu Cys
            100                 105

<210> 88
<211> 108
<212> PRT
<213> Artificial Sequence

<220>

<221> source
<223> /note="Description of Artificial Sequence: Synthetic
polypeptide"

<400> 88
Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Cys Pro Val Thr Lys Ser Phe Asn Arg Gly Glu Cys
            100                 105

<210> 89
<211> 108
<212> PRT
<213> Artificial Sequence

<220>
<221> source
<223> /note="Description of Artificial Sequence: Synthetic
polypeptide"

<400> 89
Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

```
Glu Lys His Lys Val Tyr Ala Cys Glu Val Thr His Gln Gly Leu Ser
                85                  90                  95

Ser Pro Val Cys Lys Ser Phe Asn Arg Gly Glu Cys
            100                 105
```

<210> 90
<211> 214
<212> PRT
<213> Homo sapiens

<400> 90

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190
```

Ala Cys Glu Val Thr His Gln Gly Leu Ser Ser Pro Val Thr Lys Ser
195 200 205

Phe Asn Arg Gly Glu Cys
210

<210> 91
<211> 105
<212> PRT
<213> Homo sapiens

<400> 91
Gln Pro Lys Ala Ala Pro Ser Val Thr Leu Phe Pro Pro Ser Ser Glu
1 5 10 15

Glu Leu Gln Ala Asn Lys Ala Thr Leu Val Cys Leu Ile Ser Asp Phe
20 25 30

Tyr Pro Gly Ala Val Thr Val Ala Trp Lys Ala Asp Ser Ser Pro Val
35 40 45

Lys Ala Gly Val Glu Thr Thr Thr Pro Ser Lys Gln Ser Asn Asn Lys
50 55 60

Tyr Ala Ala Ser Ser Tyr Leu Ser Leu Thr Pro Glu Gln Trp Lys Ser
65 70 75 80

His Arg Ser Tyr Ser Cys Gln Val Thr His Glu Gly Ser Thr Val Glu
85 90 95

Lys Thr Val Ala Pro Thr Glu Cys Ser
100 105

<210> 92
<211> 105
<212> PRT
<213> Artificial Sequence

<220>
<221> source
<223> /note="Description of Artificial Sequence: Synthetic polypeptide"

<400> 92
Gln Pro Lys Ala Ala Pro Ser Val Thr Leu Phe Pro Pro Ser Ser Glu
1 5 10 15

Glu Leu Gln Ala Asn Lys Ala Thr Leu Val Cys Leu Ile Ser Asp Phe
20 25 30

Tyr Pro Gly Cys Val Thr Val Ala Trp Lys Ala Asp Ser Ser Pro Val
35 40 45

Lys Ala Gly Val Glu Thr Thr Thr Pro Ser Lys Gln Ser Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
            100                 105

<210> 93
<211> 105
<212> PRT
<213> Artificial Sequence

<220>
<221> source
<223> /note="Description of Artificial Sequence: Synthetic
      polypeptide"

<400> 93
Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
            20                  25                  30

Tyr Pro Gly Ala Val Cys Val Ala Trp Lys Ala Asp Ser Ser Pro Val
        35                  40                  45

Lys Ala Gly Val Glu Thr Thr Thr Pro Ser Lys Gln Ser Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
            100                 105

<210> 94
<211> 105
<212> PRT
<213> Artificial Sequence

<220>

<221> source
<223> /note="Description of Artificial Sequence: Synthetic
      polypeptide"

<400> 94
Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
            20                  25                  30

Tyr Pro Gly Ala Val Thr Val Cys Trp Lys Ala Asp Ser Ser Pro Val
        35                  40                  45

Lys Ala Gly Val Glu Thr Thr Thr Pro Ser Lys Gln Ser Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
            100                 105

<210> 95
<211> 105
<212> PRT
<213> Artificial Sequence

<220>
<221> source
<223> /note="Description of Artificial Sequence: Synthetic
      polypeptide"

<400> 95
Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
            20                  25                  30

Tyr Pro Gly Ala Val Thr Val Ala Trp Lys Ala Asp Ser Ser Pro Val
        35                  40                  45

Cys Ala Gly Val Glu Thr Thr Thr Pro Ser Lys Gln Ser Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
85 90 95

Lys Thr Val Ala Pro Thr Glu Cys Ser
100 105

<210> 96
<211> 105
<212> PRT
<213> Artificial Sequence

<220>
<221> source
<223> /note="Description of Artificial Sequence: Synthetic polypeptide"

<400> 96
Gln Pro Lys Ala Ala Pro Ser Val Thr Leu Phe Pro Pro Ser Ser Glu
1 5 10 15

Glu Leu Gln Ala Asn Lys Ala Thr Leu Val Cys Leu Ile Ser Asp Phe
20 25 30

Tyr Pro Gly Ala Val Thr Val Ala Trp Lys Ala Asp Ser Ser Pro Val
35 40 45

Lys Ala Gly Cys Glu Thr Thr Thr Pro Ser Lys Gln Ser Asn Asn Lys
50 55 60

Tyr Ala Ala Ser Ser Tyr Leu Ser Leu Thr Pro Glu Gln Trp Lys Ser
65 70 75 80

His Arg Ser Tyr Ser Cys Gln Val Thr His Glu Gly Ser Thr Val Glu
85 90 95

Lys Thr Val Ala Pro Thr Glu Cys Ser
100 105

<210> 97
<211> 105
<212> PRT
<213> Artificial Sequence

<220>
<221> source
<223> /note="Description of Artificial Sequence: Synthetic polypeptide"

<400> 97
Gln Pro Lys Ala Ala Pro Ser Val Thr Leu Phe Pro Pro Ser Ser Glu
1 5 10 15

```
Glu Leu Gln Ala Asn Lys Ala Thr Leu Val Cys Leu Ile Ser Asp Phe
            20                  25                  30

Tyr Pro Gly Ala Val Thr Val Ala Trp Lys Ala Asp Ser Ser Pro Val
        35                  40                  45

Lys Ala Gly Val Glu Thr Thr Cys Pro Ser Lys Gln Ser Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
            100                 105


<210> 98
<211> 105
<212> PRT
<213> Artificial Sequence

<220>
<221> source
<223> /note="Description of Artificial Sequence: Synthetic
      polypeptide"

<400> 98
Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
            20                  25                  30

Tyr Pro Gly Ala Val Thr Val Ala Trp Lys Ala Asp Ser Ser Pro Val
        35                  40                  45

Lys Ala Gly Val Glu Thr Thr Thr Pro Ser Lys Gln Cys Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
            100                 105
```

<210> 99
<211> 24
<212> PRT
<213> Artificial Sequence

<220>
<221> source
<223> /note="Description of Artificial Sequence: Synthetic peptide"

<400> 99
```
Met Lys Thr Phe Ile Leu Leu Leu Trp Val Leu Leu Leu Trp Val Ile
1               5                   10                  15

Phe Leu Leu Pro Gly Ala Thr Ala
            20
```

<210> 100
<211> 40
<212> DNA
<213> Artificial Sequence

<220>
<221> source
<223> /note="Description of Artificial Sequence: Synthetic primer"

<400> 100
gtggagatct gtcgaacggt ggccgctccc agcgtgttca 40

<210> 101
<211> 39
<212> DNA
<213> Artificial Sequence

<220>
<221> source
<223> /note="Description of Artificial Sequence: Synthetic primer"

<400> 101
accgttcgac agatctccac cttggtaccc tgtccgaac 39

<210> 102
<211> 41
<212> DNA
<213> Artificial Sequence

<220>
<221> source
<223> /note="Description of Artificial Sequence: Synthetic primer"

<400> 102
agcggcaact gtcaggagag cgtcaccgag caggacagca a 41

<210> 103
<211> 40

<212> DNA
<213> Artificial Sequence

<220>
<221> source
<223> /note="Description of Artificial Sequence: Synthetic primer"

<400> 103
ctctcctgac agttgccgct ctgcagggcg ttgtccacct 40

<210> 104
<211> 40
<212> DNA
<213> Artificial Sequence

<220>
<221> source
<223> /note="Description of Artificial Sequence: Synthetic primer"

<400> 104
gagcgtcacc tgtcaggaca gcaaggactc cacctacagc 40

<210> 105
<211> 40
<212> DNA
<213> Artificial Sequence

<220>
<221> source
<223> /note="Description of Artificial Sequence: Synthetic primer"

<400> 105
ctgtcctgac aggtgacgct ctcctggctg ttgccgctct 40

<210> 106
<211> 39
<212> DNA
<213> Artificial Sequence

<220>
<221> source
<223> /note="Description of Artificial Sequence: Synthetic primer"

<400> 106
tacttcccct gtcccgtgac cgtgtcctgg aacagcgga 39

<210> 107
<211> 38
<212> DNA
<213> Artificial Sequence

<220>
<221> source
<223> /note="Description of Artificial Sequence: Synthetic primer"

<400> 107
ggtcacggga caggggaagt agtccttcac caggcagc 38

<210> 108

<400> 108
000

<210> 109
<211> 40
<212> DNA
<213> Artificial Sequence

<220>
<221> source
<223> /note="Description of Artificial Sequence: Synthetic primer"

<400> 109
cacaccttct gtgccgtgct gcagagcagc ggcctgtaca 40

<210> 110
<211> 40
<212> DNA
<213> Artificial Sequence

<220>
<221> source
<223> /note="Description of Artificial Sequence: Synthetic primer"

<400> 110
cagcacggca cagaaggtgt gcacgccgga ggtcagggct 40

<210> 111
<211> 38
<212> DNA
<213> Artificial Sequence

<220>
<221> source
<223> /note="Description of Artificial Sequence: Synthetic primer"

<400> 111
ctgttcccac ccaagtgtaa ggacaccctg atgatcag 38

<210> 112
<211> 36
<212> DNA
<213> Artificial Sequence

<220>
<221> source
<223> /note="Description of Artificial Sequence: Synthetic primer"

<400> 112
cttgggtggg aacaggaaca cggagggtcc gcccag 36

<210> 113
<211> 42
<212> DNA
<213> Artificial Sequence

<220>
<221> source
<223> /note="Description of Artificial Sequence: Synthetic primer"

<400> 113
tgcaaggtct ccaacaagtg tctgccagcc cccatcgaaa ag 42

<210> 114
<211> 40
<212> DNA
<213> Artificial Sequence

<220>
<221> source
<223> /note="Description of Artificial Sequence: Synthetic primer"

<400> 114
gttggagacc ttgcacttgt attccttgcc gttcagccag 40

<210> 115
<211> 39
<212> DNA
<213> Artificial Sequence

<220>
<221> source
<223> /note="Description of Artificial Sequence: Synthetic primer"

<400> 115
cccatcgaat gcaccatcag caaggccaag ggccagcca 39

<210> 116
<211> 40
<212> DNA
<213> Artificial Sequence

<220>
<221> source
<223> /note="Description of Artificial Sequence: Synthetic primer"

<400> 116
gctgatggtg cattcgatgg gggctggcag ggccttgttg 40

<210> 117
<211> 40
<212> DNA

<213> Artificial Sequence

<220>
<221> source
<223> /note="Description of Artificial Sequence: Synthetic primer"

<400> 117
cttgctgatg gtcttttcga tgggggctgg cagggccttg 40

<210> 118
<211> 36
<212> DNA
<213> Artificial Sequence

<220>
<221> source
<223> /note="Description of Artificial Sequence: Synthetic primer"

<400> 118
aagaccatca gcaagtgtaa gggccagcca cgggag 36

<210> 119
<211> 39
<212> DNA
<213> Artificial Sequence

<220>
<221> source
<223> /note="Description of Artificial Sequence: Synthetic primer"

<400> 119
agctgacctg caaccaggtg tccctgacct gtctggtga 39

<210> 120
<211> 40
<212> DNA
<213> Artificial Sequence

<220>
<221> source
<223> /note="Description of Artificial Sequence: Synthetic primer"

<400> 120
cacctggttg caggtcagct cgtcccggga tggaggcagg 40

<210> 121
<211> 41
<212> DNA
<213> Artificial Sequence

<220>
<221> source
<223> /note="Description of Artificial Sequence: Synthetic primer"

<400> 121
ctggtgaagg gcttctgtcc cagcgacatc gccgtggagt g 41

<210> 122
<211> 40
<212> DNA
<213> Artificial Sequence

<220>
<221> source
<223> /note="Description of Artificial Sequence: Synthetic primer"

<400> 122
gaagcccttc accagacagg tcagggacac ctggttcttg 40

<210> 123
<211> 40
<212> DNA
<213> Artificial Sequence

<220>
<221> source
<223> /note="Description of Artificial Sequence: Synthetic primer"

<400> 123
ttctacccct gcgacatcgc cgtggagtgg gagagcaacg 40

<210> 124
<211> 41
<212> DNA
<213> Artificial Sequence

<220>
<221> source
<223> /note="Description of Artificial Sequence: Synthetic primer"

<400> 124
ggcgatgtcg caggggtaga agcccttcac cagacaggtc a 41

<210> 125
<211> 39
<212> DNA
<213> Artificial Sequence

<220>
<221> source
<223> /note="Description of Artificial Sequence: Synthetic primer"

<400> 125
aacaactgta agaccacacc tccagtgctg gacagcgac 39

<210> 126
<211> 40
<212> DNA

<213> Artificial Sequence

<220>
<221> source
<223> /note="Description of Artificial Sequence: Synthetic
primer"

<400> 126
ggtcttacag ttgttctcgg gctggccgtt gctctcccac 40

<210> 127
<211> 39
<212> DNA
<213> Artificial Sequence

<220>
<221> source
<223> /note="Description of Artificial Sequence: Synthetic
primer"

<400> 127
acaccttgtg tgctggacag cgacggcagc ttcttcctg 39

<210> 128
<211> 39
<212> DNA
<213> Artificial Sequence

<220>
<221> source
<223> /note="Description of Artificial Sequence: Synthetic
primer"

<400> 128
cagcacacaa ggtgtggtct tgtagttgtt ctcgggctg 39

<210> 129
<211> 331
<212> PRT
<213> Artificial Sequence

<220>
<221> source
<223> /note="Description of Artificial Sequence: Synthetic
polypeptide"

<400> 129
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr

50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Cys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Cys Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 130
<211> 331
<212> PRT
<213> Artificial Sequence

<220>
<221> source
<223> /note="Description of Artificial Sequence: Synthetic polypeptide"

<400> 130
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Cys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Cys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 131
<211> 331
<212> PRT
<213> Artificial Sequence

<220>
<221> source
<223> /note="Description of Artificial Sequence: Synthetic polypeptide"

<400> 131
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Cys Pro Val Thr Val Ser Trp Asn Ser Gly Ala Leu Thr
35 40 45

```
Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Cys Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300
```

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 132
<211> 331
<212> PRT
<213> Artificial Sequence

<220>
<221> source
<223> /note="Description of Artificial Sequence: Synthetic polypeptide"

<400> 132
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Cys Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Cys Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 133
<211> 331
<212> PRT
<213> Artificial Sequence

<220>
<221> source
<223> /note="Description of Artificial Sequence: Synthetic polypeptide"

<400> 133
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Cys Pro Val Thr Val Ser Trp Asn Ser Gly Ala Leu Thr
35 40 45

```
Ser Gly Val His Thr Phe Cys Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300
```

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 134
<211> 331
<212> PRT
<213> Artificial Sequence

<220>
<221> source
<223> /note="Description of Artificial Sequence: Synthetic polypeptide"

<400> 134
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Cys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Cys Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 135
<211> 331
<212> PRT
<213> Artificial Sequence

<220>
<221> source
<223> /note="Description of Artificial Sequence: Synthetic polypeptide"

<400> 135
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Cys Val Leu Asp Ser Asp Gly Ser Phe
275 280 285

Phe Leu Tyr Ser Lys Leu Thr Val Asp Lys Ser Arg Trp Gln Gln Gly

290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 136
<211> 331
<212> PRT
<213> Artificial Sequence

<220>
<221> source
<223> /note="Description of Artificial Sequence: Synthetic polypeptide"

<400> 136
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg

165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Cys Asp Ile Ala Val Glu Trp Glu Ser Asn Gly Gln Pro Glu
260 265 270

Asn Asn Tyr Lys Thr Thr Pro Cys Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 137
<211> 331
<212> PRT
<213> Artificial Sequence

<220>
<221> source
<223> /note="Description of Artificial Sequence: Synthetic polypeptide"

<400> 137
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr

35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Cys Asp Ile Ala Val Glu Trp Glu Ser Asn Gly Gln Pro Glu
260 265 270

Asn Asn Cys Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 138
<211> 331
<212> PRT
<213> Artificial Sequence

<220>
<221> source
<223> /note="Description of Artificial Sequence: Synthetic polypeptide"

<400> 138
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Cys Lys Thr Thr Pro Cys Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 139
<211> 331
<212> PRT
<213> Artificial Sequence

<220>
<221> source
<223> /note="Description of Artificial Sequence: Synthetic polypeptide"

<400> 139
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Cys Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Cys Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 140
<211> 331
<212> PRT
<213> Artificial Sequence

<220>
<221> source
<223> /note="Description of Artificial Sequence: Synthetic polypeptide"

<400> 140
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Cys Leu Pro Ala Pro Ile Glu Lys Thr Ile Ser Lys Cys Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 141
<211> 331
<212> PRT
<213> Artificial Sequence

<220>
<221> source
<223> /note="Description of Artificial Sequence: Synthetic polypeptide"

<400> 141
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Cys Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210> 142
<211> 331
<212> PRT
<213> Artificial Sequence

<220>
<221> source
<223> /note="Description of Artificial Sequence: Synthetic polypeptide"

<400> 142
Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Cys Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Cys Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 143
<211> 331
<212> PRT
<213> Artificial Sequence

<220>
<221> source
<223> /note="Description of Artificial Sequence: Synthetic polypeptide"

<400> 143
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Cys Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Cys Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe

275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 144
<211> 331
<212> PRT
<213> Artificial Sequence

<220>
<221> source
<223> /note="Description of Artificial Sequence: Synthetic polypeptide"

<400> 144
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Cys Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn

145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Cys Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 145
<211> 331
<212> PRT
<213> Artificial Sequence

<220>
<221> source
<223> /note="Description of Artificial Sequence: Synthetic polypeptide"

<400> 145
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp

20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Ala Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Cys Pro Ser Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 146
<211> 331
<212> PRT
<213> Artificial Sequence

<220>
<221> source
<223> /note="Description of Artificial Sequence: Synthetic polypeptide"

<400> 146
Ser Ala Ser Thr Lys Gly Pro Ser Val Phe Pro Leu Ala Pro Ser Ser
1 5 10 15

Lys Ser Thr Ser Gly Gly Thr Ala Ala Leu Gly Cys Leu Val Lys Asp
20 25 30

Tyr Phe Pro Glu Pro Val Thr Val Ser Trp Asn Ser Gly Ala Leu Thr
35 40 45

Ser Gly Val His Thr Phe Pro Ala Val Leu Gln Ser Ser Gly Leu Tyr
50 55 60

Ser Leu Ser Ser Val Val Thr Val Pro Ser Ser Ser Leu Gly Thr Gln
65 70 75 80

Thr Tyr Ile Cys Asn Val Asn His Lys Pro Ser Asn Thr Lys Val Asp
85 90 95

Lys Lys Val Glu Pro Lys Ser Cys Asp Lys Thr His Thr Cys Pro Pro
100 105 110

Cys Pro Ala Pro Glu Leu Leu Gly Gly Pro Ser Val Phe Leu Phe Pro
115 120 125

Pro Lys Pro Lys Asp Thr Leu Met Ile Ser Arg Thr Pro Glu Val Thr
130 135 140

Cys Val Val Val Asp Val Ser His Glu Asp Pro Glu Val Lys Phe Asn
145 150 155 160

Trp Tyr Val Asp Gly Val Glu Val His Asn Ala Lys Thr Lys Pro Arg
165 170 175

Glu Glu Gln Tyr Asn Ser Thr Tyr Arg Val Val Ser Val Leu Thr Val
180 185 190

Leu His Gln Asp Trp Leu Asn Gly Lys Glu Tyr Lys Cys Lys Val Ser
195 200 205

Asn Lys Cys Leu Pro Ala Pro Ile Glu Lys Thr Ile Ser Lys Ala Lys
210 215 220

Gly Gln Pro Arg Glu Pro Gln Val Tyr Thr Leu Pro Pro Ser Arg Glu
225 230 235 240

Glu Met Thr Lys Asn Gln Val Ser Leu Thr Cys Leu Val Lys Gly Phe
245 250 255

Tyr Pro Cys Asp Ile Ala Val Glu Trp Glu Ser Asn Gly Gln Pro Glu
260 265 270

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 147
<211> 331
<212> PRT
<213> Artificial Sequence

<220>
<221> source
<223> /note="Description of Artificial Sequence: Synthetic polypeptide"

<400> 147
Ser Ala Ser Thr Lys Gly Pro Ser Val Phe Pro Leu Ala Pro Ser Ser
1 5 10 15

```
Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Cys Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270
```

Asn Asn Tyr Lys Thr Thr Pro Pro Val Leu Asp Ser Asp Gly Ser Phe
275 280 285

Phe Leu Tyr Ser Lys Leu Thr Val Asp Lys Ser Arg Trp Gln Gln Gly
290 295 300

Asn Val Phe Ser Cys Ser Val Met His Glu Ala Leu His Asn His Tyr
305 310 315 320

Thr Gln Lys Ser Leu Ser Leu Ser Pro Gly Lys
325 330

<210> 148
<211> 108
<212> PRT
<213> Artificial Sequence

<220>
<221> source
<223> /note="Description of Artificial Sequence: Synthetic polypeptide"

<400> 148
Lys Arg Thr Val Ala Ala Pro Ser Val Phe Ile Phe Pro Pro Ser Asp
1 5 10 15

Glu Gln Leu Lys Ser Gly Thr Ala Ser Val Val Cys Leu Leu Asn Asn
20 25 30

Phe Tyr Pro Arg Glu Ala Lys Val Gln Trp Lys Val Asp Asn Ala Leu
35 40 45

Gln Ser Gly Asn Cys Gln Glu Ser Val Thr Cys Gln Asp Ser Lys Asp
50 55 60

Ser Thr Tyr Ser Leu Ser Ser Thr Leu Thr Leu Ser Lys Ala Asp Tyr
65 70 75 80

Glu Lys His Lys Val Tyr Ala Cys Glu Val Thr His Gln Gly Leu Ser
85 90 95

Ser Pro Val Cys Lys Ser Phe Asn Arg Gly Glu Cys
100 105

<210> 149
<211> 108
<212> PRT
<213> Artificial Sequence

<220>
<221> source

<223> /note="Description of Artificial Sequence: Synthetic polypeptide"

<400> 149

Lys Arg Thr Val Ala Ala Pro Ser Val Phe Ile Phe Pro Pro Ser Asp
1               5                   10                  15

Glu Gln Leu Lys Ser Gly Thr Ala Ser Val Val Cys Leu Leu Asn Asn
            20                  25                  30

Phe Tyr Pro Arg Glu Ala Lys Val Gln Trp Lys Val Asp Asn Ala Leu
        35                  40                  45

Gln Ser Gly Asn Ser Gln Glu Ser Val Thr Glu Gln Asp Ser Lys Asp
    50                  55                  60

Ser Thr Tyr Ser Leu Ser Ser Thr Leu Thr Leu Ser Lys Ala Asp Tyr
65                  70                  75                  80

Glu Lys His Lys Val Tyr Ala Cys Glu Val Thr His Gln Gly Leu Ser
                85                  90                  95

Ser Pro Val Thr Lys Ser Phe Asn Arg Gly Glu Cys
            100                 105

<210> 150
<211> 331
<212> PRT
<213> Homo sapiens

<400> 150

Ser Ala Ser Thr Lys Gly Pro Ser Val Phe Pro Leu Ala Pro Ser Ser
1               5                   10                  15

Lys Ser Thr Ser Gly Gly Thr Ala Ala Leu Gly Cys Leu Val Lys Asp
            20                  25                  30

Tyr Phe Pro Glu Pro Val Thr Val Ser Trp Asn Ser Gly Ala Leu Thr
        35                  40                  45

Ser Gly Val His Thr Phe Pro Ala Val Leu Gln Ser Ser Gly Leu Tyr
    50                  55                  60

Ser Leu Ser Ser Val Val Thr Val Pro Ser Ser Ser Leu Gly Thr Gln
65                  70                  75                  80

Thr Tyr Ile Cys Asn Val Asn His Lys Pro Ser Asn Thr Lys Val Asp
                85                  90                  95

Lys Lys Val Glu Pro Lys Ser Cys Asp Lys Thr His Thr Cys Pro Pro

```
                100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Glu
225                 230                 235                 240

Glu Met Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu
            260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330
```

<210> 151
<211> 328
<212> PRT
<213> Homo sapiens

<400> 151
Ser Thr Lys Gly Pro Ser Val Phe Pro Leu Ala Pro Ser Ser Lys Ser
1               5                   10                  15

Thr Ser Gly Gly Thr Ala Ala Leu Gly Cys Leu Val Lys Asp Tyr Phe
            20                  25                  30

Pro Glu Pro Val Thr Val Ser Trp Asn Ser Gly Ala Leu Thr Ser Gly
        35                  40                  45

Val His Thr Phe Pro Ala Val Leu Gln Ser Ser Gly Leu Tyr Ser Leu
    50                  55                  60

Ser Ser Val Val Thr Val Pro Ser Ser Ser Leu Gly Thr Gln Thr Tyr
65                  70                  75                  80

Ile Cys Asn Val Asn His Lys Pro Ser Asn Thr Lys Val Asp Lys Lys
                85                  90                  95

Val Glu Pro Lys Ser Cys Asp Lys Thr His Thr Cys Pro Pro Cys Pro
            100                 105                 110

Ala Pro Glu Leu Leu Gly Gly Pro Ser Val Phe Leu Phe Pro Pro Lys
        115                 120                 125

Pro Lys Asp Thr Leu Met Ile Ser Arg Thr Pro Glu Val Thr Cys Val
    130                 135                 140

Val Val Asp Val Ser His Glu Asp Pro Glu Val Lys Phe Asn Trp Tyr
145                 150                 155                 160

Val Asp Gly Val Glu Val His Asn Ala Lys Thr Lys Pro Arg Glu Glu
                165                 170                 175

Gln Tyr Asn Ser Thr Tyr Arg Val Val Ser Val Leu Thr Val Leu His
            180                 185                 190

Gln Asp Trp Leu Asn Gly Lys Glu Tyr Lys Cys Lys Val Ser Asn Lys
        195                 200                 205

Ala Leu Pro Ala Pro Ile Glu Lys Thr Ile Ser Lys Ala Lys Gly Gln
    210                 215                 220

Pro Arg Glu Pro Gln Val Tyr Thr Leu Pro Pro Ser Arg Asp Glu Leu
225                 230                 235                 240

Thr Lys Asn Gln Val Ser Leu Thr Cys Leu Val Lys Gly Phe Tyr Pro

245 250 255

Ser Asp Ile Ala Val Glu Trp Glu Ser Asn Gly Gln Pro Glu Asn Asn
260 265 270

Tyr Lys Thr Thr Pro Pro Val Leu Asp Ser Asp Gly Ser Phe Phe Leu
275 280 285

Tyr Ser Lys Leu Thr Val Asp Lys Ser Arg Trp Gln Gln Gly Asn Val
290 295 300

Phe Ser Cys Ser Val Met His Glu Ala Leu His Asn His Tyr Thr Gln
305 310 315 320

Lys Ser Leu Ser Leu Ser Pro Gly
325

<210> 152
<211> 324
<212> PRT
<213> Homo sapiens

<400> 152
Ser Thr Lys Gly Pro Ser Val Phe Pro Leu Ala Pro Cys Ser Arg Ser
1 5 10 15

Thr Ser Glu Ser Thr Ala Ala Leu Gly Cys Leu Val Lys Asp Tyr Phe
20 25 30

Pro Glu Pro Val Thr Val Ser Trp Asn Ser Gly Ala Leu Thr Ser Gly
35 40 45

Val His Thr Phe Pro Ala Val Leu Gln Ser Ser Gly Leu Tyr Ser Leu
50 55 60

Ser Ser Val Val Thr Val Pro Ser Ser Asn Phe Gly Thr Gln Thr Tyr
65 70 75 80

Thr Cys Asn Val Asp His Lys Pro Ser Asn Thr Lys Val Asp Lys Thr
85 90 95

Val Glu Arg Lys Cys Cys Val Glu Cys Pro Pro Cys Pro Ala Pro Pro
100 105 110

Val Ala Gly Pro Ser Val Phe Leu Phe Pro Pro Lys Pro Lys Asp Thr
115 120 125

Leu Met Ile Ser Arg Thr Pro Glu Val Thr Cys Val Val Val Asp Val
130 135 140

Ser His Glu Asp Pro Glu Val Gln Phe Asn Trp Tyr Val Asp Gly Val
145 150 155 160

Glu Val His Asn Ala Lys Thr Lys Pro Arg Glu Glu Gln Phe Asn Ser
165 170 175

Thr Phe Arg Val Val Ser Val Leu Thr Val Val His Gln Asp Trp Leu
180 185 190

Asn Gly Lys Glu Tyr Lys Cys Lys Val Ser Asn Lys Gly Leu Pro Ala
195 200 205

Pro Ile Glu Lys Thr Ile Ser Lys Thr Lys Gly Gln Pro Arg Glu Pro
210 215 220

Gln Val Tyr Thr Leu Pro Pro Ser Arg Glu Glu Met Thr Lys Asn Gln
225 230 235 240

Val Ser Leu Thr Cys Leu Val Lys Gly Phe Tyr Pro Ser Asp Ile Ser
245 250 255

Val Glu Trp Glu Ser Asn Gly Gln Pro Glu Asn Asn Tyr Lys Thr Thr
260 265 270

Pro Pro Met Leu Asp Ser Asp Gly Ser Phe Phe Leu Tyr Ser Lys Leu
275 280 285

Thr Val Asp Lys Ser Arg Trp Gln Gln Gly Asn Val Phe Ser Cys Ser
290 295 300

Val Met His Glu Ala Leu His Asn His Tyr Thr Gln Lys Ser Leu Ser
305 310 315 320

Leu Ser Pro Gly

<210> 153
<211> 375
<212> PRT
<213> Homo sapiens

<400> 153
Ser Thr Lys Gly Pro Ser Val Phe Pro Leu Ala Pro Cys Ser Arg Ser
1 5 10 15

Thr Ser Gly Gly Thr Ala Ala Leu Gly Cys Leu Val Lys Asp Tyr Phe
20 25 30

Pro Glu Pro Val Thr Val Ser Trp Asn Ser Gly Ala Leu Thr Ser Gly
35 40 45

Val His Thr Phe Pro Ala Val Leu Gln Ser Ser Gly Leu Tyr Ser Leu
50 55 60

Ser Ser Val Val Thr Val Pro Ser Ser Ser Leu Gly Thr Gln Thr Tyr
65 70 75 80

Thr Cys Asn Val Asn His Lys Pro Ser Asn Thr Lys Val Asp Lys Arg
85 90 95

Val Glu Leu Lys Thr Pro Leu Gly Asp Thr Thr His Thr Cys Pro Arg
100 105 110

Cys Pro Glu Pro Lys Ser Cys Asp Thr Pro Pro Pro Cys Pro Arg Cys
115 120 125

Pro Glu Pro Lys Ser Cys Asp Thr Pro Pro Pro Cys Pro Arg Cys Pro
130 135 140

Glu Pro Lys Ser Cys Asp Thr Pro Pro Pro Cys Pro Arg Cys Pro Ala
145 150 155 160

Pro Glu Leu Leu Gly Gly Pro Ser Val Phe Leu Phe Pro Pro Lys Pro
165 170 175

Lys Asp Thr Leu Met Ile Ser Arg Thr Pro Glu Val Thr Cys Val Val
180 185 190

Val Asp Val Ser His Glu Asp Pro Glu Val Gln Phe Lys Trp Tyr Val
195 200 205

Asp Gly Val Glu Val His Asn Ala Lys Thr Lys Pro Arg Glu Glu Gln
210 215 220

Tyr Asn Ser Thr Phe Arg Val Val Ser Val Leu Thr Val Leu His Gln
225 230 235 240

Asp Trp Leu Asn Gly Lys Glu Tyr Lys Cys Lys Val Ser Asn Lys Ala
245 250 255

Leu Pro Ala Pro Ile Glu Lys Thr Ile Ser Lys Thr Lys Gly Gln Pro
260 265 270

Arg Glu Pro Gln Val Tyr Thr Leu Pro Pro Ser Arg Glu Glu Met Thr
275 280 285

Lys Asn Gln Val Ser Leu Thr Cys Leu Val Lys Gly Phe Tyr Pro Ser
290 295 300

Asp Ile Ala Val Glu Trp Glu Ser Ser Gly Gln Pro Glu Asn Asn Tyr
305 310 315 320

Asn Thr Thr Pro Pro Met Leu Asp Ser Asp Gly Ser Phe Phe Leu Tyr
325 330 335

Ser Lys Leu Thr Val Asp Lys Ser Arg Trp Gln Gln Gly Asn Ile Phe
340 345 350

Ser Cys Ser Val Met His Glu Ala Leu His Asn Arg Phe Thr Gln Lys
355 360 365

Ser Leu Ser Leu Ser Pro Gly
370 375

<210> 154
<211> 325
<212> PRT
<213> Homo sapiens

<400> 154
Ser Thr Lys Gly Pro Ser Val Phe Pro Leu Ala Pro Cys Ser Arg Ser
1 5 10 15

Thr Ser Glu Ser Thr Ala Ala Leu Gly Cys Leu Val Lys Asp Tyr Phe
20 25 30

Pro Glu Pro Val Thr Val Ser Trp Asn Ser Gly Ala Leu Thr Ser Gly
35 40 45

Val His Thr Phe Pro Ala Val Leu Gln Ser Ser Gly Leu Tyr Ser Leu
50 55 60

Ser Ser Val Val Thr Val Pro Ser Ser Ser Leu Gly Thr Lys Thr Tyr
65 70 75 80

Thr Cys Asn Val Asp His Lys Pro Ser Asn Thr Lys Val Asp Lys Arg
85 90 95

Val Glu Ser Lys Tyr Gly Pro Pro Cys Pro Ser Cys Pro Ala Pro Glu
100 105 110

Phe Leu Gly Gly Pro Ser Val Phe Leu Phe Pro Pro Lys Pro Lys Asp
115 120 125

Thr Leu Met Ile Ser Arg Thr Pro Glu Val Thr Cys Val Val Val Asp
    130                 135                 140

Val Ser Gln Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Tyr Arg Val Val Ser Val Leu Thr Val Leu His Gln Asp Trp
            180                 185                 190

Leu Asn Gly Lys Glu Tyr Lys Cys Lys Val Ser Asn Lys Gly Leu Pro
        195                 200                 205

Ser Ser Ile Glu Lys Thr Ile Ser Lys Ala Lys Gly Gln Pro Arg Glu
    210                 215                 220

Pro Gln Val Tyr Thr Leu Pro Pro Ser Gln Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Val Leu Asp Ser Asp Gly Ser Phe Phe Leu Tyr Ser Arg
        275                 280                 285

Leu Thr Val Asp Lys Ser Arg Trp Gln Glu Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Leu Gly
                325

<210> 155
<211> 328
<212> PRT
<213> Homo sapiens

<400> 155
Ser Thr Lys Gly Pro Ser Val Phe Pro Leu Ala Pro Ser Ser Lys Ser
1               5                   10                  15

Thr Ser Gly Gly Thr Ala Ala Leu Gly Cys Leu Val Lys Asp Tyr Phe
20 25 30

Pro Glu Pro Val Thr Val Ser Trp Asn Ser Gly Ala Leu Thr Ser Gly
35 40 45

Val His Thr Phe Pro Ala Val Leu Gln Ser Ser Gly Leu Tyr Ser Leu
50 55 60

Ser Ser Val Val Thr Val Pro Ser Ser Ser Leu Gly Thr Gln Thr Tyr
65 70 75 80

Ile Cys Asn Val Asn His Lys Pro Ser Asn Thr Lys Val Asp Lys Lys
85 90 95

Val Glu Pro Lys Ser Cys Asp Lys Thr His Thr Cys Pro Pro Cys Pro
100 105 110

Ala Pro Glu Leu Leu Gly Gly Pro Ser Val Phe Leu Phe Pro Pro Lys
115 120 125

Pro Lys Asp Thr Leu Met Ile Ser Arg Thr Pro Glu Val Thr Cys Val
130 135 140

Val Val Asp Val Ser His Glu Asp Pro Glu Val Lys Phe Asn Trp Tyr
145 150 155 160

Val Asp Gly Val Glu Val His Asn Ala Lys Thr Lys Pro Arg Glu Glu
165 170 175

Gln Tyr Asn Ser Thr Tyr Arg Val Val Ser Val Leu Thr Val Leu His
180 185 190

Gln Asp Trp Leu Asn Gly Lys Glu Tyr Lys Cys Lys Val Ser Asn Lys
195 200 205

Ala Leu Pro Ala Pro Ile Glu Lys Thr Ile Ser Lys Ala Lys Gly Gln
210 215 220

Pro Arg Glu Pro Gln Val Tyr Thr Leu Pro Pro Ser Arg Glu Glu Met
225 230 235 240

Thr Lys Asn Gln Val Ser Leu Thr Cys Leu Val Lys Gly Phe Tyr Pro
245 250 255

Ser Asp Ile Ala Val Glu Trp Glu Ser Asn Gly Gln Pro Glu Asn Asn
260 265 270

```
Tyr Lys Thr Thr Pro Pro Val Leu Asp Ser Asp Gly Ser Phe Phe Leu
        275                 280                 285

Tyr Ser Lys Leu Thr Val Asp Lys Ser Arg Trp Gln Gln Gly Asn Val
    290                 295                 300

Phe Ser Cys Ser Val Met His Glu Ala Leu His Asn His Tyr Thr Gln
305                 310                 315                 320

Lys Ser Leu Ser Leu Ser Pro Gly
                325

<210> 156
<211> 6
<212> PRT
<213> Artificial Sequence

<220>
<221> source
<223> /note="Description of Artificial Sequence: Synthetic
      6xHis tag"

<400> 156
His His His His His His
1               5
```